(19) Europäisches Patentamt
European Patent Office
Office européen des brevets

(11) EP 4 206 182 A1

(12) **EUROPEAN PATENT APPLICATION**
published in accordance with Art. 153(4) EPC

(43) Date of publication:
**05.07.2023 Bulletin 2023/27**

(21) Application number: **21861653.0**

(22) Date of filing: **26.08.2021**

(51) International Patent Classification (IPC):
***C07C 229/60*** *(2006.01)* ***C07D 317/36*** *(2006.01)*
***C07D 333/38*** *(2006.01)* ***C07D 493/08*** *(2006.01)*
***C07D 307/68*** *(2006.01)* ***C07C 69/24*** *(2006.01)*
***C07C 69/75*** *(2006.01)* ***C07C 69/76*** *(2006.01)*
***C07C 69/78*** *(2006.01)* ***C07C 69/92*** *(2006.01)*

(52) Cooperative Patent Classification (CPC):
**C07C 69/24; C07C 69/75; C07C 69/76; C07C 69/78; C07C 69/92; C07C 229/60; C07D 307/68; C07D 317/36; C07D 333/38; C07D 493/08**

(86) International application number:
**PCT/JP2021/031281**

(87) International publication number:
**WO 2022/045231 (03.03.2022 Gazette 2022/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.08.2020 JP 2020142569**
**26.08.2020 JP 2020142570**
**27.01.2021 JP 2021011210**
**02.08.2021 JP 2021126616**

(71) Applicant: **Mitsui Chemicals, Inc.**
**Tokyo 104-0028 (JP)**

(72) Inventors:
- **YAMADA Wataru**
  **Sodegaura-shi, Chiba 299-0265 (JP)**
- **YANO Takaaki**
  **Sodegaura-shi, Chiba 299-0265 (JP)**
- **TAKANO Shotaro**
  **Sodegaura-shi, Chiba 299-0265 (JP)**
- **KIMURA Takashi**
  **Kuga-gun, Yamaguchi 740-0061 (JP)**
- **ISOGAI Makoto**
  **Kuga-gun, Yamaguchi 740-0061 (JP)**
- **NAKANO Takashi**
  **Sodegaura-shi, Chiba 299-0265 (JP)**
- **MOORTHI Sunil Krzysztof**
  **Sodegaura-shi, Chiba 299-0265 (JP)**

(74) Representative: **J A Kemp LLP**
**80 Turnmill Street**
**London EC1M 5QU (GB)**

# (54) ESTER COMPOUND

(57) The ester compound of the present invention is represented by the following formula (1), wherein $R^1$ to $R^{24}$ are each independently a hydrogen atom, a halogen atom, a hydrocarbon group, or a hetero atom-containing hydrocarbon group; $R^1$ to $R^{10}$, $R^{23}$, and $R^{24}$ may be bonded to one another to form a ring or may form a multiple bond at which adjacent substituents are directly bonded; $R^{11}$ to $R^{24}$ may be bonded to one another to form a ring, or adjacent substituents may be bonded to one another to form a multiple bond; at least one set in $R^1$ to $R^{24}$ is bonded to one another to form a ring structure; n2 to n5 each independently represent an integer of 0 to 2; n1 and n6 each independently represent an integer of 0 or 1; and $L^1$ and $L^2$ are each independently a hydrocarbon group or a hetero atom-containing hydrocarbon group.

EP 4 206 182 A1

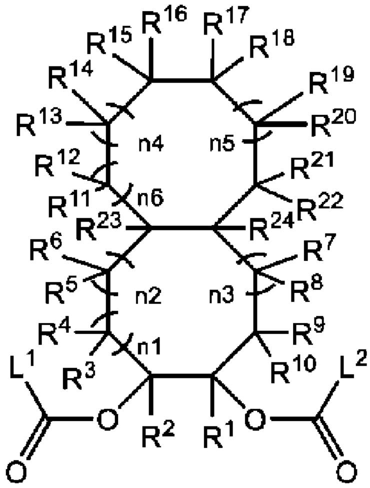

(1)

## Description

### Technical Field

The present invention relates to a novel ester compound.

### Background Art

As conventional techniques relating to ester compounds, there are many disclosures relating to applications as additives for resin additives, cosmetics and external preparations for skin, microbicidal compositions, antioxidants, and chelators, for example. As one of such applications, there is known an aspect for use in a Mg compound-supported titanium catalyst for use in olefin polymerization.

A catalyst for olefin polymerization is one of techniques greatly developed up to the present, which was triggered by the discovery of so-called Ziegler-Natta catalysts, for which Ziegler reported in 1953 that ethylene was polymerized even at low pressures by use of a combination of titanium tetrachloride and an organoaluminum compound and Natta subsequently reported the first propylene polymerization by use of a combination of titanium trichloride and a halogen-containing organoaluminum compound. Meanwhile, it has been found that catalysts containing titanium tetrachloride, a magnesium compound, and a Lewis base, which are referred to as third generation catalysts, can achieve both high polymerization activity (high productivity) and high stereoregularity in propylene polymerization. This provided one opportunity to allow propylene polymers (polypropylene) to spread around the world.

A Lewis base (hereinafter, also referred to as an "internal donor"), one of major components of the above third generation catalyst component (hereinafter, also referred to as a "solid titanium catalyst component"), was found to greatly affect catalyst performance, and various Lewis bases have been developed so far.

As Lewis bases for use in Ziegler-Natta catalysts, ethyl benzoate, phthalic esters, 1,3-diketone (Patent Literature 1), malonic ester (Patent Literature 2), succinic ester (Patent Literature 3), 2,4-pentanediol diester (Patent Literature 4), naphthalenediol diester (Patent Literature 5), and catechol diester (Patent Literature 6), for example, have been reported. Mainly enterprises vigorously make research and development in this field even today.

With respect to elementary reactions for synthesis of various ester compound, a large number of approaches have been disclosed (e.g., Patent Literatures 7 to 11 and Non-Patent Literatures 1 to 19).

### Citation List

#### Patent Literature

Patent Literature 1: JP 2005-226076A
Patent Literature 2: JP 2000-516987A
Patent Literature 3: JP 2002-542347A
Patent Literature 4: JP 2005-517746A
Patent Literature 5: JP 2011-529888A
Patent Literature 6: JP 2014-500390A
Patent Literature 7: JP 2008-247796A
Patent Literature 8: WO2008/062553
Patent Literature 9: US 2018/0149973A1
Patent Literature 10: US 2002/0162991A1
Patent Literature 11: JP 2008-037756A


#### Non-Patent Literature

Non-Patent Literature 1: Journal of the American Chemical Society, 1952, 74, 1027-1029
Non-Patent Literature 2: Journal of Organic Chemistry, 1971, 36, 3979-3987
Non-Patent Literature 3: Organic Letters, 2004, 6, 1589-1592
Non-Patent Literature 4: Journal of the American Chemical Society, 1957, 79, 2822-2824
Non-Patent Literature 5: Organic Synthesis 1991, 70, 47-53
Non-Patent Literature 6: Organic Synthesis 1997, 75, 153-160

Non-Patent Literature 7: Catalysis Letters 2012, 142, 124-130
Non-Patent Literature 8: Organic Synthesis 1997, 74, 91-100
Non-Patent Literature 9: The Fourth Series of Experimental Chemistry, vol. 20, Synthesis of Organic Compound II, Alcohols and amines, p.39
Non-Patent Literature 10: Journal of Organic Chemistry 1959, 24, 54-55
Non-Patent Literature 11: Angewandte Chemie International Edition, 1978, 17, 522-524
Non-Patent Literature 12: Bulletin of the Chemical Society of Japan, 1967, 40, 2380-2382
Non-Patent Literature 13: Organic Synthesis, 1952, 32, 41
Non-Patent Literature 14: Macromolecules, 2017, 50, 580-586
Non-Patent Literature 15: Journal of Organic Chemistry, 1980, 45, 2301-2304
Non-Patent Literature 16: Journal of Organic Chemistry, 2009, 74, 405-407
Non-Patent Literature 17: Journal of Organic Chemistry, 1988, 53, 2120-2122
Non-Patent Literature 18: Journal of Organic Chemistry, 1963, 28, 2572-2577
Non-Patent Literature 19: European Journal of Organic Chemistry, 2017, 24, 3501-3504

## Summary of Invention

### Technical Problem

Propylene polymers, while having heat resistance and rigidity similar to those of general-purpose engineering plastics, have an advantage of generating a smaller amount of toxic gas even when combustion-treated, because of being constituted substantially only by carbon and hydrogen.

With recent advances in molding techniques, use of a propylene polymer having higher stereoregularity than before has a possibility of developing higher physical properties (such as rigidity and heat resistance). For this reason, the market has required propylene polymers having higher stereoregularity. From the viewpoint of resource saving and environmental protection, methods for producing a propylene polymer at a high productivity also have been required.

It is thus an object of the present invention to provide an internal donor component suitable for a solid titanium catalyst component capable of producing a propylene polymer having extremely high stereoregularity at a high productivity (high activity), when used mainly for solid titanium catalyst component.

### Solution to Problem

As a result of diligent study to solve the above problems, the present inventors have found that an ester compound having a specific cyclic structure is suitable as a Lewis base for a solid titanium catalyst component, for example, having completed the present invention. The present invention relates to the following [1] to [28], for example.

[1] An ester compound represented by the following general formula (1):

[Chem. 1]

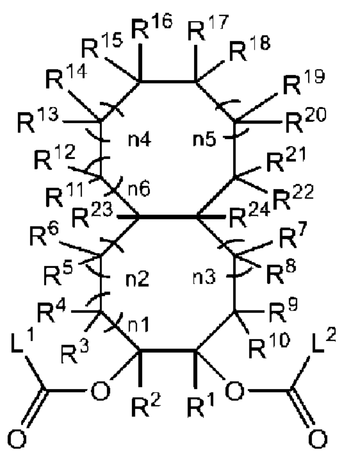

(1)

wherein, in the formula (1), $R^1$ to $R^{24}$ are each independently a hydrogen atom, a halogen atom, a hydrocarbon

group, or a hetero atom-containing hydrocarbon group; $R^1$ to $R^{10}$, $R^{23}$, and $R^{24}$ are optionally bonded to one another to form a ring or optionally form a multiple bond at which adjacent substituents are directly bonded; $R^{11}$ to $R^{24}$ are optionally bonded to one another to form a ring, or adjacent substituents are optionally bonded to one another to form a multiple bond; at least one set in $R^1$ to $R^{24}$ is bonded to one another to form a ring structure; n2 to n5 each independently represent an integer of 0 to 2; n1 and n6 each independently represent an integer of 0 or 1; and $L^1$ and $L^2$ are each independently a hydrocarbon group or a hetero atom-containing hydrocarbon group.

[2] The ester compound according to [1], wherein $L^1$ and $L^2$ are each independently a hydrocarbon group or a hetero atom-containing hydrocarbon group having 1 to 20 carbon atoms.

[3] The ester compound according to [1], wherein $L^1$ and $L^2$ are each independently a hydrocarbon group or a hetero atom-containing hydrocarbon group having 4 or more carbon atoms.

[4] The ester compound according to [1], represented by any of the following general formulas (2) to (4):

[Chem. 2]

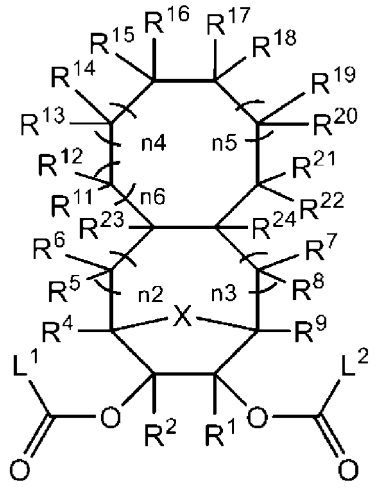

(2)

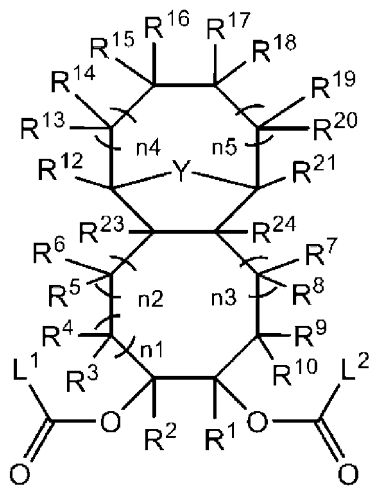

(3)

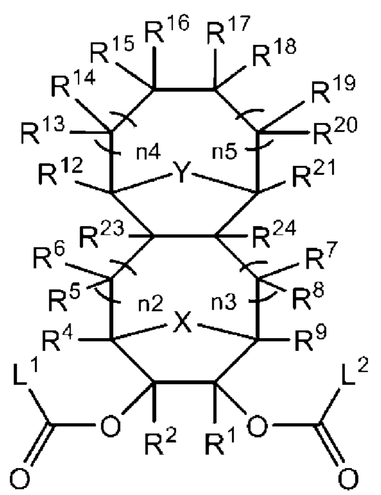

(4)

wherein, in the formulas (2) to (4), $R^1$ to $R^{24}$ are each independently a hydrogen atom, a halogen atom, a hydrocarbon group, or a hetero atom-containing hydrocarbon group; $R^1$ to $R^{10}$, $R^{23}$, and $R^{24}$ are optionally bonded to one another to form a ring or optionally form a multiple bond at which adjacent substituents are directly bonded; $R^{11}$ to $R^{24}$ are optionally bonded to one another to form a ring, or adjacent substituents are optionally bonded to one another to

form a multiple bond; X and Y are each independently a hydrocarbon group, a hetero atom, or a hetero atom-containing hydrocarbon group; n2 to n5 each independently represent an integer of 0 to 2; n1 and n6 each independently represent an integer of 0 or 1; and $L^1$ and $L^2$ are each independently a hydrocarbon group or a hetero atom-containing hydrocarbon group having 4 or more carbon atoms.

[5] The ester compound according to [3] or [4], wherein n1 and n6 are 1, and n2 to n5 are all 0.

[6] The ester compound according to [1], represented by the following general formula (5) or (6):

[Chem. 3]

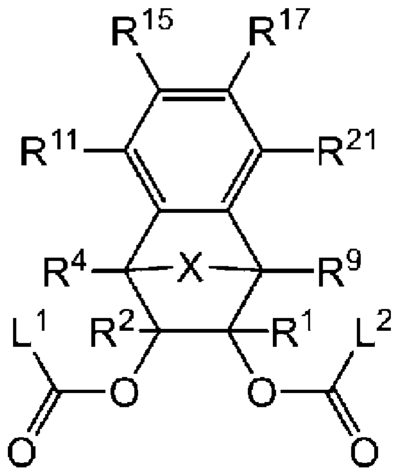

(5)

wherein, in the formula (5), $R^1$ and $R^2$ are each independently a hydrogen atom or a hydrocarbon group, $R^4$ and $R^9$ are each independently a hydrogen atom, a hydrocarbon group, or a hetero atom-containing hydrocarbon group, $R^{11}$, $R^{15}$, $R^{17}$, and $R^{21}$ are each independently a hydrogen atom, a halogen atom, hydrocarbon group, or a hetero atom-containing hydrocarbon group; $R^{11}$, $R^{15}$, $R^{17}$, and $R^{21}$ are optionally bonded to one another to form a ring; X is a hydrocarbon group, a hetero atom, or a hetero atom-containing hydrocarbon group; and $L^1$ and $L^2$ are each independently a hydrocarbon group or a hetero atom-containing hydrocarbon group having 4 or more carbon atoms;

[Chem. 4]

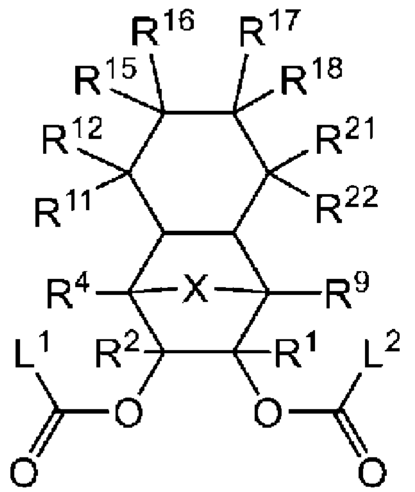

(6)

wherein, in the formula (6), $R^1$ and $R^2$ are each independently a hydrogen atom or a hydrocarbon group, $R^4$, $R^9$, $R^{11}$, $R^{12}$, $R^{15}$ to $R^{18}$, $R^{21}$, and $R^{22}$ are each independently a hydrogen atom, a hydrocarbon group, or a hetero atom-containing hydrocarbon group; $R^{11}$, $R^{12}$, $R^{15}$ to $R^{18}$, $R^{21}$, and $R^{22}$ are optionally bonded to one another to form a ring or optionally form a multiple bond at which adjacent substituents are bonded to one another; X is a hydrocarbon

group, a hetero atom, or a hetero atom-containing hydrocarbon group; and $L^1$ and $L^2$ are each independently a hydrocarbon group or a hetero atom-containing hydrocarbon group having 4 or more carbon atoms.
[7] The ester compound according to [1], represented by the following general formula (7) or (8):

[Chem. 5]

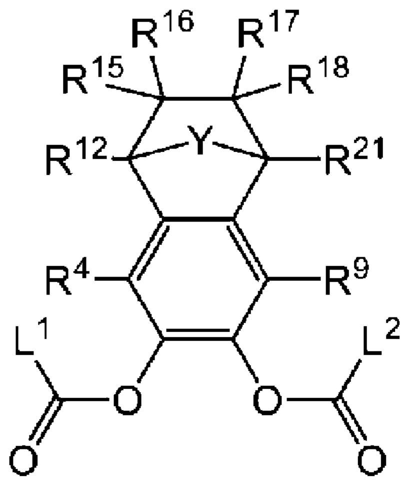

(7)

wherein, in the formula (7), $R^4$, $R^9$, $R^{12}$, $R^{15}$ to $R^{18}$, and $R^{21}$ are each independently a hydrogen atom, a hydrocarbon group, or a hetero atom-containing hydrocarbon group; $R^{15}$ to $R^{18}$ are optionally bonded to one another to form a ring or optionally form a multiple bond at which adjacent substituents are bonded to one another; Y is a hydrocarbon group, a hetero atom, or a hetero atom-containing hydrocarbon group; and $L^1$ and $L^2$ are each independently a hydrocarbon group or a hetero atom-containing hydrocarbon group having 4 or more carbon atoms;

[Chem. 6]

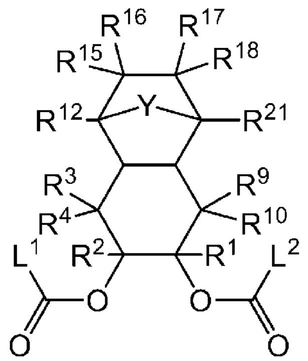

(8)

wherein, in the formula (8), $R^1$ and $R^2$ are each independently a hydrogen atom or a hydrocarbon group, $R^3$, $R^4$, $R^9$, $R^{10}$, $R^{12}$, $R^{15}$ to $R^{18}$, and $R^{21}$ are each independently a hydrogen atom, a hydrocarbon group, or a hetero atom-containing hydrocarbon group; $R^{15}$ to $R^{18}$ are optionally bonded to one another to form a ring or optionally form a multiple bond at which adjacent substituents are bonded to one another; Y is a hydrocarbon group, a hetero atom, or a hetero atom-containing hydrocarbon group; and $L^1$ and $L^2$ are each independently a hydrocarbon group or a hetero atom-containing hydrocarbon group having 4 or more carbon atoms.
[8] The ester compound according to [1], represented by the following general formula (9):

[Chem. 7]

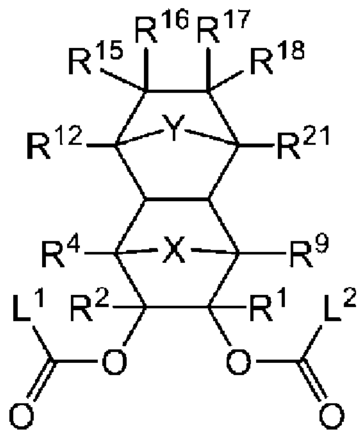

(9)

wherein, in the formula (9), $R^1$ and $R^2$ are each independently a hydrogen atom or a hydrocarbon group, $R^4$, $R^9$, $R^{12}$, $R^{15}$ to $R^{18}$, and $R^{21}$ are each independently a hydrogen atom, a hydrocarbon group, or a hetero atom-containing hydrocarbon group; $R^{15}$ to $R^{18}$ are optionally bonded to one another to form a ring or optionally form a multiple bond at which adjacent substituents are bonded to one another; X and Y are each independently a hydrocarbon group, a hetero atom, or a hetero atom-containing hydrocarbon group; and $L^1$ and $L^2$ are each independently a hydrocarbon group or a hetero atom-containing hydrocarbon group having 4 or more carbon atoms.

[9] The ester compound according to [1], represented by the following general formula (31):

[Chem. 8]

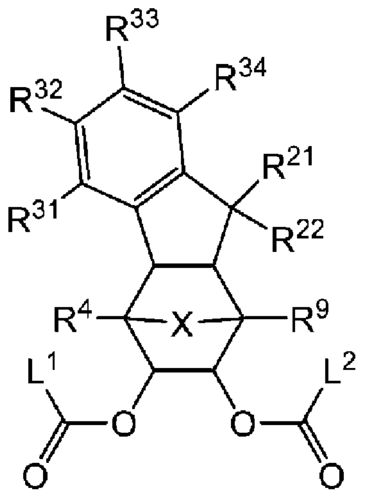

(3 1)

wherein, in the formula (31), $R^{31}$ to $R^{34}$ are each independently a hydrogen atom, a halogen atom, a hydrocarbon group, or a hetero atom-containing hydrocarbon group, $R^4$, $R^9$, $R^{21}$, and $R^{22}$ are each independently a hydrogen atom, a hydrocarbon group, or a hetero atom-containing hydrocarbon group, $R^4$, $R^9$, $R^{21}$, $R^{22}$, and $R^{31}$ to $R^{34}$ are optionally bonded to one another to form a ring; $L^1$ and $L^2$ are each independently a hydrocarbon group or a hetero atom-containing hydrocarbon group; and X is a hydrocarbon group, hetero atom or a hetero atom-containing hydrocarbon group.

[10] The ester compound according to any one of [4] and [6] to [9], wherein X and Y are each independently a divalent group selected from the following general formula group (10):

[Chem. 9]

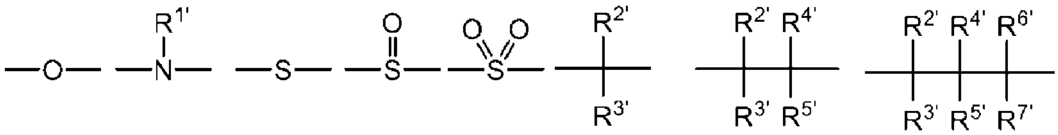

(10)

wherein, in the group (10), $R^{1'}$ to $R^{7'}$ are each independently a hydrogen atom, a hydrocarbon group, or a hetero atom-containing hydrocarbon group, and $R^{2'}$ to $R^{7'}$ are optionally bonded to one another to form a ring, or adjacent substituents are optionally directly bonded to form a multiple bond.

[11] The ester compound according to any one of [4] and [6] to [9], wherein X and Y are a divalent group selected from the following general formula group (11):

[Chem. 10]

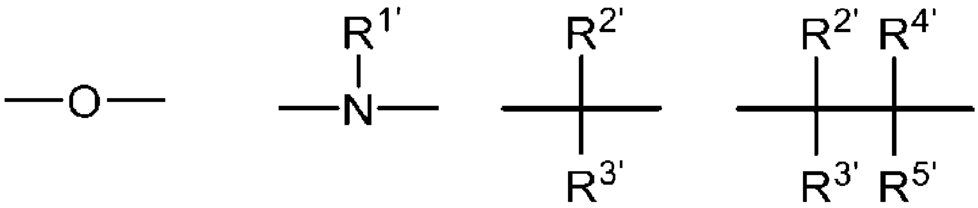

(11)

wherein, in the group (11), $R^{1'}$ to $R^{5'}$ are each independently a hydrogen atom, a hydrocarbon group having 1 to 20 carbon atoms, or a hetero atom-containing hydrocarbon group having 1 to 20 carbon atoms, and $R^{2'}$ to $R^{5'}$ are optionally bonded to one another to form a ring, or adjacent substituents are optionally directly bonded to form a multiple bond.

[12] The ester compound according to [9], wherein X is a divalent group represented by the following general formula (13) :

[Chem. 11]

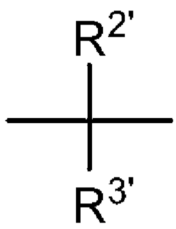

(13)

wherein, in the formula (13), $R^{2'}$ and $R^{3'}$ are each independently a hydrogen atom, a hydrocarbon group having 1 to 20 carbon atoms, or a hetero atom-containing hydrocarbon group having 1 to 20 carbon atoms, and $R^{2'}$ and $R^{3'}$ are optionally bonded to one another to form a ring.

[13] The ester compound according to [10], wherein $R^{1'}$ to $R^{7'}$ are each independently a hydrogen atom or a hydrocarbon group having 1 to 10 carbon atoms.

[14] The ester compound according to [11], wherein $R^{1'}$ to $R^{5'}$ are each independently a hydrogen atom or a hydrocarbon group having 1 to 10 carbon atoms.

[15] The ester compound according to [12], wherein $R^{2'}$ and $R^{3'}$ are each independently a hydrogen atom or a hydrocarbon group having 1 to 10 carbon atoms.

[16] The ester compound according to [12], wherein $R^{2'}$ and $R^{3'}$ are all hydrogen atoms.

[17] The ester compound according to any one of [1] to [16], wherein $R^1$ to $R^{24}$ are each independently a hydrogen atom, a hydrocarbon group having 1 to 20 carbon atoms, or a hetero atom-containing hydrocarbon group having 1 to 20 carbon atoms.

[18] The ester compound according to any one of [1] to [16], wherein $R^1$ to $R^{24}$ are each independently a hydrogen atom, a hydrocarbon group having 1 to 10 carbon atoms, or a hetero atom-containing hydrocarbon group having 1

to 10 carbon atoms.

[19] The ester compound according to [9], wherein $R^{31}$ to $R^{34}$ are each independently a hydrogen atom, a halogen atom, a hydrocarbon group having 1 to 20 carbon atoms, or a hetero atom-containing hydrocarbon group having 1 to 20 carbon atoms.

[20] The ester compound according to [9], wherein $R^{31}$ to $R^{34}$ are each independently a hydrogen atom, a hydrocarbon group having 1 to 10 carbon atoms, or a hetero atom-containing hydrocarbon group having 1 to 10 carbon atoms.

[21] The ester compound according to [9], wherein $R^{31}$ to $R^{34}$ are all hydrogen atoms, $R^4$, $R^9$, $R^{21}$, and $R^{22}$ are each independently a hydrogen atom, a hydrocarbon group having 1 to 6 carbon atoms, or a hetero atom-containing hydrocarbon group having 1 to 6 carbon atoms, and $L^1$ and $L^2$ are each independently a hydrocarbon group having 1 to 10 carbon atoms or a hetero atom-containing hydrocarbon group having 1 to 10 carbon atoms.

[22] The ester compound according to [9], wherein $R^{31}$ to $R^{34}$, $R^{21}$ and $R^{22}$ are all hydrogen atoms, $R^4$ and $R^9$ are each independently a hydrogen atom or a hydrocarbon group having 1 to 6 carbon atoms, and $L^1$ and $L^2$ are each independently selected from hydrocarbon groups having 1 to 10 carbon atoms.

[23] The ester compound according to any one of [1] to [8], wherein $R^1$ and $R^2$ are hydrogen atoms.

[24] The ester compound according to any one of [1] to [8], wherein $R^1$, $R^2$, $R^{23}$, and $R^{24}$ are all hydrogen atoms, and $R^3$ to $R^{22}$ are each independently a hydrogen atom or a substituted or unsubstituted alkyl group having 1 to 4 carbon atoms.

[25] The ester compound according to any one of [1] to [8], wherein $L^1$ and $L^2$ are each independently a hydrocarbon group or a hetero atom-containing hydrocarbon group having 4 to 20 carbon atoms.

[26] The ester compound according to any one of [1] to [8], wherein $L^1$ and $L^2$ are each independently a hydrocarbon group or a hetero atom-containing hydrocarbon group having 4 to 10 carbon atoms.

[27] The ester compound according to [4], [6], or [8], wherein the $R^4$ and/or $R^9$ are/is a hydrocarbon group or a hetero atom-containing hydrocarbon group.

[28] The ester compound according to [4], [6], or [8], wherein the $R^4$ and/or $R^9$ are/is a hydrocarbon group or an oxygen atom-containing hydrocarbon group.

## Advantageous Effect of Invention

The ester compound of the present invention can be used in resin additives, cosmetics and external preparations for skin, microbicidal compositions, antioxidants, chelators, and Ziegler-Natta catalysts, for example.

## Description of Embodiment

Hereinafter, an ester compound according to the present invention will be described in detail.

The ester compound according to the present invention (hereinafter, also referred to as the "ester compound (A)") is represented by the following general formula (1).

[Chem. 12]

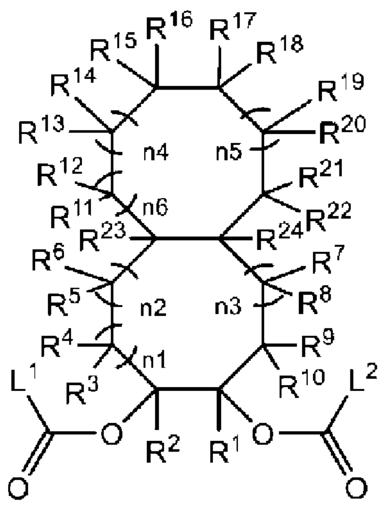

(1)

In the formula (1), $R^1$ to $R^{24}$ are each independently a hydrogen atom, a halogen atom, a hydrocarbon group, or a hetero atom-containing hydrocarbon group. $R^1$ to $R^{10}$, $R^{23}$, and $R^{24}$ may be bonded to one another to form a ring or may form a multiple bond at which adjacent substituents are directly bonded. $R^{11}$ to $R^{24}$ may be bonded to one another to form a ring, or adjacent substituents may be bonded to one another to form a multiple bond. At least one set in $R^1$ to $R^{24}$ is bonded to one another to form a ring structure. n2 to n5 each independently represent an integer of 0 to 2. n1 and n6 each independently represent an integer of 0 or 1. $L^1$ and $L^2$ are each independently a hydrocarbon group or a hetero atom-containing hydrocarbon group. Of those described above, any of n4, n5, and n6 is preferably 1 or 2. Particularly, when any two or more of $R^{11}$ to $R^{24}$ are bonded to form an aromatic ring structure, any of n4, n5, and n6 is preferably 1 or 2.

An example of a preferable aspect of the ester compound (A) of the present invention includes compounds represented by the following general formulas (2) to (4).

[Chem. 13]

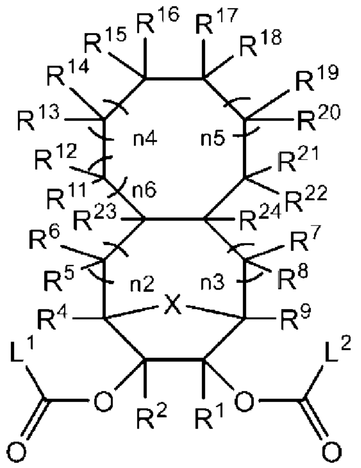

(2)

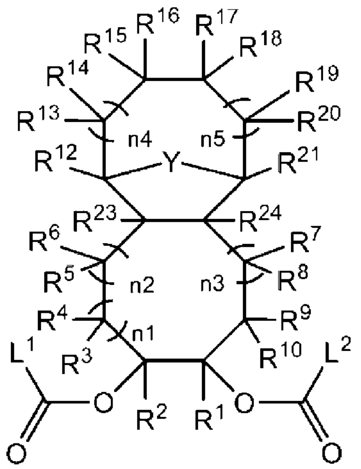

(3)

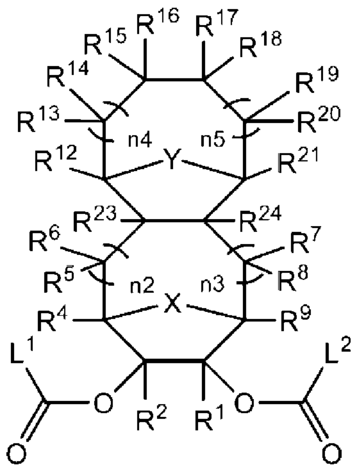

(4)

wherein, in the formulas (2) to (4), $R^1$ to $R^{24}$ are each independently a hydrogen atom, a halogen atom, a hydrocarbon group, or a hetero atom-containing hydrocarbon group; $R^1$ to $R^{10}$, $R^{23}$, and $R^{24}$ are optionally bonded to one another to form a ring or optionally form a multiple bond at which adjacent substituents are directly bonded; $R^{11}$ to $R^{24}$ are optionally bonded to one another to form a ring, or adjacent substituents are optionally bonded to one another to form a multiple bond; X and Y are each independently a hydrocarbon group, a hetero atom, or a hetero atom-containing hydrocarbon group; n2 to n5 each independently represent an integer of 0 to 2; n1 and n6 each independently represent

an integer of 0 or 1; and $L^1$ and $L^2$ are each independently a hydrocarbon group or a hetero atom-containing hydrocarbon group having 4 or more carbon atoms.

An example of a more preferable aspect of the ester compound (A) of the present invention also includes compounds represented by the following general formulas (5) to (9).

[Chem. 14]

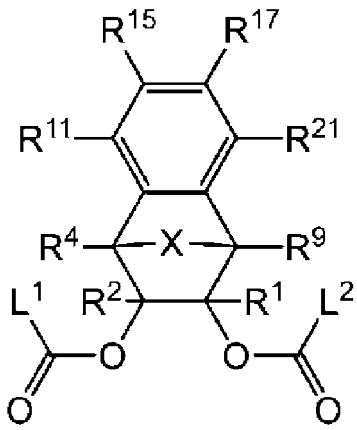

(5)

wherein, in the formula (5), $R^1$ and $R^2$ are each independently a hydrogen atom or a hydrocarbon group, $R^4$ and $R^9$ are each independently a hydrogen atom, a hydrocarbon group, or a hetero atom-containing hydrocarbon group, $R^{11}$, $R^{15}$, $R^{17}$, and $R^{21}$ are each independently a hydrogen atom, a halogen atom, hydrocarbon group, or a hetero atom-containing hydrocarbon group; $R^{11}$, $R^{15}$, $R^{17}$, and $R^{21}$ are optionally bonded to one another to form a ring; X is a hydrocarbon group, a hetero atom, or a hetero atom-containing hydrocarbon group; and $L^1$ and $L^2$ are each independently a hydrocarbon group or a hetero atom-containing hydrocarbon group having 4 or more carbon atoms.

[Chem. 15]

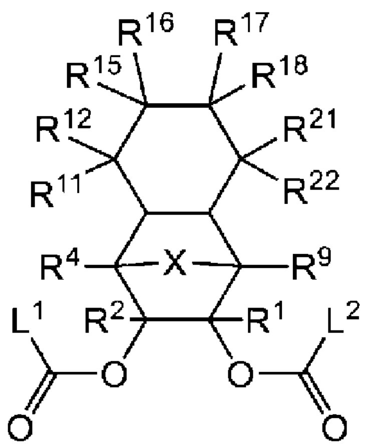

(6)

wherein, in the formula (6), $R^1$ and $R^2$ are each independently a hydrogen atom or a hydrocarbon group, $R^4$, $R^9$, $R^{11}$, $R^{12}$, $R^{15}$ to $R^{18}$, $R^{21}$, and $R^{22}$ are each independently a hydrogen atom, a hydrocarbon group, or a hetero atom-containing hydrocarbon group; $R^{11}$, $R^{12}$, $R^{15}$ to $R^{18}$, $R^{21}$, and $R^{22}$ are optionally bonded to one another to form a ring or optionally form a multiple bond at which adjacent substituents are bonded to one another; X is a hydrocarbon group, a hetero atom, or a hetero atom-containing hydrocarbon group; and $L^1$ and $L^2$ are each independently a hydrocarbon group or a hetero atom-containing hydrocarbon group having 4 or more carbon atoms.

[Chem. 16]

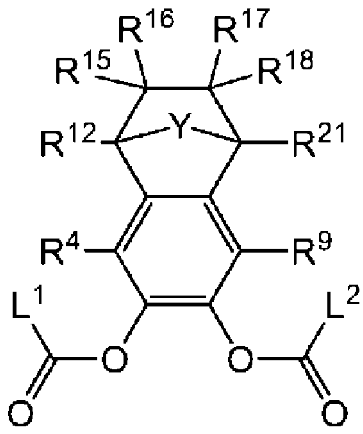

(7)

wherein, in the formula (7), $R^4$, $R^9$, $R^{12}$, $R^{15}$ to $R^{18}$, and $R^{21}$ are each independently a hydrogen atom, a hydrocarbon group, or a hetero atom-containing hydrocarbon group; $R^{15}$ to $R^{18}$ are optionally bonded to one another to form a ring or optionally form a multiple bond at which adjacent substituents are bonded to one another; Y is a hydrocarbon group, a hetero atom, or a hetero atom-containing hydrocarbon group; and $L^1$ and $L^2$ are each independently a hydrocarbon group or a hetero atom-containing hydrocarbon group having 4 or more carbon atoms.

[Chem. 17]

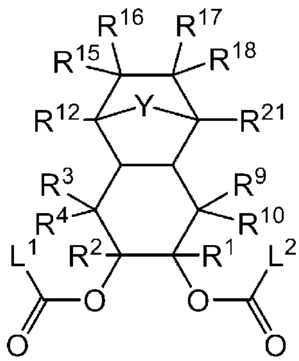

(8)

wherein, in the formula (8), $R^1$ and $R^2$ are each independently a hydrogen atom or a hydrocarbon group, $R^3$, $R^4$, $R^9$, $R^{10}$, $R^{12}$, $R^{15}$ to $R^{18}$, and $R^{21}$ are each independently a hydrogen atom, a hydrocarbon group, or a hetero atom-containing hydrocarbon group; $R^{15}$ to $R^{18}$ are optionally bonded to one another to form a ring or optionally form a multiple bond at which adjacent substituents are bonded to one another; Y is a hydrocarbon group, a hetero atom, or a hetero atom-containing hydrocarbon group; and $L^1$ and $L^2$ are each independently a hydrocarbon group or a hetero atom-containing hydrocarbon group having 4 or more carbon atoms.

In the case of the structure of the formula (8), $R^3$, $R^4$, $R^9$ and $R^{10}$ are each independently preferably a substituent selected from a hydrogen atom, a halogen atom, a hydrocarbon group, and a halogen-containing hydrocarbon group, more preferably selected from a hydrogen atom, a hydrocarbon group, and a halogen-containing hydrocarbon group, and particularly preferably selected from hydrogen and a hydrocarbon group. Preferable examples of the hydrocarbon group described above are more specifically substituted or unsubstituted alkyl groups having 1 to 20 carbon atoms, substituted or unsubstituted cycloalkyl groups having 1 to 20 carbon atoms, substituted or unsubstituted alkenyl groups having 2 to 20 carbon atoms, substituted or unsubstituted alkynyl groups having 2 to 20 carbon atoms, and substituted or unsubstituted aryl groups having 6 to 20 carbon atoms. Preferable examples of the halogen-containing hydrocarbon group described above are substituents in which one or more hydrogen atom in a substituted or unsubstituted alkyl group having 1 to 20 carbon atoms, a substituted or unsubstituted cycloalkyl group having 1 to 20 carbon atoms, a substituted or unsubstituted alkenyl group having 2 to 20 carbon atoms, a substituted or unsubstituted alkynyl group having 2 to 20 carbon atoms, or a substituted or unsubstituted aryl group having 6 to 20 carbon atoms are replaced with a halogen atom.

[Chem. 18]

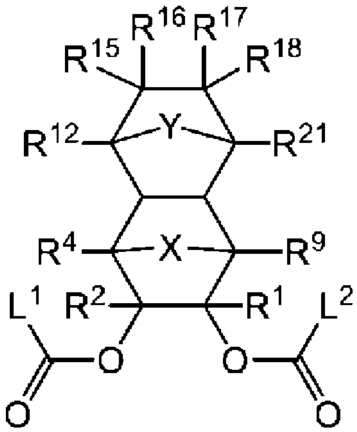

(9)

wherein, in the formula (9), $R^1$ and $R^2$ are each independently a hydrogen atom or a hydrocarbon group, $R^4$, $R^9$, $R^{12}$, $R^{15}$ to $R^{18}$, and $R^{21}$ are each independently a hydrogen atom, a hydrocarbon group, or a hetero atom-containing hydrocarbon group; $R^{15}$ to $R^{18}$ are optionally bonded to one another to form a ring or optionally form a multiple bond at which adjacent substituents are bonded to one another; X and Y are each independently a hydrocarbon group, a hetero atom, or a hetero atom-containing hydrocarbon group; and $L^1$ and $L^2$ are each independently a hydrocarbon group or a hetero atom-containing hydrocarbon group having 4 or more carbon atoms.

Of these ester compounds, ester compounds represented by the formulas (5), (8), and (9) are preferable, compounds represented by the formulas (5) and (9) are more preferable, and ester compounds represented by the formula (5) are most preferable.

An example of a preferable aspect of the ester compound (A) of the present invention also includes a compound represented by the following general formula (31).

[Chem. 19]

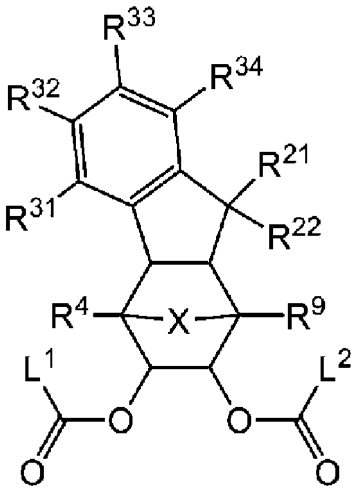

(31)

Detailed description of the structure of substituents in the formula (31) will be mentioned below.

<$R^1$ to $R^{24}$>

In the formulas (1) and the like, $R^1$ to $R^{24}$ are each independently a hydrogen atom, a halogen atom, a hydrocarbon group, or a hetero atom-containing hydrocarbon group.

Examples of the hydrocarbon group can include substituted or unsubstituted alkyl groups, substituted or unsubstituted cycloalkyl groups, substituted or unsubstituted alkenyl groups, substituted or unsubstituted alkynyl groups,

and substituted or unsubstituted aryl groups.

Examples of the hetero atom-containing hydrocarbon group can include substituted or unsubstituted hetero atom-containing alkyl groups and substituted or unsubstituted heteroaryl groups.

Examples of the hydrocarbon group and the hetero atom-containing hydrocarbon group include alkyl groups, cycloalkyl groups, alkenyl groups, alkynyl groups, aryl groups, hetero atom-containing alkyl groups, and heteroaryl groups. These groups preferably have 1 to 20 carbon atoms. The lower limit value is preferably 2, more preferably 3, and particularly preferably 4. However, the preferable lower limit value in the case of an aryl group is 6. Meanwhile, the upper limit value is preferably 18, more preferably 15, further preferably 10, and particularly preferably 6. A heteroaryl group preferably has one or more 5 or more-membered ring structures, more preferably has one or more 5 to 7-membered ring structures, and further preferably has one or more 5-membered ring or 6-membered ring structure.

At least one substituent of the $R^1$ to $R^{24}$ may be preferably a substituent other than hydrogen. Further, one or more of the carbon atoms forming the cyclic structure may be preferably a quaternary carbon. In an aspect as described above, when the ester compound of the present invention is used as a component of a catalyst for olefin polymerization, the performance balance may be improved.

As described above, $R^1$ to $R^{10}$, $R^{23}$, and $R^{24}$ may be bonded to one another to form a ring, and $R^{11}$ to $R^{24}$ may be bonded to one another to form a ring. The moiety forming a ring may be formed with a single bond or may include a double bond. A structure including a carbon-carbon double bond may be preferable. The moiety forming a ring may be preferably a structure further including a ring structure, and an aspect may be preferable in which a double bond, particularly preferably a carbon-carbon double bond is included in the ring structure. Specific examples of the structure of the moiety forming a ring like this are the same as structure examples of X and Y mentioned below. In the present invention, the carbon-carbon double bond includes an aromatic structure.

To carbon to which the substituents that are bonded one another to form a ring are bonded (hereinafter, the carbon may be referred to as "B4C"), "another substituent" (hereinafter, may be referred to as "B4S") is usually bonded (e.g., when $R^3$ is bonded directly to $R^{10}$ to form a ring, $R^4$ and $R^9$ correspond thereto). In the present invention, the "another substituent" may be preferably a hydrocarbon group and/or a hetero atom-containing hydrocarbon group mentioned below. As the hetero atom-containing hydrocarbon group, an oxygen-containing hydrocarbon group is particularly preferable. The hydrocarbon group is more specifically an aliphatic group having 1 to 10 carbon atoms, alicyclic group, or aromatic group and more preferably an aliphatic group having 1 to 6 carbon atoms, alicyclic group, or aromatic group. The hetero atom-containing hydrocarbon group is more specifically a hetero atom-containing aliphatic group having 1 to 10 carbon atoms, alicyclic group, or aromatic group and more preferably a hetero atom-containing aliphatic group having 1 to 6 carbon atoms, alicyclic group, or aromatic group. The hetero atom is preferably oxygen. The oxygen-containing hydrocarbon group is further preferably an alkoxy group. More specific examples of the position for such a substituent can include $R^4$ and/or $R^9$ in the formulas (2), (4), (5), (6), and (9) and $R^{12}$ and/or $R^{21}$ in the formulas (3), (4), (7), and (8). More preferable are/is the $R^4$ and/or $R^9$. When the substituent at such a position is a group having a structure as above and is used as a component of the catalyst for olefin polymerization, the molecular weight of a polymer to be obtained may be easily controlled via hydrogen, in addition to polymerization activity and stereoregularity.

In $R^1$ to $R^{24}$, adjacent substituents may be bonded directly to form a multiple bond, for example a double bond or triple bond. Further, an aromatic ring structure in which these substituents are bonded is also within the scope of this invention. Examples thereof can include aromatic ring structures represented by the formulas (5) and (7).

When $R^1$ and the like form a ring structure, substituents forming the ring are selected from substituents other than a hydrogen atom and halogen atoms and are preferably hydrocarbon groups. At least one set in $R^1$ to $R^{24}$ is bonded to one another to form a ring structure. Within the ring to be formed, substituents to form the ring of the at least one set are preferably separated by 2 or more carbons and more preferably separated by 3 or more carbons. Such a structure is preferably a ring structure including X or Y in the formulas (2) to (4) and more preferably a ring structure including X or Y in the formulas (5) to (9).

When adjacent substituents are bonded to form a ring, substituents selected from $R^3$ to $R^6$, $R^7$ to $R^{10}$, and $R^{11}$ to $R^{22}$ are preferably bonded to one another to form the ring. At this time, the substituents forming the ring preferably include no carbon atoms in a bridgehead position from the synthetic viewpoint. Carbon atoms in a bridgehead position refer to carbon atoms sharing 2 or more rings. For example, in the case of the formula (2), the carbon atom to which X and $R^4$ are bonded, the carbon atom to which X and $R^9$ are bonded, the carbon atom to which $R^{23}$ is bonded, and the carbon atom to which $R^{24}$ is bonded are referred to. Preferable examples of such structures include structures included in the formulas (5) to (9). As the structure included in the formula (5), particularly preferable are a structure of a ring formed by bonding of $R^{11}$ and $R^{15}$ to one another, a structure of a ring formed by bonding of $R^{15}$ and $R^{17}$ to one another, a structure of a ring formed by bonding of $R^{17}$ and $R^{21}$ to one another, and a structure composed of these combinations. As the structure included in the formula (6), particularly preferable are a structure of a ring formed by bonding of $R^{11}$ or $R^{12}$ and $R^{15}$ or $R^{16}$, a structure of a ring formed by bonding of $R^{15}$ or $R^{16}$ and $R^{17}$ or $R^{18}$, a structure of a ring formed by bonding of $R^{17}$ or $R^{18}$ and $R^{21}$ or $R^{22}$, and a structure composed of these combinations. As the structure included in the formulas (7) to (9), a structure of a ring formed by bonding of $R^{15}$ or $R^{16}$ and $R^{17}$ or $R^{18}$ is particularly preferable.

One example of an ester compound (A) having a structure in which $R^{11}$ and $R^{15}$ are bonded to one another to form a ring in the formula (5) is shown below.

[Chem. 20]

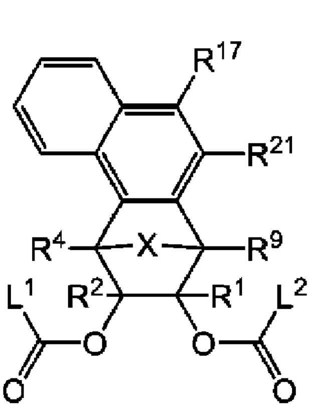

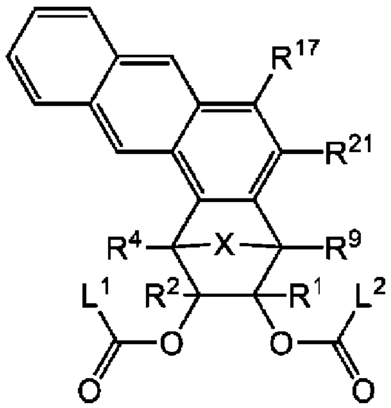

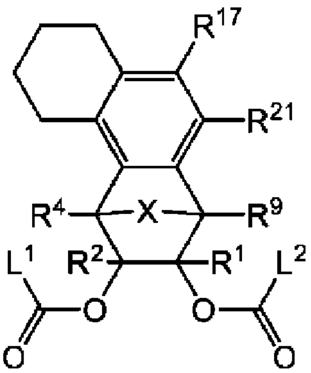

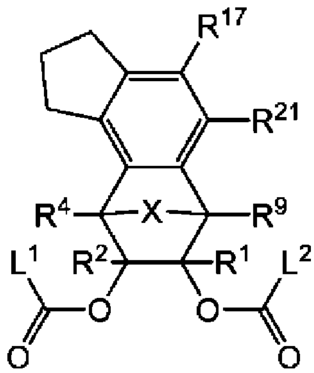

One example of an ester compound (A) having a structure in which $R^{15}$ and $R^{17}$ are bonded to one another to form a ring in the formula (5) is shown below.

[Chem. 21]

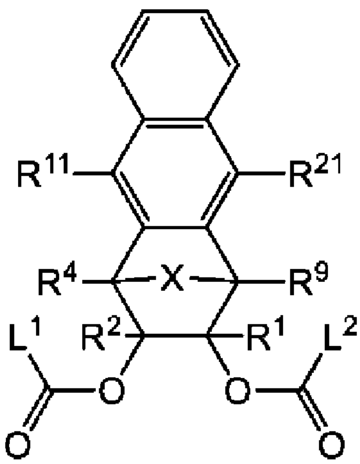

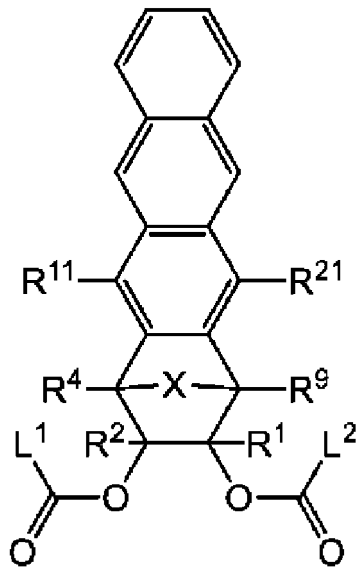

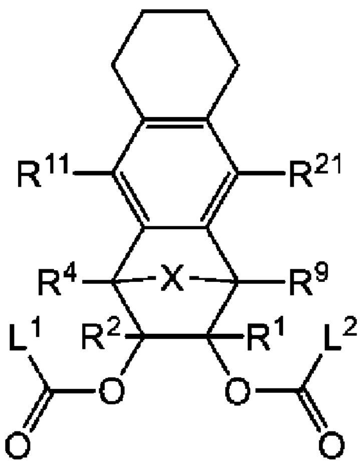

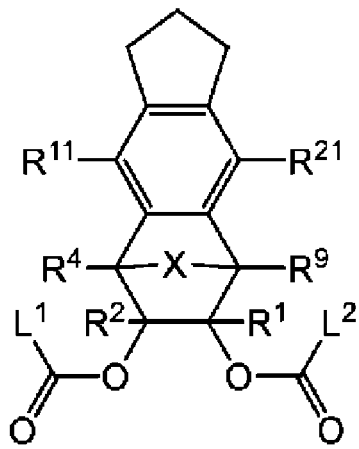

One example of an ester compound (A) having a structure in which $R^{17}$ and $R^{21}$ are bonded to one another to form a ring in the formula (5) is shown below.

[Chem. 22]

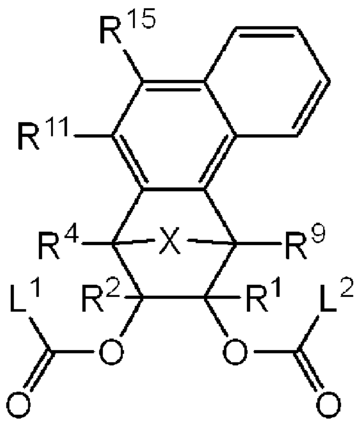

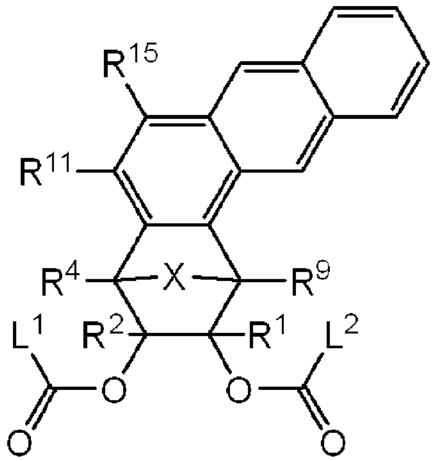

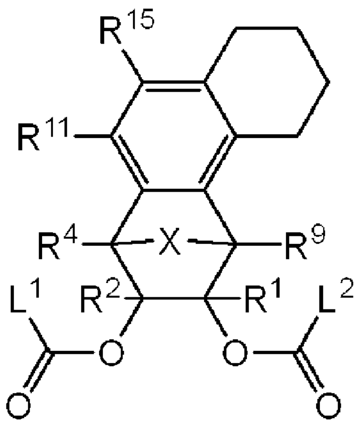

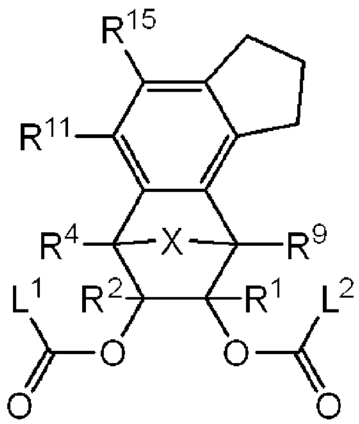

One example of an ester compound (A) having a structure in which $R^{11}$ or $R^{12}$ is bonded to $R^{15}$ or $R^{16}$ to form a ring in the formula (6) is shown below.

[Chem. 23]

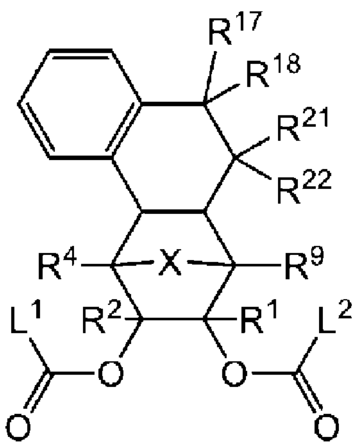

One example of an ester compound (A) having a structure in which $R^{15}$ or $R^{16}$ is bonded to $R^{17}$ or $R^{18}$ to form a ring in the formula (6) is shown below.

[Chem. 24]

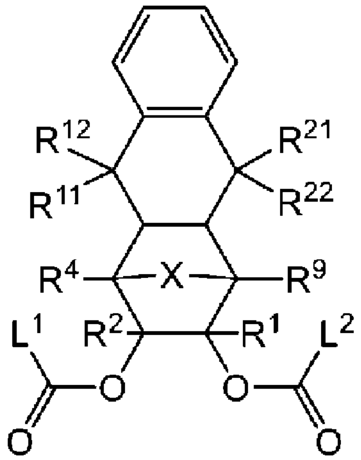

One example of an ester compound (A) having a structure, in which $R^{17}$ or $R^{18}$ is bonded to $R^{21}$ or $R^{22}$ to form a ring in the formula (6), is shown below.

[Chem. 25]

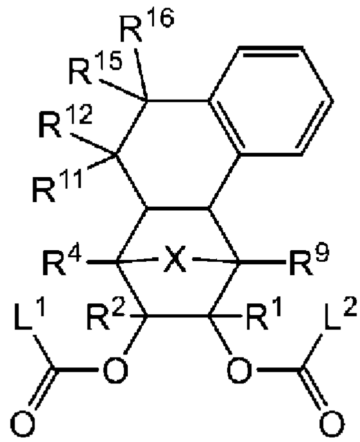

One example of an ester compound (A) having both a structure in which $R^{11}$ or $R^{12}$ is bonded to $R^{15}$ or $R^{16}$ to form a ring and a structure in which $R^{17}$ or $R^{18}$ is bonded to $R^{21}$ or $R^{22}$ to form a ring in the formula (6) is shown below.

[Chem. 26]

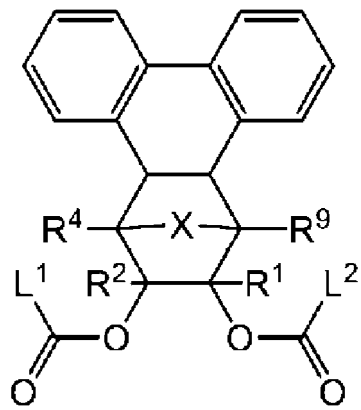

One example of an ester compound (A) having a structure in which $R^{15}$ or $R^{16}$ is bonded to $R^{17}$ or $R^{18}$ to form a ring in the formula (7) is shown below.

[Chem. 27]

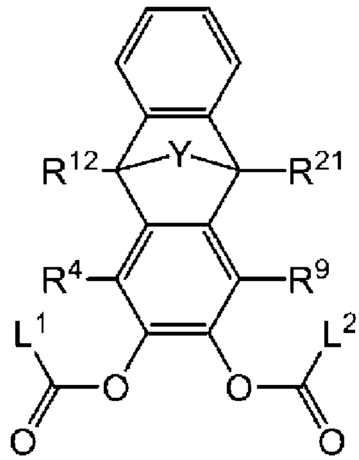

One example of an ester compound (A) having a structure in which $R^{15}$ or $R^{16}$ is bonded to $R^{17}$ or $R^{18}$ to form a ring in the formula (8) is shown below.

[Chem. 28]

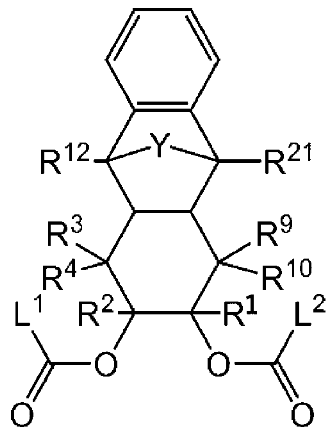

One example of an ester compound (A) having a structure in which $R^{15}$ or $R^{16}$ is bonded to $R^{17}$ or $R^{18}$ to form a ring in the formula (9) is shown below.

[Chem. 29]

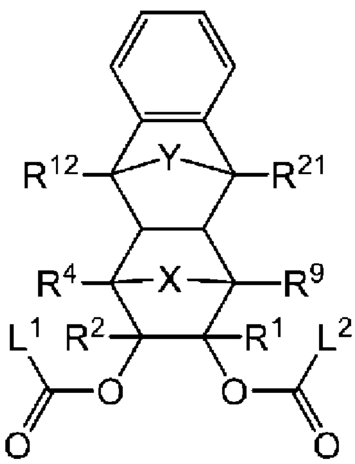

Preferable $R^{15}$ to $R^{18}$ are each independently a hydrogen atom, a halogen atom, a substituted or unsubstituted alkyl group having 1 to 20 carbon atoms, a substituted or unsubstituted cycloalkyl group having 1 to 20 carbon atoms, a substituted or unsubstituted alkenyl group having 2 to 20 carbon atoms, a substituted or unsubstituted alkynyl group having 2 to 20 carbon atoms, a substituted or unsubstituted aryl group having 6 to 20 carbon atoms, a substituted or unsubstituted hetero atom-containing alkyl group having 1 to 20 carbon atoms, or a substituted or unsubstituted heteroaryl group having 2 to 20 carbon atoms. The $R^3$, $R^4$, $R^9$, and $R^{10}$ are each independently preferably a substituent selected from a hydrogen atom, a halogen atom, a hydrocarbon group, and a halogen-containing hydrocarbon group, more preferably selected from a hydrogen atom, a hydrocarbon group, and a halogen-containing hydrocarbon group, and particularly preferably selected from hydrogen and a hydrocarbon group. Preferable examples of the hydrocarbon group include substituted or unsubstituted alkyl groups having 1 to 20 carbon atoms, substituted or unsubstituted cycloalkyl groups having 1 to 20 carbon atoms, substituted or unsubstituted alkenyl groups having 2 to 20 carbon atoms, substituted or unsubstituted alkynyl groups having 2 to 20 carbon atoms, and substituted or unsubstituted aryl groups having 6 to 20 carbon atoms. Preferable examples of the halogen-containing hydrocarbon group include substituents in which one or more hydrogen atoms in a substituted or unsubstituted alkyl group having 1 to 20 carbon atoms, a substituted or unsubstituted cycloalkyl group having 1 to 20 carbon atoms, a substituted or unsubstituted alkenyl group having 2 to 20 carbon atoms, a substituted or unsubstituted alkynyl group having 2 to 20 carbon atoms, or a substituted or unsubstituted aryl group having 6 to 20 carbon atoms are replaced with a halogen atom.

Preferably, $R^1$ to $R^{24}$ are each independently a hydrogen atom, a halogen atom, a substituted or unsubstituted alkyl group having 1 to 20 carbon atoms, a substituted or unsubstituted cycloalkyl group having 1 to 20 carbon atoms, a substituted or unsubstituted alkenyl group having 2 to 20 carbon atoms, a substituted or unsubstituted alkynyl group having 2 to 20 carbon atoms, a substituted or unsubstituted aryl group having 6 to 20 carbon atoms, a substituted or unsubstituted hetero atom-containing alkyl group having 1 to 20 carbon atoms, or a substituted or unsubstituted heteroaryl group having 2 to 20 carbon atoms.

More preferably, $R^1$ to $R^{24}$ are each independently a hydrogen atom, a substituted or unsubstituted alkyl group having 1 to 10 carbon atoms, a substituted or unsubstituted cycloalkyl group having 1 to 10 carbon atoms, a substituted or unsubstituted alkenyl group having 2 to 10 carbon atoms, a substituted or unsubstituted alkynyl group having 2 to 10 carbon atoms, a substituted or unsubstituted aryl group having 6 to 15 carbon atoms, a substituted or unsubstituted hetero atom-containing alkyl group having 1 to 10 carbon atoms, or a substituted or unsubstituted heteroaryl group having 2 to 10 carbon atoms.

Further preferably, $R^1$ to $R^{24}$ are each independently a hydrogen atom, a substituted or unsubstituted alkyl group having 1 to 6 carbon atoms, a substituted or unsubstituted cycloalkyl group having 1 to 6 carbon atoms, a substituted or unsubstituted aryl group having 6 to 10 carbon atoms, a substituted or unsubstituted hetero atom-containing alkyl group having 1 to 6 carbon atoms, or a substituted or unsubstituted heteroaryl group having 3 to 10 carbon atoms.

Further more preferably, $R^1$ to $R^{24}$ are each independently a hydrogen atom or a substituted or unsubstituted alkyl group having 1 to 4 carbon atoms.

Particularly preferably, $R^1$, $R^2$, $R^{23}$, and $R^{24}$ are all hydrogen atoms, and $R^3$ to $R^{22}$ are each independently a

hydrogen atom or a substituted or unsubstituted alkyl group having 1 to 4 carbon atoms.

Carbon to which $R^1$ to $R^{24}$ are bonded forms 2 or more ring structures, as shown by the general formula (1). One or more of the ring structures are preferably alicyclic structures. That is, at least not all the rings are preferably aromatic ring structures.

<$R^{31}$ to $R^{34}$>

In the formula (31), $R^{31}$ to $R^{34}$ are each independently a hydrogen atom, a halogen atom, a hydrocarbon group, or a hetero atom-containing hydrocarbon group.

Examples of the hydrocarbon group can include substituted or unsubstituted alkyl groups, substituted or unsubstituted cycloalkyl groups, substituted or unsubstituted alkenyl groups, substituted or unsubstituted alkynyl groups, and substituted or unsubstituted aryl groups.

Examples of the hetero atom-containing hydrocarbon group can include substituted or unsubstituted hetero atom-containing alkyl groups and substituted or unsubstituted heteroaryl groups.

Examples of the hydrocarbon group and the hetero atom-containing hydrocarbon group include alkyl groups, cycloalkyl groups, alkenyl groups, alkynyl groups, aryl groups, hetero atom-containing alkyl groups, and heteroaryl groups. These groups preferably have 1 to 20 carbon atoms. The lower limit value is preferably 2, more preferably 3, and particularly preferably 4. However, the preferable lower limit value in the case of an aryl group is 6. Meanwhile, the upper limit value is preferably 18, more preferably 15, further preferably 10, and particularly preferably 6. A heteroaryl group preferably has one or more 5 or more-membered ring structures, more preferably has one or more 5 to 7-membered ring structures, and further preferably has one or more 5-membered ring or 6-membered ring structure.

Preferable $R^{31}$ to $R^{34}$ are each independently a hydrogen atom, a halogen atom, a substituted or unsubstituted alkyl group having 1 to 20 carbon atoms, a substituted or unsubstituted cycloalkyl group having 1 to 20 carbon atoms, a substituted or unsubstituted alkenyl group having 2 to 20 carbon atoms, a substituted or unsubstituted alkynyl group having 2 to 20 carbon atoms, a substituted or unsubstituted aryl group having 6 to 20 carbon atoms, a substituted or unsubstituted hetero atom-containing alkyl group having 1 to 20 carbon atoms, or a substituted or unsubstituted heteroaryl group having 2 to 20 carbon atoms.

More preferable $R^{31}$ to $R^{34}$ are each independently a hydrogen atom, a substituted or unsubstituted alkyl group having 1 to 10 carbon atoms, a substituted or unsubstituted cycloalkyl group having 1 to 10 carbon atoms, a substituted or unsubstituted alkenyl group having 2 to 10 carbon atoms, a substituted or unsubstituted alkynyl group having 2 to 10 carbon atoms, a substituted or unsubstituted aryl group having 6 to 15 carbon atoms, a substituted or unsubstituted hetero atom-containing alkyl group having 1 to 10 carbon atoms, or a substituted or unsubstituted heteroaryl group having 2 to 10 carbon atoms.

Further preferable $R^{31}$ to $R^{34}$ are each independently a hydrogen atom, a substituted or unsubstituted alkyl group having 1 to 6 carbon atoms, a substituted or unsubstituted cycloalkyl group having 1 to 6 carbon atoms, a substituted or unsubstituted aryl group having 6 to 10 carbon atoms, a substituted or unsubstituted hetero atom-containing alkyl group having 1 to 6 carbon atoms, or a substituted or unsubstituted heteroaryl group having 3 to 6 carbon atoms.

$R^{31}$ to $R^{34}$ may have a structure of a ring formed by bonding of $R^{31}$ to $R^{34}$ to one another.

Particularly preferable $R^{31}$ to $R^{34}$ are each independently a hydrogen atom or a substituted or unsubstituted alkyl group having 1 to 4 carbon atoms, and most preferable $R^{31}$ to $R^{34}$ are all hydrogen atoms. $R^{31}$ to $R^{34}$, $R^{21}$, $R^{22}$, $R^4$, and $R^9$ may be bonded to one another to form a ring, or adjacent substituents may be directly bonded to form a multiple bond.

Hereinafter, the structure of the ester compound represented by the formula (31) will be described in detail.

As described above, $R^{31}$ to $R^{34}$, $R^{21}$, $R^{22}$, $R^4$, and $R^9$ may form a ring in which the substituents are bonded to one another, or adjacent substituents are bonded directly to form a multiple bond, for example, a double bond or triple bond. Further, an aromatic ring structure in which these substituents are bonded is also within the scope of this invention. An example thereof can include an aromatic ring structure in which $R^{34}$, $R^{21}$, and $R^{22}$ are bonded.

When $R^{31}$ to $R^{34}$, $R^{21}$, $R^{22}$, $R^4$, and $R^9$ are bonded to one another to form a ring structure, the substituents forming the ring are selected from substituents other than a hydrogen atom and halogen atoms and are preferably hydrocarbon groups. $R^{31}$ to $R^{34}$, $R^{21}$, and $R^{22}$ are preferably bonded to one another to form a ring, and more preferable is a structure selected from a structure of a ring formed by bonding of $R^{31}$ and $R^{32}$ to one another, a structure of a ring formed by bonding of $R^{32}$ and $R^{33}$ to one another, a structure of a ring formed by bonding of $R^{33}$ and $R^{34}$ to one another, a structure of a ring formed by bonding of $R^{21}$ and $R^{22}$ to one another, a structure in which $R^{34}$, $R^{21}$, and $R^{22}$ are bonded to one another, and combinations thereof.

One example of the ester compound (A) having a structure in which $R^{31}$ and $R^{32}$ are bonded to one another to form a ring is shown below.

[Chem. 30]

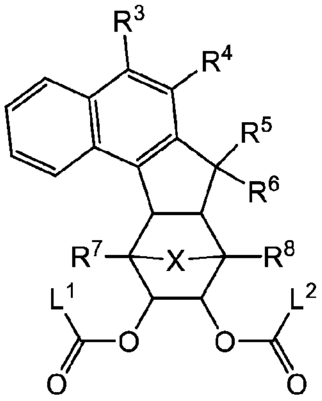

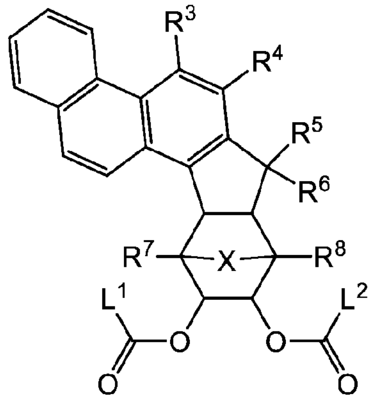

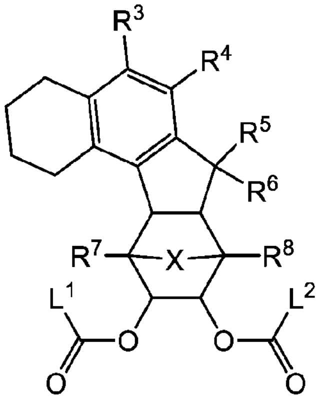

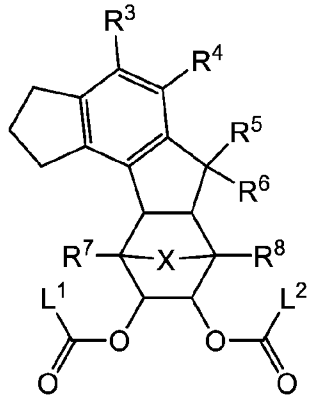

One example of the ester compound (A) having a structure in which $R^{32}$ and $R^{33}$ are bonded to one another to form a ring is shown below.

[Chem. 31]

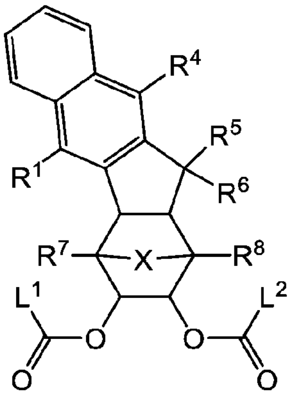

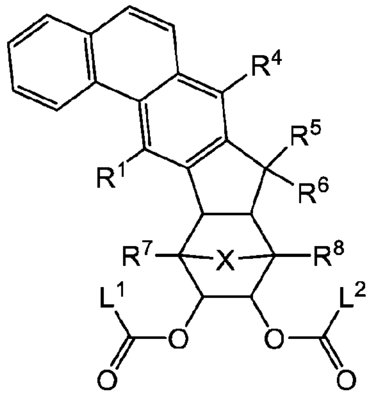

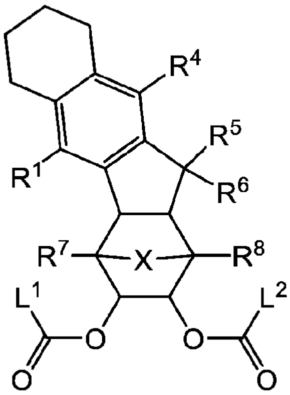

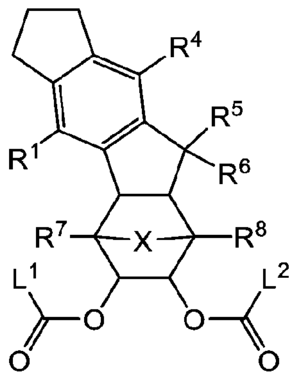

One example of the ester compound (A) having a structure in which $R^{33}$ and $R^{34}$ are bonded to one another to form a ring is shown below.

[Chem. 32]

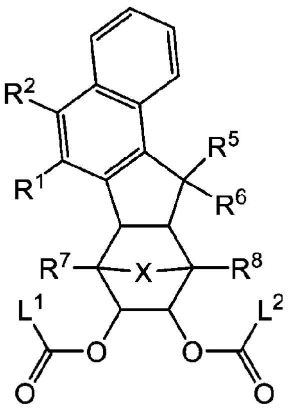
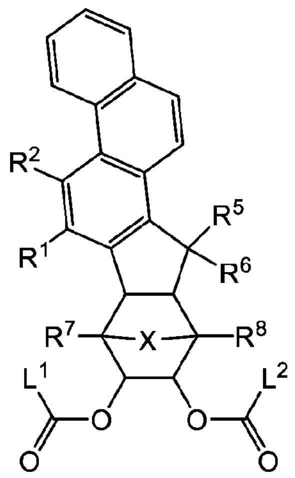
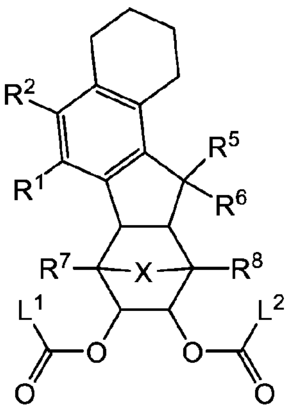
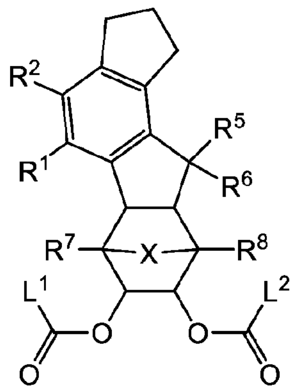

One example of the ester compound (A) having a structure in which $R^{21}$ and $R^{22}$ are bonded to one another to form a ring is shown below.

[Chem. 33]

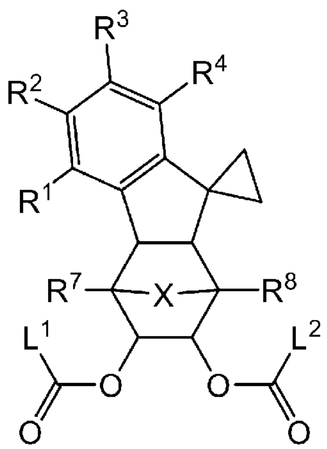
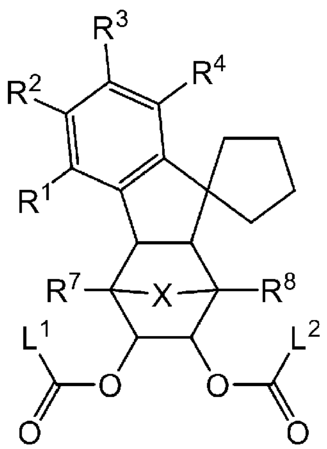
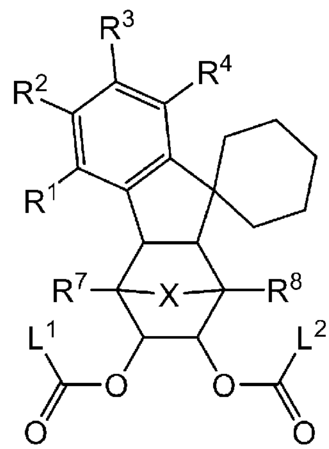

One example of the ester compound (A) having a structure in which $R^{34}$, $R^{21}$, and $R^{22}$ are bonded to one another to form a ring is shown below.

[Chem. 34]

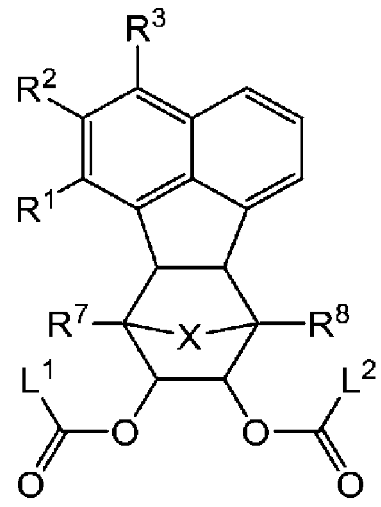

Preferable $R^{21}$, $R^{22}$, $R^4$, and $R^9$ are each independently a hydrogen atom, a substituted or unsubstituted alkyl group having 1 to 20 carbon atoms, a substituted or unsubstituted cycloalkyl group having 1 to 20 carbon atoms, a substituted or unsubstituted alkenyl group having 2 to 20 carbon atoms, a substituted or unsubstituted alkynyl group having 2 to 20 carbon atoms, a substituted or unsubstituted aryl group having 6 to 20 carbon atoms, a substituted or unsubstituted hetero atom-containing alkyl group having 1 to 20 carbon atoms, or a substituted or unsubstituted heteroaryl group having 2 to 20 carbon atoms.

More preferable $R^{21}$, $R^{22}$, $R^4$, and $R^9$ are each independently a hydrogen atom, a substituted or unsubstituted alkyl group having 1 to 10 carbon atoms, a substituted or unsubstituted cycloalkyl group having 1 to 10 carbon atoms, a substituted or unsubstituted alkenyl group having 2 to 10 carbon atoms, a substituted or unsubstituted alkynyl group having 2 to 10 carbon atoms, a substituted or unsubstituted aryl group having 6 to 15 carbon atoms, a substituted or unsubstituted hetero atom-containing alkyl group having 1 to 10 carbon atoms, or a substituted or unsubstituted heteroaryl group having 2 to 10 carbon atoms.

Further preferable $R^{21}$, $R^{22}$, $R^4$, and $R^9$ are each independently a hydrogen atom, a substituted or unsubstituted alkyl group having 1 to 6 carbon atoms, a substituted or unsubstituted cycloalkyl group having 1 to 6 carbon atoms, a substituted or unsubstituted alkenyl group having 2 to 6 carbon atoms, a substituted or unsubstituted alkynyl group having 2 to 6 carbon atoms, a substituted or unsubstituted aryl group having 6 to 10 carbon atoms, a substituted or unsubstituted hetero atom-containing alkyl group having 1 to 6 carbon atoms, or a substituted or unsubstituted heteroaryl group having 2 to 6 carbon atoms.

$R^{21}$, $R^{22}$, $R^4$, and $R^9$ may have a structure in which $R^{21}$, $R^{22}$, $R^4$, and $R^9$ are bonded to one another to form a ring, and an example thereof may include a ring structure formed by bonding of $R^{21}$ and $R^{22}$ to one another.

Particularly preferable $R^{21}$, $R^{22}$, $R^4$, and $R^9$ are each independently a hydrogen atom or a substituted or unsubstituted alkyl group having 1 to 4 carbon atoms, and most preferable $R^{21}$, $R^{22}$, $R^4$, and $R^9$ are all hydrogen atoms.

<$L^1$ and $L^2$>

In the formulas (1) and the like, $L^1$ and $L^2$ are each independently a hydrocarbon group or a hetero atom-containing hydrocarbon group.

Examples of the hydrocarbon group can include substituted or unsubstituted alkyl groups, substituted or unsubstituted cycloalkyl groups, substituted or unsubstituted alkenyl groups, substituted or unsubstituted alkynyl groups, and substituted or unsubstituted aryl groups.

Examples of the hetero atom-containing hydrocarbon group can include substituted or unsubstituted hetero atom-containing alkyl groups and substituted or unsubstituted heteroaryl groups.

Examples of the hydrocarbon group and the hetero atom-containing hydrocarbon group include alkyl groups, cycloalkyl groups, alkenyl groups, alkynyl groups, aryl groups, hetero atom-containing alkyl groups, and heteroaryl groups. These groups preferably have 1 to 20 carbon atoms. The lower limit value is preferably 2, more preferably 3, and particularly preferably 4. However, the preferable lower limit value in the case of an aryl group is 6. Meanwhile, the upper limit value is preferably 18, more preferably 15, further preferably 10, and particularly preferably 6. A heteroaryl group preferably has one or more 5 or more-membered ring structures, more preferably has one or more 5 to 7-membered ring structures, and further preferably has one or more 5-membered ring or 6-membered ring structure.

The preferable number of carbon atoms described above is selected from in the range of 4 or more or of 1 to 20. In the latter case, the number is more preferably from 1 to 10. In the former case, the number is more preferably from 4 to 20.

Preferable $L^1$ and $L^2$ in the former are each independently a substituted or unsubstituted alkyl group having 4 to 20 carbon atoms, a substituted or unsubstituted cycloalkyl group having 4 to 20 carbon atoms, a substituted or unsubstituted alkenyl group having 4 to 20 carbon atoms, a substituted or unsubstituted alkynyl group having 4 to 20 carbon atoms, a substituted or unsubstituted aryl group having 6 to 20 carbon atoms, a substituted or unsubstituted hetero atom-containing alkyl group having 4 to 20 carbon atoms, or a substituted or unsubstituted heteroaryl group having 4 to 20 carbon atoms.

More preferable $L^1$ and $L^2$ are each independently a substituted or unsubstituted alkyl group having 4 to 10 carbon atoms, a substituted or unsubstituted aryl group having 6 to 15 carbon atoms, a substituted or unsubstituted hetero atom-containing alkyl group having 4 to 10 carbon atoms, or a substituted or unsubstituted heteroaryl group having 4 to 15 carbon atoms.

Further preferable $L^1$ and $L^2$ are each independently a substituted or unsubstituted aryl group having 6 to 10 carbon atoms and a substituted or unsubstituted heteroaryl group having 4 to 10 carbon atoms, and particularly preferably a substituted or unsubstituted aryl group having 6 to 10 carbon atoms. Especially preferable are aryl groups containing a substituent other than hydrogen. The substituents other than hydrogen are hydrocarbon groups having 1 to 10 carbon atoms and hetero atom-containing hydrocarbon groups having 1 to 10 carbon atoms. The hetero atom is specifically an element of Group 16 of the periodic table and more specifically oxygen. Specific examples of the hydrocarbon group can include a methyl group, an ethyl group, an isopropyl group, a n-butyl group, a s-butyl group, and a t-butyl group, and specific examples of the hetero atom-containing hydrocarbon group can include a methoxy group, an ethoxy group, an isopropoxy group, a n-butoxy group, a s-butoxy group, and a t-butoxy group, as preferable examples.

<n1 to n6>

In the formulas (1) and the like, n2 to n5 represent integers of 0 to 2, and n1 and n6 represent integers of 0 or 1.

n2 to n5 are preferably from 0 to 2, more preferably 0 or 1, and particularly preferably 0.
n1 and n6 are preferably 0 or 1 and more preferably 1.

<X and Y>

In the formulas (2) to (9) and (31), X and Y are each independently a hydrocarbon group, a hetero atom, or a hetero atom-containing hydrocarbon group and preferably each independently a divalent group selected from the groups shown in the following general formula group (10).

[Chem. 35]

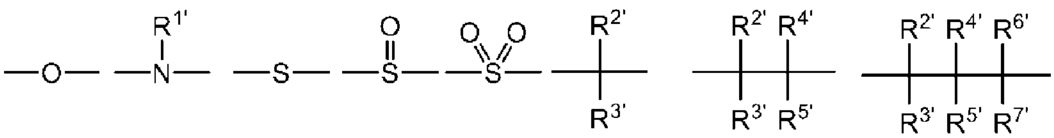

(10)

wherein, in the group (10), $R^{1'}$ to $R^{7'}$ are each independently a hydrogen atom, a hydrocarbon group, or a hetero atom-containing hydrocarbon group, and $R^{2'}$ to $R^{7'}$ are optionally bonded to one another to form a ring, or adjacent substituents are optionally directly bonded to form a multiple bond.

Preferable $R^{1'}$ to $R^{7'}$ are each independently a hydrogen atom, a substituted or unsubstituted hydrocarbon group having 1 to 10 carbon atoms, or a substituted or unsubstituted hetero atom-containing hydrocarbon group having 1 to 10 carbon atoms.

More preferable $R^{1'}$ to $R^{7'}$ are each independently a hydrogen atom or a substituted or unsubstituted hydrocarbon group having 1 to 6 carbon atoms, and most preferable $R^{1'}$ to $R^{7'}$ are all hydrogen atoms.

As previously described, $R^{2'}$ to $R^{7'}$ may be bonded to one another to form a monocyclic ring or a polycyclic ring. Alternatively, $R^{1'}$ to $R^{7'}$ can be bonded with the $R^1$ to $R^{24}$ to form a ring structure.

X and Y are preferably divalent groups selected from the groups shown in the following general formula group (11).

[Chem. 36]

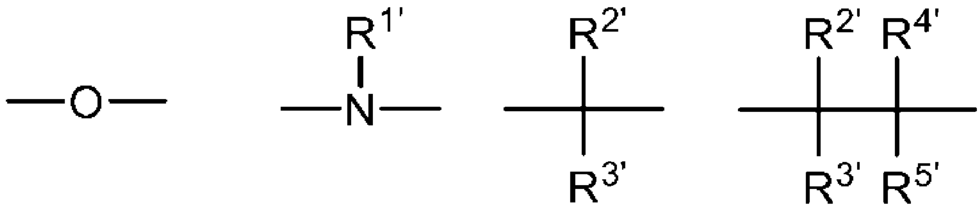

(11)

wherein, in the group (11), $R^{1'}$ to $R^{5'}$ are each independently a hydrogen atom, a hydrocarbon group having 1 to 20 carbon atoms, or a hetero atom-containing hydrocarbon group having 1 to 20 carbon atoms, and $R^{2'}$ to $R^{5'}$ are optionally bonded to one another to form a ring, or adjacent substituents are optionally directly bonded to form a multiple bond.

When X and Y are divalent groups selected from the groups shown in the general formula group (11), $R^{1'}$ to $R^{5'}$ are preferably each independently a hydrogen atom or a hydrocarbon group having 1 to 10 carbon atoms.

X and Y are further preferably divalent groups selected from the groups shown in the following general formula group (12) .

[Chem. 37]

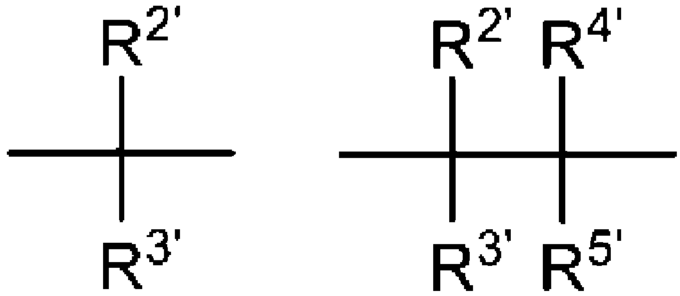

(12)

In the formula (12), $R^{2'}$ to $R^{5'}$ are each independently a hydrogen atom, a hydrocarbon group having 1 to 20 carbon atoms, or a hetero atom-containing hydrocarbon group having 1 to 20 carbon atoms, and $R^{2'}$ to $R^{5'}$ may be bonded to one another to form a ring.

X and Y are particularly preferably divalent groups shown in the following general formula (13).

[Chem. 38]

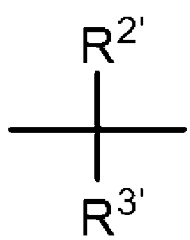

(13)

In the formula (13), $R^{2'}$ and $R^{3'}$ are each independently selected from a hydrogen atom, a hydrocarbon group having 1 to 20 carbon atoms, or a hetero atom-containing hydrocarbon group having 1 to 20 carbon atoms, and $R^{2'}$ and $R^{3'}$ may be bonded to one another to form a ring.

In the ester compound (A) represented by the formula (31), when X is a divalent group shown in the formula (13), $R^{2'}$ and $R^{3'}$ are preferably each independently a hydrogen atom or a hydrocarbon group having 1 to 10 carbon atoms, and more preferably both are hydrogen atoms.

As mentioned above, in the formulas (10), (11), and (12), $R^{2'}$ to $R^{7'}$ may be preferably bonded to one another

to further form a ring, or adjacent substituents may be preferably directly bonded to form a multiple bond. As the multiple bond, a carbon-carbon double bond is preferable. The moiety further forming a ring by bonding of the $R^{2'}$ to $R^{7'}$ to one another preferably has a structure including a carbon-carbon double bond. Of those described above, $R^{2'}$ to $R^{5'}$ are preferably bonded to one another to form a ring, and more preferably, a substituted or unsubstituted aryl group is included in the multiple bond. An example of the substituent of such X and Y is shown below.

[Chem. 39]

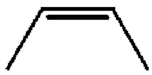
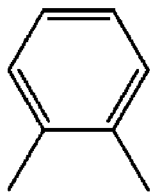
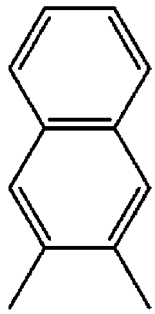

Examples of the hydrocarbon group can include substituted or unsubstituted alkyl groups, substituted or unsubstituted cycloalkyl groups, substituted or unsubstituted alkynyl groups, substituted or unsubstituted alkenyl groups, and substituted or unsubstituted aryl groups.

Examples of the hetero atom-containing hydrocarbon group can include substituted or unsubstituted hetero atom-containing alkyl groups and substituted or unsubstituted heteroaryl groups.

Examples of the hydrocarbon group and the hetero atom-containing hydrocarbon group include alkyl groups, cycloalkyl groups, alkenyl groups, alkynyl groups, aryl groups, hetero atom-containing alkyl groups, and heteroaryl groups. These groups preferably have 1 to 20 carbon atoms. The lower limit value is preferably 2, more preferably 3, and particularly preferably 4. However, in the case of an aryl group, the preferable lower limit value is 6, and the upper limit value is preferably 20, more preferably 15, further preferably 10, and particularly preferably 6. A heteroaryl group preferably has one or more 5 or more-membered ring structures, more preferably has one or more 5 to 7-membered ring structures, and further preferably has one or more 5-membered ring or 6-membered ring structure.

More specific examples of the groups and atoms exemplified for $R^1$ to $R^{24}$, $R^{31}$ to $R^{34}$, $L^1$, $L^2$, and $R^{1'}$ to $R^{7'}$ are shown hereinafter.

Examples of the halogen atom include fluorine, chlorine, bromine, and iodine. As the hydrocarbon group, various structures can be exemplified such as aliphatic, branched aliphatic, alicyclic, and aromatic structures as described below.

Examples of the substituted or unsubstituted alkyl group include a methyl group, an ethyl group, a n-propyl group, an isopropyl group, a n-butyl group, an isobutyl group, a sec-butyl group, a tert-butyl group, a neopentyl group, a n-hexyl group, a thexyl group, a cumyl group, and a trityl group.

Examples of the substituted or unsubstituted alkenyl group include a vinyl group, an allyl group, a propenyl group, an isopropenyl group, a butenyl group, an isobutenyl group, a pentenyl group, and a hexenyl group.

Examples of the substituted or unsubstituted alkynyl group include an ethynyl group, a propynyl group, a butynyl group, a pentynyl group, a hexynyl group, a heptynyl group, and an octynyl group.

Examples of the substituted or unsubstituted cycloalkyl group include a cyclopropyl group, a cyclobutyl group, a cyclopentyl group, a cyclohexyl group, a methylcyclohexyl group, a cycloheptyl group, a cyclooctyl group, an adamantyl group, a cyclopentadienyl group, an indenyl group, and a fluorenyl group.

Examples of the substituted or unsubstituted aryl group include aromatic hydrocarbon groups such as a phenyl group, a methylphenyl group, a dimethylphenyl group, a diisopropylphenyl group, a dimethylisopropylphenyl group, a tert-butylphenyl group, a di-tert-butylphenyl group, a naphthyl group, a biphenyl group, a terphenyl group, a phenanthryl group, and an anthracenyl group and hetero atom-substituted aryl groups such as a methoxyphenyl group, a dimethylaminophenyl group, a nitrophenyl group, and a trifluoromethylphenyl group.

Examples of the substituted or unsubstituted hetero atom-containing hydrocarbon group include hetero atom-containing alkyl groups such as a methoxymethyl group, a methoxyethyl group, a benzyloxy group, an ethoxymethyl group, and an ethoxyethyl group, and heteroaryl groups such as a furyl group, a pyrrolyl group, a thienyl group, a pyrazolyl group, a pyridyl group, a carbazolyl group, an imidazolyl group, a dimethylfuryl group, a N-methylpyrrolyl group, a N-phenylpyrrolyl group, a diphenylpyrrolyl group, a thiazolyl group, a quinolyl group, a benzofuryl group, a triazolyl group, and a tetrazolyl group. The number of carbon atoms contained in a substituent containing carbon as described above is, as previously described, preferably from 1 to 20, more preferably from 1 to 10, further preferably from 1 to 6, and

particularly preferably from 1 to 4. The number of carbon atoms when the substituent has an aryl group structure is preferably from 4 to 20, more preferably from 4 to 10, and further preferably from 6 to 10. $R^1$ to $R^{24}$, when being substituents other than a hydrogen atom, are preferably selected from the hydrocarbon groups and oxygen-containing hydrocarbon substituents described above. As the oxygen-containing hydrocarbon group, an alkoxy group is further preferable. With such a structure, in use as the internal donor of a catalyst for olefin polymerization, effects may be developed such as achievement of an olefin polymer having a broader molecular weight distribution.

<Specific examples of ester compound (A)>

Hereinafter, specific examples of the ester compound (A) of the present invention will be shown, but the ester compound (A) of the present invention is not limited thereto.

[Chem. 40]

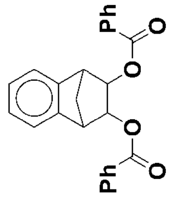

A-1

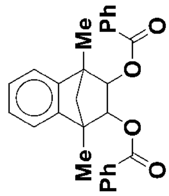

A-2

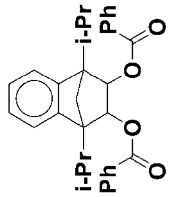

A-3

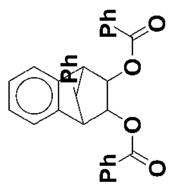

A-4

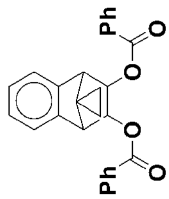

A-5

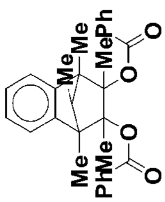

A-6

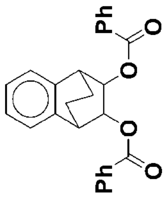

A-7

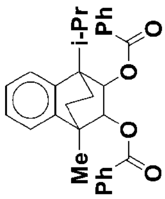

A-8

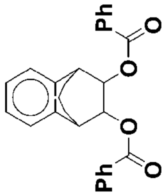

A-9

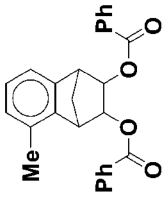

A-10

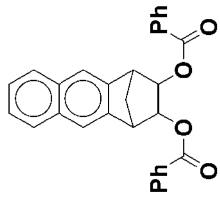

A-11

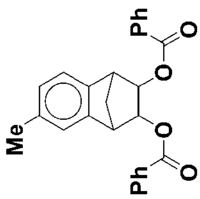

A-12

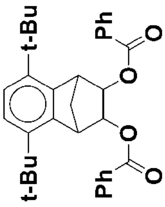

A-13

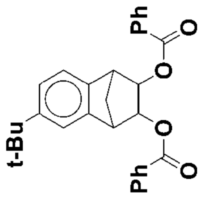

A-14

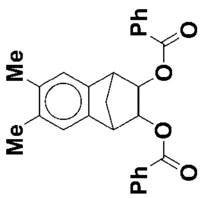

A-15

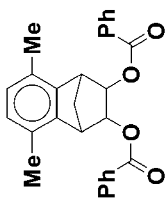

A-16

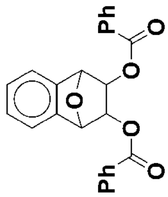

A-17

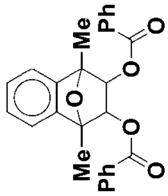

A-18

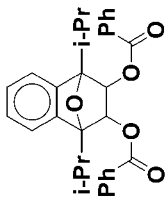

A-19

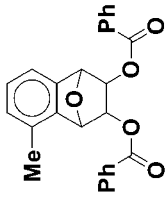

A-20

[Chem. 41]

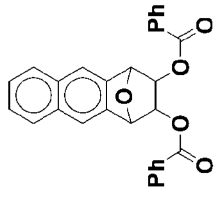
A-21

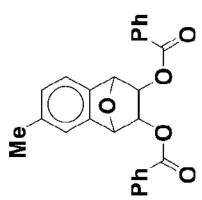
A-22

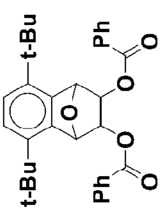
A-23

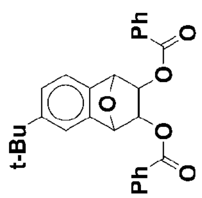
A-24

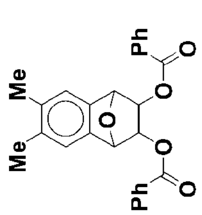
A-25

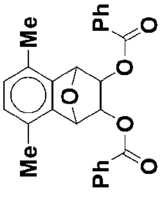
A-26

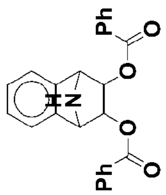
A-27

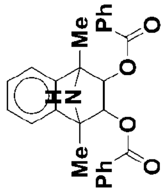
A-28

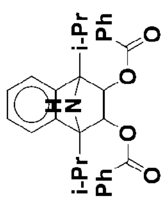
A-29

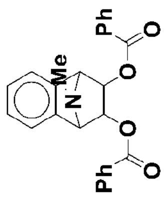
A-30

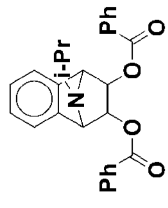
A-31

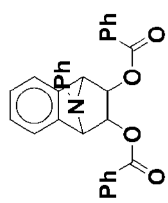
A-32

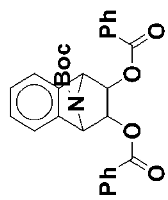
A-33

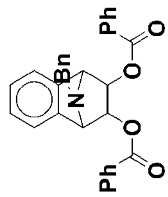
A-34

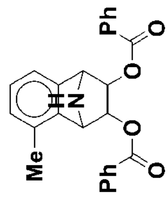
A-35

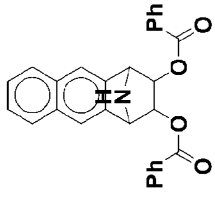
A-36

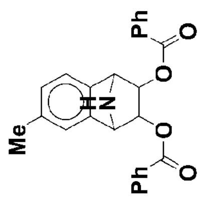
A-37

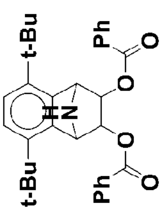
A-38

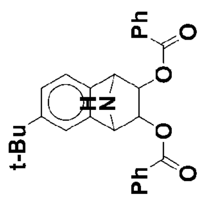
A-39

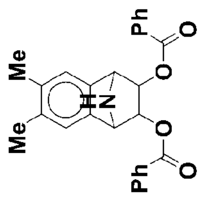
A-40

[Chem. 42]

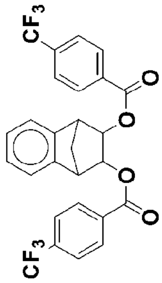

A-44

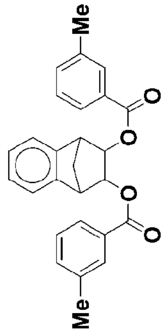

A-48

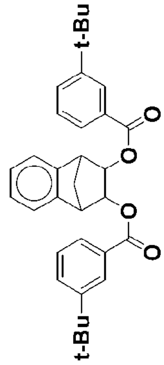

A-52

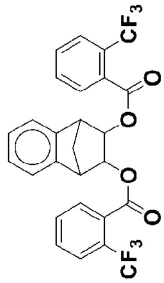

A-56

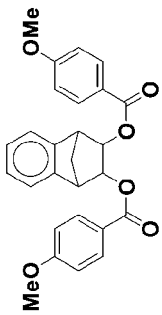

A-43

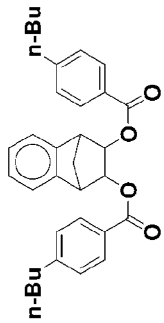

A-47

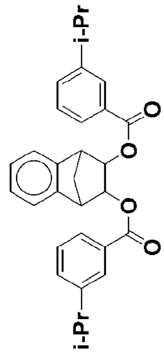

A-51

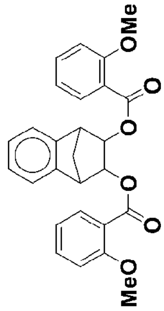

A-55

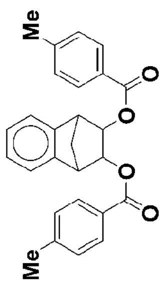

A-42

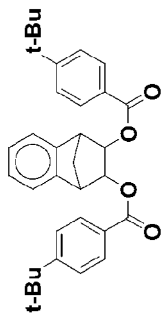

A-46

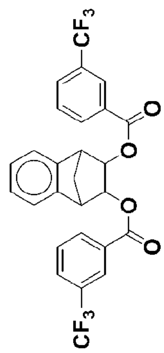

A-50

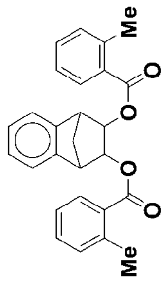

A-54

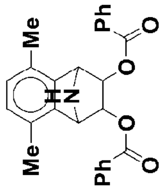

A-41

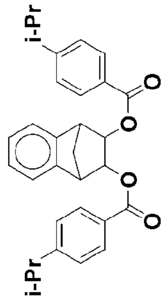

A-45

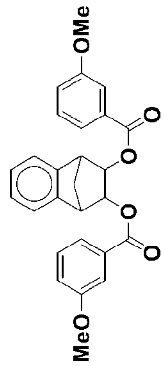

A-49

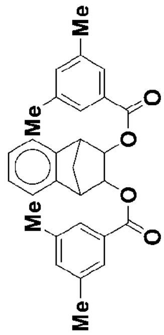

A-53

[Chem. 43]

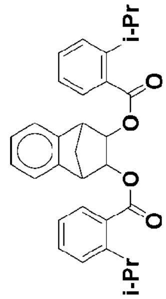
A-57

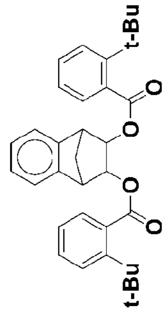
A-58

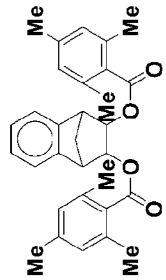
A-59

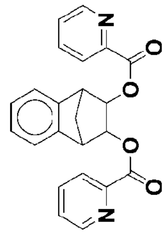
A-60

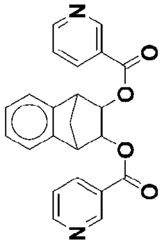
A-61

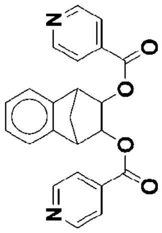
A-62

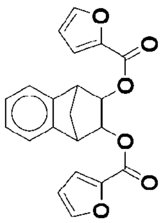
A-63

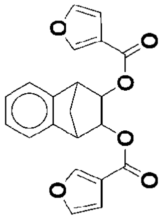
A-64

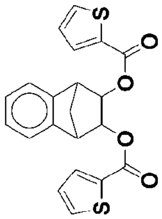
A-65

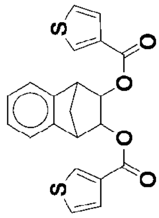
A-66

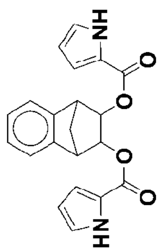
A-67

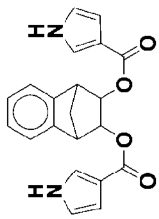
A-68

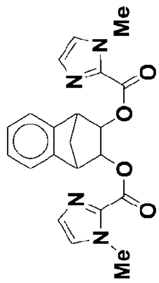
A-69

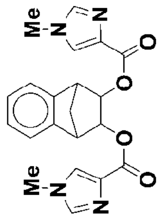
A-70

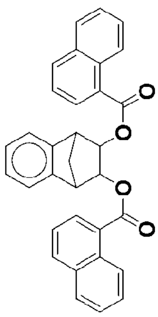
A-71

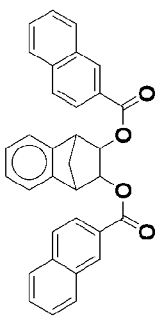
A-72

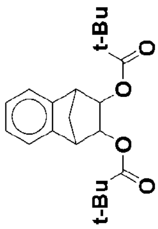
A-73

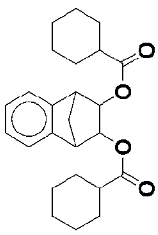
A-74

[Chem. 44]

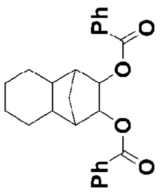
B-1

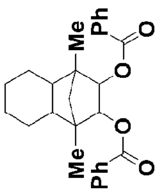
B-2

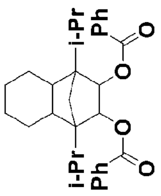
B-3

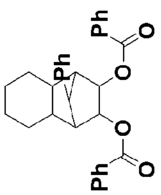
B-4

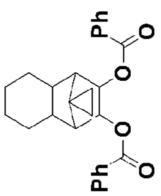
B-5

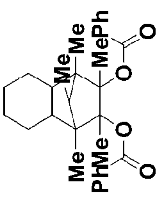
B-6

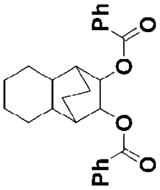
B-7

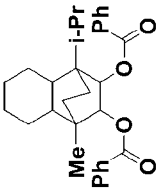
B-8

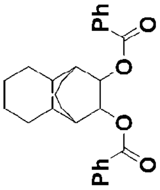
B-9

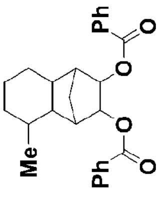
B-10

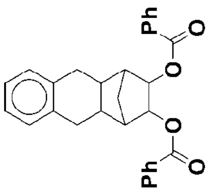
B-11

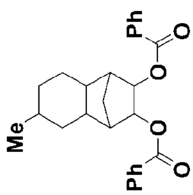
B-12

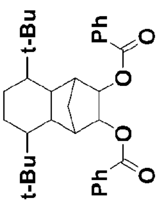
B-13

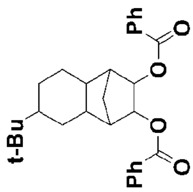
B-14

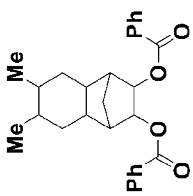
B-15

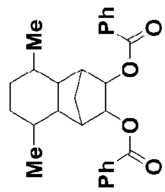
B-16

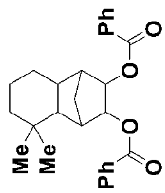
B-17

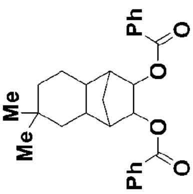
B-18

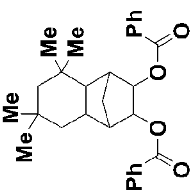
B-19

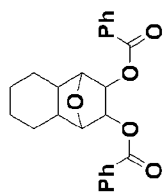
B-20

[Chem. 45]

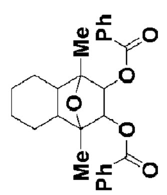
B-21

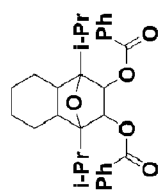
B-22

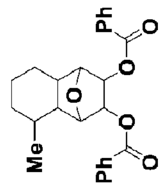
B-23

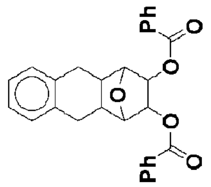
B-24

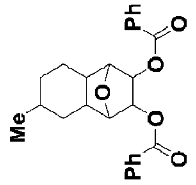
B-25

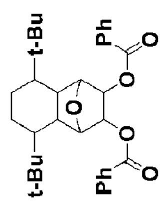
B-26

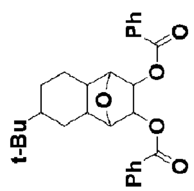
B-27

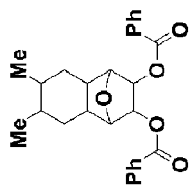
B-28

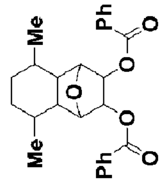
B-29

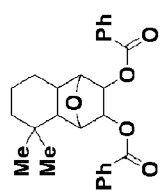
B-30

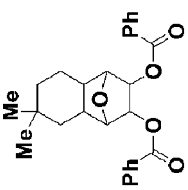
B-31

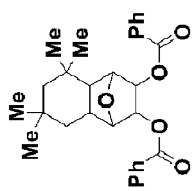
B-32

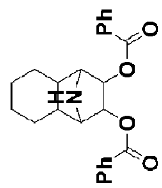
B-33

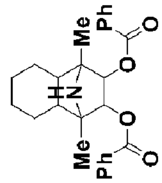
B-34

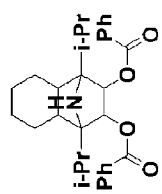
B-35

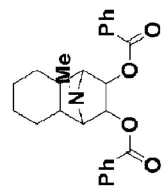
B-36

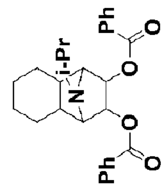
B-37

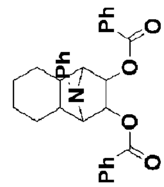
B-38

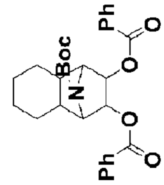
B-39

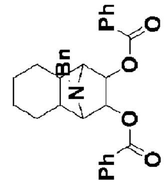
B-40

[Chem. 46]

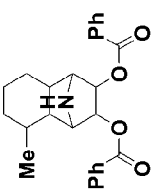
B-41

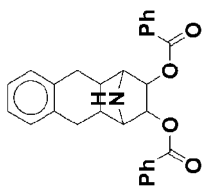
B-42

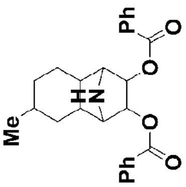
B-43

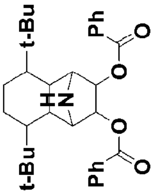
B-44

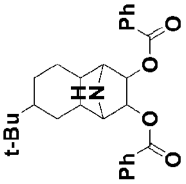
B-45

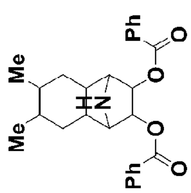
B-46

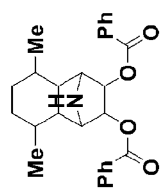
B-47

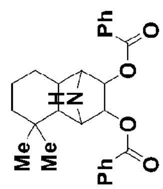
B-48

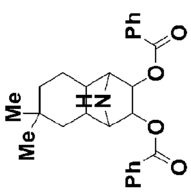
B-49

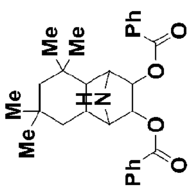
B-50

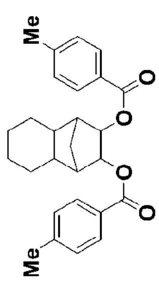
B-51

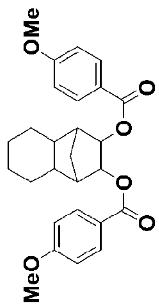
B-52

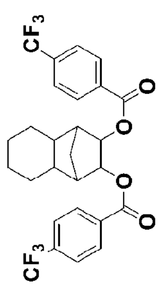
B-53

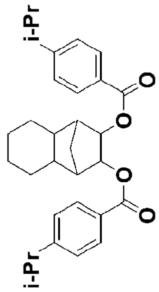
B-54

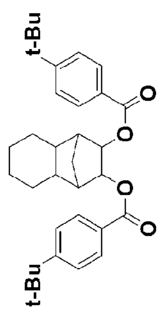
B-55

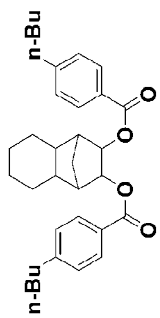
B-56

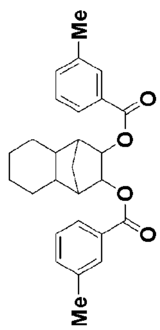
B-57

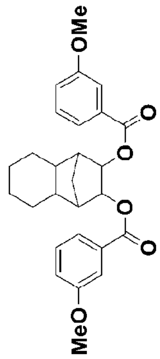
B-58

[Chem. 47]

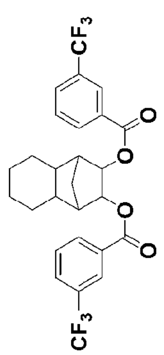

B-59

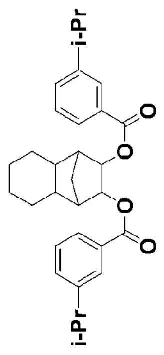

B-60

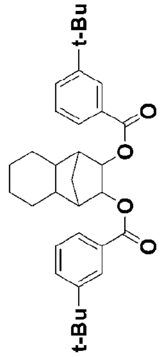

B-61

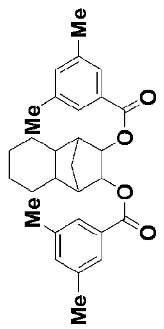

B-62

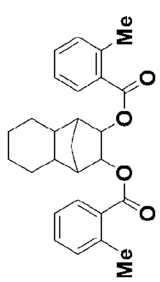

B-63

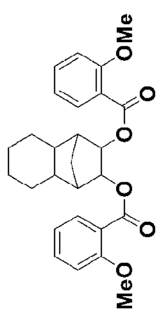

B-64

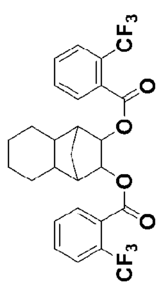

B-65

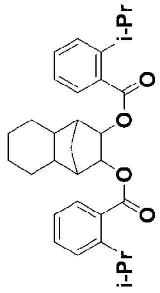

B-66

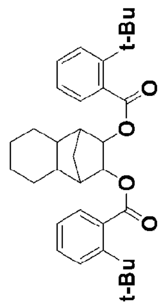

B-67

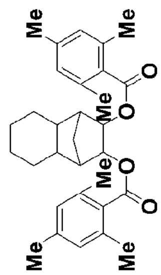

B-68

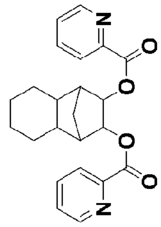

B-69

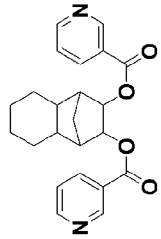

B-70

[Chem. 48]

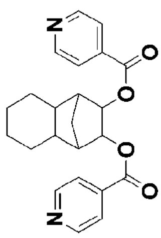
B-71

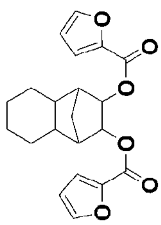
B-72

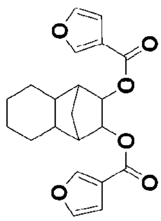
B-73

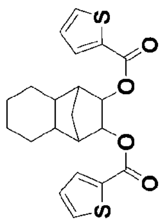
B-74

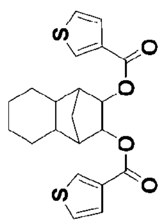
B-75

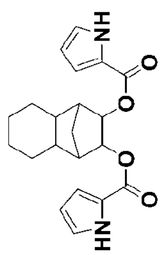
B-76

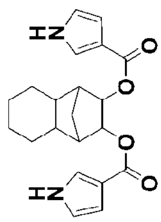
B-77

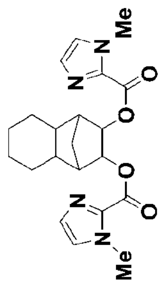
B-78

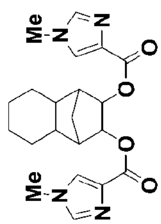
B-79

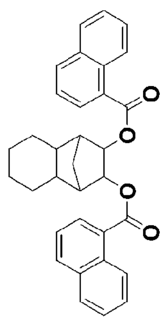
B-80

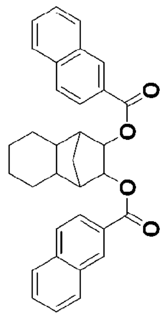
B-81

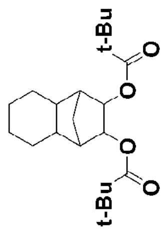
B-82

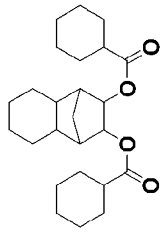
B-83

[Chem. 49]

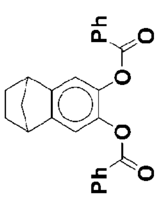
C-1

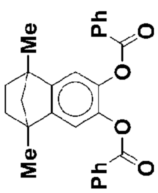
C-2

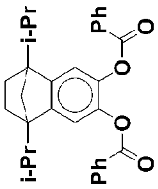
C-3

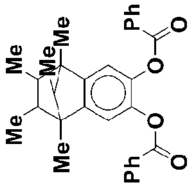
C-4

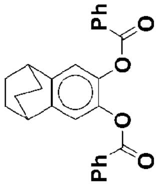
C-5

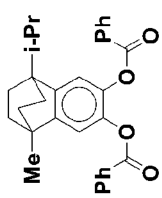
C-6

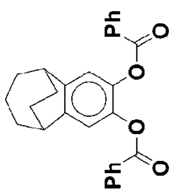
C-7

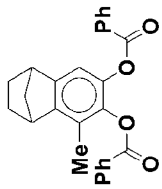
C-8

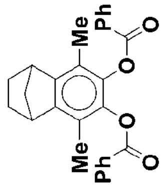
C-9

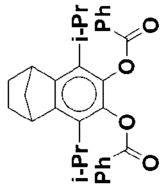
C-10

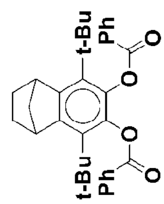
C-11

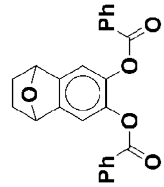
C-12

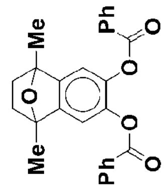
C-13

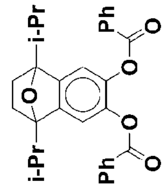
C-14

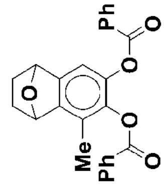
C-15

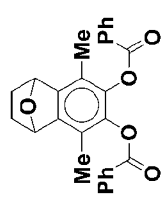
C-16

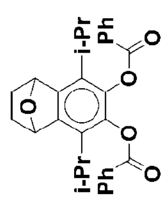
C-17

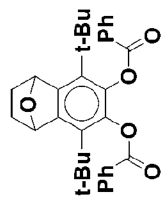
C-18

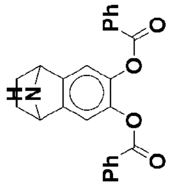
C-19

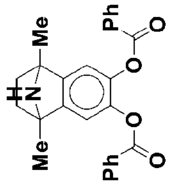
C-20

[Chem. 50]

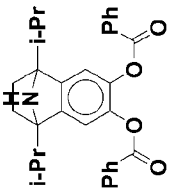

C-21

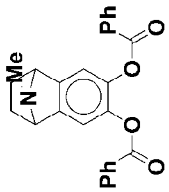

C-22

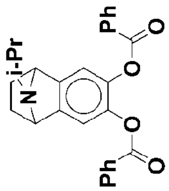

C-23

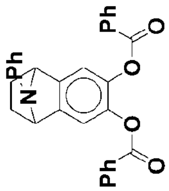

C-24

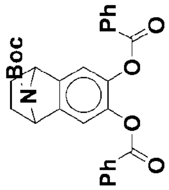

C-25

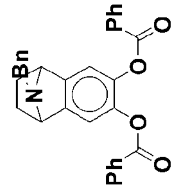

C-26

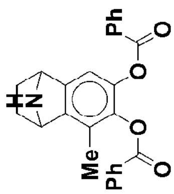

C-27

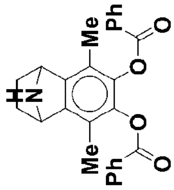

C-28

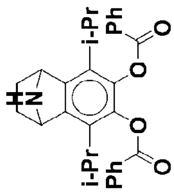

C-29

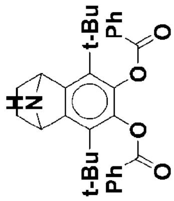

C-30

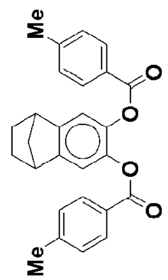

C-31

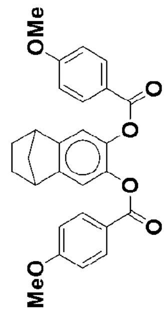

C-32

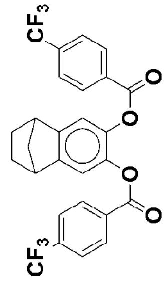

C-33

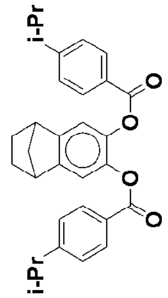

C-34

[Chem. 51]

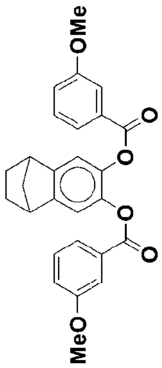
C-38

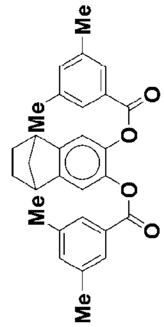
C-42

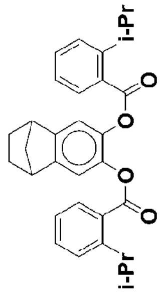
C-46

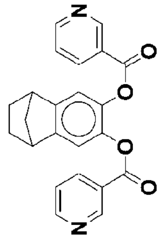
C-50

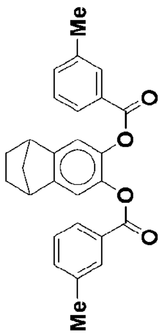
C-37

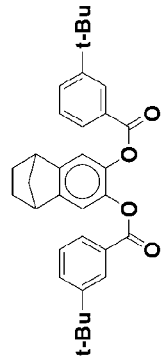
C-41

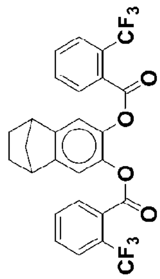
C-45

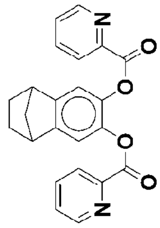
C-49

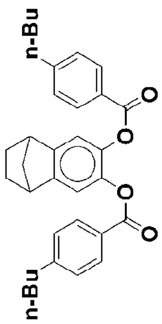
C-36

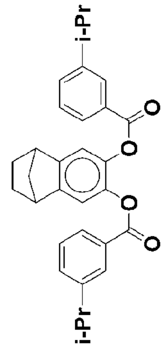
C-40

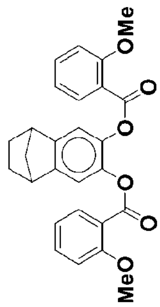
C-44

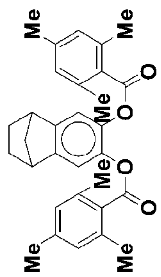
C-48

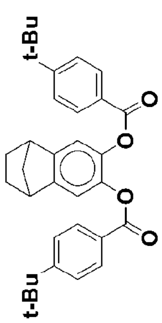
C-35

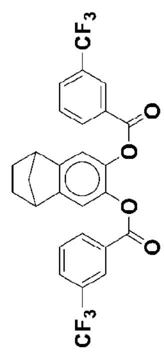
C-39

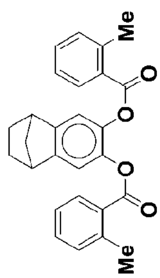
C-43

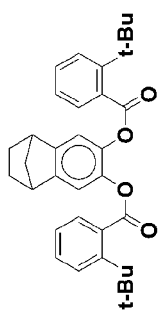
C-47

[Chem. 52]

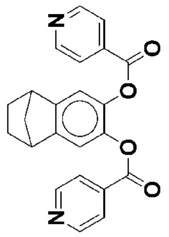
C-51

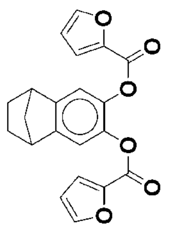
C-52

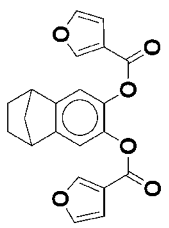
C-53

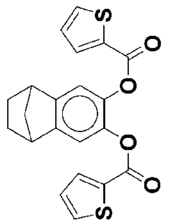
C-54

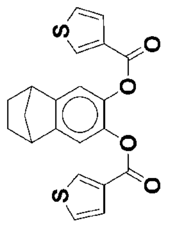
C-55

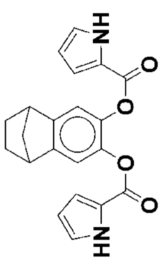
C-56

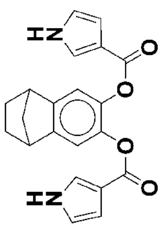
C-57

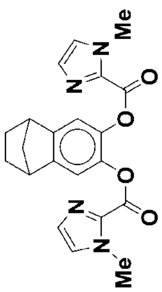
C-58

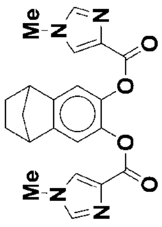
C-59

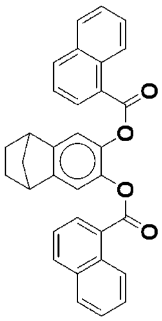
C-60

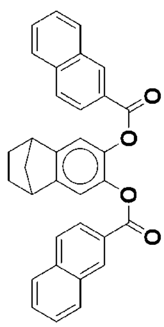
C-61

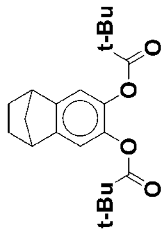
C-62

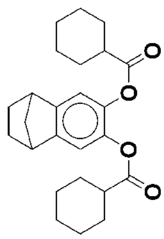
C-63

[Chem. 53]

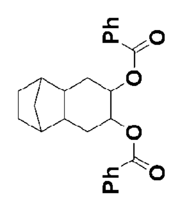
D-1

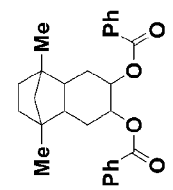
D-2

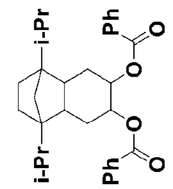
D-3

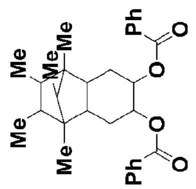
D-4

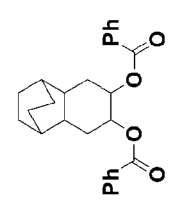
D-5

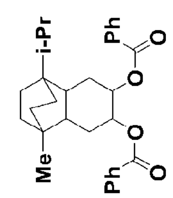
D-6

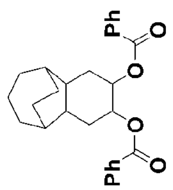
D-7

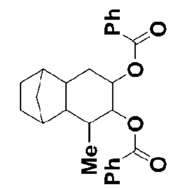
D-8

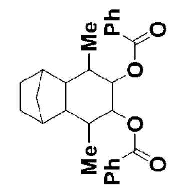
D-9

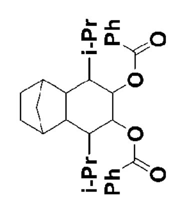
D-10

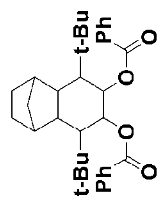
D-11

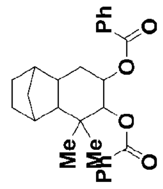
D-12

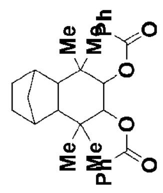
D-13

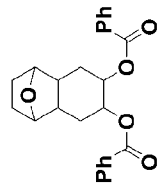
D-14

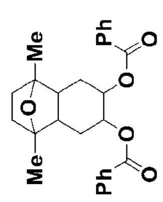
D-15

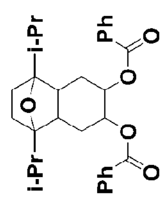
D-16

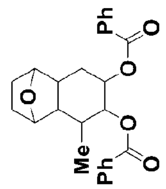
D-17

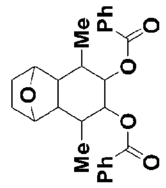
D-18

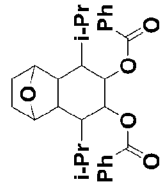
D-19

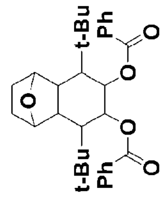
D-20

[Chem. 54]

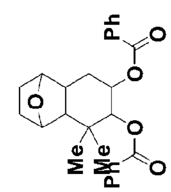

D-21

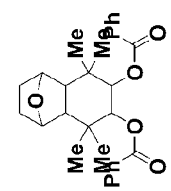

D-22

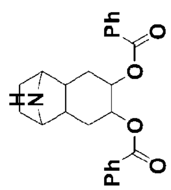

D-23

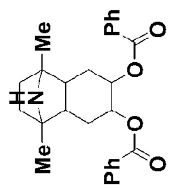

D-24

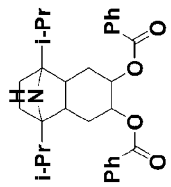

D-25

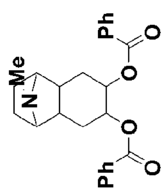

D-26

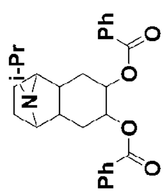

D-27

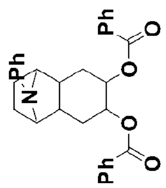

D-28

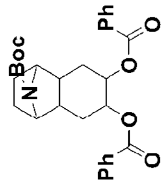

D-29

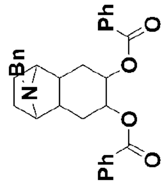

D-30

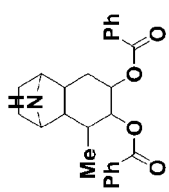

D-31

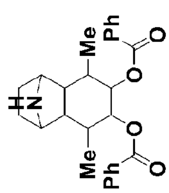

D-32

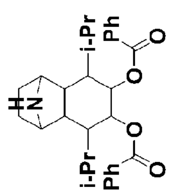

D-33

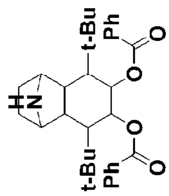

D-34

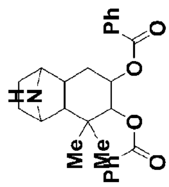

D-35

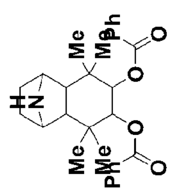

D-36

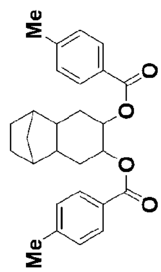

D-37

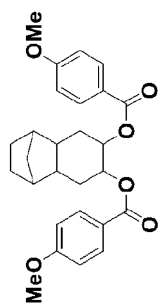

D-38

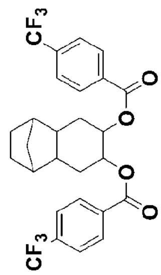

D-39

[Chem. 55]

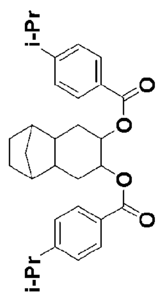
D-40

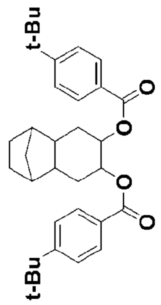
D-41

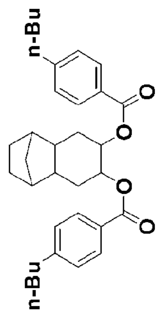
D-42

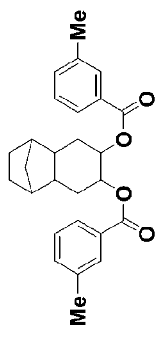
D-43

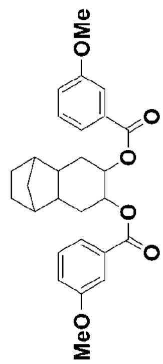
D-44

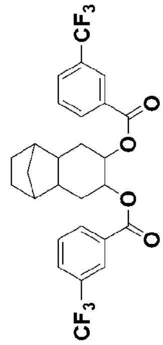
D-45

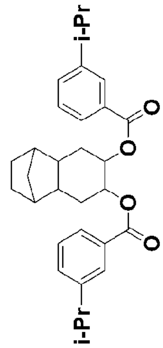
D-46

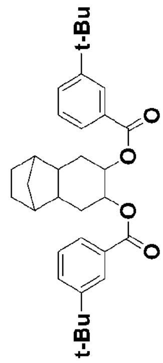
D-47

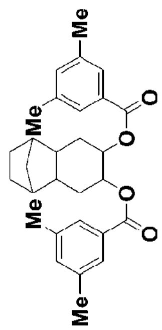
D-48

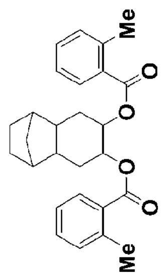
D-49

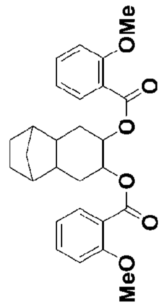
D-50

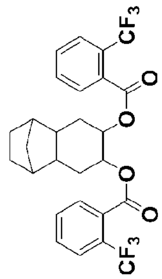
D-51

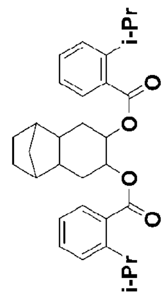
D-52

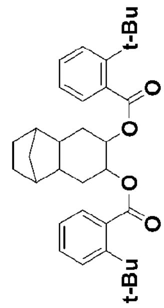
D-53

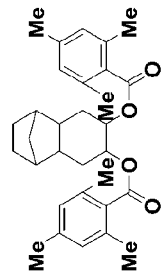
D-54

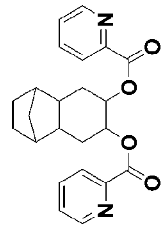
D-55

[Chem. 56]

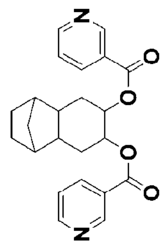
D-56

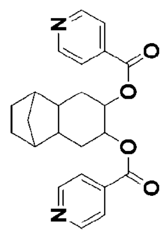
D-57

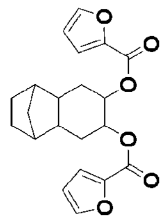
D-58

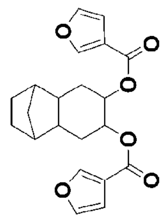
D-59

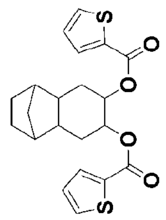
D-60

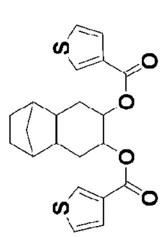
D-61

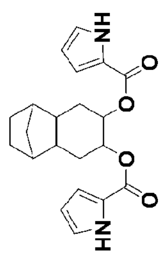
D-62

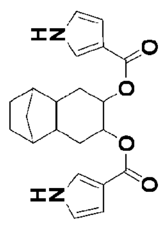
D-63

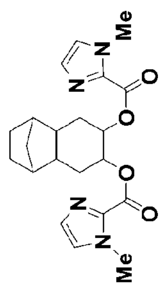
D-64

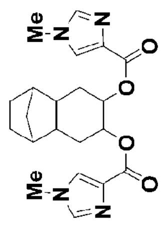
D-65

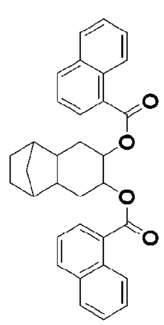
D-66

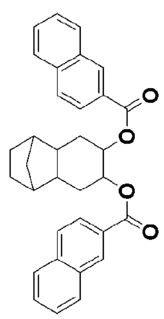
D-67

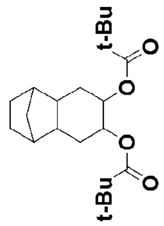
D-68

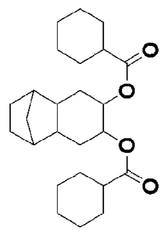
D-69

[Chem. 57]

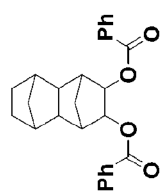

E-1

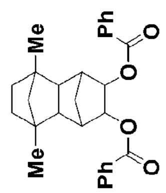

E-2

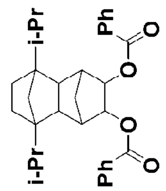

E-3

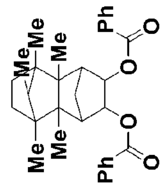

E-4

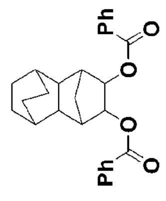

E-5

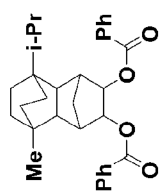

E-6

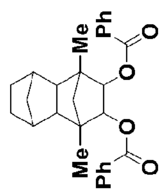

E-7

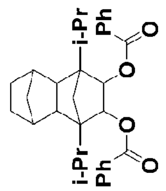

E-8

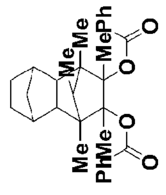

E-9

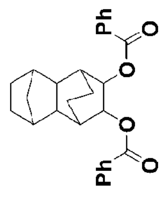

E-10

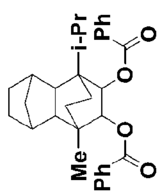

E-11

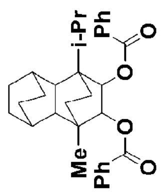

E-12

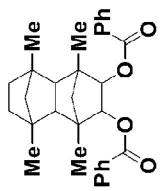

E-13

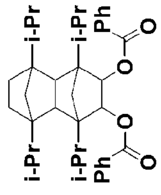

E-14

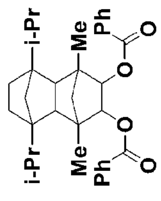

E-15

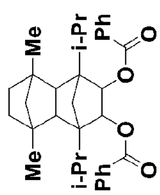

E-16

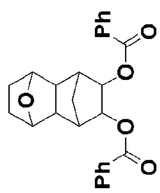

E-17

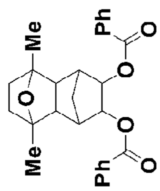

E-18

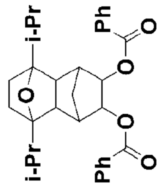

E-19

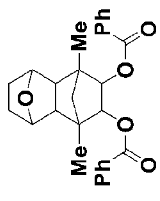

E-20

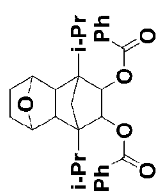

E-21

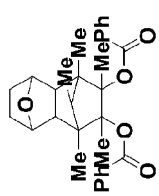

E-22

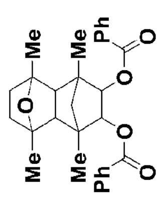

E-23

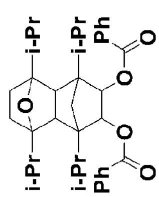

E-24

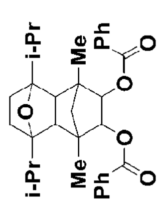

E-25

[Chem. 58]

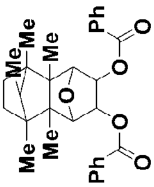

E-30

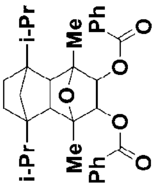

E-35

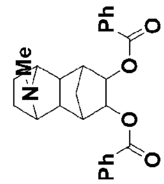

E-40

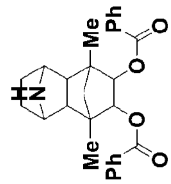

E-45

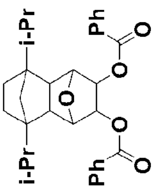

E-29

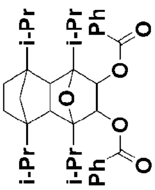

E-34

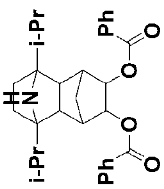

E-39

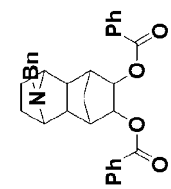

E-44

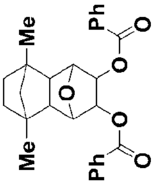

E-28

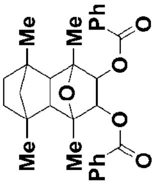

E-33

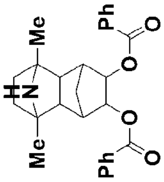

E-38

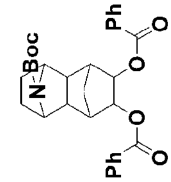

E-43

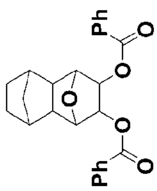

E-27

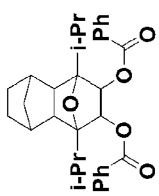

E-32

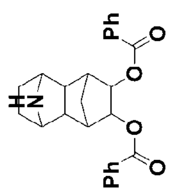

E-37

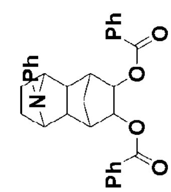

E-42

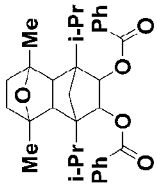

E-26

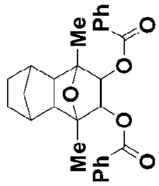

E-31

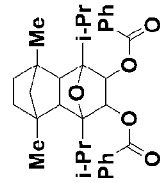

E-36

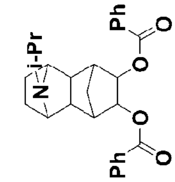

E-41

[Chem. 59]

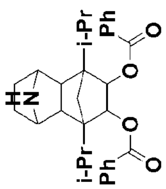
E-46

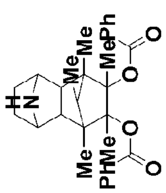
E-47

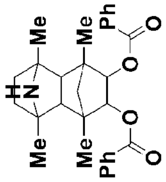
E-48

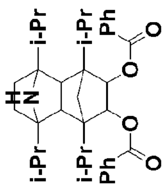
E-49

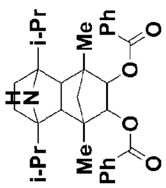
E-50

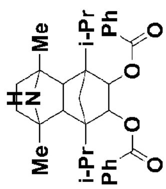
E-51

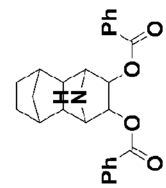
E-52

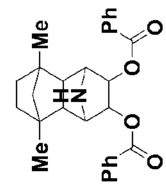
E-53

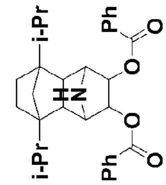
E-54

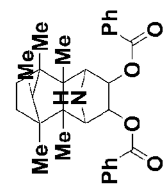
E-55

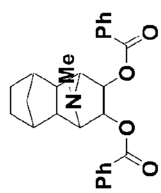
E-56

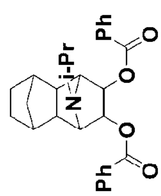
E-57

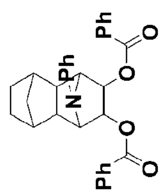
E-58

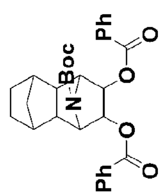
E-59

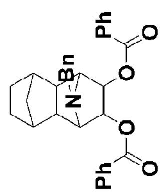
E-60

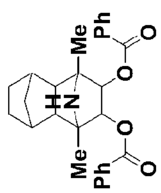
E-61

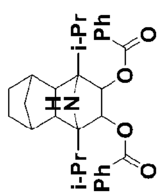
E-62

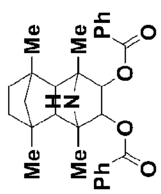
E-63

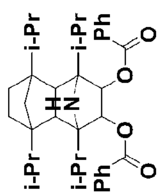
E-64

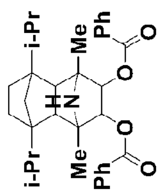
E-65

[Chem. 60]

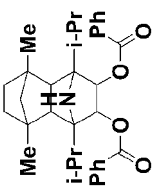
E-66

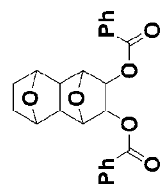
E-67

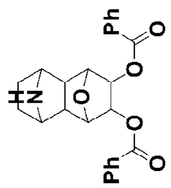
E-68

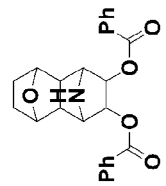
E-69

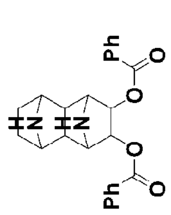
E-70

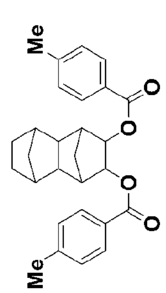
E-71

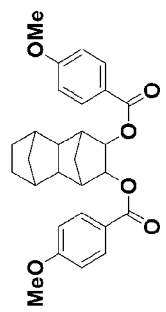
E-72

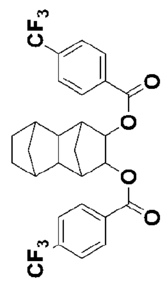
E-73

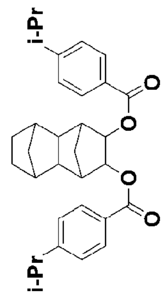
E-74

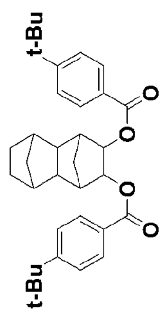
E-75

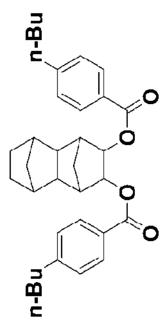
E-76

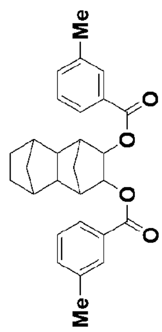
E-77

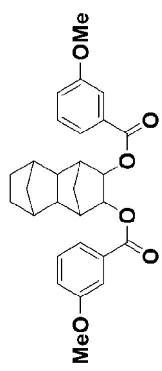
E-78

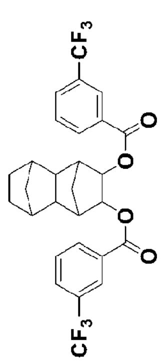
E-79

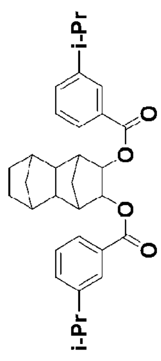
E-80

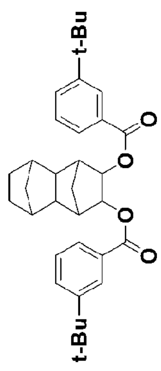
E-81

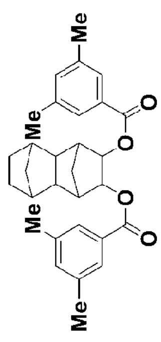
E-82

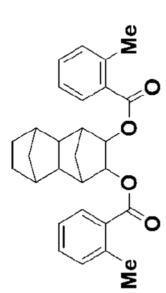
E-83

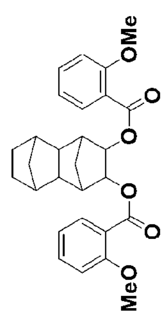
E-84

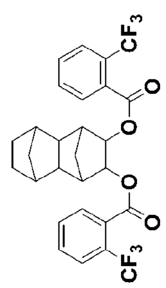
E-85

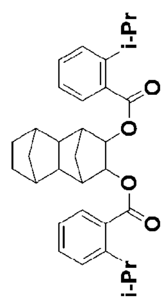
E-86

[Chem. 61]

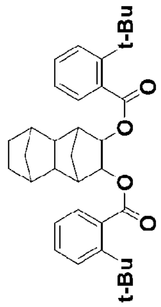
E-87

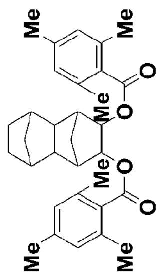
E-88

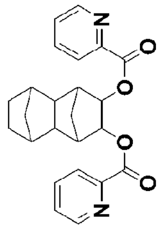
E-89

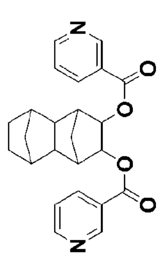
E-90

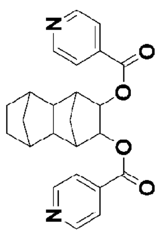
E-91

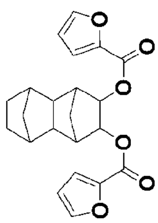
E-92

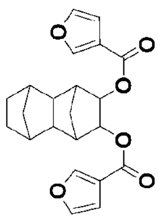
E-93

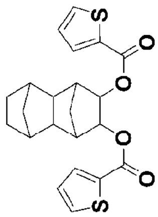
E-94

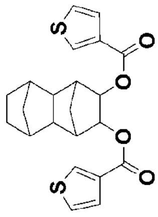
E-95

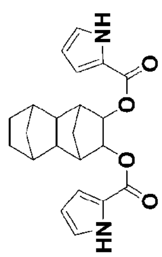
E-96

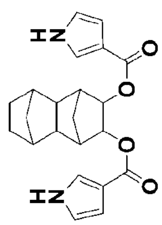
E-97

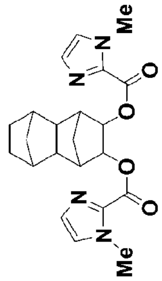
E-98

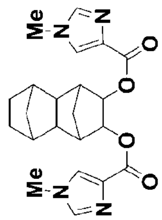
E-99

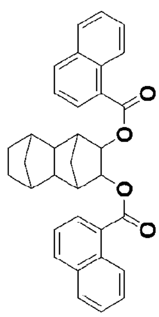
E-100

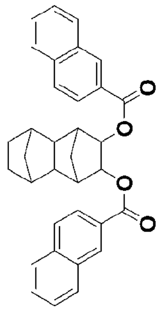
E-101

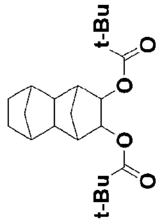
E-102

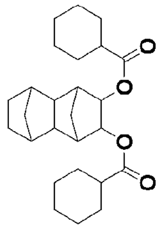
E-103

[Chem. 62]

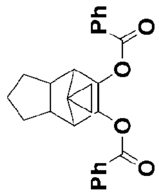
F-5

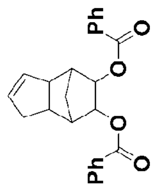
F-10

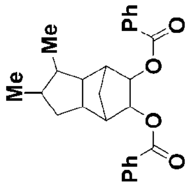
F-15

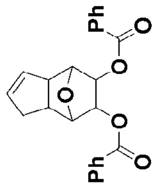
F-20

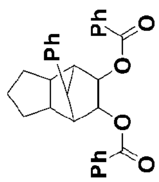
F-4

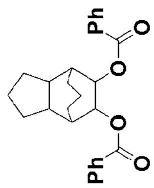
F-9

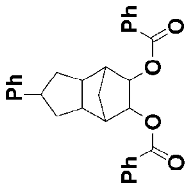
F-14

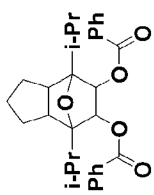
F-19

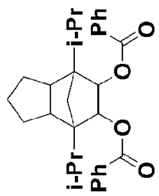
F-3

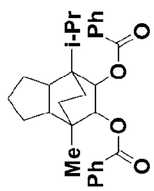
F-8

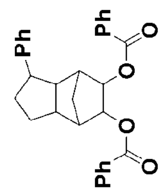
F-13

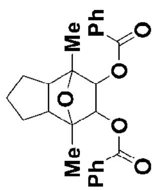
F-18

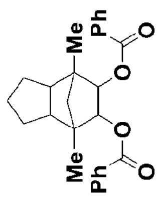
F-2

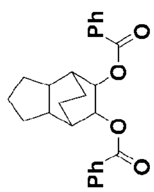
F-7

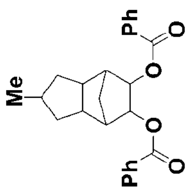
F-12

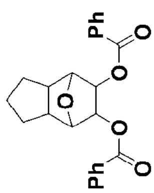
F-17

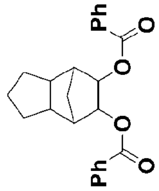
F-1

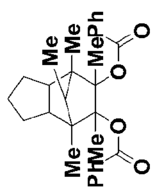
F-6

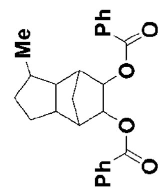
F-11

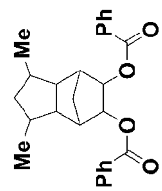
F-16

[Chem. 63]

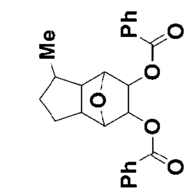
F-21

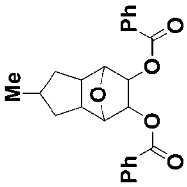
F-22

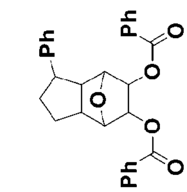
F-23

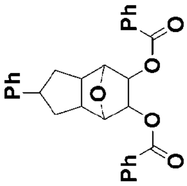
F-24

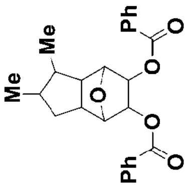
F-25

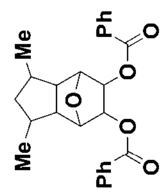
F-26

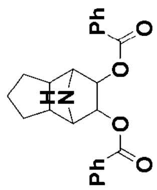
F-27

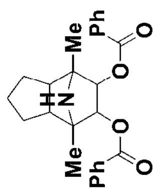
F-28

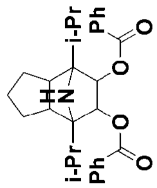
F-29

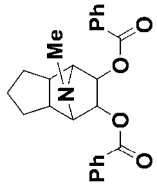
F-30

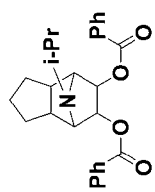
F-31

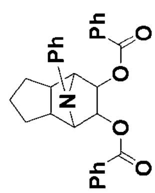
F-32

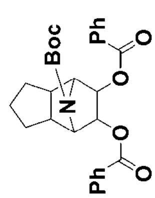
F-33

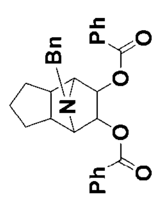
F-34

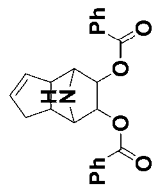
F-35

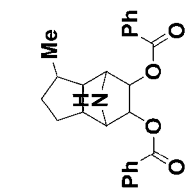
F-36

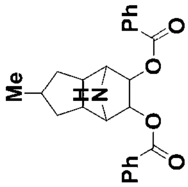
F-37

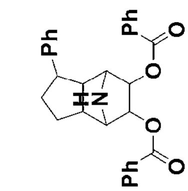
F-38

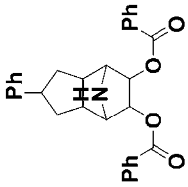
F-39

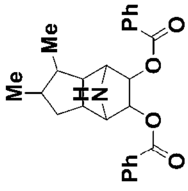
F-40

[Chem. 64]

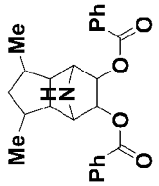
F-41

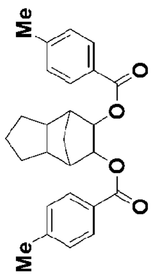
F-42

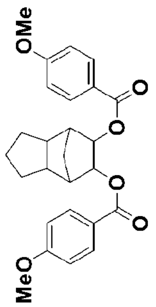
F-43

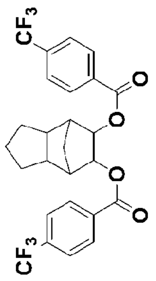
F-44

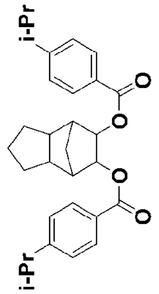
F-45

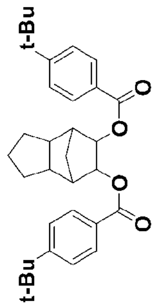
F-46

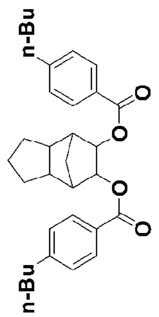
F-47

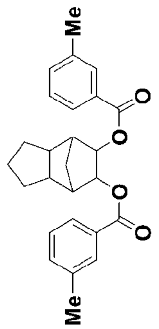
F-48

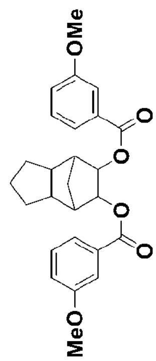
F-49

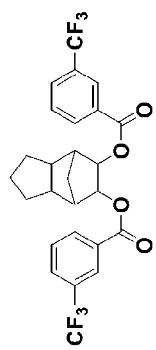
F-50

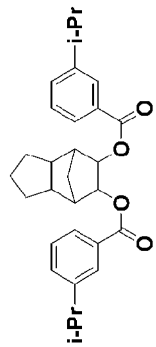
F-51

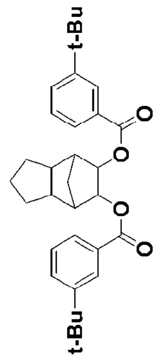
F-52

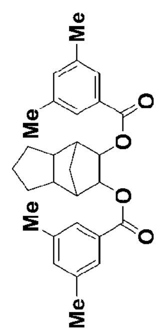
F-53

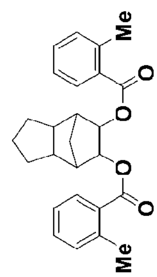
F-54

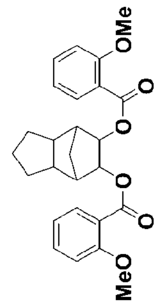
F-55

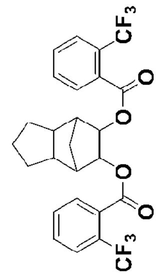
F-56

[Chem. 65]

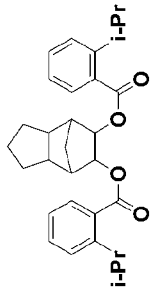
F-57

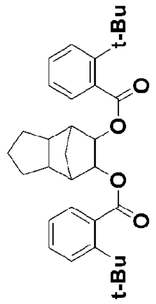
F-58

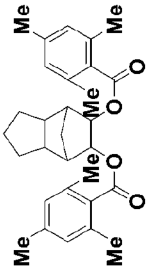
F-59

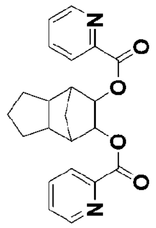
F-60

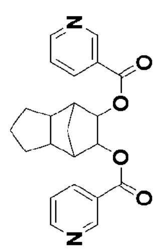
F-61

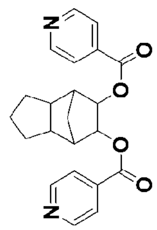
F-62

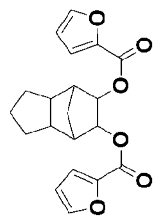
F-63

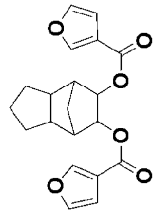
F-64

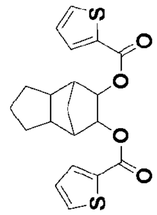
F-65

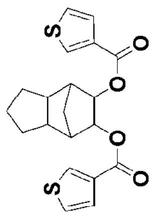
F-66

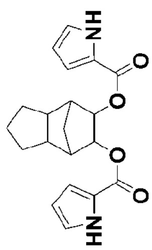
F-67

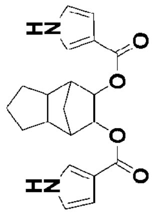
F-68

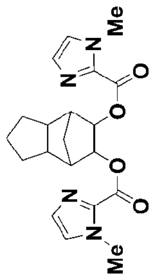
F-69

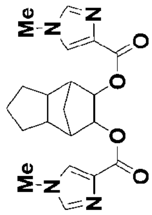
F-70

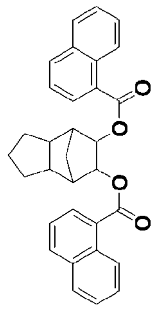
F-71

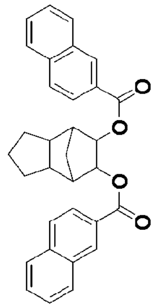
F-72

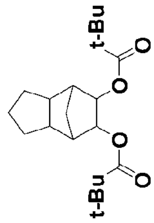
F-73

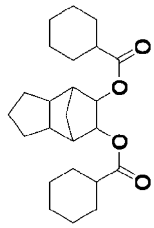
F-74

[Chem. 66]

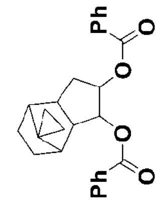
G-5

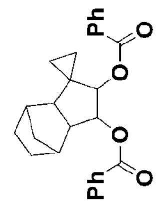
G-10

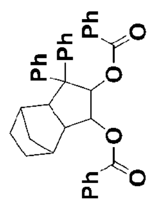
G-15

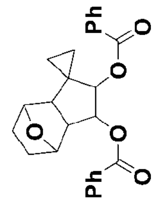
G-20

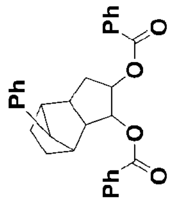
G-4

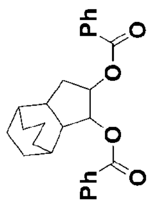
G-9

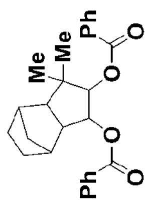
G-14

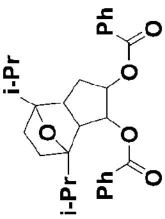
G-19

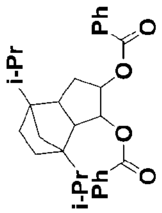
G-3

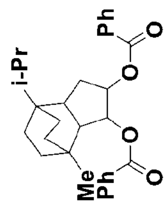
G-8

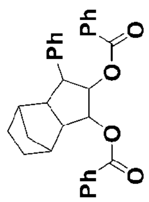
G-13

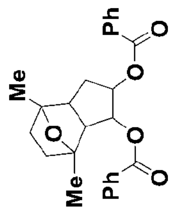
G-18

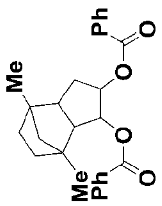
G-2

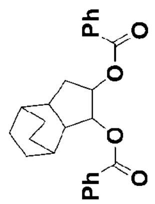
G-7

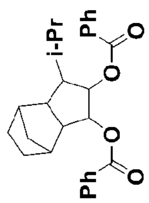
G-12

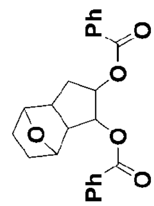
G-17

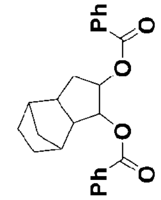
G-1

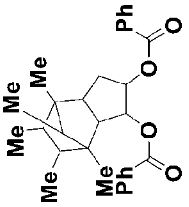
G-6

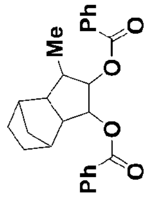
G-11

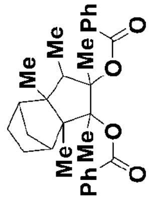
G-16

[Chem. 67]

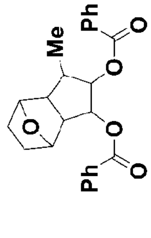
G-21

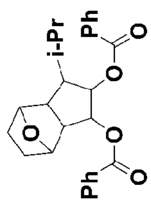
G-22

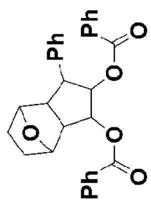
G-23

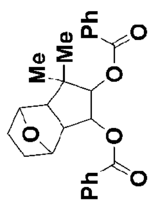
G-24

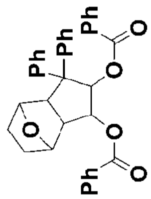
G-25

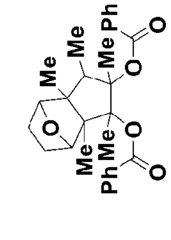
G-26

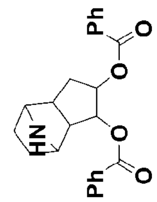
G-27

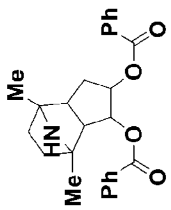
G-28

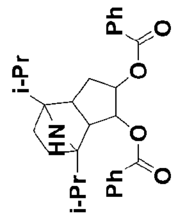
G-29

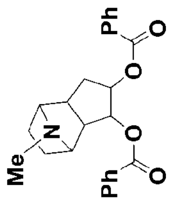
G-30

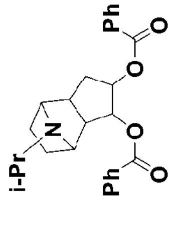
G-31

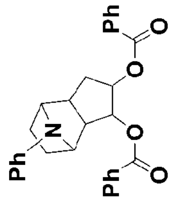
G-32

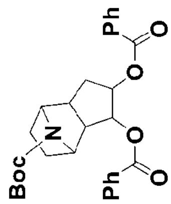
G-33

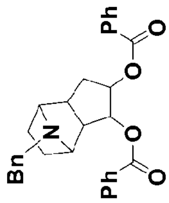
G-34

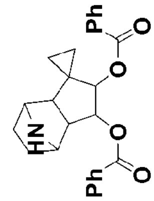
G-35

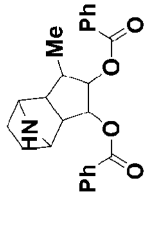
G-36

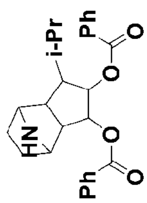
G-37

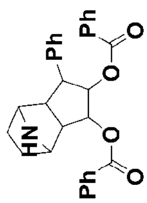
G-38

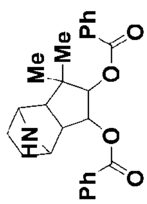
G-39

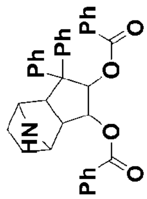
G-40

[Chem. 68]

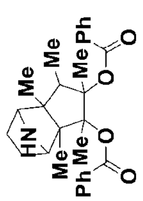
G-41

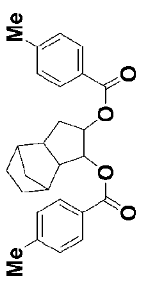
G-42

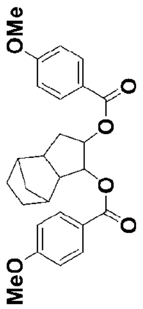
G-43

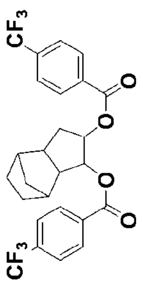
G-44

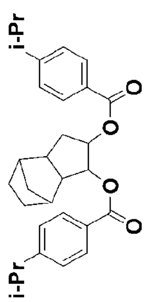
G-45

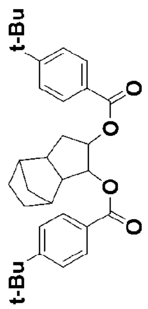
G-46

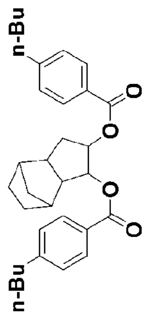
G-47

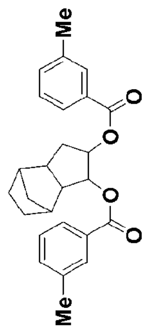
G-48

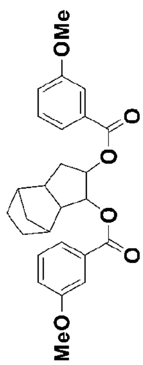
G-49

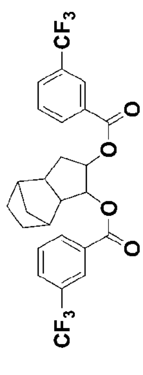
G-50

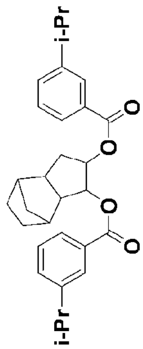
G-51

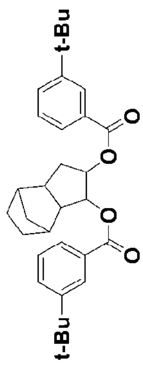
G-52

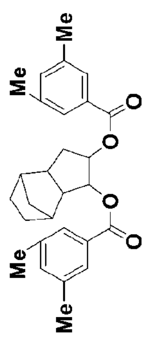
G-53

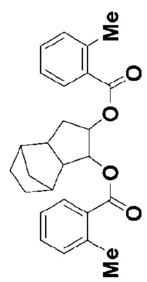
G-54

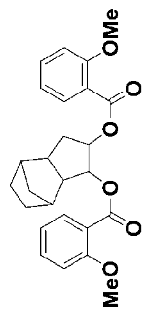
G-55

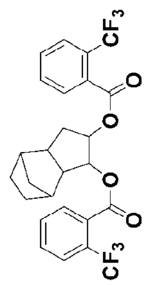
G-56

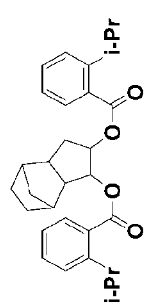
G-57

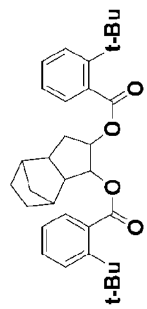
G-58

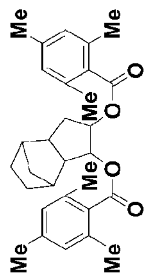
G-59

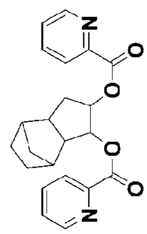
G-60

[Chem. 69]

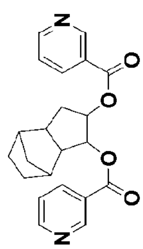
G-61

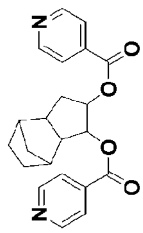
G-62

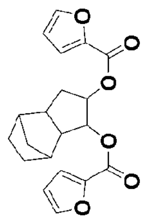
G-63

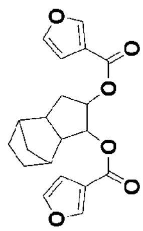
G-64

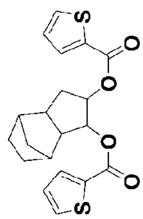
G-65

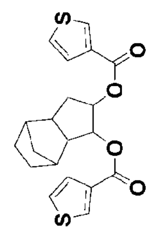
G-66

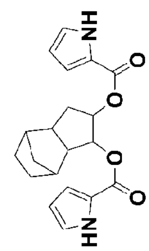
G-67

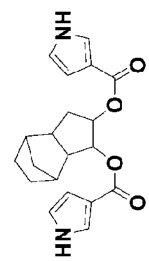
G-68

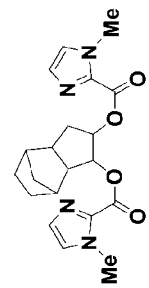
G-69

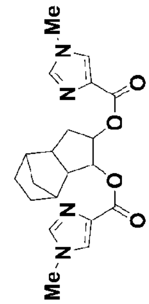
G-70

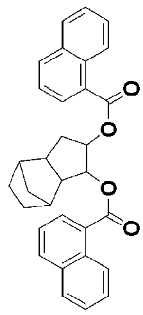
G-71

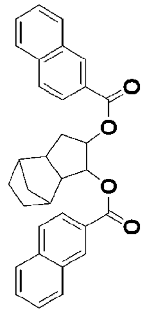
G-72

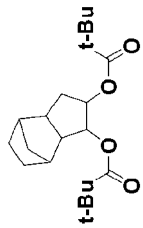
G-73

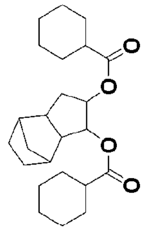
G-74

[Chem. 70]

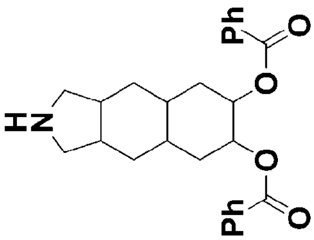
I-5

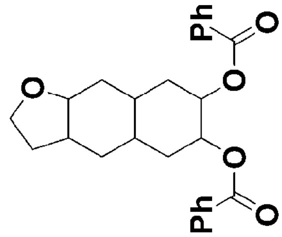
I-10

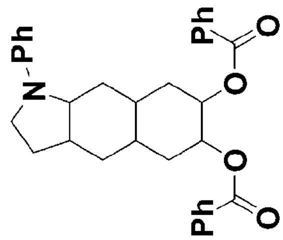
I-15

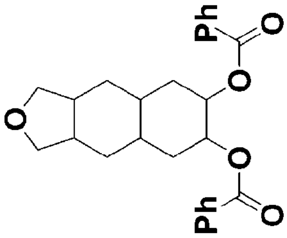
I-4

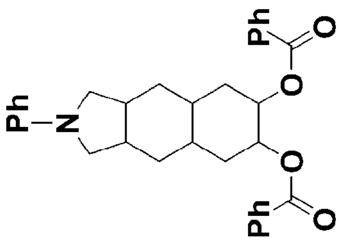
I-9

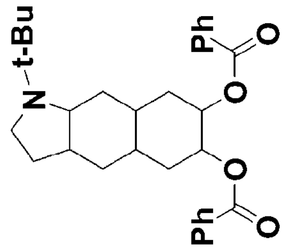
I-14

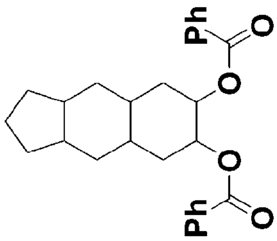
I-3

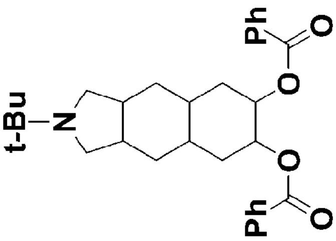
I-8

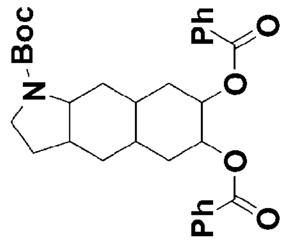
I-13

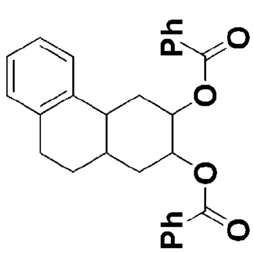
I-2

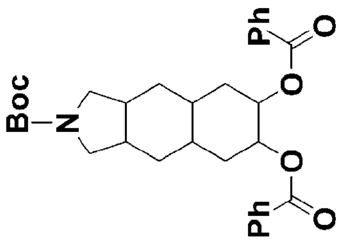
I-7

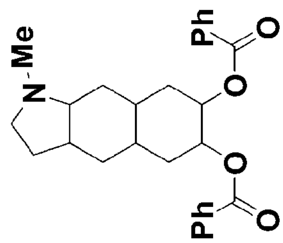
I-12

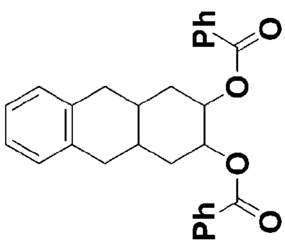
I-1

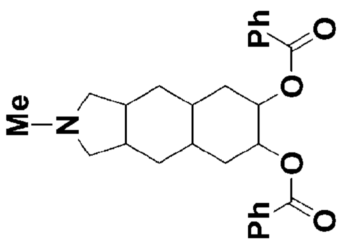
I-6

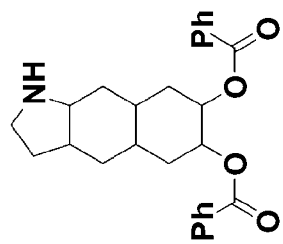
I-11

[Chem. 71]

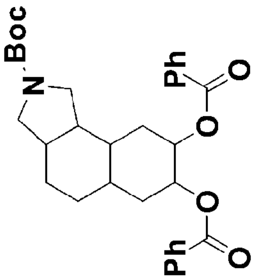

I-20

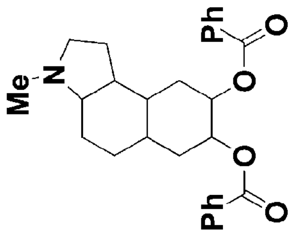

I-25

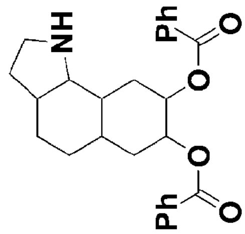

I-30

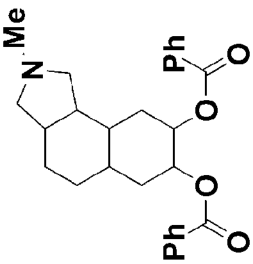

I-19

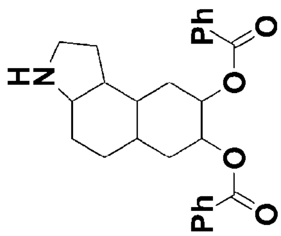

I-24

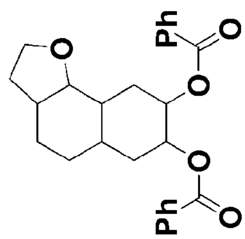

I-29

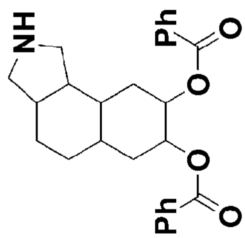

I-18

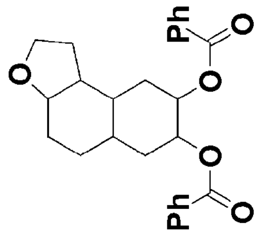

I-23

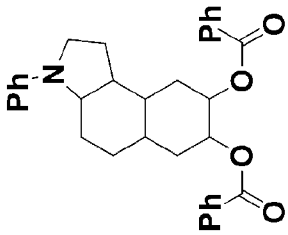

I-28

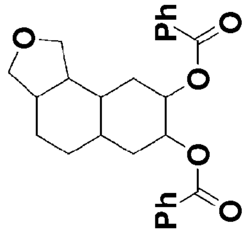

I-17

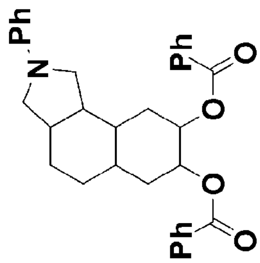

I-22

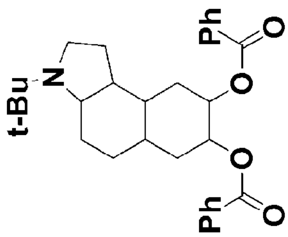

I-27

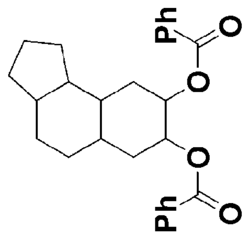

I-16

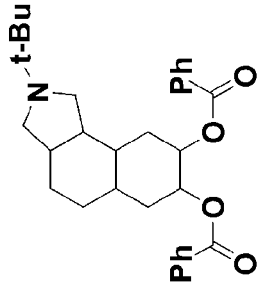

I-21

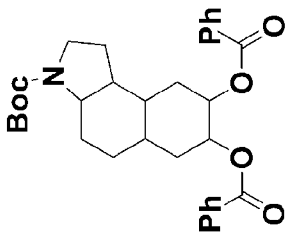

I-26

[Chem. 72]

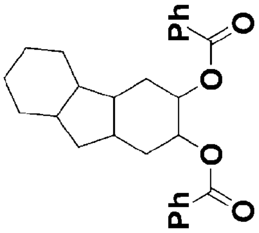
I-36

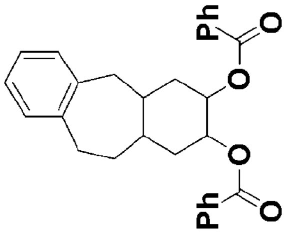
I-68

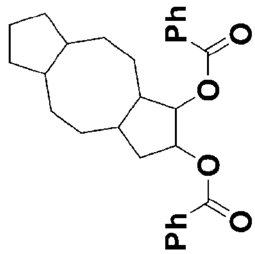
I-75

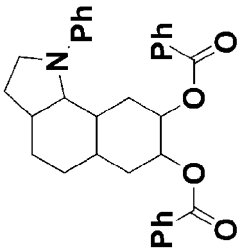
I-34

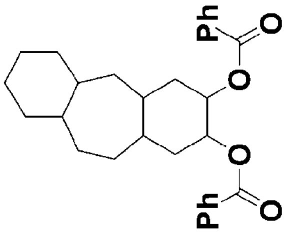
I-67

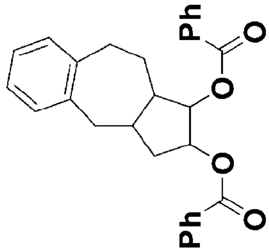
I-74

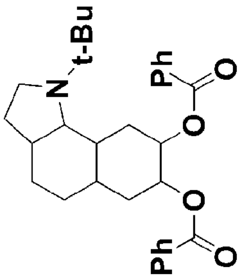
I-33

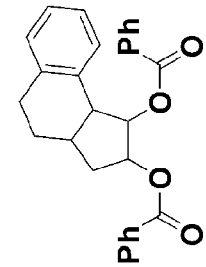
I-62

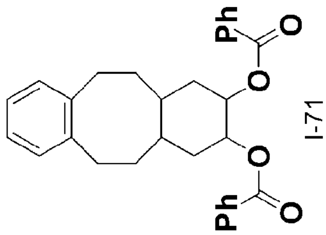
I-71

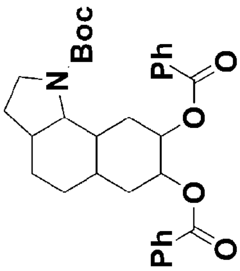
I-32

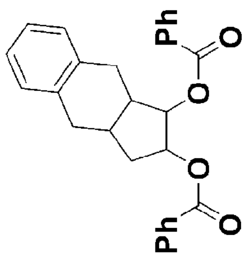
I-61

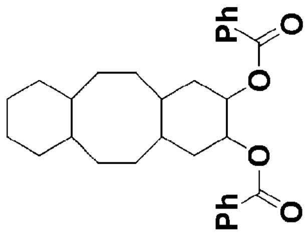
I-70

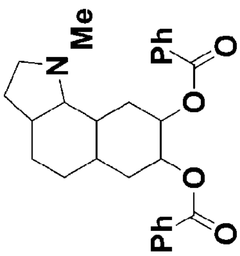
I-31

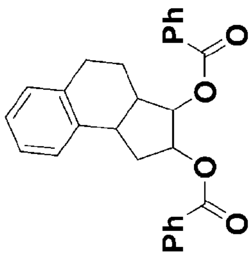
I-56

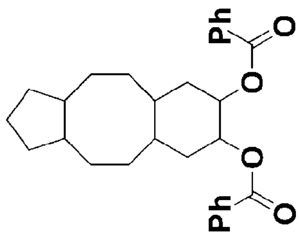
I-69

[Chem. 73]

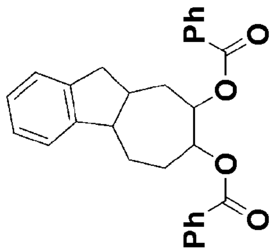
I-80

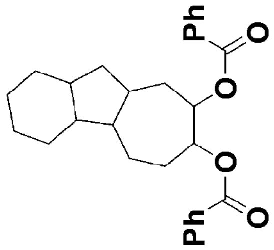
I-79

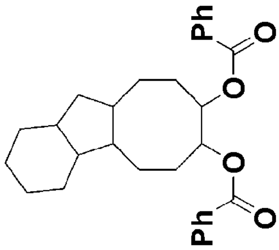
I-85

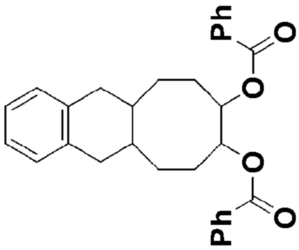
I-89

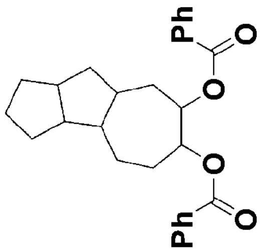
I-78

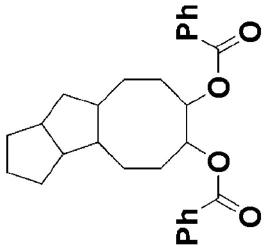
I-84

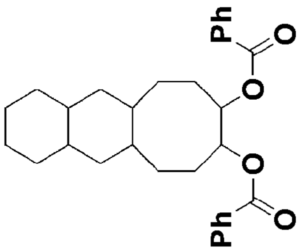
I-88

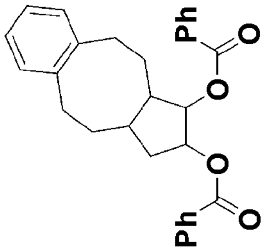
I-77

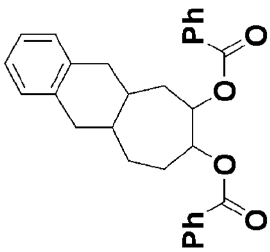
I-83

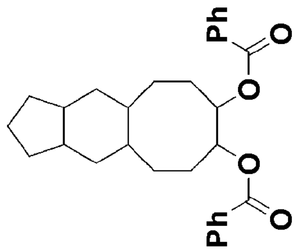
I-87

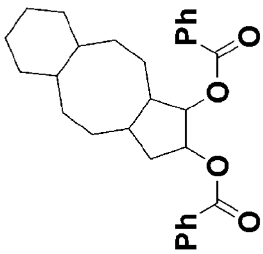
I-76

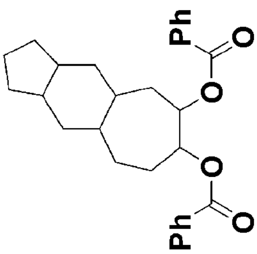
I-81

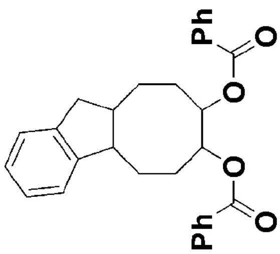
I-86

[Chem. 74]

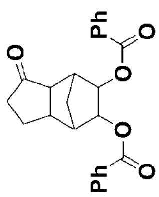
F-75

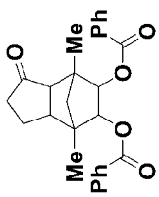
F-76

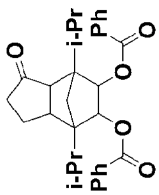
F-77

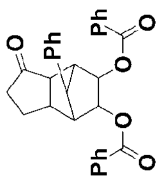
F-78

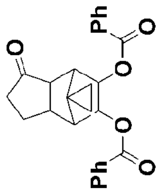
F-79

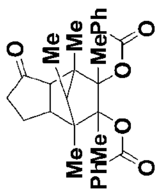
F-80

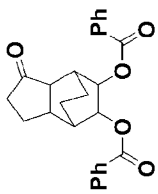
F-81

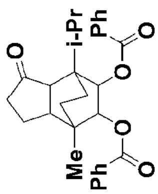
F-82

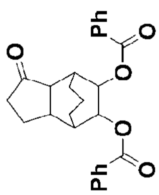
F-83

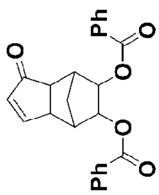
F-84

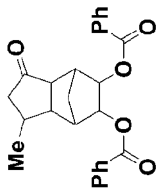
F-85

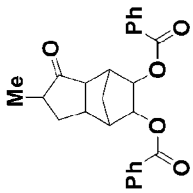
F-86

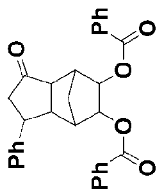
F-87

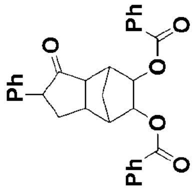
F-88

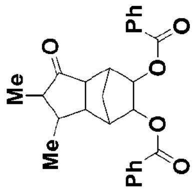
F-39

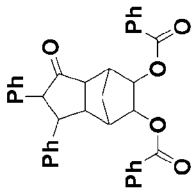
F-90

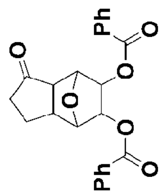
F-91

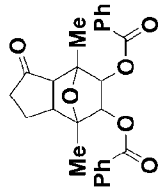
F-92

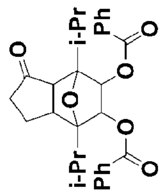
F-93

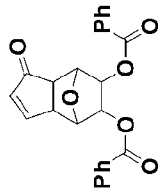
F-94

[Chem. 75]

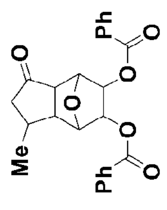
F-95

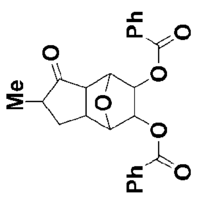
F-96

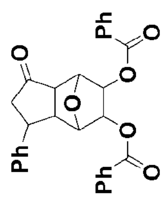
F-97

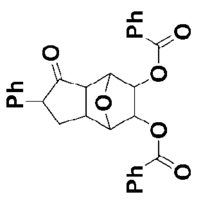
F-98

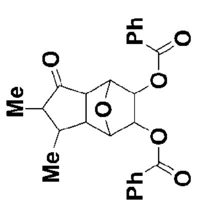
F-99

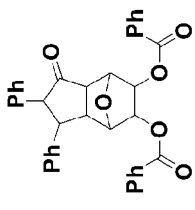
F-100

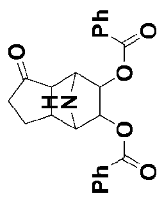
F-101

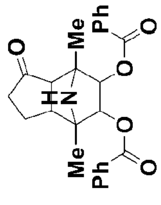
F-102

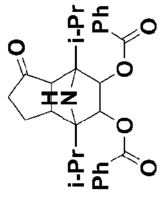
F-103

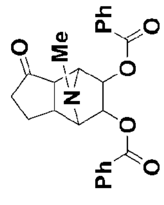
F-104

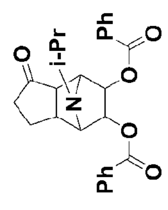
F-105

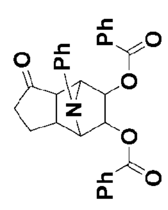
F-106

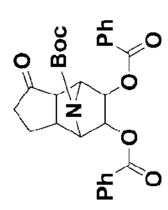
F-107

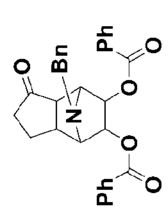
F-108

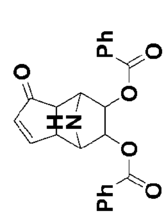
F-109

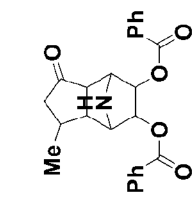
F-110

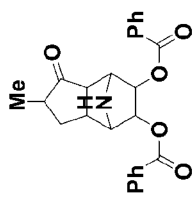
F-111

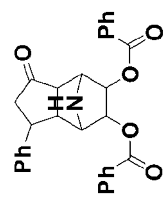
F-112

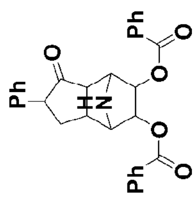
F-113

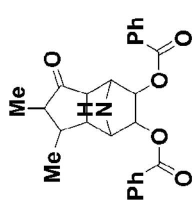
F-114

[Chem. 76]

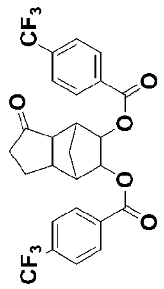
F-118

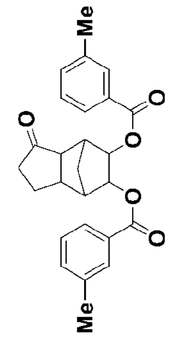
F-122

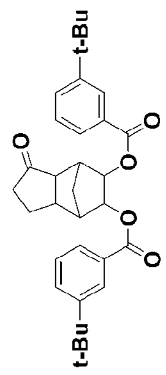
F-126

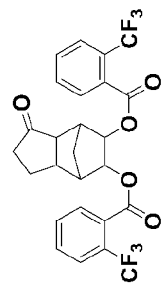
F-130

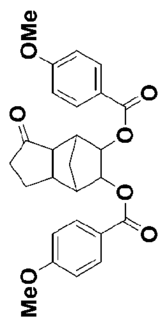
F-117

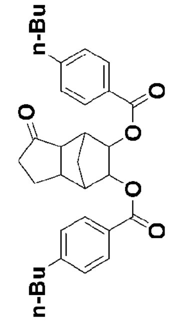
F-121

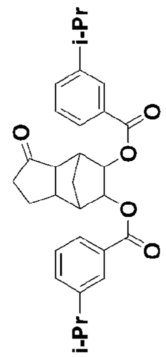
F-125

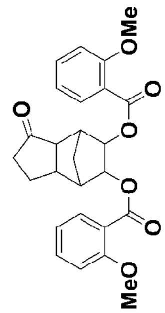
F-129

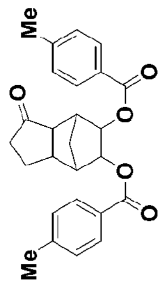
F-116

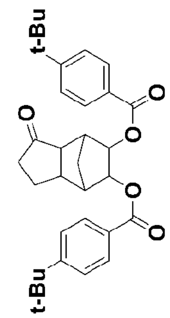
F-120

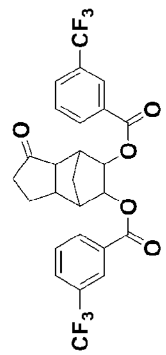
F-124

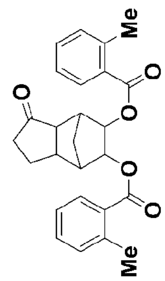
F-128

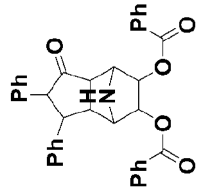
F-115

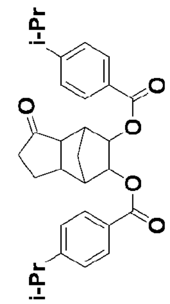
F-119

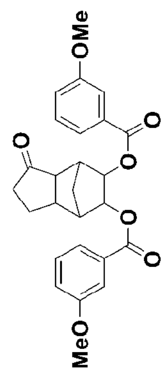
F-123

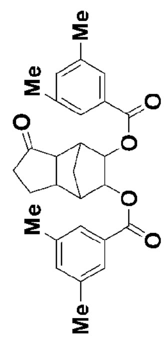
F-127

[Chem. 77]

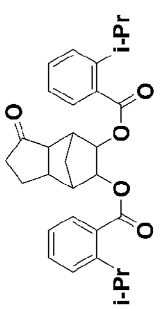
F-131

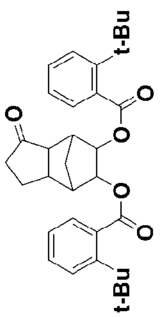
F-132

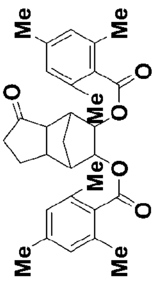
F-133

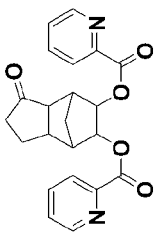
F-134

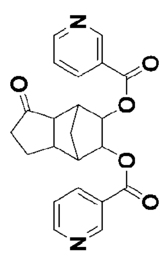
F-135

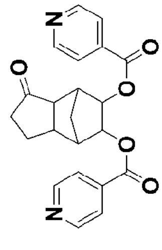
F-136

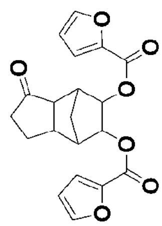
F-137

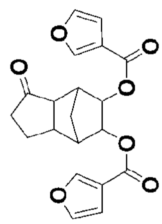
F-138

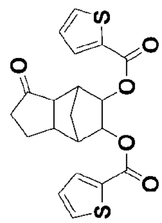
F-139

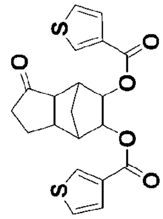
F-140

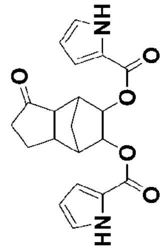
F-141

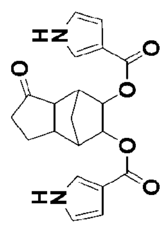
F-142

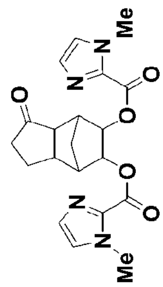
F-143

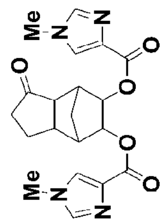
F-144

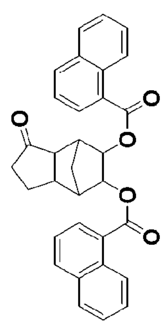
F-145

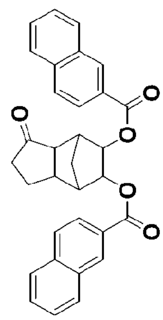
F-146

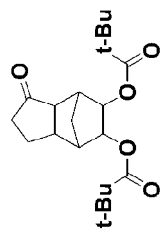
F-147

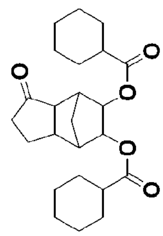
F-148

[Chem. 78]

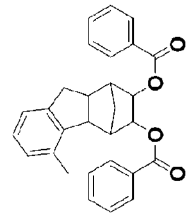
5

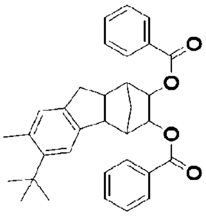
10

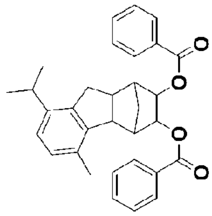
15

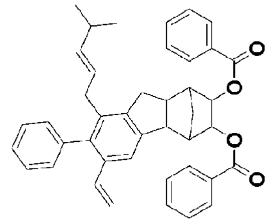
20

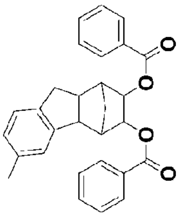
4

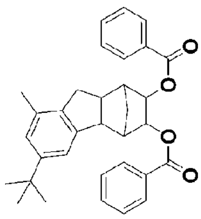
9

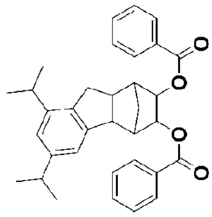
14

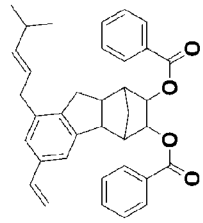
19

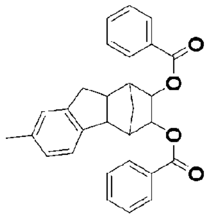
3

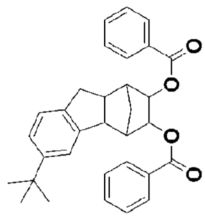
8

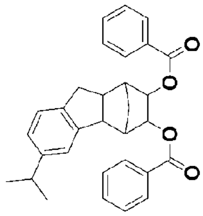
13

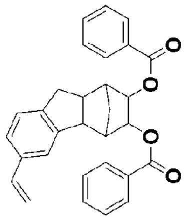
18

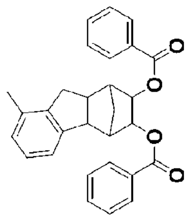
2

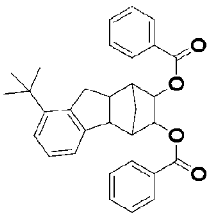
7

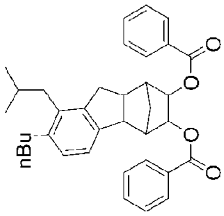
12

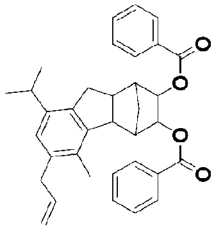
17

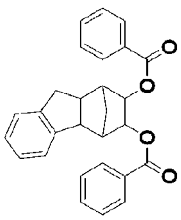
1

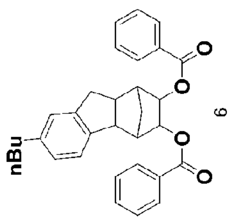
6

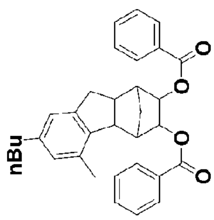
11

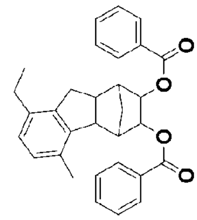
16

[Chem. 79]

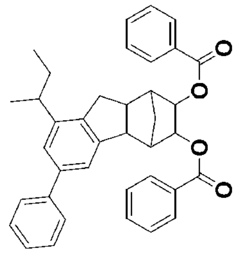

25

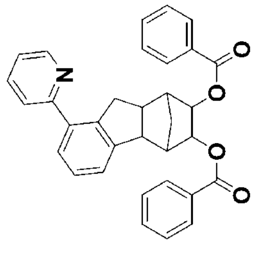

30

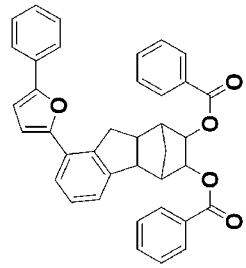

35

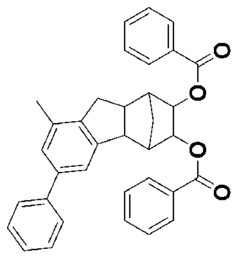

24

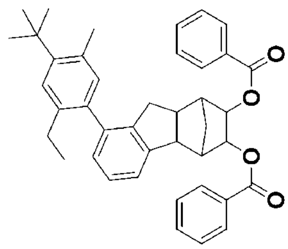

29

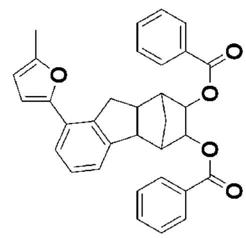

34

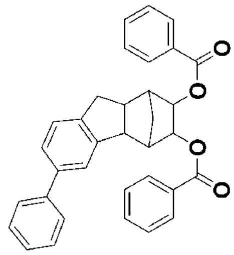

23

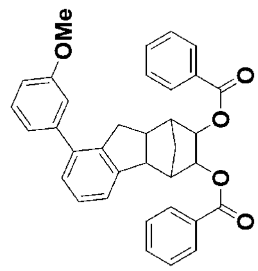

28

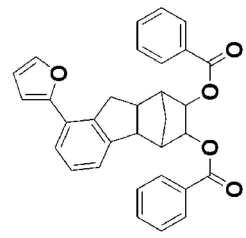

33

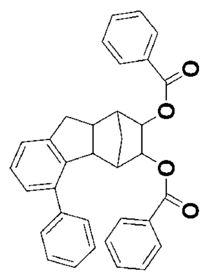

22

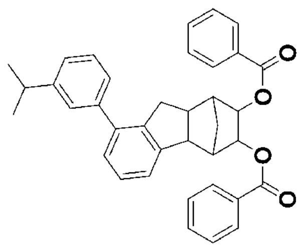

27

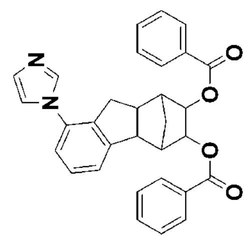

32

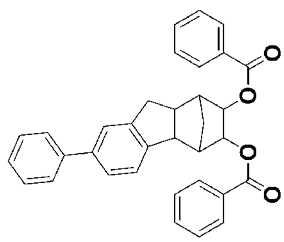

21

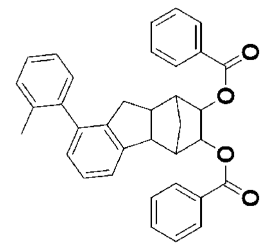

26

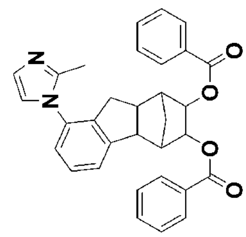

31

[Chem. 80]

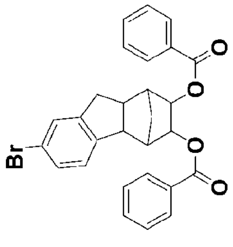
40

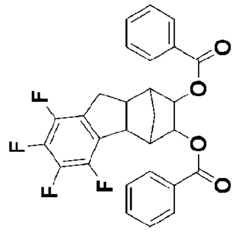
50

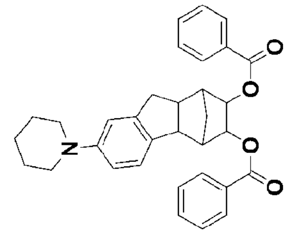
39

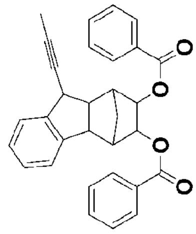
45

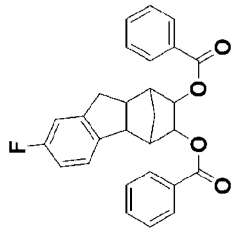
49

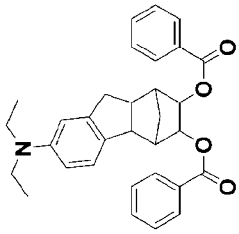
38

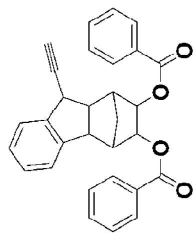
44

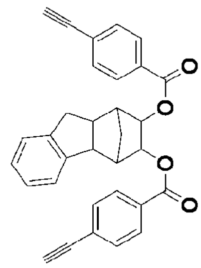
48

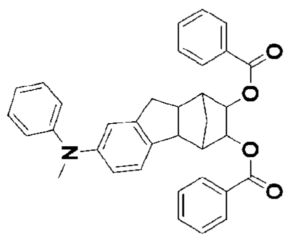
37

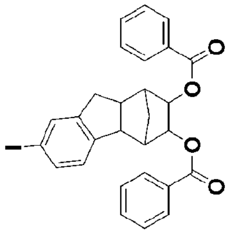
42

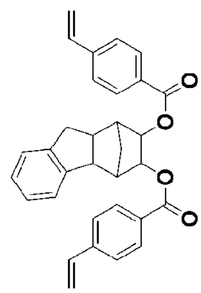
47

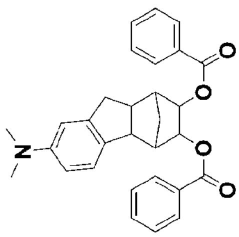
36

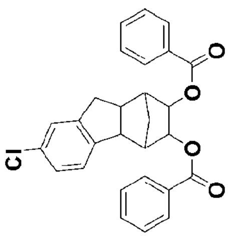
41

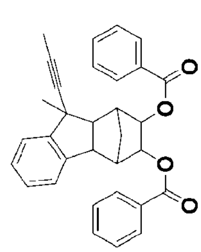
46

[Chem. 81]

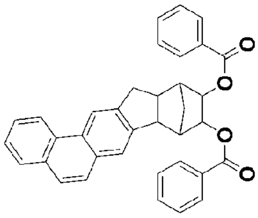
55

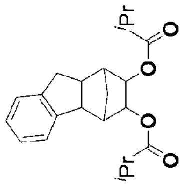
65

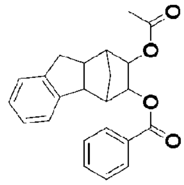
70

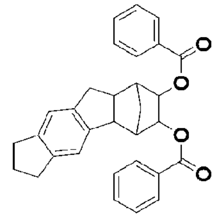
54

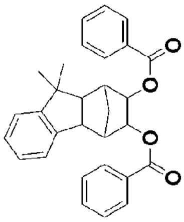
59

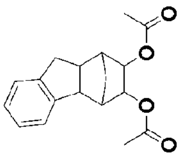
64

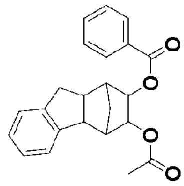
69

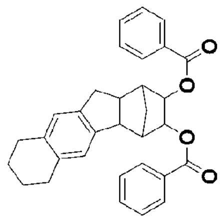
53

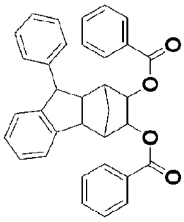
58

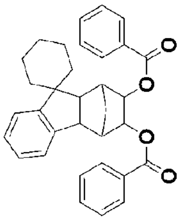
63

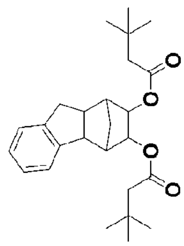
68

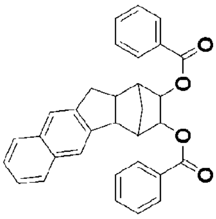
52

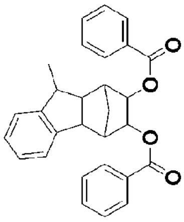
57

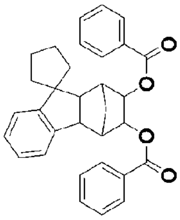
62

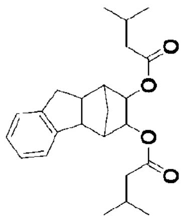
67

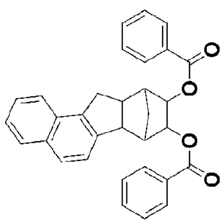
51

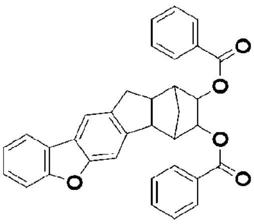
56

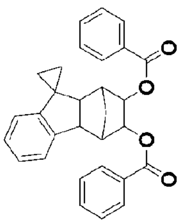
61

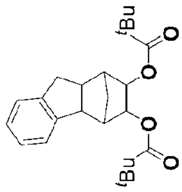
66

[Chem. 82]

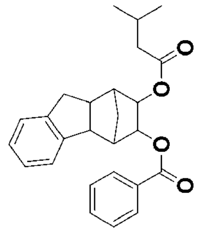
75

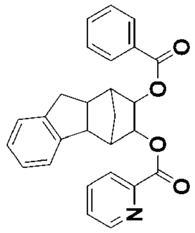
80

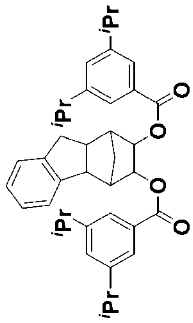
85

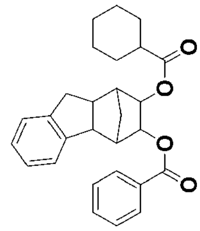
90

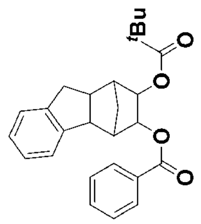
74

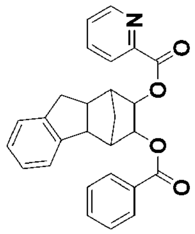
79

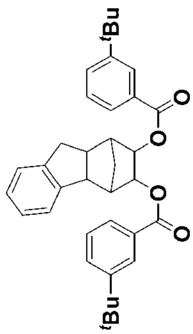
84

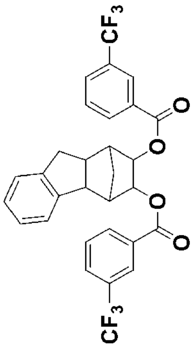
89

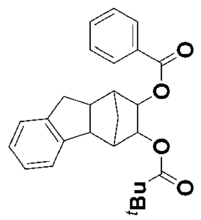
73

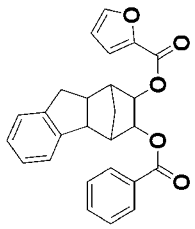
78

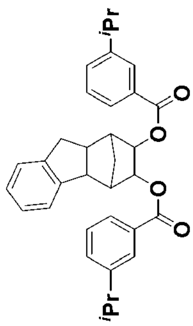
83

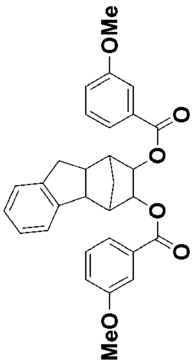
88

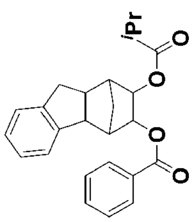
72

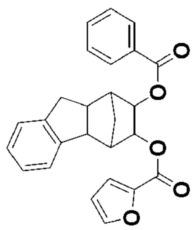
77

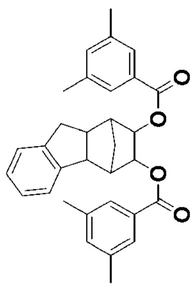
82

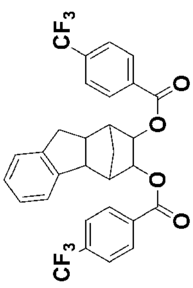
87

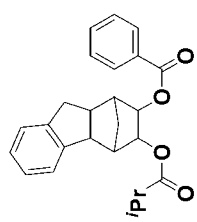
71

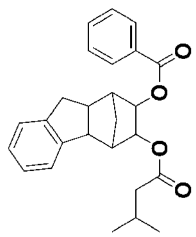
76

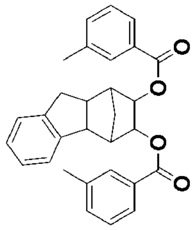
81

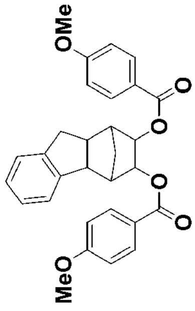
86

[Chem. 83]

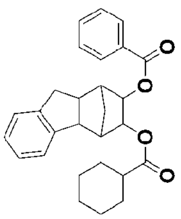
91

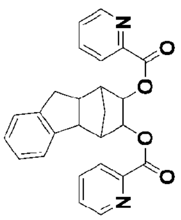
92

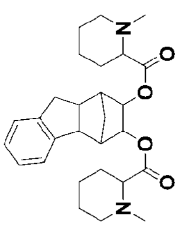
93

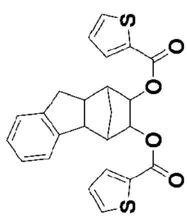
96

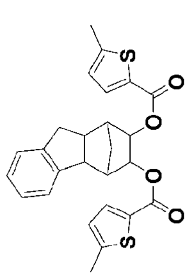
97

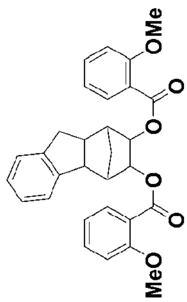
98

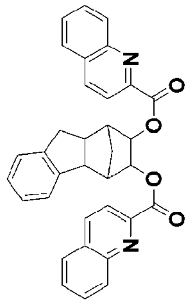
99

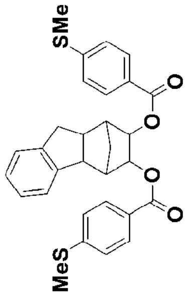
100

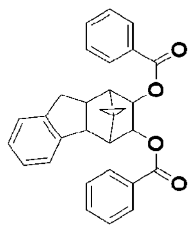
101

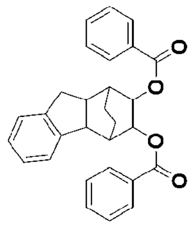
102

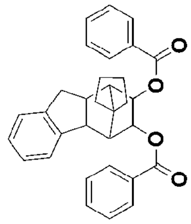
103

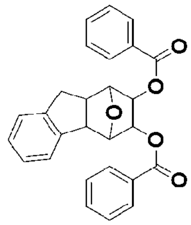
104

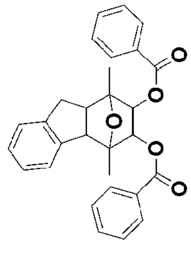
105

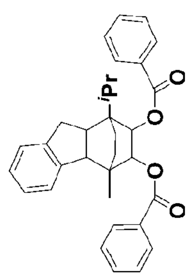
106

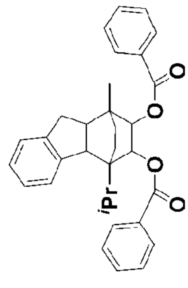
107

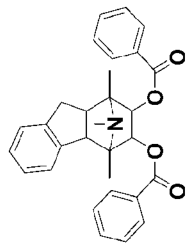
108

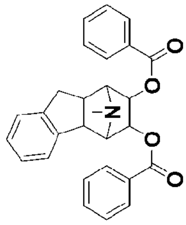
109

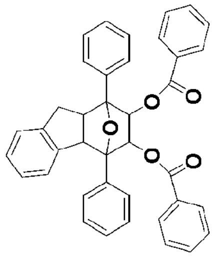
110

[Chem. 84]

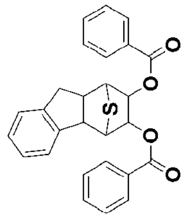
111

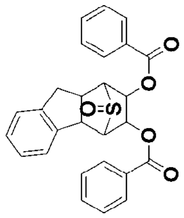
112

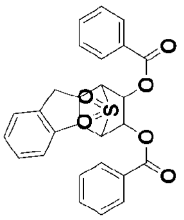
113

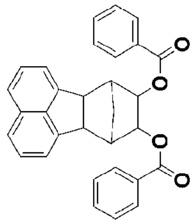
115

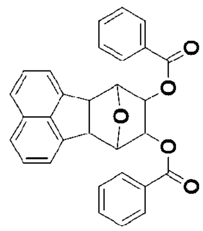
116

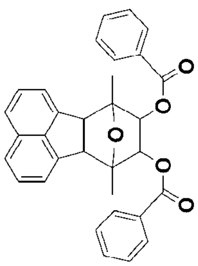
117

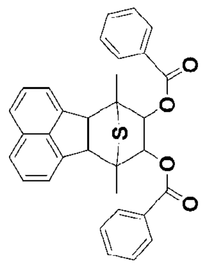
118

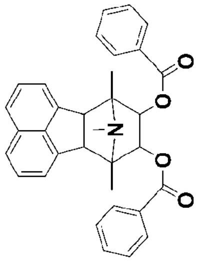
119

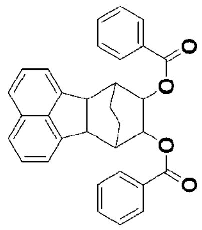
120

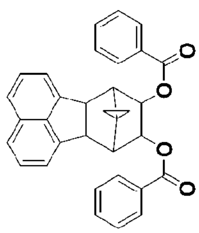
121

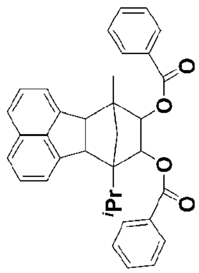
122

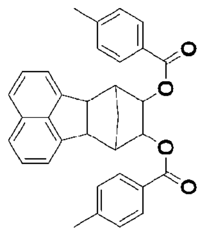
123

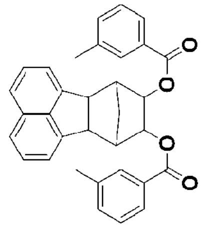
124

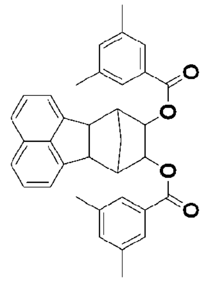
125

[Chem. 85]

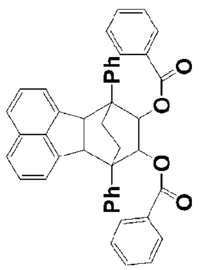

130

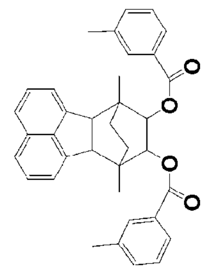

135

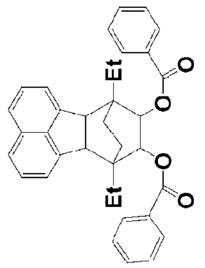

129

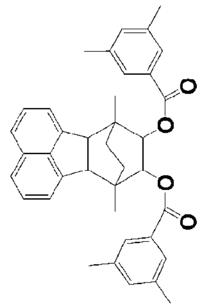

134

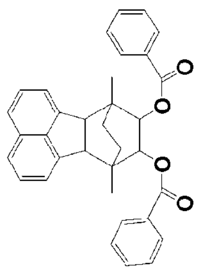

128

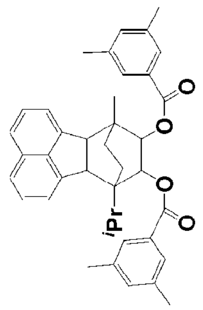

133

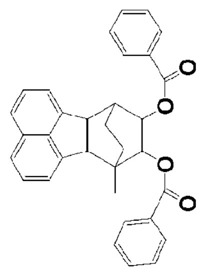

127

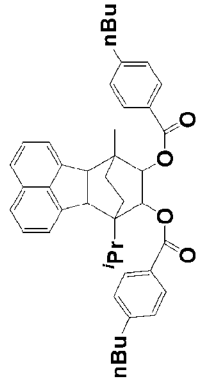

132

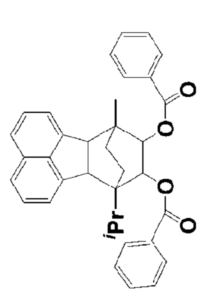

126

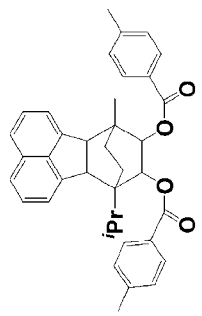

131

[Chem. 86]

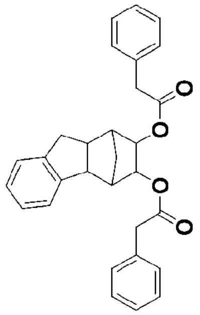

140

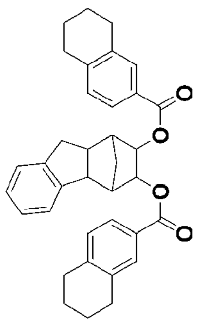

145

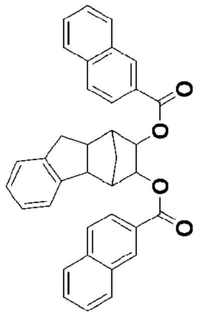

139

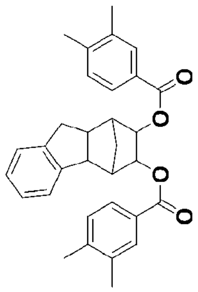

144

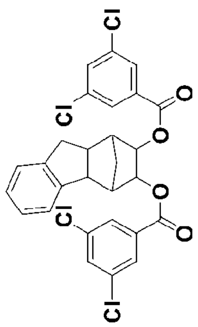

143

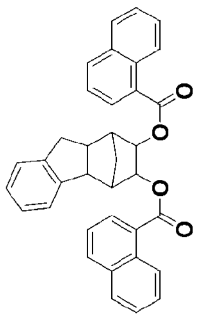

138

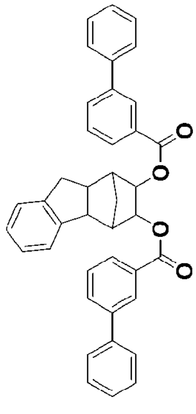

147

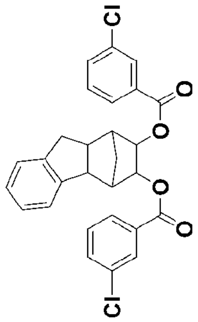

142

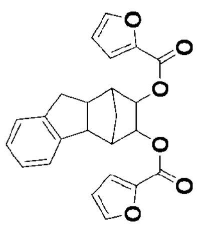

137

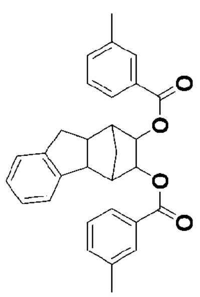

136

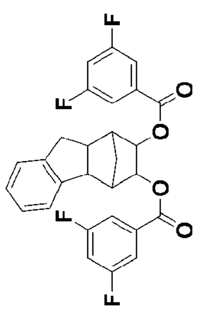

141

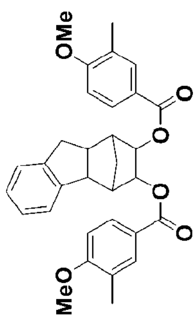

146

Examples of a compound of which the moiety forming a ring by bonding of $R^1$ to $R^{24}$ has a structure having a ring structure further including a double bond or a ring structure, particularly a double bond can include compounds of

the following structures.

[Chem. 87]

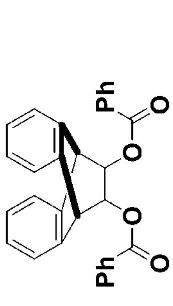
H-1

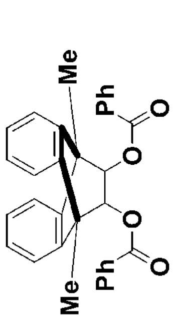
H-2

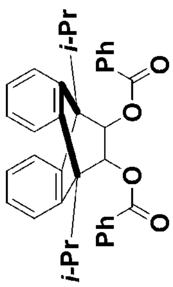
H-3

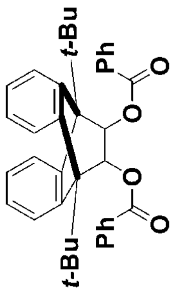
H-4

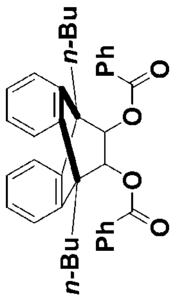
H-5

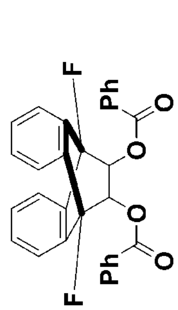
H-6

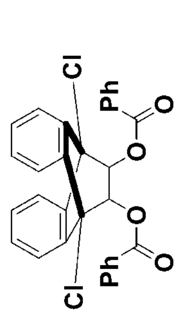
H-7

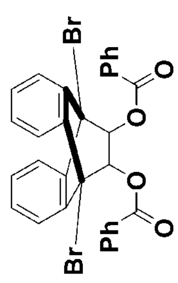
H-8

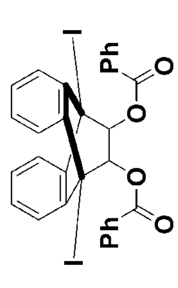
H-9

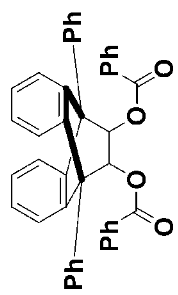
H-10

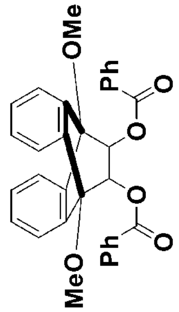
H-11

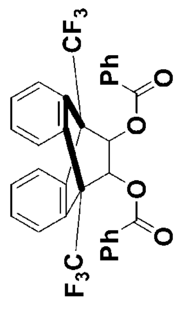
H-12

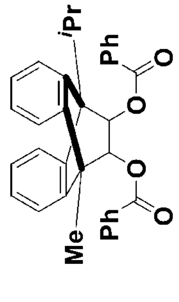
H-13

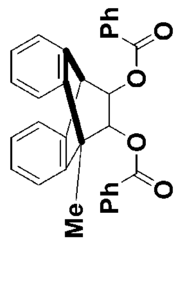
H-14

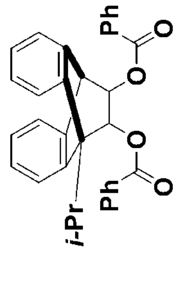
H-15

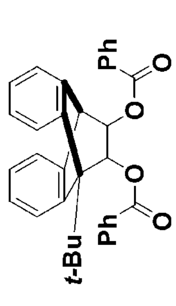
H-16

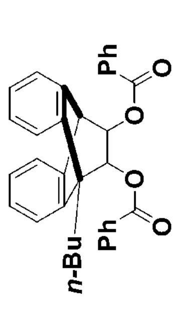
H-17

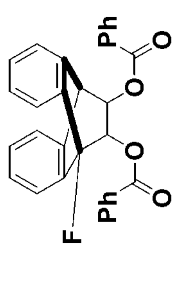
H-18

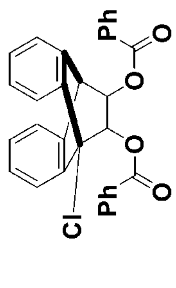
H-19

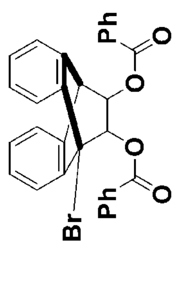
H-20

[Chem. 88]

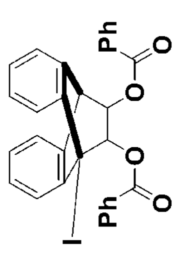
H-21

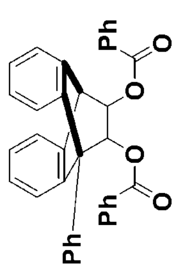
H-22

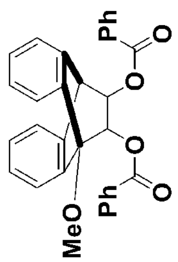
H-23

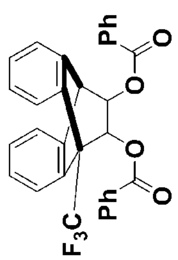
H-24

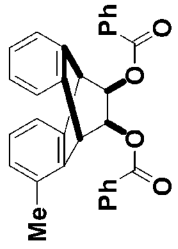
H-25

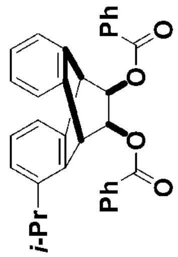
H-26

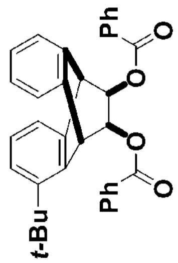
H-27

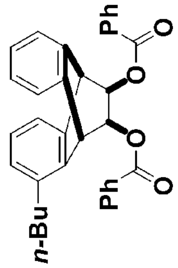
H-28

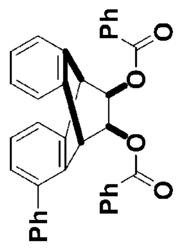
H-29

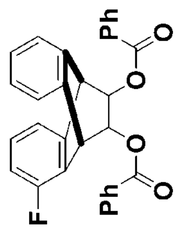
H-30

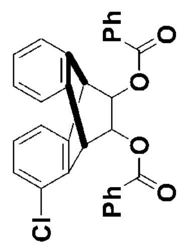
H-31

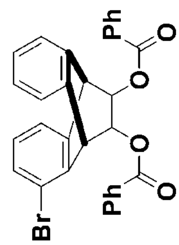
H-32

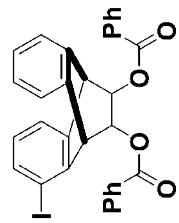
H-33

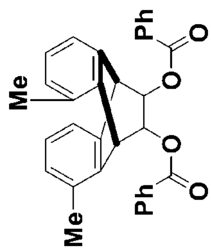
H-34

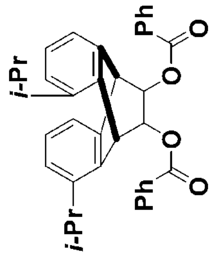
H-35

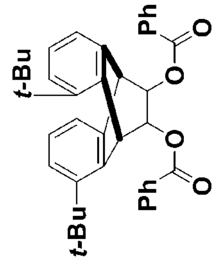
H-36

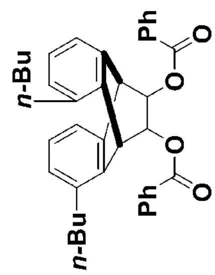
H-37

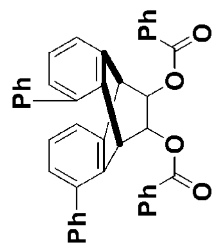
H-38

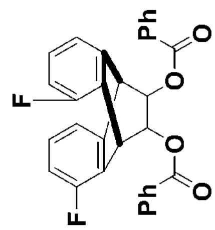
H-39

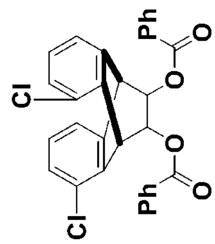
H-40

[Chem. 89]

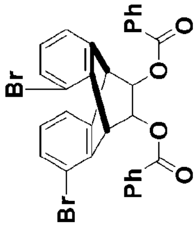
H-41

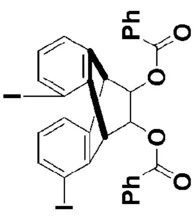
H-42

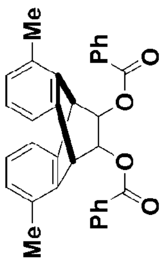
H-43

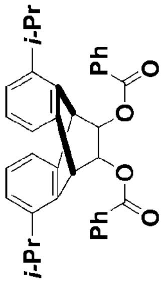
H-44

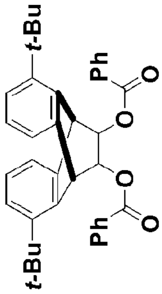
H-45

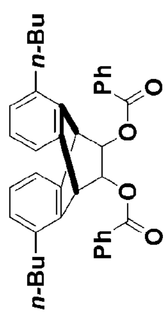
H-46

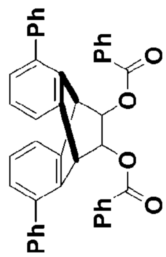
H-47

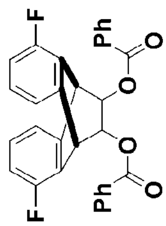
H-48

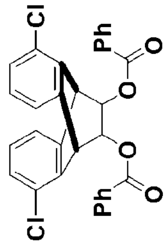
H-49

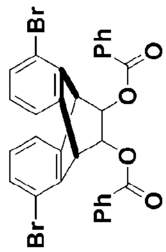
H-50

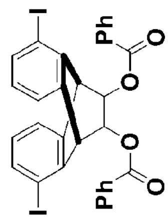
H-51

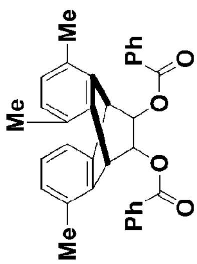
H-52

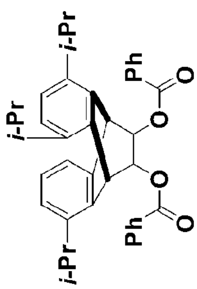
H-53

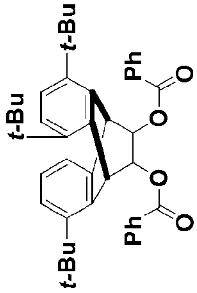
H-54

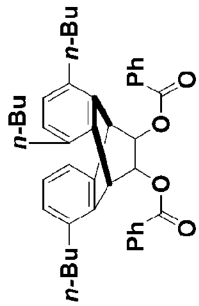
H-55

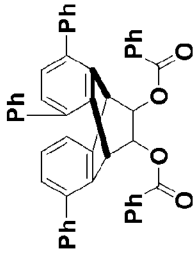
H-56

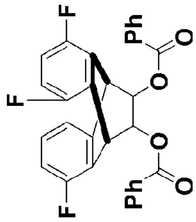
H-57

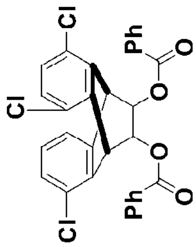
H-58

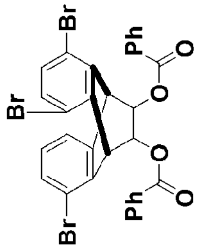
H-59

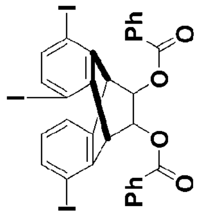
H-60

[Chem. 90]

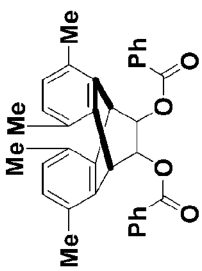
H-61

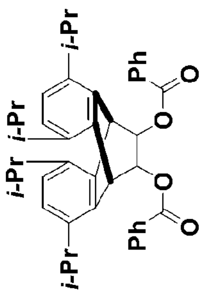
H-62

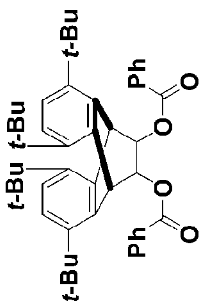
H-63

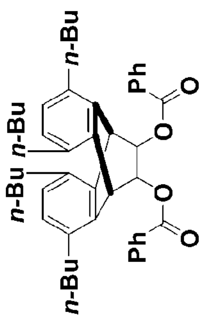
H-64

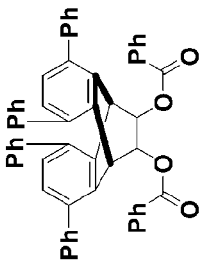
H-65

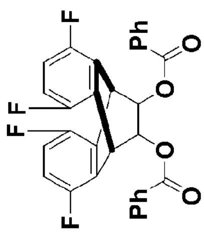
H-66

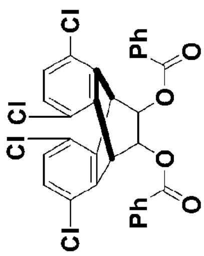
H-67

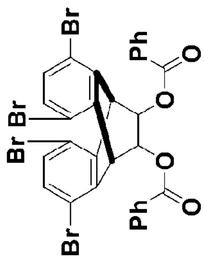
H-68

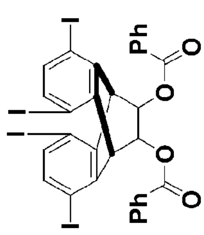
H-69

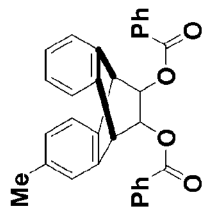
H-70

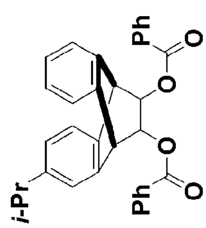
H-71

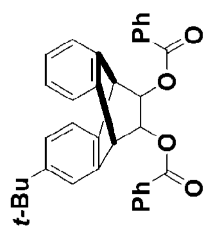
H-72

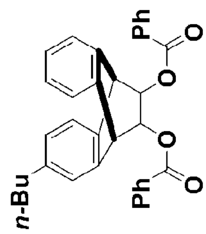
H-73

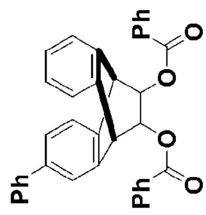
H-74

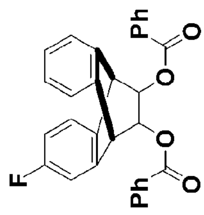
H-75

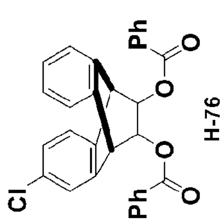
H-76

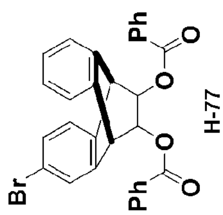
H-77

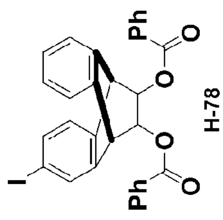
H-78

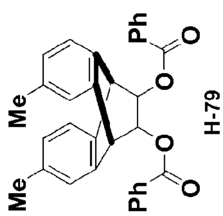
H-79

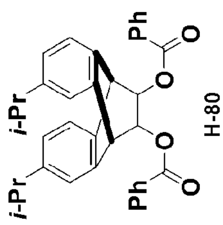
H-80

[Chem. 91]

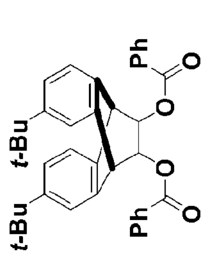
H-81

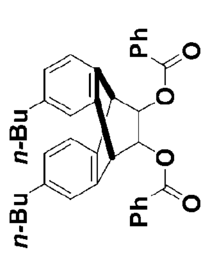
H-82

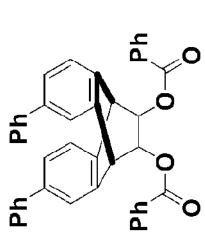
H-83

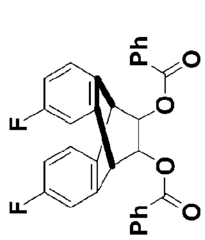
H-84

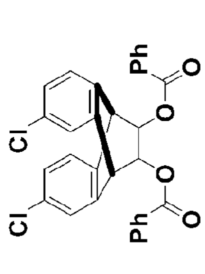
H-85

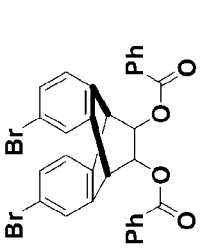
H-86

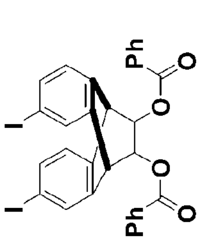
H-87

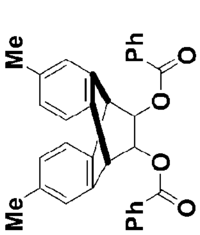
H-88

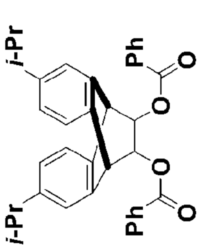
H-89

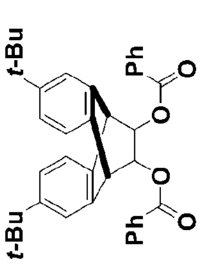
H-90

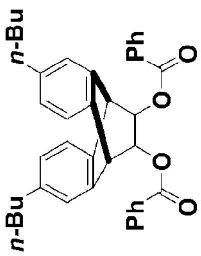
H-91

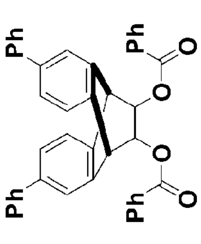
H-92

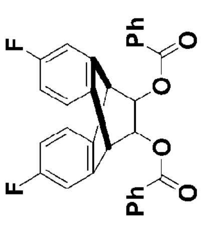
H-93

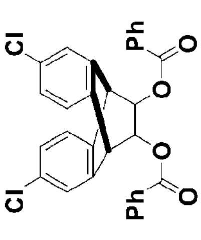
H-94

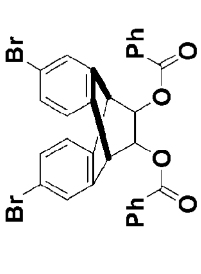
H-95

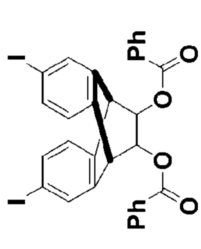
H-96

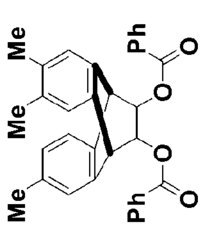
H-97

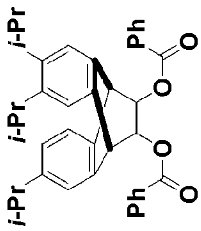
H-98

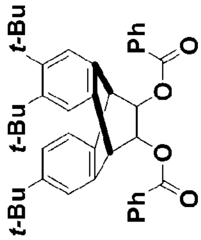
H-99

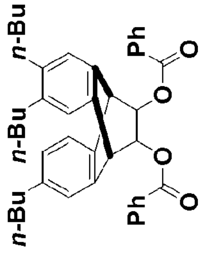
H-100

[Chem. 92]

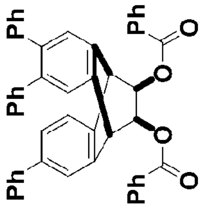
H-101

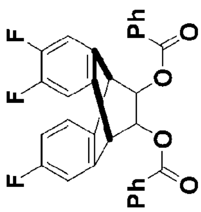
H-102

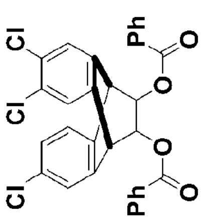
H-103

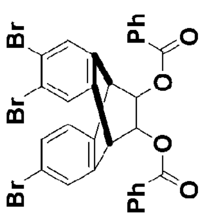
H-104

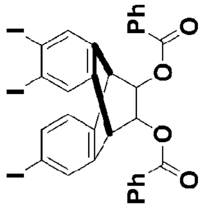
H-105

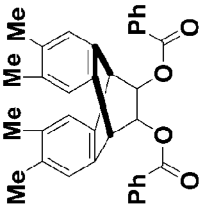
H-106

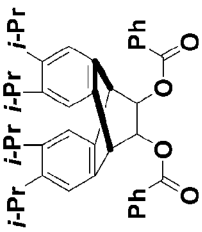
H-107

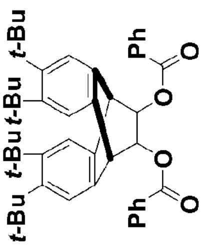
H-108

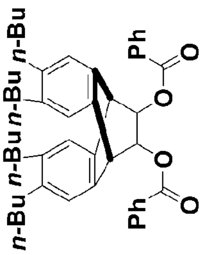
H-109

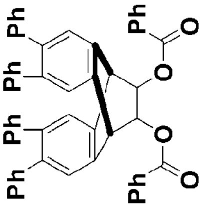
H-110

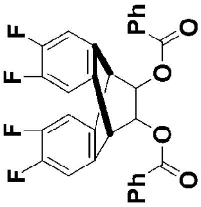
H-111

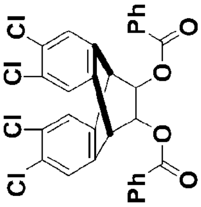
H-112

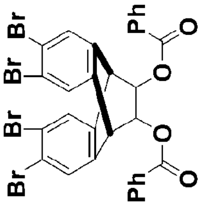
H-113

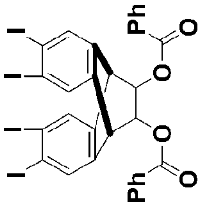
H-114

[Chem. 93]

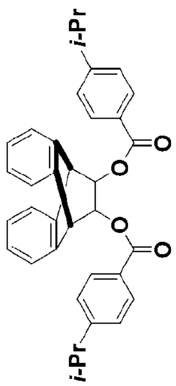
H-118

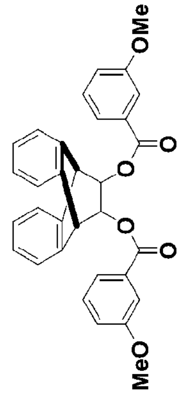
H-122

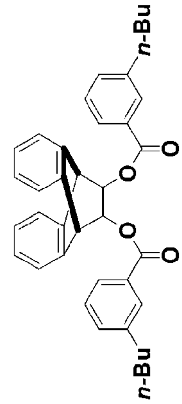
H-126

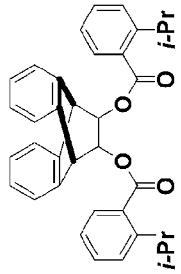
H-130

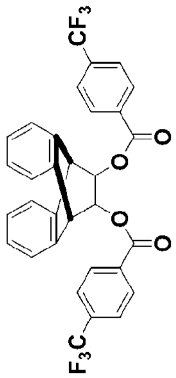
H-117

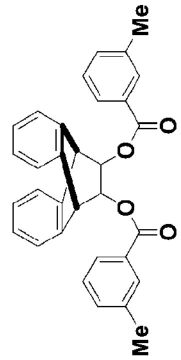
H-121

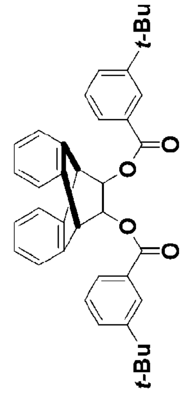
H-125

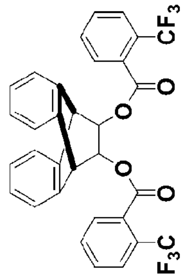
H-129

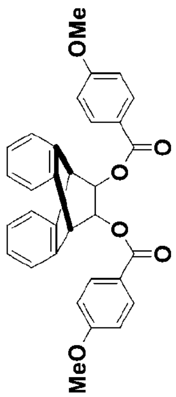
H-116

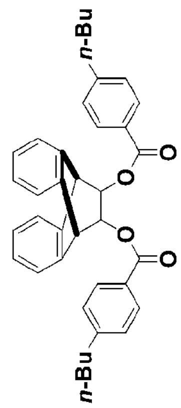
H-120

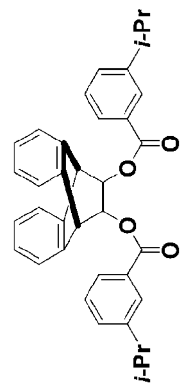
H-124

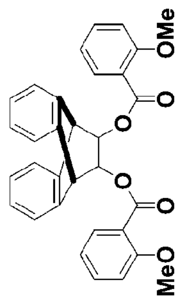
H-128

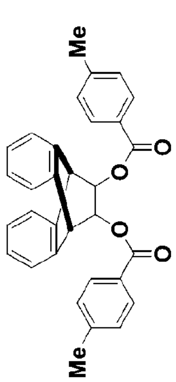
H-115

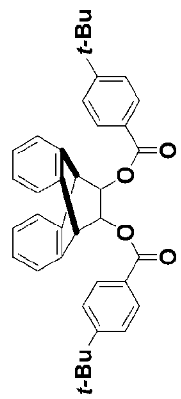
H-119

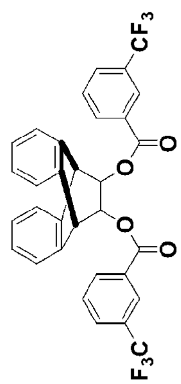
H-123

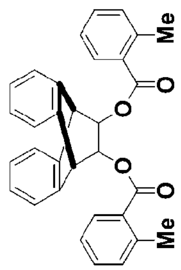
H-127

[Chem. 94]

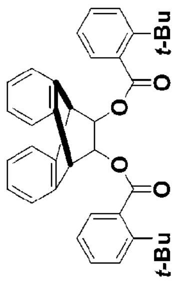
H-131

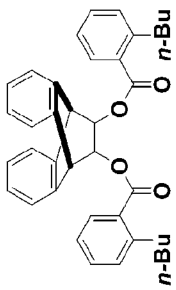
H-132

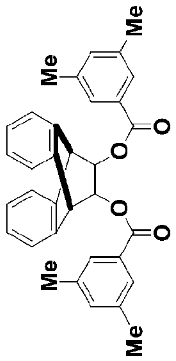
H-133

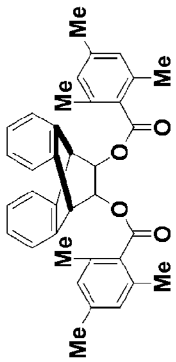
H-134

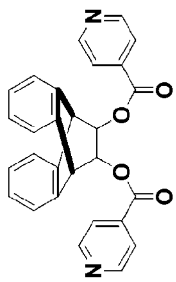
H-135

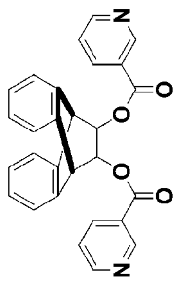
H-136

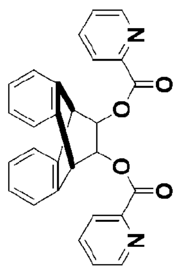
H-137

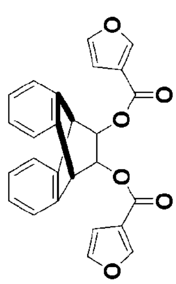
H-138

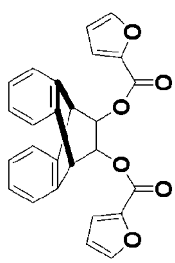
H-139

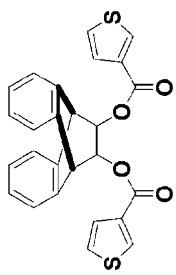
H-140

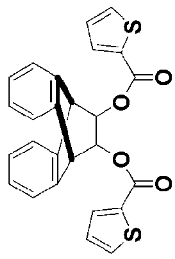
H-141

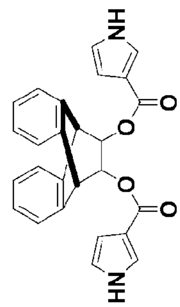
H-142

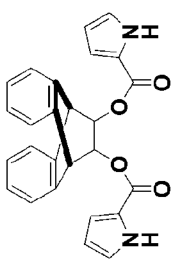
H-143

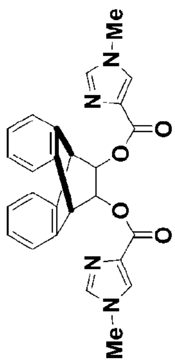
H-144

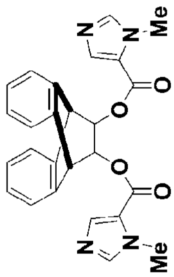
H-145

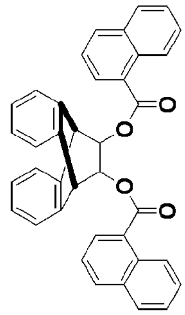
H-146

[Chem. 95]

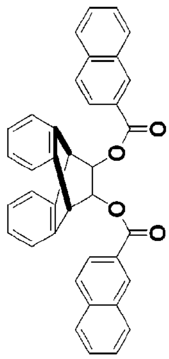
H-147

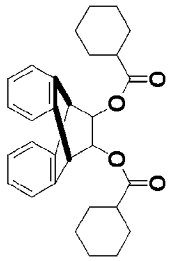
H-148

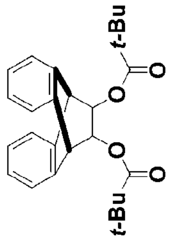
H-149

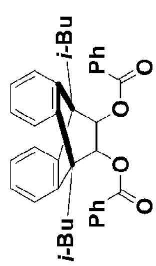
H-150

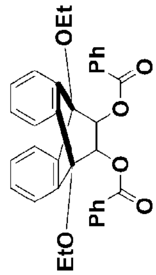
H-151

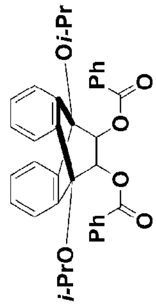
H-152

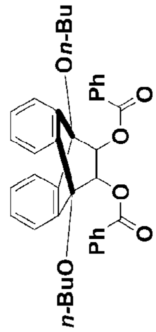
H-153

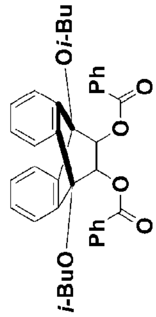
H-154

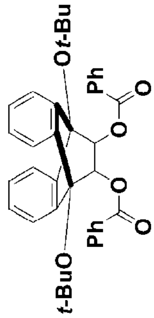
H-155

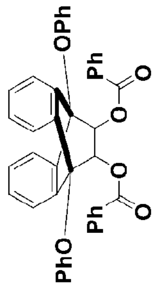
H-156

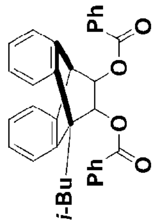
H-157

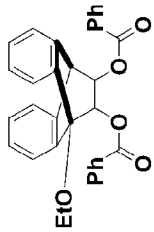
H-158

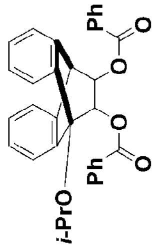
H-159

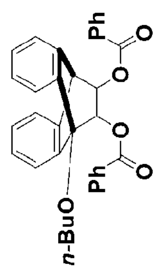
H-160

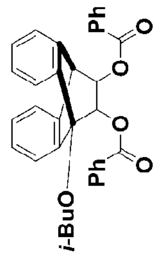
H-161

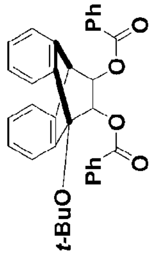
H-162

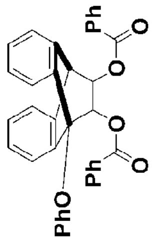
H-163

[Chem. 96]

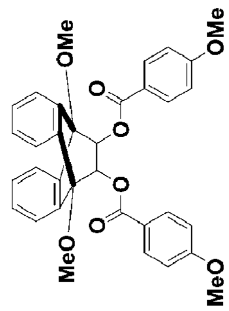

H-168

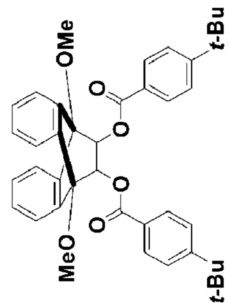

H-167

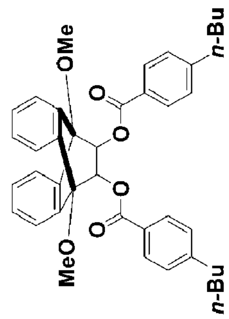

H-166

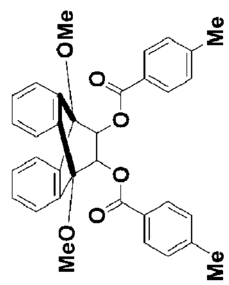

H-165

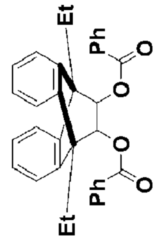

H-164

In the structural formulas described above, a methyl group is denoted as "Me", an ethyl group is denoted as "Et", a propyl group is denoted as "Pr", a butyl group is denoted as "Bu", and a phenyl group is denoted as "Ph", and [n] represents "normal", [i] represents "iso", and "t" represents "tertially".

In the ester compound (A) of the present invention, the $OCOL^1$ group and $OCOL^2$ group bonded to the alicyclic structure may form a cis structure or a trans structure derived from the alicyclic structure, and the ester compound of the cis structure is preferably the main component. The main component here refers to the content of the compound of the cis structure exceeding 50 mol% and preferably the content being 70 mol% or more.

Although the reason why the ester compound (A) of the present invention is suitable as a Lewis base (internal donor) component of the solid titanium catalyst component is currently unknown, the present inventors presume the reason as follows.

The ester compound (A) of the present invention has a structure in which rings are connected and preferably also has a crosslinked structure (structure corresponding to the X or Y described above). This requirement restricts the conformation of the compound, and that fixes the distance between the two adjacent ester groups bonded to the rings and the orientation of the ester groups. Consequently, when the ester is coordinated to magnesium chloride, the coordination mode for the ester is limited, and a rigid polymerization environment including titanium atoms, magnesium chloride, and the ester compound is presumably formed. When the polymerization proceeds under the environment, it is conceived that the orientation of the polymer chain and the insert direction of propylene are highly regulated to thereby form a catalyst suitable for high stereoregular polymerization. A skeleton in which the ester is bonded to a ring including a crosslinked structure (e.g., the formulas (5), (6), and (9)) is subjected to a restriction on the distance and the orientation of the ester moieties, and thus is a more excellent skeleton as the internal donor for a Ziegler catalyst. Even with a skeleton in which the ester moieties are directly bonded to the cyclohexane skeleton (e.g., the formula (8)), it is conceived that fusing a ring structure including a crosslinked structure to the cyclohexane skeleton causes the conformation of the cyclohexane skeleton to be restricted and a similar effect is consequently achieved. As mentioned above, the structure having a 6-membered ring structure has been described here. On the basis of this presumption, a similar effect can be expected from an ester compound having the structure represented by the formula (1), more preferably the polycyclic structure represented by the formulas (2) to (4), even if the compound is other than the compounds specifically exemplified. It is obvious that the similar effect is developed even with a 5-membered ring to 10-membered ring structure.

The ester compound (A) of the present invention, which has a structure in which rings are connected as described above, is presumed to have moderate rigidity as a compound and a relatively smaller displacement of the structure. For this reason, in the case of use in a catalyst for olefin polymerization mentioned below, it is expected that a stable structure is kept while a moderate interaction is provided when the ester group-containing compound (A) is coordinated to a titanium compound or magnesium compound. Therefore, it is conceived that the stereospecificity and activity are positively influenced by the interaction. Meanwhile, an alicyclic structure as described above will exhibit various structures liable to be displaced in respect of a local structure, such as a chair type or a boat type. Accordingly, a polymer having a broad molecular weight distribution can be expected to be produced.

The ester compound (A) of the present invention represented by the formula (31) has a particular multicyclic structure as described above. The compound also has a specific asymmetric structure. Having such a structure allows the compound to have moderate rigidity. Thus, when active species for olefin polymerization are formed by an interaction with a magnesium compound or titanium compound and polymerization reaction occurs, there is conceived to be a possibility of fewer displacements or fluctuations as for the structures. Meanwhile, in the case of an ester compound (A) having an asymmetric structure as that of the formula (31), there may be more conformations in the case of an interaction with a magnesium compound or titanium compound than those of a symmetric compound. Thus, there is a possibility of formation of active species having various micro structures. It is conceived that this respect can be advantageous for achieving aspects including a polymer having a broad molecular weight distribution or a polymer having high stereoregularity. As the compound preferably has a specific asymmetric structure including an aryl structure, the compound may have enabled a robust coordination structure to be achieved with the magnesium compound or titanium compound.

From the viewpoint as above, the ester compound (A) of the present invention is presumably suitable as a Lewis base (internal donor) component of the solid titanium catalyst component.

<Method for producing ester compound (A)>

The method for producing the ester compound (A) of the present invention is not particularly limited. For example, the ester compound (A) can be obtained by the reaction of a dihydroxylation of a corresponding olefin, followed by an esterification of the diol obtained by the dihydroxylation. In addition to that, the ester compound (A) can be also obtained by the successive reaction of introducing a carbonate group to a particular polycyclic compound such as anthracene, a hydrolysis of the carbonate group, and then an esterification of the diol obtained by the hydrolysis. More specifically, the ester compound (A) can be produced as follows.

<<Synthesis of olefin>>

The olefin shown in the following formula (21) can be synthesized by a Diels-Alder reaction of cyclopentadiene

and norbornene, for example (Non-Patent Literature 1). The olefin shown in the following general formula (33) can be synthesized by a Diels-Alder reaction of a substituted indene and a substituted diene (Patent Literature 11). For the diene, a dimer of a diene (e.g., dicyclopentadiene), which is a precursor, can be also used as the raw material. The product obtained by the Diels-Alder reaction is often a mixture of an endo form and an exo form (see the following formulas (22) and (34)), but either of the isomers can be applied to the present invention. That is, any of the mixture, only the endo form, or only the exo form may be applied. These endo form structure and exo form structure are often reflected also on the structure of the ester compound as a final product.

Allowing a benzyne to react with a diene enables an olefin to be obtained (Non-Patent Literatures 2, 3, and 14). A cyclic olefin can be obtained by decarbonylating and decarboxylating an alicyclic dicarboxylic anhydride in the presence of a nickel complex (e.g., tetrakis triphenylphosphine nickel) with a reagent that can function as a ligand (e.g., triphenylphosphine) to the nickel complex (Patent Literatures 7 and 8).

<<Synthesis of anthracene>>

When a compound of which the moiety forming a ring by bonding of the $R^1$ to $R^{24}$ has a cyclic structure having a ring structure including a double bond or a ring structure, especially the one that includes a double bond, the compound can be synthesized by subjecting anthracene to a Diels-Alder reaction with vinylene carbonate to give a carbonate compound, and then subjecting the carbonate compound to a hydrolysis, followed by an esterification of the diol.

Anthracene can be synthesized, for example, by reacting anthraquinone with an organometallic reagent (e.g., an alkyl lithium or Grignard reagent) and then conducting reduction (e.g., a reaction using tin(II) chloride or sodium hypophosphite) (the following formula (14(1)), see Patent Literature 9 and Non-Patent Literatures 15 and 16).

Anthracene can be synthesized also by subjecting a dihalogenoanthracene to a displacement reaction by a metal reagent (e.g., lithium or magnesium) and a halogenated alkyl and to a coupling reaction using a transition metal catalyst (e.g., nickel or palladium) and an organometallic reagent (e.g., a boronic ester or Grignard reagent) (the formulas (14(2)) and (14(3)), see Patent Literature 10 and Non-Patent Literature 17). Additionally, a dialkoxy anthracene can be obtained by reacting anthraquinone with a reducing agent (e.g., zinc) and an electrophile (e.g., a halogenated alkyl or sulfonic ester) (the formula (14(4)), see Non-Patent Literature 18).

[Chem. 97]

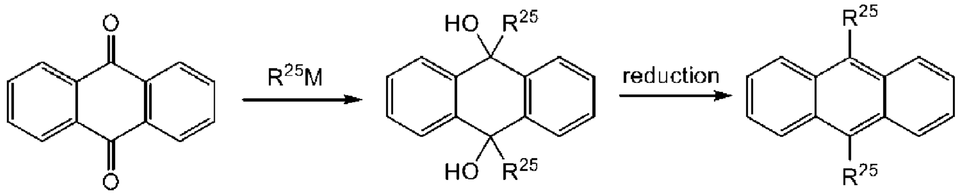


(14 (1))

In the formula (14(1)), $R^{25}M$ represents an organometallic reagent, $R^{25}$ represents an alkyl group, and M represents a metal or metal halide. An example of M includes Li, MgBr, MgCl, and MgI.

[Chem. 98]

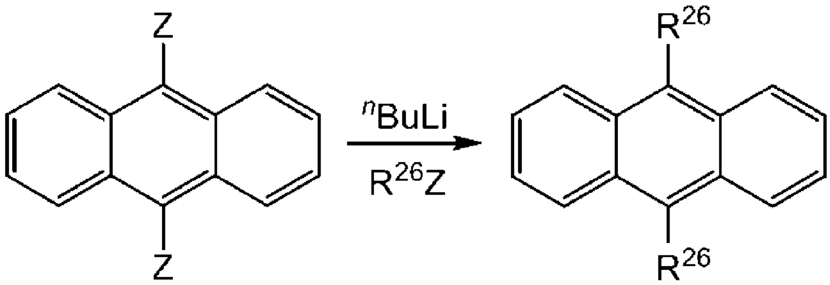


(14 (2))

In the formula (14(2)), $R^{26}Z$ represents an electrophile, Z represents a halogen atom, and $R^{26}$ represents an alkyl group.

[Chem. 99]

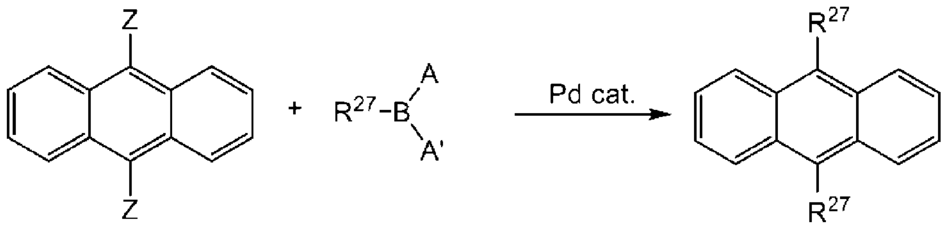


(1 4 (3))

In the formula (14(3)), Z represents a halogen atom, $R^{27}$ represents an alkyl group or aryl group, and A and A' represents a hydroxyl group or a crosslinked structure in which A and A' are bonded. An example of the structural formula of a boric acid compound and a boric ester (preferably a cyclic boric ester compound), which are boron-containing compounds including the structures of the above $R^{27}$, A, and A', can include structures represented by the following formula groups (15).

[Chem. 100]

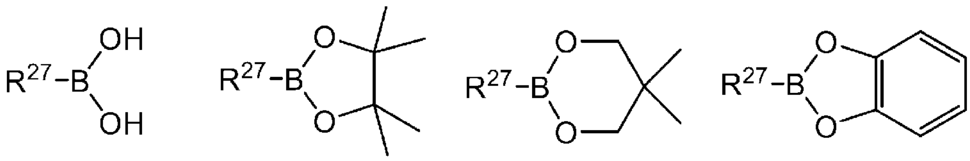

(1 5)

[Chem. 101]

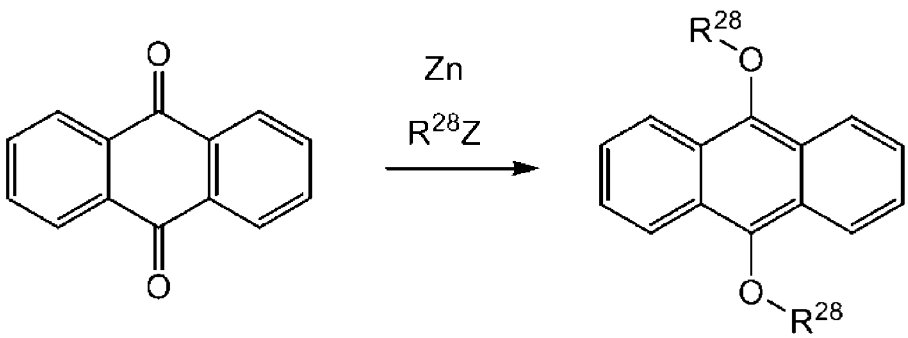


(1 4 (4))

In the formula 14(4), $R^{28}Z$ represents an electrophile, Z represents a halogen atom, and $R^{28}$ represents an alkyl group.

<<Synthesis of diol>>

Diol forms (the formula (23) and the formula (35)), which are precursors of the ester, can be produced with corresponding olefins (the formula (21) and the formula (33)) as the raw material. For example, a reaction of an olefin with potassium permanganate (Non-Patent Literature 4) or osmium tetroxide (Non-Patent Literature 5) enables a diol form (the formula (23) and the formula (35)) to be obtained directly.

As a different method, diol forms (the formula (23) and the formula (35)) can be obtained by epoxidizing the olefin moiety using m-chloroperoxybenzoic acid (Non-Patent Literature 6);tert-butyl peroxide (Non-Patent Literature 7); dimethyldioxirane (Non-Patent Literature 8); formic acid and hydrogen peroxide solution (Non-Patent Literature 9); hydrogen peroxide solution and a molybdenum catalyst; or hydrogen peroxide solution and a tungsten catalyst (Non-Patent Literature 10) followed by an acidic or alkali hydrolysis reaction.

[Chem. 102]

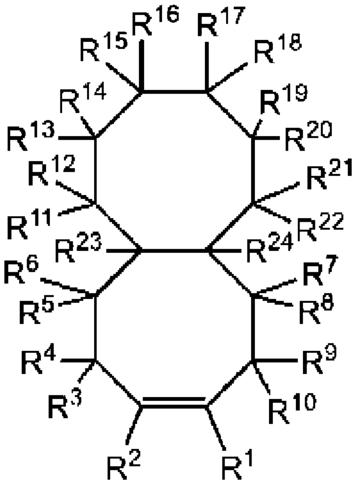

(2 1)

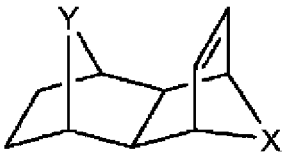

endo form

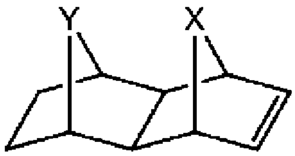

exo form

(2 2)

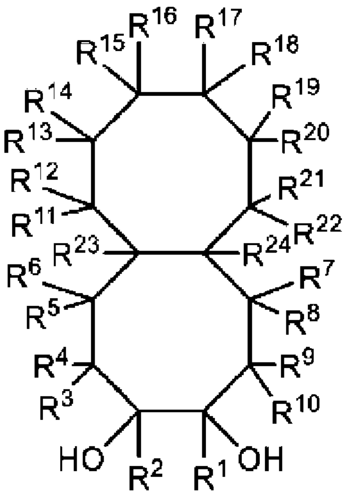

(2 3)

[Chem. 103]

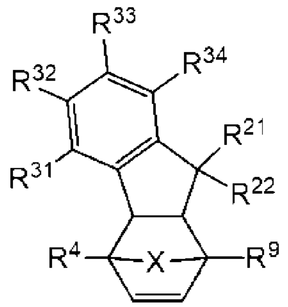

(33)

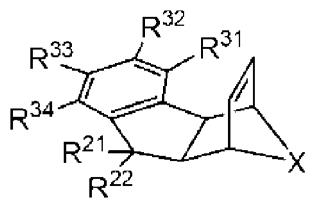

endo form

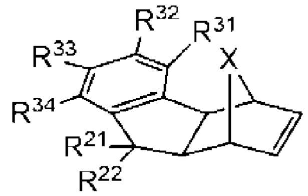

exo form

(34)

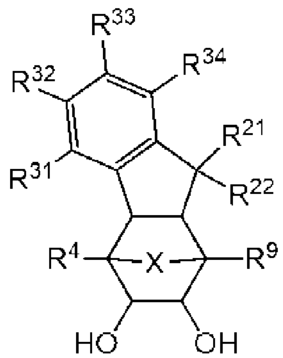

(35)

A diol compound can also be obtained after a hydrolysis of the cyclic carbonate that is obtained from the aforementioned diene or anthracene compound. The details are as follows.

The cyclic carbonate (the formula (24)), which is a precursor of the diol form (the formula (23)), can be produced by a Diels-Alder reaction of a corresponding diene and vinylene carbonate (Non-Patent Literature 19). As described above, for the diene, a dimer of the diene, which is a precursor, also can be used as the raw material. The product obtained by the Diels-Alder reaction is often a mixture of an endo form and an exo form, and even the present Diels-Alder isomer can be applied. In this reaction, a polycyclic carbonate of a particular structure as shown in examples mentioned below can be obtained by using a polycyclic aromatic compound such as anthracene instead of the diene.

Acidic- or alkali-hydrolyzing the cyclic carbonate of the formula (24) enables the diol form (the formula (23)) to be obtained (Non-Patent Literature 19).

[Chem. 104]

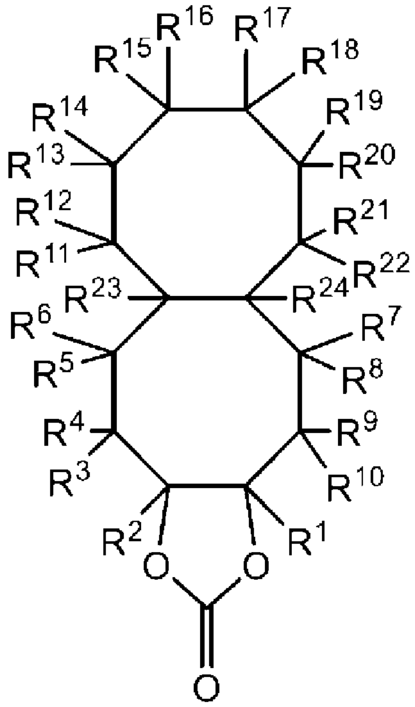

(24)

<<Synthesis of ester>>

An ester form corresponding to the formula (1) can be synthesized by allowing the diol form (the formula (23)) and an acid chloride to react in the presence of a base (the formula 25). Examples of the base that can be used include, but are not particularly limited to, sodium hydroxide, potassium hydroxide, and amine bases. The ester form can be synthesized also by a synthesis method in which the diol form and a carboxylic acid are allowed to react in the presence of an acid catalyst or by using a condensing agent such as DCC (Non-Patent Literature 11) (the formula (26)). When one equivalent of an acid chloride or carboxylic acid is allowed to react with the diol form (the formula (3')), an isomer corresponding to the formula (24) may be formed, but subsequently allowing an acid chloride or carboxylic acid to react enables a compound corresponding to the formula (1) to be obtained. At this time, $L^1$ and $L^2$ may be the same or different. The ester form can be synthesized also by allowing the diol form to react with a carboxylic acid in the presence of an azocarboxylic ester and triphenylphosphine (Non-Patent Literature 12).

An ester form corresponding to the formula (31) can be synthesized by allowing the diol form (the formula (35)) and an acid chloride in the presence of a base, as shown in the following formula (37). Examples of the base that can be used include, but are not particularly limited to, sodium hydroxide, potassium hydroxide, and amine bases. The ester form can be synthesized also by a synthesis method in which the diol form and a carboxylic acid are allowed to react in the presence of an acid catalyst or by using a condensing agent such as DCC (Non-Patent Literature 11) (see the formula (38)). When one equivalent of an acid chloride or carboxylic acid is allowed to react with the diol form (the formula (35), an isomer corresponding to the formula (36) may be formed, but subsequently allowing an acid chloride or carboxylic acid to react enables a compound corresponding to the formula (31) to be obtained. At this time, $L^1$ and $L^2$ may be the same or different. The ester form can be synthesized also by allowing the diol form to react with a carboxylic acid in the presence of an azocarboxylic ester and triphenylphosphine (Non-Patent Literature 12).

[Chem. 105]

[Chem. 106]

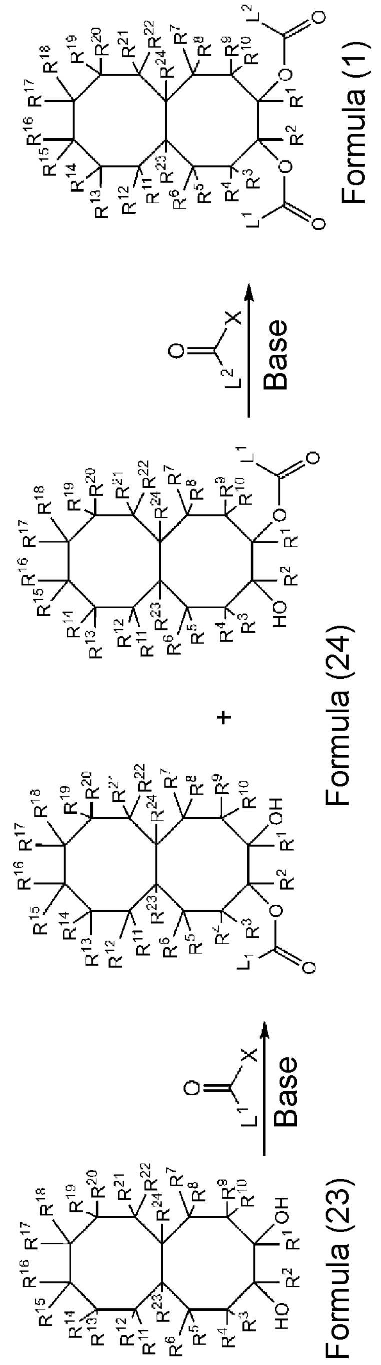


(25)

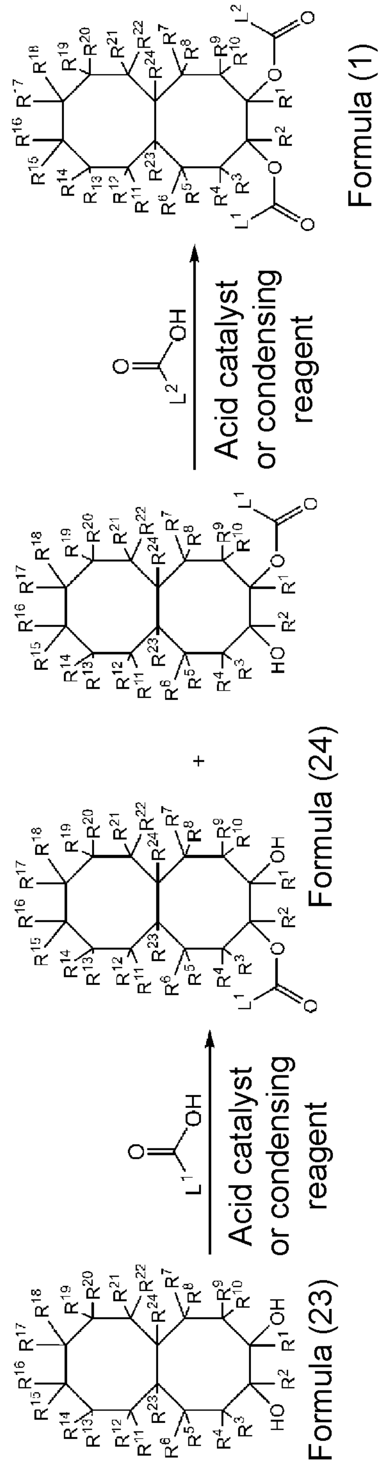


(26)

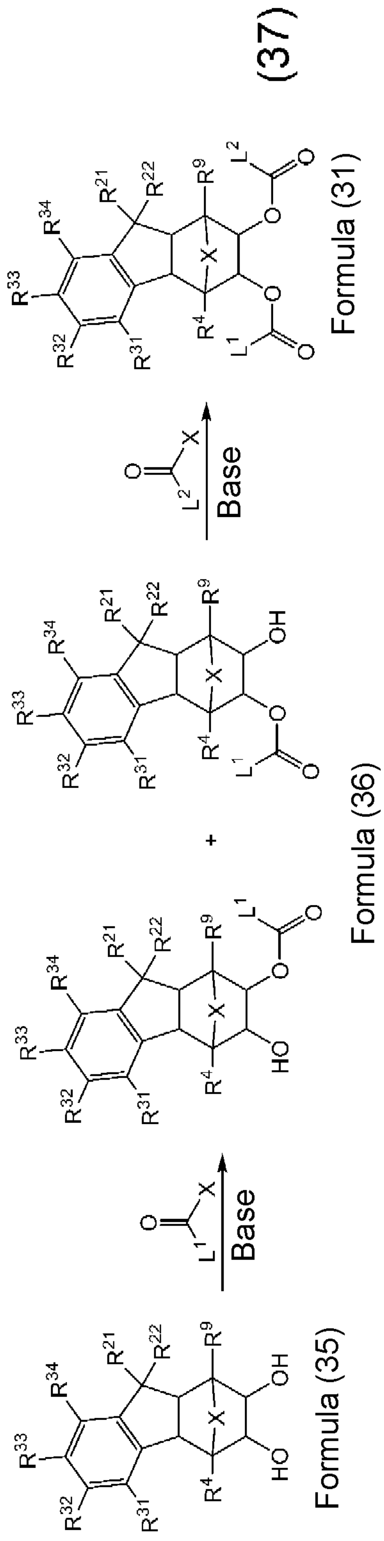


Formula (35) Formula (36) Formula (31) (37)

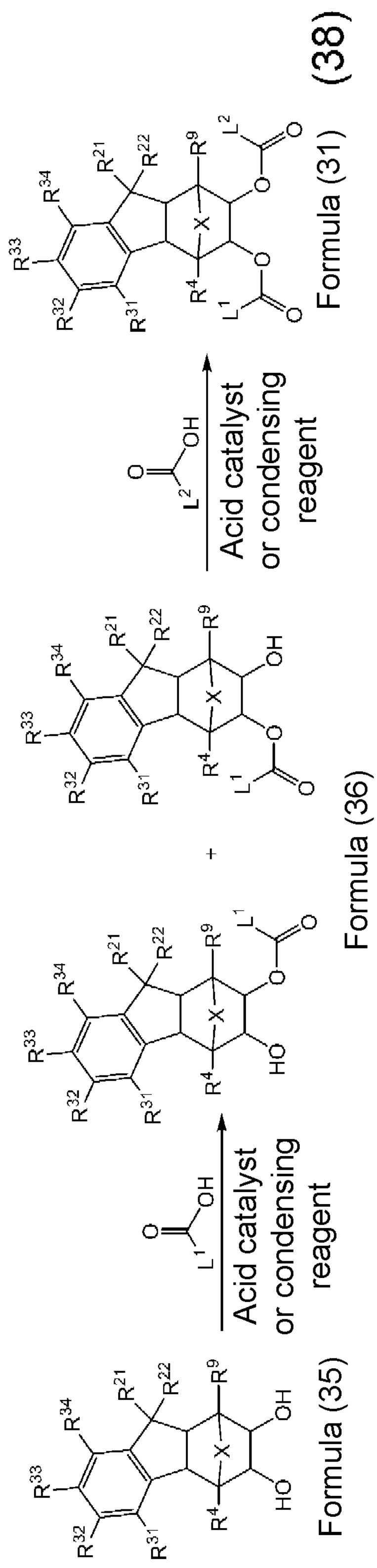


Formula (35) Formula (36) Formula (31) (38)

In the method for synthesizing an ester compound as described above, the ester compound may be obtained as a mixture of an endo form and an exo form, as described previously. From the structural viewpoint, the endo form tends to be easily produced. The isomer ratio when the compound is obtained as a mixture of these is not particularly limited, and a preferable example can include an endo form/exo form ratio of 100/0 to 50/50, preferably of 95/5 to 60/40, and further preferably of 90/10 to 65/35.

These endo form and exo form often can be separated by a known column chromatography using silica gel.

Thus, the endo form and exo form each can be isolated. The isomer ratio can be changed also by an isomerization reaction using a solid acid catalyst such as zeolite. It is certainly possible to adjust a desired isomer ratio by combining each of the compounds isolated at a specific ratio. Depending on applications in which the ester compound of the present invention is applied, it is expected that either of the endo form and exo form may exhibit a suitable effect or that a suitable effect may be exhibited at a specific isomer ratio. In such cases, it is only required to adjust the isomer ratio within 100/0 to 0/100 by a method as described above, for example, before use.

As the ester compound (A) of the present invention, each of the endo form and the exo form may be used singly or an isomer mixture may be used in various applications, as described above.

The ester compound (A) of the present invention is suitable as the Lewis base component of the solid titanium catalyst component, as mentioned previously, but is not limited to this application. It is needless to say that the ester compound has a possibility to be applied in known additive applications such as additives to various resins, cosmetics and external preparations for skin, microbicidal compositions, antioxidants, and chelators.

## Examples

A method for synthesizing the ester compound of the present invention will be exemplified in the following Examples. Compounds of structural formulas disclosed in the following Examples and Comparative Examples represent the structures of the main component of stereoisomers and may include other stereoisomers. In the present invention, the main component refers to a component exceeding 50 mol%, preferably of 70 mol% or more.

(Method for measuring melting behavior of compound)

When the obtained ester compound was solid, using a model DSC7020 Differential Scanning Calorimeter manufactured by Hitachi High-Tech Science Corporation, an appropriate amount of a specimen was placed in the aluminum pan, and the melting behavior was measured under the following conditions.

Onset temperature: 25°C
Finish temperature: 300°C
Temperature increase rate: 10°C/minute

The peak temperature considered to be the melting point was observed. When the peak temperature was difficult to identify, probably due to the influence of an isomer mixture or a compound beginning to decompose at high temperature, for example, the temperature at which heat absorption was finished was defined as the melting completion temperature.

(Method for determining isomer composition)

The isomers were separated by a conventional silica column chromatography. From the results of the NMR analysis of the isolated isomers and the NMR analysis of the mixture, the chemical shifts specific to the isomers were identified, and the isomer ratio was identified with the absorption intensity ratio thereof.

(Structure analysis method by $^1H$ NMR)

A model JNM-EX270 nuclear magnetic resonance apparatus manufactured by JEOL Ltd., and a small amount of tetramethyl silane was added to deuterated chloroform as the solvent. The conditions included: measurement temperature of room temperature, observed nucleus of $^1H$ (270 MHz), sequence of a single pulse, a 45° pulse, repeating time of 5.5 seconds or more, and integrated frequency of 16 to 64 times or more. As the reference chemical shift, the hydrogen of the tetramethylsilane was set to 0 ppm. Peaks of $^1H$ derived from an organic acid compound, for example, were assigned by a conventional method.

[Example A1]

<Synthesis of compound 1>

A compound 1 shown below was synthesized by a method mentioned below according to the following reaction formula.

[Chem. 107]

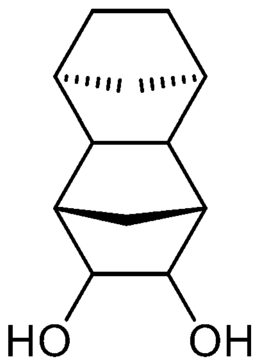

(Compound 1)

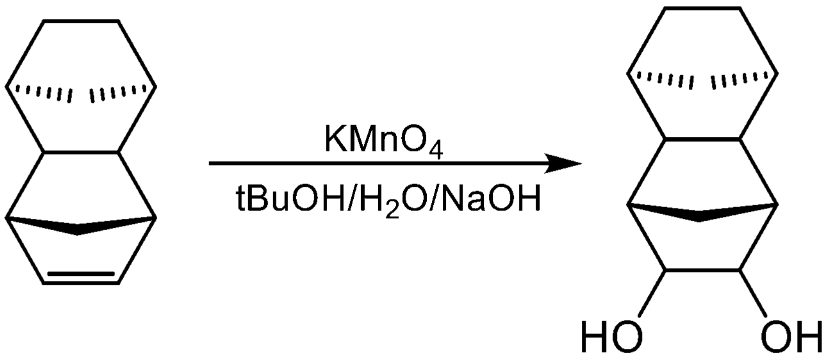


To a 1 L 3-neck flask, 8.62 g (53.8 mmol) of the endo form tetracyclododecene, 240 mL of tert-butyl alcohol, and 50 mL of water were added, and the internal temperature was cooled to 0°C under stirring. To another flask, 12.0 g of potassium permanganate, 250 mL of water, and 2.68 g of sodium hydroxide were added and stirred to prepare a potassium permanganate aqueous solution. The potassium permanganate aqueous solution was slowly added dropwise to the tetracyclododecene solution prepared previously such that the internal temperature did not exceed 5°C. After the dropwise addition, stirring was continued at 0°C for an hour. A saturated sodium metabisulfite aqueous solution was prepared and slowly added dropwise to the reaction solution until a white precipitate was formed. The precipitate formed was removed by filtration, and the filtrate was extracted with ethyl acetate 3 times. The organic layer was washed with brine and dried over magnesium sulfate. The magnesium sulfate was filtered off, and the obtained organic layer was concentrated in a rotary evaporator to thereby give 7.24 g of a crude product of the compound 1. The compound 1 obtained was used in the next <Synthesis of compound 2> without further purification.

<Synthesis of compound 2>

A compound 2 shown below was synthesized by a method mentioned below according to the following reaction formula.

[Chem. 108]

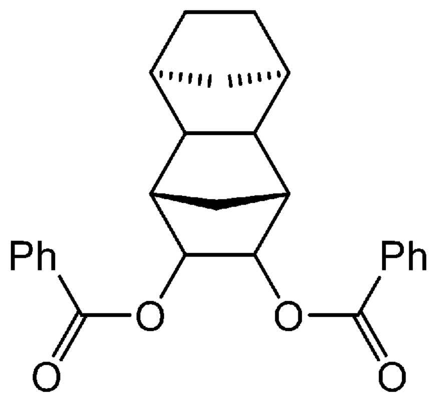

(Compound 2)

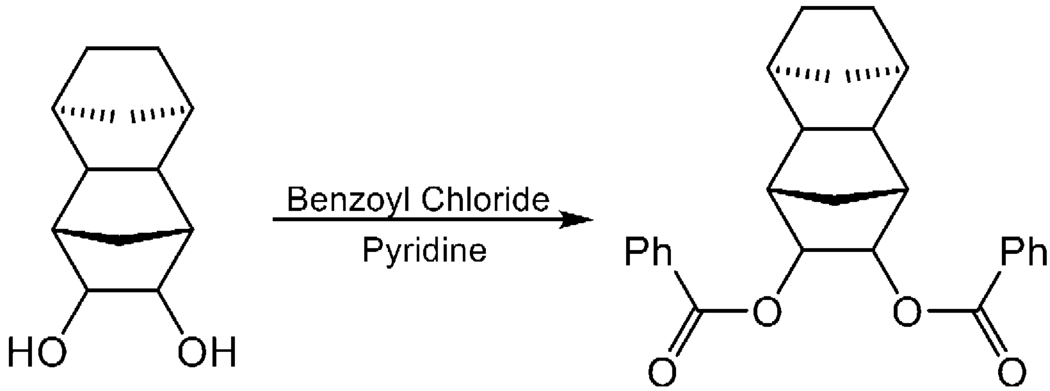


Under a nitrogen atmosphere, 5 g (25.7 mmol) of the compound 1 and 31 mL of dehydrated pyridine were added to a 100 mL 3-neck flask and stirred. Under cooling in an ice bath, 6.3 mL of benzoyl chloride was slowly added thereto. After the addition, the temperature was raised to room temperature, and stirring was conducted overnight. Under cooling in an ice bath again, 5 mL of methanol was added. The organic layer was separated by adding chloroform and water to the reaction solution. The organic layer was washed with water 3 times, a saturated ammonium chloride aqueous solution, and brine, and dried over magnesium sulfate. The magnesium sulfate was filtered off, and the obtained organic layer was concentrated in a rotary evaporator to thereby give 10.37 g of a crude product. The crude product was purified by silica gel column chromatography to give 6.67 g (16.6 mmol, white solid) of the compound 2. The $^1$H-NMR data of the compound 2 obtained are shown below.

$^1$H NMR (270 MHz, $CDCl_3$, TMS as an internal standard): $\delta$ 0.97-1.28 (m, 3H), 1.32-1.70 (m, 4H), 1.90 (br s, 2H), 2.15 (d, J=10.2 Hz, 1H), 2.39 (br s, 2H), 2.53 (br s, 2H), 5.53-5.59 (m, 2H), 7.19-7.33 (m, 4H), 7.40-7.49 (m, 2H), 7.80-7.92 (m, 4H).

The melting completion temperature of the compound 2 obtained was 141°C.

[Example A2]

<Synthesis of compound 3>

A compound 3 shown below was synthesized by a method mentioned below.

[Chem. 109]

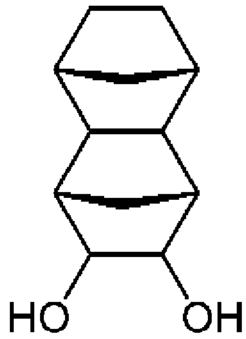

(Compound 3)

To a 1 L 3-neck flask, 10.18 g (63.5 mmol) of the exo form tetracyclododecene, 240 mL of tert-butyl alcohol, and 60 mL of water were added, and the internal temperature was cooled to 0°C under stirring. To another flask, 13.81 g of potassium permanganate, 300 mL of water, and 3.00 g of sodium hydroxide were added and stirred to prepare a potassium permanganate aqueous solution. The potassium permanganate aqueous solution was slowly added dropwise to the tetracyclododecene solution prepared previously such that the internal temperature did not exceed 5°C. After the dropwise addition, stirring was continued at 0°C for an hour. A saturated sodium metabisulfite aqueous solution was prepared and slowly added dropwise to the reaction solution until a white precipitate was formed. The precipitate formed was removed by filtration, and the filtrate was extracted with ethyl acetate 3 times. The organic layer was washed with brine and dried over magnesium sulfate. The magnesium sulfate was filtered off, and the obtained organic layer was concentrated in a rotary evaporator to thereby give 3.42 g (17.6 mmol) of a crude product of the compound 3. The compound 3 obtained was used in the next <Synthesis of compound 4> without further purification.

<Synthesis of compound 4>

A compound 4 shown below was synthesized by a method mentioned below.

[Chem. 110]

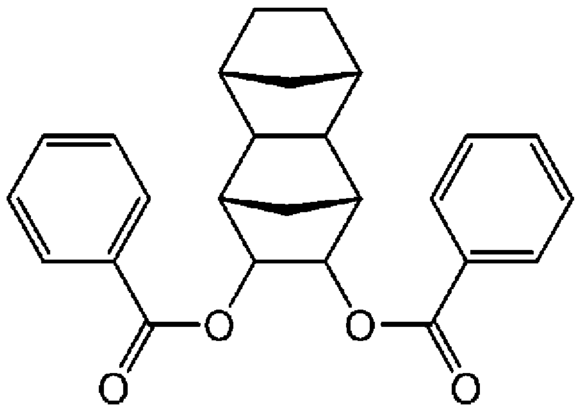

(Compound 4)

Under a nitrogen atmosphere, 4.0 g (20.6 mmol) of the compound 3, 4.2 mL of dehydrated pyridine, and 100 mL of dehydrated chloroform were added to a 200 mL 3-neck flask and stirred. Under cooling in an ice bath, 4.9 mL of benzoyl chloride was slowly added thereto. After the addition, the temperature was raised to room temperature, and stirring was conducted overnight. Under cooling in an ice bath again, 5 mL of methanol was added. The organic layer was separated by adding chloroform and water to the reaction solution. The organic layer was washed with water 3 times, a saturated ammonium chloride aqueous solution, and brine, and dried over magnesium sulfate. The magnesium sulfate was filtered off, and the obtained organic layer was concentrated in a rotary evaporator to thereby give 11.3 g of a crude product. The crude product was purified by silica gel column chromatography to give 4.44 g (11.0 mmol, white solid) of the compound 4. The $^1$H-NMR data of the compound 4 obtained are shown below.

$^1$H NMR (270 MHz, $CDCl_3$, TMS as an internal standard): δ 0.96 (d, J=10.6 Hz 1H), 1.13-1.22 (m, 2H), 1.43-1.60 (m, 5H), 1.77-2.05 (m, 2H), 2.23 (br s, 2H), 2.41 (br s, 2H), 4.99-5.01 (m, 2H), 7.21-7.29 (m, 4H), 7.42-7.49 (m, 2H), 7.81-7.87 (m, 4H).

The melting completion temperature of the compound 4 obtained was 163°C.

[Example A3]

<Synthesis of compound 5>

A compound 5 shown below was synthesized by a method mentioned below.

[Chem. 111]

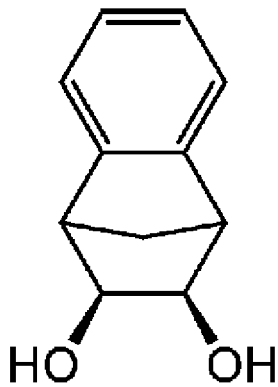

(Compound 5)

To a 2 L 3-neck flask, 15.53 g (110.8 mmol) of benzonorbornadiene, 425 mL of tert-butyl alcohol, and 105 mL of water were added, and the internal temperature was cooled to 0°C under stirring. To another flask, 23.7 g of potassium permanganate, 527 mL of water, and 5.07 g of sodium hydroxide were added and stirred to prepare a potassium permanganate aqueous solution. The potassium permanganate aqueous solution was slowly added dropwise to the benzonorbornadiene solution prepared previously such that the internal temperature did not exceed 5°C. After the dropwise addition, stirring was continued at 0°C for an hour. A saturated sodium metabisulfite aqueous solution was prepared and slowly added dropwise to the reaction solution until a white precipitate was formed. The precipitate formed was removed by filtration, and the filtrate was extracted with ethyl acetate 3 times. The organic layer was washed with brine and dried over magnesium sulfate. The magnesium sulfate was filtered off, and the obtained organic layer was concentrated in a rotary evaporator to thereby give 8.19 g of the compound 5. The $^{1}$H-NMR data of the compound 5 obtained are shown below.

$^{1}$H NMR (270 MHz, $CDCl_3$, TMS as an internal standard): $\delta$ 1.89-1.97 (m, 1H), 2.20-2.27 (m, 1H), 2.74-2.82 (m, 2H), 3.20-3.25 (m, 2H), 3.80-3.87 (m, 2H), 7.06-7.13 (m, 2H), 7.15-7.22 (m, 2H).

<Synthesis of compound 6>

A compound 6 shown below was synthesized by a method mentioned below.

[Chem. 112]

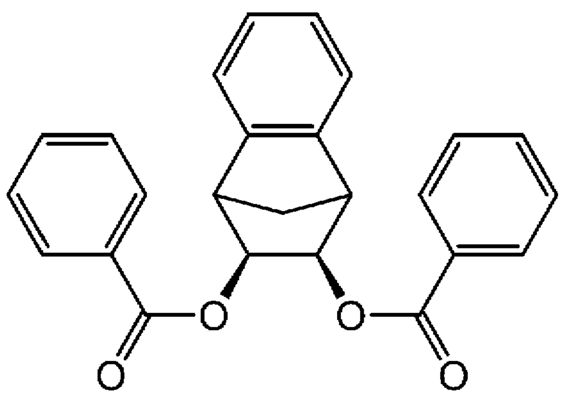

(Compound 6)

Under a nitrogen atmosphere, 9.57 g (54.3 mmol) of the compound 5 and 70 mL of dehydrated pyridine were added to a 300 mL 3-neck flask and stirred. Under cooling in an ice bath, 13.4 mL of benzoyl chloride was slowly added

thereto. After the addition, the temperature was raised to room temperature, and stirring was conducted overnight. Under cooling in an ice bath again, 5 mL of methanol was added. To a beaker was added 200 mL of water, and the reaction solution was added thereto under stirring, and a precipitate formed was filtered off. The precipitate was washed with hexane, and then a recrystallization operation was conducted with ethanol to give 16.36 g of a solid. The small amount of the residual pyridine was removed with silica gel column chromatography to give 15.48 g (40.3 mmol, white solid) of the compound 6. The $^1$H-NMR data of the compound 6 obtained are shown below.

$^1$H NMR (270 MHz, $CDCl_3$, TMS as an internal standard): δ 2.12-2.20 (m, 1H), 2.57-2.64 (m, 1H), 3.57 (br s, 2H), 5.18 (d, J=1.6 Hz, 2H), 7.12-7.22 (m, 2H), 7.24-7.37 (m, 6H), 7.44-7.53 (m, 4H), 7.87-7.96 (m, 2H).

The melting completion temperature of the compound 6 obtained was 139°C.

[Example A4]

<Synthesis of compound 7>

A compound 7 shown below was synthesized by a method mentioned below according to the following reaction formula.

[Chem. 113]

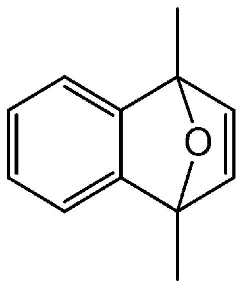

(Compound 7)

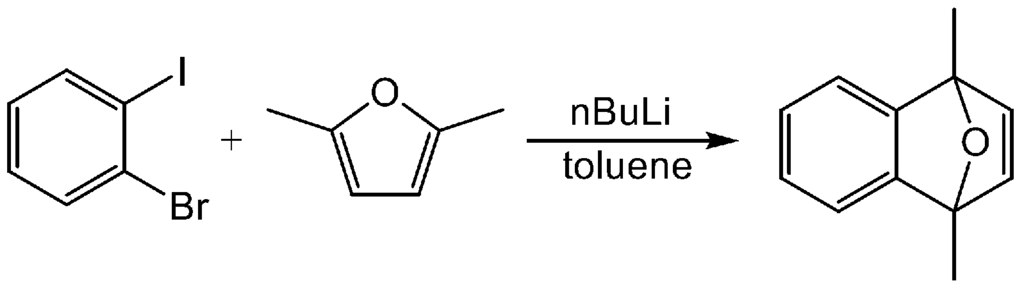


Under a nitrogen atmosphere, 200 mL of dehydrated toluene, 6.36 mL of 1-bromo-2-iodobenzene, and 10.5 mL of 2,5-dimethylfuran were added to a 1 L 3-neck flask and cooled to -20°C under stirring. 47 mL of a n-butyllithium hexane solution (1.6 M) was slowly added dropwise, and after the dropwise addition, stirred at -20°C for an hour. The temperature was gradually raised to room temperature, and stirring was continued overnight. Under cooling in an ice bath, water was slowly added dropwise, and ethyl acetate was added to separate the organic layer. The organic layer was washed with brine and dried over magnesium sulfate. The magnesium sulfate was filtered off, and the obtained organic layer was concentrated in a rotary evaporator. The resultant crude product was purified by silica gel column chromatography to give 4.05 g (23.5 mmol) of the compound 7. The $^1$H-NMR data of the compound 7 obtained are shown below.

$^1$H NMR (270 MHz, $CDCl_3$, TMS as an internal standard): δ 1.90 (s, 6H), 6.77 (s, 2H), 6.97 (dd, J=5.3, 3.0 Hz, 2H), 7.13 (dd, J=5.3, 3.0 Hz, 2H).

<Synthesis of compound 8>

A compound 8 shown below was synthesized by a method mentioned below.

[Chem. 114]

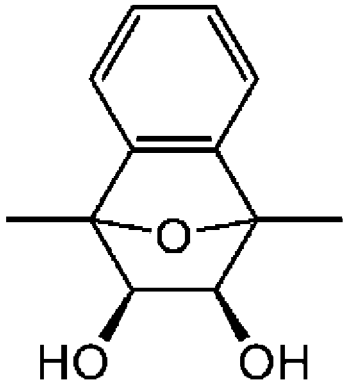

(Compound 8)

To a 2 L 3-neck flask, 4.05 g (23.5 mmol) of the compound 7, 129 mL of tert-butyl alcohol, and 32 mL of water were added, and the internal temperature was cooled to 0°C under stirring. To another flask, 7.23 g of potassium permanganate, 161 mL of water, and 1.61 g of sodium hydroxide were added and stirred to prepare a potassium permanganate aqueous solution. The potassium permanganate aqueous solution was slowly added dropwise to the solution of the compound 7 prepared previously such that the internal temperature did not exceed 5°C. After the dropwise addition, stirring was continued at 0°C for an hour. A saturated sodium metabisulfite aqueous solution was prepared and slowly added dropwise to the reaction solution until a white precipitate was formed. The precipitate formed was removed by filtration, and the filtrate was extracted with ethyl acetate 3 times. The organic layer was washed with brine and dried over magnesium sulfate. The magnesium sulfate was filtered off, and the obtained organic layer was concentrated in a rotary evaporator to thereby give 3.03 g of a crude product. The crude product was purified by silica gel column chromatography to give 2.02 g (10.7 mmol) of the compound 8. The $^1$H-NMR data of the compound 8 obtained are shown below.

$^1$H NMR (270 MHz, $CDCl_3$, TMS as an internal standard): $\delta$ 1.76 (s, 6H), 2.84 (br s, 2H), 3.76 (br s, 2H), 7.15-7.25 (m, 4H) .

<Synthesis of compound 9>

A compound 9 shown below was synthesized by a method mentioned below.

[Chem. 115]

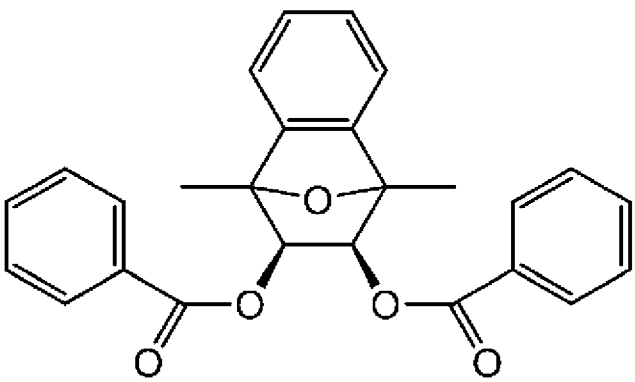

(Compound 9)

Under a nitrogen atmosphere, 2.15 g (10.4 mmol) of the compound 8 and 10 mL of dehydrated pyridine were added to a 50 mL 3-neck flask and stirred. Under cooling in an ice bath, 2.8 mL of benzoyl chloride was slowly added thereto. After the addition, the temperature was raised to room temperature, and stirring was conducted overnight. Under cooling in an ice bath again, 5 mL of methanol was added. To a beaker were added 100 mL of water and 100 mL of ethyl acetate to separate the organic layer. The organic layer was washed with water 3 times, washed with a saturated ammonium chloride aqueous solution and brine, and dried over magnesium sulfate. The magnesium sulfate was filtered, and the filtrate was concentrated in a rotary evaporator to thereby give 4.78 g of a crude product. The crude product

was purified by silica gel column chromatography to give 3.29 g (7.9 mmol, white solid) of the compound 9. The $^{1}$H-NMR data of the compound 9 obtained are shown below.

$^{1}$H NMR (270 MHz, $CDCl_3$, TMS as an internal standard): δ 1.85 (s, 6H), 5.34 (s, 2H), 7.22-7.55 (m, 10H), 7.91-8.02 (m, 4H) .

The melting completion temperature of the compound 9 obtained was 196°C.

[Example A5]

<Synthesis of compound 10>

A compound 10 shown below was synthesized by a method mentioned below according to the following reaction formula.

[Chem. 116]

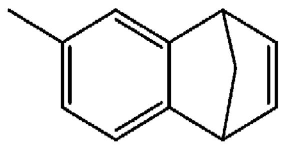

(Compound 10)

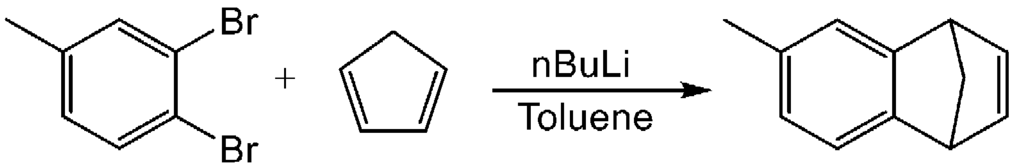


Under a nitrogen atmosphere, 16.0 g of 3,4-dibromotoluene, 120 mL of dehydrated toluene, and 4.23 g of cyclopentadiene were added to a 500 mL 3-neck flask and stirred. As for the cyclopentadiene used for the reaction, in reference to Non-Patent Literature 13, dicyclopentadiene was pyrolyzed at 160 to 170°C, and a component distilled at 40°C to 67°C was rapidly used. The internal temperature was cooled to 0°C, and 40 mL of a n-butyllithium hexane solution (1.6 M) was slowly added dropwise. After the dropwise addition, the temperature was gradually raised to room temperature, and stirring was conducted at room temperature for 4 hours. After the reaction, a saturated ammonium chloride aqueous solution was added, and then diethyl ether was added. The organic layer was separated, and the organic layer was washed with water and brine. After the organic layer was dried over magnesium sulfate, the magnesium sulfate was filtered off, and the obtained organic layer was concentrated in a rotary evaporator to thereby give 14.32 g of a crude product. The crude product was purified by silica gel column chromatography to give 4.19 g (26.8 mmol) of the compound 10. The $^{1}$H-NMR data of the compound 10 obtained are shown below.

$^{1}$H NMR (270 MHz, $CDCl_3$, TMS as an internal standard): δ 2.19-2.24 (m, 1H), 2.27-2.33 (m, 4H), 3.85 (s, 2H), 6.71-6.81 (m, 2H), 7.06-7.12 (m, 3H).

<Synthesis of compound 11>

A compound 11 shown below was synthesized by a method mentioned below.

[Chem. 117]

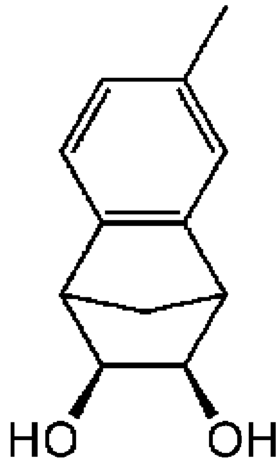

(Compound 11)

To a 500 mL 3-neck flask, 4.19 g (26.8 mmol) of the compound 10, 107 mL of tert-butyl alcohol, and 27 mL of water were added, and the internal temperature was cooled to 0°C under stirring. To another flask, 6.40 g of potassium permanganate, 135 mL of water, and 1.42 g of sodium hydroxide were added and stirred to prepare a potassium permanganate aqueous solution. The potassium permanganate aqueous solution was slowly added dropwise to the solution of the compound 10 prepared previously such that the internal temperature did not exceed 5°C. After the dropwise addition, stirring was continued at 0°C for an hour. A saturated sodium metabisulfite aqueous solution was prepared and slowly added dropwise to the reaction solution until a white precipitate was formed. The precipitate formed was removed by filtration, and the filtrate was extracted with ethyl acetate 4 times. The organic layer was washed with brine and dried over magnesium sulfate. The magnesium sulfate was filtered off, and the obtained organic layer was concentrated in a rotary evaporator to thereby give 3.80 g of a crude product. The crude product was purified by silica gel column chromatography to give 3.04 g (16.0 mmol) of the compound 11. The $^1$H-NMR data of the compound 11 obtained are shown below.

$^1$H NMR (270 MHz, $CDCl_3$, TMS as an internal standard): δ 1.86-1.93 (m, 1H), 2.18-2.24 (m, 1H), 2.30 (s, 3H), 2.78-2.86 (m, 2H), 3.15-3.19 (m, 2H), 3.78-3.84 (m, 2H), 6.90 (d, J=7.3 Hz, 1H), 7.02 (s, 1H), 7.07 (d, J=7.3 Hz, 1H).

<Synthesis of compound 12>

A compound 12 shown below was synthesized by a method mentioned below.

[Chem. 118]

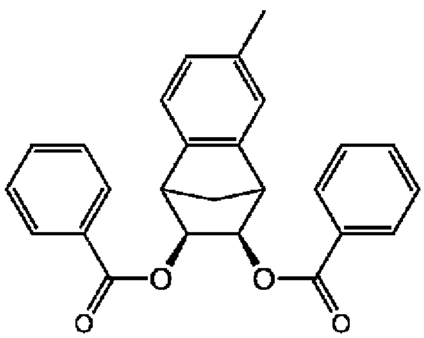

(Compound 12)

Under a nitrogen atmosphere, 2.94 g (15.5 mmol) of the compound 11, 80 mL of dehydrated chloroform, and 4.6 g of benzoyl chloride were added to a 200 mL 3-neck flask and stirred. Under cooling in an ice bath, 2.6 mL of dehydrated pyridine was slowly added dropwise. After the addition, the temperature was raised to room temperature, and stirring was conducted overnight. Under cooling in an ice bath again, 5 mL of methanol was added. After water and chloroform was added to the reaction solution and stirred, the organic layer was separated. The organic layer was

washed with brine and then dried over magnesium sulfate. The magnesium sulfate was filtered off, and the obtained organic layer was concentrated in a rotary evaporator to thereby give 7.91 g of a crude product. The crude product was purified by silica gel column chromatography to give 4.82 g (12.1 mmol, white solid) of the compound 12. The $^{1}$H-NMR data of the compound 12 obtained are shown below.

$^{1}$H NMR (270 MHz, $CDCl_3$, TMS as an internal standard): δ 2.08-2.17 (m, 1H), 2.35 (s, 3H), 2.55-2.63 (m, 1H), 3.49-3.55 (m, 2H), 5.13-5.18 (m, 2H), 6.96-7.04 (m, 1H), 7.15-7.39 (m, 6H), 7.44-7.52 (m, 2H), 7.87-7.95 (m, 4H).

The melting completion temperature of the compound 12 obtained was 155°C.

[Example A6]

<Synthesis of compound 13>

A compound 13 shown below was synthesized by a method mentioned below according to the following reaction formula.

[Chem. 119]

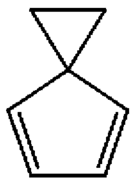

(Compound 13)

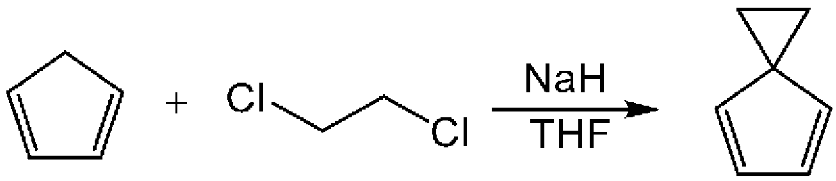


Under a nitrogen atmosphere, 21.7 g of sodium hydride (64.6% liquid paraffin dispersion) was added to a 200 mL 3-neck flask, and 135 mL of dehydrated tetrahydrofuran was added thereto and stirred. Under cooling in an ice bath, 19.3 g of cyclopentadiene was slowly added dropwise. As for the cyclopentadiene used for the reaction, in reference to Non-Patent Literature 13, dicyclopentadiene was pyrolyzed at 160 to 170°C, and a component distilled at 40°C to 67°C was rapidly used. After the dropwise addition, the mixture was stirred at room temperature for 20 minutes. Then, under cooling in an ice bath, 28.9 g of 1,2-dichloroethane was slowly added dropwise. After the dropwise addition, the temperature was raised to room temperature, and stirring was conducted for 8 hours. To a beaker were added 100 mL of tetrahydrofuran and 10.5 g of water to prepare hydrous THF. While the flask after the reaction was cooled in an ice bath, the hydrous tetrahydrofuran was slowly added dropwise. Ice water was provided in another flask, and the reaction solution was transferred into the ice water. Thereafter, pentane was added, and the organic layer was separated. Subsequently, the organic layer was washed twice with 0.5 N hydrochloric acid, twice with water, and once with brine, and dried over magnesium sulfate. The magnesium sulfate was filtered off, and the obtained organic layer was distilled, and the component at 664 to 665 torr and the distillation temperatures of 88 to 92°C was collected to give 18.09 g of the compound 13.

<Synthesis of compound 14>

A compound 14 shown below was synthesized by a method mentioned below according to the following reaction formula.

[Chem. 120]

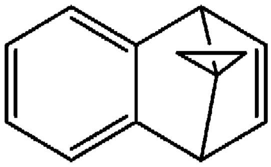

(Compound 14)

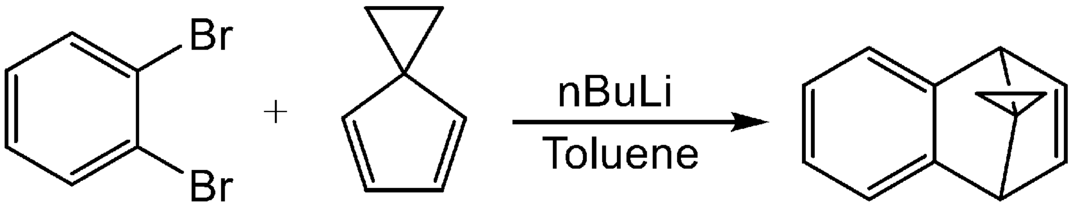


Under a nitrogen atmosphere, 15.1 g (64.0 mmol) of 1,2-dibromobenzene, 130 mL of dehydrated toluene, and 5.90 g of the compound 13 were added to a 500 mL 3-neck flask and stirred. The internal temperature was cooled to 0°C, and 40 mL of a n-butyllithium hexane solution (1.6 M) was slowly added dropwise. After the dropwise addition, the temperature was gradually raised to room temperature, and stirring was conducted at room temperature for 4 hours. After the reaction, a saturated ammonium chloride aqueous solution was added, and then diethyl ether was added. The organic layer was separated and washed with water and brine in the order mentioned. After the organic layer was dried over magnesium sulfate, the magnesium sulfate was filtered off, and the obtained organic layer was concentrated in a rotary evaporator to thereby give 15.44 g of a crude product. The crude product was purified by silica gel column chromatography to give 7.70 g (45.8 mmol) of the compound 14.

<Synthesis of compound 14-2>

A compound 14-2 shown below was synthesized by a method mentioned below.

[Chem. 121]

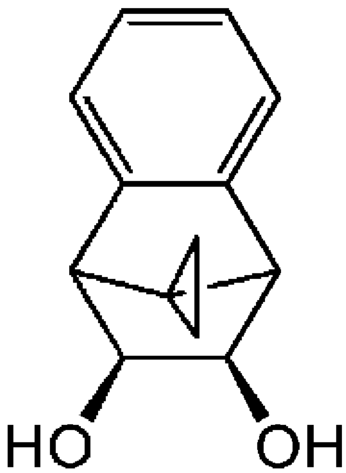

(Compound 14-2)

To a 500 mL 3-neck flask, 7.7 g (45.8 mmol) of the compound 14, 178 mL of tert-butyl alcohol, and 44 mL of water were added, and the internal temperature was cooled to 0°C under stirring. To another flask, 10.6 g of potassium permanganate, 222 mL of water, and 2.32 g of sodium hydroxide were added and stirred to prepare a potassium permanganate aqueous solution. The potassium permanganate aqueous solution was slowly added dropwise to the solution of the compound 14 prepared previously such that the internal temperature did not exceed 5°C. After the dropwise addition, stirring was continued at 0°C for an hour. A saturated sodium metabisulfite aqueous solution was prepared and slowly added dropwise to the reaction solution until a white precipitate was formed. The precipitate formed

was removed by filtration, and the filtrate was extracted with ethyl acetate 4 times. The organic layer was washed with brine and dried over magnesium sulfate. The magnesium sulfate was filtered off, and the obtained organic layer was concentrated in a rotary evaporator to thereby give 5.63 g of a crude product. The crude product was purified by silica gel column chromatography to give 3.85 g (19.0 mmol) of the compound 14-2. The $^1$H-NMR data of the compound 14-2 obtained are shown below.

$^1$H NMR (270 MHz, $CDCl_3$, TMS as an internal standard): δ 0.29-0.35 (m, 2H), 0.89-0.95 (m, 2H), 2.69-2.79 (m, 4H), 3.91-3.96 (m, 2H), 7.08-7.22 (m, 4H).

<Synthesis of compound 15>

A compound 15 shown below was synthesized by a method mentioned below.

[Chem. 122]

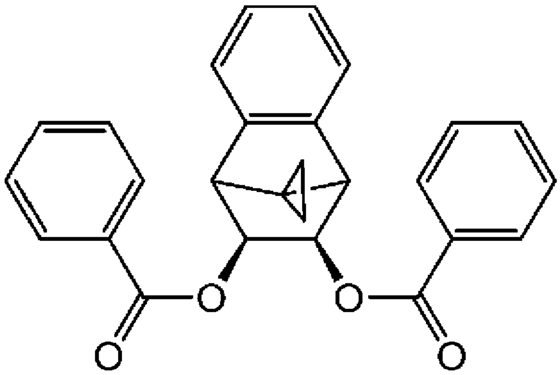

(Compound 15)

Under a nitrogen atmosphere, 4.06 g (24.1 mmol) of the compound 14-2, 105 mL of dehydrated chloroform, and 5.95 g of benzoyl chloride were added to a 200 mL 3-neck flask and stirred. Under cooling in an ice bath, 3.4 mL of dehydrated pyridine was slowly added dropwise. After the addition, the temperature was raised to room temperature, and stirring was conducted overnight. Under cooling in an ice bath again, 5 mL of methanol was added. After water and chloroform was added to the reaction solution and stirred, the organic layer was separated. The organic layer was washed with brine and then dried over magnesium sulfate. The magnesium sulfate was filtered off, and the obtained organic layer was concentrated in a rotary evaporator to thereby give 9.65 g of a crude product. The crude product was purified by silica gel column chromatography to give 3.88 g (9.5 mmol, white solid) of the compound 15. The $^1$H-NMR data of the compound 15 obtained are shown below.

$^1$H NMR (270 MHz, $CDCl_3$, TMS as an internal standard): δ 0.46-0.56 (m, 2H), 1.05-1.14 (m, 2H), 3.04 (s, 2H), 5.28 (s, 2H), 7.17-7.37 (m, 8H), 7.44-7.53 (m, 2H), 7.88-7.96 (m, 4H).

The melting completion temperature of the compound 15 obtained was 111°C.

[Example A7]

<Synthesis of compound 16>

A compound 16 shown below was synthesized by a method mentioned below according to the following reaction formula.

[Chem. 123]

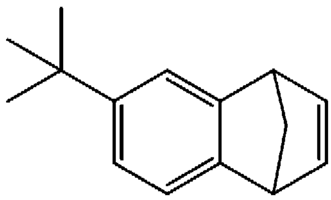

(Compound 16)

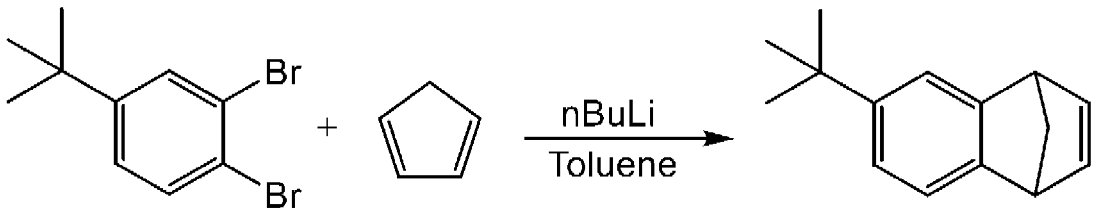


Under a nitrogen atmosphere, 20.0 g (68.5 mmol) of 1,2-dibromo-4-tert-butylbenzene, 130 mL of dehydrated toluene, and 4.54 g of cyclopentadiene were added to a 500 mL 3-neck flask and stirred. As for the cyclopentadiene used for the reaction, in reference to Non-Patent Literature 13, dicyclopentadiene was pyrolyzed at 160 to 170°C, and a component distilled at 40°C to 67°C was rapidly used. The internal temperature was cooled to 0°C, and 43 mL of a n-butyllithium hexane solution (1.6 M) was slowly added dropwise. After the dropwise addition, the temperature was gradually raised to room temperature, and stirring was conducted at room temperature for 12 hours. After the reaction, a saturated ammonium chloride aqueous solution was added, and then diethyl ether was added. The organic layer was separated and washed with water and brine in the order mentioned. After the organic layer was dried over magnesium sulfate, the magnesium sulfate was filtered off, and the obtained organic layer was concentrated in a rotary evaporator to thereby give 19.67 g of a crude product. The crude product was purified by silica gel column chromatography to give 5.57 g (28.1 mmol) of the compound 16. The $^1$H-NMR data of the compound 16 obtained are shown below.

$^1$H NMR (270 MHz, $CDCl_3$, TMS as an internal standard): δ 1.30 (s, 9H), 2.21-2.33 (m, 2H), 3.83-3.87 (m, 2H), 6.76-6.80 (m, 2H), 6.93 (dd, J=7.2, 1.6 Hz, 1H), 7.13 (d, J=7.2 Hz, 1H), 7.29 (d, J=1.6 Hz, 1H).

<Synthesis of compound 17>

A compound 17 shown below was synthesized by a method mentioned below.

[Chem. 124]

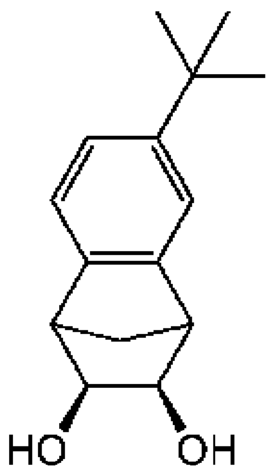

(Compound 17)

To a 500 mL 3-neck flask, 5.57 g (28.1 mmol) of the compound 16, 109 mL of tert-butyl alcohol, and 27 mL of water were added, and the internal temperature was cooled to 0°C under stirring. To another flask, 6.55 g of potassium

permanganate, 135 mL of water, and 1.55 g of sodium hydroxide were added and stirred to prepare a potassium permanganate aqueous solution. The potassium permanganate aqueous solution was slowly added dropwise to the solution of the compound 16 prepared previously such that the internal temperature did not exceed 5°C. After the dropwise addition, stirring was continued at 0°C for an hour. A saturated sodium metabisulfite aqueous solution was prepared and slowly added dropwise to the reaction solution until a white precipitate was formed. The precipitate formed was removed by filtration, and the filtrate was extracted with ethyl acetate 4 times. The organic layer was washed with brine and dried over magnesium sulfate. The magnesium sulfate was filtered off, and the obtained organic layer was concentrated in a rotary evaporator. The crude product was purified by silica gel column chromatography to give 4.11 g (17.7 mmol) of the compound 17. The $^1$H-NMR data of the compound 17 obtained are shown below.

$^1$H NMR (270 MHz, $CDCl_3$, TMS as an internal standard): δ 1.29 (s, 9H), 1.88-1.95 (m, 1H), 2.19-2.25 (m, 1H), 2.89 (br s, 2H), 3.17-3.20 (m, 2H), 3.80-3.84 (m, 2H), 7.09-7.12 (m, 2H), 7.22-7.24 (m, 1H).

<Synthesis of compound 18>

A compound 18 shown below was synthesized by a method mentioned below.

[Chem. 125]

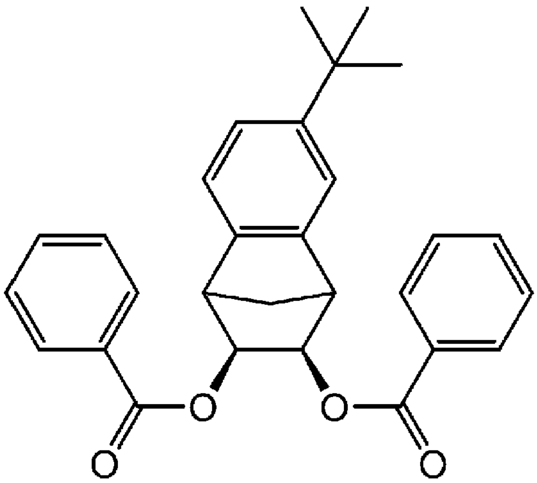

(Compound 18)

Under a nitrogen atmosphere, 4.11 g (9.3 mmol) of the compound 17 and 17.7 mL of dehydrated pyridine were added to a 50 mL 3-neck flask and stirred. Under cooling in an ice bath, 4.3 mL of benzoyl chloride was slowly added thereto. After the addition, the temperature was raised to room temperature, and stirring was conducted overnight. Under cooling in an ice bath again, 5 mL of methanol was added. To a beaker were added 100 mL of water and 100 mL of ethyl acetate to separate the organic layer. The organic layer was washed with water 3 times and once each with a saturated ammonium chloride aqueous solution and brine in the order mentioned, and dried over magnesium sulfate. The magnesium sulfate was filtered, and the filtrate was concentrated in a rotary evaporator to thereby give 9.34 g of a crude product. The crude product was purified by silica gel column chromatography to give 6.13 g of a solid component. The obtained solid was recrystallized with hexane to give 4.0 g (9.0 mmol, white solid) of the compound 18. The $^1$H-NMR data of the compound 18 obtained are shown below.

$^1$H NMR (270 MHz, $CDCl_3$, TMS as an internal standard): δ 1.33 (s, 9H), 2.11-2.19 (m, 1H), 2.56-2.63 (m, 1H), 3.53 (s, 2H), 5.15-5.19 (m, 2H), 7.18-7.32 (m, 6H), 7.37-7.39 (m, 1H), 7.44-7.53 (m, 2H), 7.88-7.94 (m, 4H).

The melting completion temperature of the compound 18 obtained was 115°C.

[Example A8]

<Synthesis of compound 19>

A compound 19 shown below was synthesized by a method mentioned below.

[Chem. 126]

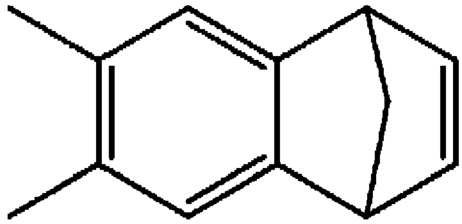

(Compound 19)

Under a nitrogen atmosphere, 20.0 g (75.8 mmol) of 1,2-dibromo-4,5-dimethylbenzene, 150 mL of dehydrated toluene, and 5.01 g of cyclopentadiene were added to a 500 mL 3-neck flask and stirred. As for the cyclopentadiene used for the reaction, in reference to Non-Patent Literature 13, dicyclopentadiene was pyrolyzed at 160 to 170°C, and a component distilled at 40°C to 67°C was rapidly used. The internal temperature was cooled to 0°C, and 47.5 mL of a n-butyllithium hexane solution (1.6 M) was slowly added dropwise. After the dropwise addition, the temperature was gradually raised to room temperature, and stirring was conducted at room temperature for 12 hours. After the reaction, a saturated ammonium chloride aqueous solution was added, and then diethyl ether was added. The organic layer was separated and washed with water and brine in the order mentioned. After the organic layer was dried over magnesium sulfate, the magnesium sulfate was filtered off, and the obtained organic layer was concentrated in a rotary evaporator to thereby give 18.64 g of a crude product. The crude product was purified by silica gel column chromatography to give 3.89 g (22.8 mmol) of the compound 19. The $^1$H-NMR data of the compound 19 obtained are shown below.

$^1$H NMR (270 MHz, $CDCl_3$, TMS as an internal standard): $\delta$ 2.17-2.22 (m, 7H), 2.29 (dt, J=6.9, 1.6 Hz, 1H), 3, 81-3.85 (m, 2H), 6.77 (t, J=2.0 Hz, 2H), 7.03 (s, 2H).

<Synthesis of compound 20>

A compound 20 shown below was synthesized by a method mentioned below.

[Chem. 127]

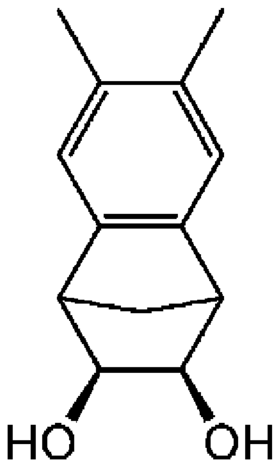

(Compound 20)

To a 500 mL 3-neck flask, 5.57 g (22.8 mmol) of the compound 19, 89 mL of tert-butyl alcohol, and 22 mL of water were added, and the internal temperature was cooled to 0°C under stirring. To another flask, 5.33 g of potassium permanganate, 111 mL of water, and 1.22 g of sodium hydroxide were added and stirred to prepare a potassium permanganate aqueous solution. The potassium permanganate aqueous solution was slowly added dropwise to the solution of the compound 19 prepared previously such that the internal temperature did not exceed 5°C. After the dropwise addition, stirring was continued at 0°C for an hour. A saturated sodium metabisulfite aqueous solution was prepared and slowly added dropwise to the reaction solution until a white precipitate formed. The precipitate formed was removed by filtration, and the filtrate was extracted with ethyl acetate 4 times. The organic layer was washed with brine and dried over magnesium sulfate. The magnesium sulfate was filtered off, and the obtained organic layer was concentrated in a rotary evaporator. The crude product was purified by silica gel column chromatography to give 2.25

g (11.0 mmol) of the compound 20. The $^1$H-NMR data of the compound 20 obtained are shown below.

$^1$H NMR (270 MHz, $CDCl_3$, TMS as an internal standard): δ 1.83-1.90 (m, 1H), 2.17-2.23 (m, 7H), 2.70-2.77 (m, 2H), 3.12-3.17 (m, 2H), 3.77-3.83 (m, 2H), 6.98 (s, 2H).

<Synthesis of compound 21>

A compound 21 shown below was synthesized by a method mentioned below.

[Chem. 128]

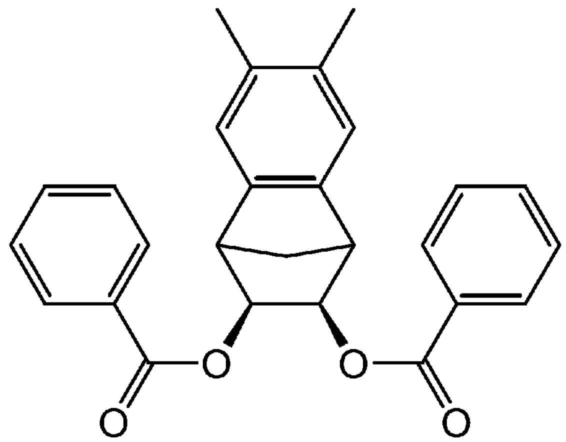

(Compound 21)

Under a nitrogen atmosphere, 2.25 g (11.0 mmol) of the compound 20 and 11.0 mL of dehydrated pyridine were added to a 50 mL 3-neck flask and stirred. Under cooling in an ice bath, 2.7 mL of benzoyl chloride was slowly added thereto. After the addition, the temperature was raised to room temperature, and stirring was conducted overnight. Under cooling in an ice bath again, 5 mL of methanol was added. To a beaker were added 100 mL of water and 100 mL of ethyl acetate to separate the organic layer. The organic layer was washed with water 3 times, once each with a saturated ammonium chloride aqueous solution and brine in the order mentioned, and dried over magnesium sulfate. The magnesium sulfate was filtered, and the filtrate was concentrated in a rotary evaporator to thereby give 4.82 g of a crude product. The crude product was purified by silica gel column chromatography to give 4.44 g (10.8 mmol, pale yellow solid) of a compound 21. The $^1$H-NMR data of the compound 21 obtained are shown below.

$^1$H NMR (270 MHz, $CDCl_3$, TMS as an internal standard): δ 2.09 (d, J = 9.8 Hz, 1H), 2.26 (s, 6H), 2.57 (d, J = 9.8 Hz, 1H), 3.49 (s, 2H), 5.13 (d, J = 1.3 Hz, 2H), 7.13 (s, 2H), 7.20-7.31 (m, 4H), 7.44-7.52 (m, 2H), 7.87-7.93 (m, 4H).

The melting completion temperature of the compound 21 obtained was 152°C.

[Example A9]

<Synthesis of compound 22>

A compound 22 shown below was synthesized by a method mentioned below.

[Chem. 129]

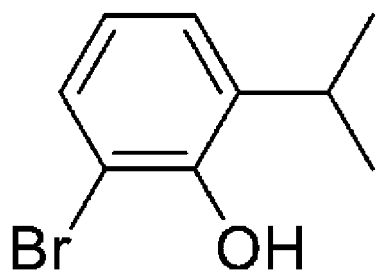

(Compound 22)

A 1 L 3-neck flask including a magnetic stirring bar sufficiently dried by heating was equipped with a dropping

funnel, a thermometer, and a three-way cock. Under a nitrogen atmosphere, 6.00 mL (44.1 mmol, 1 equivalent) of 2-isopropyl phenol was loaded followed by addition of 100 mL of dichloromethane and 0.62 mL (4.41 mmol, 0.1 equivalents) of diisopropylamine. Next, under a nitrogen atmosphere, 8.21 g (46.1 mmol, 1.05 equivalents) of N-bromosuccinimide (NBS) dissolved in 400 mL of dichloromethane was slowly added dropwise to the reaction solution prepared previously under room temperature conditions, and the reaction solution after the dropwise addition finished was stirred for an hour at room temperature. After the reaction finished, hydrochloric acid (2 M) was added until the pH reached 1, and after addition of 100 mL of water, extraction was conducted with dichloromethane 3 times. The gathered organic layer was dried over sodium sulfate and then concentrated in a rotary evaporator. The resultant crude product was purified by silica gel column chromatography (eluent: hexane) to result in 9.40 g (43.7 mmol, yield 910) of the compound 22. The obtained compound exhibited a good agreement with the spectrum of the identical compound synthesized in "J. Med. Chem. 2017, 60, 3618-3625". The $^1$H-NMR data of the compound 22 obtained are shown below.

$^1$H NMR (270 MHz, $CDCl_3$, TMS as an internal standard): δ 1.24 (d, J=6.9 Hz, 6H), 3.32 (sep, J=6.9 Hz, 1H), 5.57 (s, 1H), 6.77 (t, J=7.5 Hz, 1H), 7.14 (dd, J=7.5, 1.7 Hz, 1H) 7.29 (dd, J=7.5, 1.7 Hz, 1H).

Synthesis reference: Bull. Chem. Soc. Jpn. 1993, 66, 1576-1579.

<Synthesis of compound 23>

A compound 23 shown below was synthesized by a method mentioned below.

[Chem. 130]

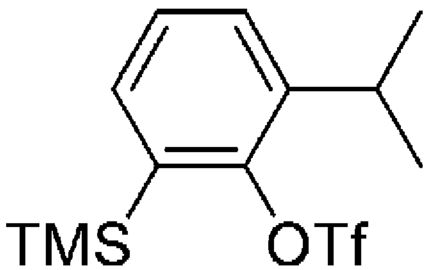

(Compound 23)

A 500 mL 3-neck flask including a magnetic stirring bar sufficiently dried by heating was equipped with a reflux condenser, a flat plug, and a three-way cock. Under a nitrogen atmosphere, 9.40 g (43.7 mmol, 1 equivalent) of the compound 22, 85 mL of THF, and 12.0 mL (56.8 mmol, 1.3 equivalents) of 1,1,1,3,3,3-hexamethyldisilazane (HMDS) were added thereto, the flat plug was replaced with a thermometer, followed by stirring under heating in an oil bath at 80°C. After stirring overnight followed by allowing to cool to room temperature, the pressure was reduced under a nitrogen atmosphere, and THF and unreacted HMDS were removed from the reaction system. The product in this reaction was not purified and used as was in the following reaction.

The container including the product of the reaction described above was equipped with a dropping funnel and, after addition of 120 mL of THF, cooled to -78°C. 38.3 mL (1.6 M, 61.2 mmol, 1.4 equivalents) of a solution of n-BuLi in hexane was slowly added dropwise, and after the dropwise addition finished, stirring was conducted at -78°C for 30 minutes. Subsequently, 10.0 mL (61.0 mmol, 1.4 equivalents) of trifluoromethanesulfonic anhydride ($Tf_2O$) was slowly added dropwise under -78°C conditions. After the dropwise addition finished, stirring was conducted at -78°C for 30 minutes, and then the reaction solution was allowed to reach room temperature. The reaction solution was cooled again to 0°C, and a saturated sodium hydrogen carbonate aqueous solution was slowly added until the pH reached approximately 7 to 8. The solution was extracted with ethyl acetate 3 times, and the gathered organic layer was dried over sodium sulfate and then concentrated in a rotary evaporator. The resultant crude product was purified by silica gel column chromatography (eluent: hexane) to result in 9.93 g (29.2 mmol, yield 67%) of the compound 23. The obtained compound 23 exhibited a good agreement with the spectrum of the identical compound synthesized in "Angew. Chem. Int. Ed. 2011, 50, 5674-5677". The $^1$H-NMR data of the compound 23 obtained are shown below.

$^1$H NMR (270 MHz, $CDCl_3$, TMS as an internal standard): δ 0.38 (s, 9H), 1.24 (d, J=6.9 Hz, 6H), 3.31 (sep, J=6.9 Hz, 1H), 7.31-7.43 (m, 3H).

Synthesis reference: Org. Lett. 2013, 15, 5722-5725.

<Synthesis of compound 24>

A compound 24 shown below was synthesized by a method mentioned below.

[Chem. 131]

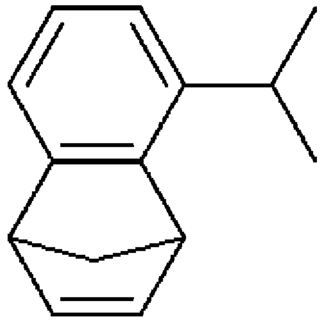

(Compound 24)

A 1 L 3-neck flask including a magnetic stirring bar sufficiently dried by heating was equipped with a reflux condenser, a flat plug, and a three-way cock. Under a nitrogen atmosphere, 22.2 g (146 mmol, 5 equivalents) of cesium fluoride and 290 mL of acetonitrile were loaded thereto. Subsequently, 12.3 mL (146 mmol, 5 equivalents) of cyclopentadiene, obtained by pyrolysis of dicyclopentadiene immediately before, was added to the reaction solution. Immediately thereafter, 9.93 g (29.2 mmol, 1 equivalent) of the compound 23 was added thereto. After the flat plug was replaced with a thermometer, the solution was heated in an oil bath to 40°C and stirred under heating for 16 hours. After the reaction finished followed by allowing to cool to room temperature, the reaction solution was allowed to pass through silica gel (eluent: ethyl acetate) and concentrated in a rotary evaporator. The resultant crude product was purified by silica gel column chromatography (eluent: hexane) to result in 5.32 g of a solution containing the compound 24 and a small amount of impurities. The solution was used in the next reaction without further purification. The $^1$H-NMR data of the compound 24 obtained are shown below.

$^1$H NMR (270 MHz, $CDCl_3$, TMS as an internal standard): δ 1.20 (d, J =6.9 Hz, 3H), 1.28 (d, J=6.9 Hz, 3H), 2.17-2.21 (m, 1H), 2.26-2.30 (m, 1H), 3.13 (sep, J=6.9 Hz, 1H), 3.86-3.88 (m, 1H), 4.08-4.09 (m, 1H), 6.77-6.93 (m, 4H), 7.07 (d, J=6.3 Hz, 1H).

Synthesis reference: Macromolecules 2017, 50, 580-586.

<Synthesis of compound 25>

A compound 25 shown below was synthesized by a method mentioned below.

[Chem. 132]

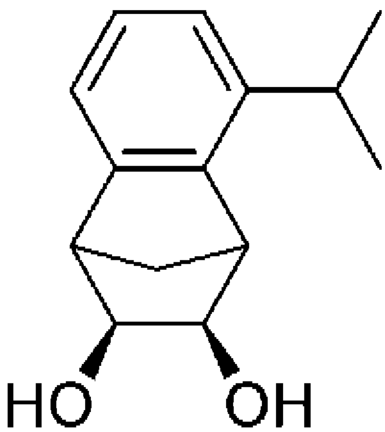

(Compound 25)

A 500 mL 3-neck flask including a magnetic stirring bar was equipped with a dropping funnel, a thermometer, and a three-way cock. Under a nitrogen atmosphere, 120 mL of $^t$BuOH, 35 mL of water, and 5.32 g (1 equivalent) of the mixed solution of the compound 24 obtained in <Synthesis of compound 24> were loaded thereto, and the reaction solution was cooled to 0°C. 1.45 g (36.2 mmol, 1.25 equivalents) of NaOH and 6.86 g (43.4 mmol, 1.5 equivalents) of $KMnO_4$ were dissolved in 130 mL of water, and the solution was slowly added dropwise to the reaction solution. After the dropwise addition finished, stirring was conducted under 0°C conditions for further 30 minutes, and then unreacted

$KMnO_4$ was quenched using a saturated aqueous solution of sodium metabisulfite. After stirring was conducted at room temperature for a while, sodium hydrogen carbonate was added until the pH of the reaction solution reached approximately 7 to 8, and the resulting white precipitate was removed by filtration. The filtered solution was extracted with ethyl acetate 3 times. The gathered organic layer was dried over sodium sulfate and then concentrated in a rotary evaporator. The resultant crude product was purified by silica gel column chromatography (after developed with 1 L of an eluent: hexane, developed with hexane:ethyl acetate = 3:1) to result in 5.31 g (24.3 mmol, yield 84%) of the compound 25. The $^1$H-NMR data of the compound 25 obtained are shown below.

$^1$H NMR (270 MHz, $CDCl_3$, TMS as an internal standard): δ 1.22 (d, J =6.9 Hz, 3H), 1.27 (d, J = 6.9 Hz, 3H), 1.86-1.92 (m, 1H), 2.20-2.24 (m, 1H), 2.71 (d, J = 4.9 Hz, 1H), 2.86 (d, J = 4.9 Hz, 1H), 3.09 (sep, J = 6.9 Hz, 1H), 3.20 (br s, 1H), 3.40 (br s, 1H), 3.78-3.87 (m, 2H), 6.99-7.10 (m, 3H). Synthesis reference: J. Org. Chem. 2017, 82, 9715-9730.

<Synthesis of compound 26>

A compound 26 shown below was synthesized by a method mentioned below.

[Chem. 133]

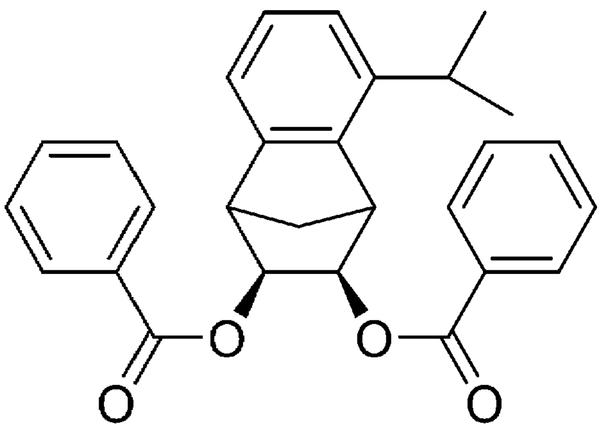

(Compound 26)

A 100 mL 3-neck flask including a magnetic stirring bar sufficiently dried by heating was equipped with a flat plug, a thermometer, and a three-way cock. Under a nitrogen atmosphere, 5.31 g (24.3 mmol, 1 equivalent) of the compound 25 and about 10 mL of pyridine were added thereto, and the flat plug was replaced with a dropping funnel. After the reaction solution was cooled to 0°C, 7.0 mL (60.3 mmol, 2.5 equivalents) of benzoyl chloride was slowly added dropwise. After the dropwise addition finished, the temperature was raised to room temperature, and stirring was conducted overnight. After the reaction finished, the solution was cooled to 0°C, 10 mL of methanol was added, and stirring was conducted for an hour. After addition of about 20 mL of water and about 30 mL of dichloromethane, the solution was extracted with dichloromethane 3 times, and the gathered organic layer was washed with a saturated ammonium chloride aqueous solution twice. After drying over sodium sulfate, the solution was concentrated in a rotary evaporator. The resultant crude product was purified by silica gel column chromatography (eluent: hexane:ethyl acetate = 20:1) and recrystallization with hexane to result in 5.19 g (12.2 mmol, yield 50%, white solid) of the compound 26. The $^1$H-NMR data of the compound 26 obtained are shown below.

$^1$H NMR (270 MHz, $CDCl_3$, TMS as an internal standard): δ 1.32 (d, J =6.9 Hz, 3H), 1.33 (d, J = 6.9 Hz, 3H), 2.10-2.15 (m, 1H), 2.57-2.61 (m, 1H), 3.19 (sep, J = 6.9 Hz, 1H), 3.56 (br s, 1H), 3.75 (br s, 1H), 5.12-5.19 (m, 2H), 7.08-7.17 (m, 3H), 7.22-7.35 (overlapping with signal of $CHCl_3$, m, 4H), 7.45-7.54 (m, 2H), 7.87-7.96 (m, 4H).

The melting completion temperature of the compound 26 obtained was 114°C.

[Example A10]

<Synthesis of compound 27>

A compound 27 shown below was synthesized by a method mentioned below.

[Chem. 134]

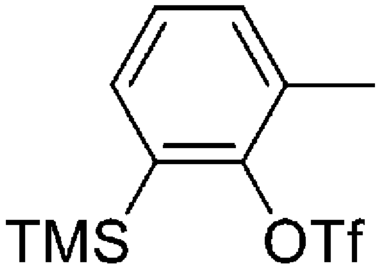

(Compound 27)

7.26 g (23.2 mmol, yield 48%) of the compound 27 was obtained in accordance with the operation and equivalence relationship described in <Synthesis of compound 23> except that 6.00 mL (48.4 mmol) of 6-bromo-o-cresol was used instead of using 9.40 g (43.7 mmol) of the compound 22 in <Synthesis of compound 23>. The obtained compound 27 exhibited a good agreement with the spectrum of the identical compound synthesized in "J. Org. Chem. 2015, 80, 11618-11623". The $^1$H-NMR data of the compound 27 obtained are shown below.

$^1$H NMR (270 MHz, $CDCl_3$, TMS as an internal standard): δ 0.38 (s, 9H), 2.38 (s, 3H), 7.28-7.30 (m, 2H), 7.38-7.47 (m, 1H) .

<Synthesis of compound 28>

A compound 28 shown below was synthesized by a method mentioned below.

[Chem. 135]

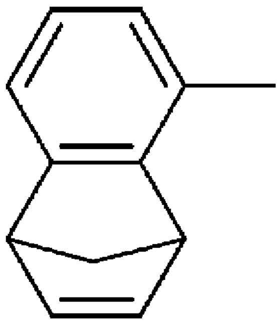

(Compound 28)

3.39 g (17 mmol, yield 74%) of the compound 28 contaminated with a small amount of hexane was obtained in accordance with the operation and equivalence relationship described in <Synthesis of compound 24> except that 7.26 g (23.2 mmol) of the compound 27 was used instead of using 9.93 g (29.2 mmol) of the compound 23 in <Synthesis of compound 24>. The $^1$H-NMR data of the compound 28 obtained are shown below.

$^1$H NMR (270 MHz, $CDCl_3$, TMS as an internal standard): δ 2.17-2.21 (m, 1H), 2.26-2.31 (m, 4H), 3.85-3.88 (m, 1H), 3.99-4.00 (m, 1H), 6.73-6.85 (m, 4H), 7.05 (d, J = 6.9 Hz, 1H) .

<Synthesis of compound 29>

A compound 29 shown below was synthesized by a method mentioned below.

[Chem. 136]

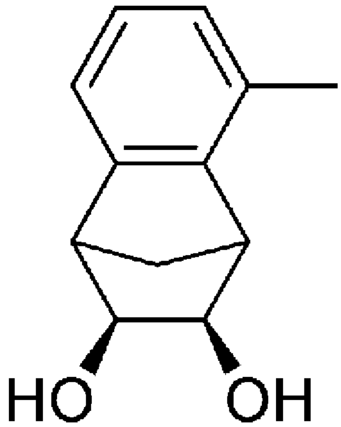

(Compound 29)

A 500 mL 3-neck flask including a magnetic stirring bar was equipped with a dropping funnel, a thermometer, and a three-way cock. Under a nitrogen atmosphere, 70 mL of $^{t}$BuOH, 20 mL of water, and 3.39 g (17 mmol, 1 equivalent) of the compound 28 contaminated with a small amount of hexane obtained in <Synthesis of compound 28> were loaded thereto, and the reaction solution was cooled to 0°C. 0.85 g (21.3 mmol, 1.25 equivalents) of NaOH and 4.03 g (25.5 mmol, 1.5 equivalents) of $KMnO_4$ were dissolved in 80 mL of water, and the solution was slowly added dropwise to the reaction solution. After the dropwise addition finished, stirring was conducted under 0°C conditions for further 30 minutes, and then unreacted $KMnO_4$ was quenched using a saturated aqueous solution of sodium metabisulfite. After stirring was conducted at room temperature for a while, sodium hydrogen carbonate was added until the pH of the reaction solution reached approximately 7 to 8, and the resulting white precipitate was removed by filtration. The filtered solution was extracted with ethyl acetate 3 times. The gathered organic layer was dried over sodium sulfate and then concentrated in a rotary evaporator. The resultant crude product was purified by silica gel column chromatography (eluent: hexane:ethyl acetate = 3:1) to result in 2.19 g (11.5 mmol, yield 68%) of the compound 29. The $^{1}$H-NMR data of the compound 29 obtained are shown below.

$^{1}$H NMR (270 MHz, $CDCl_3$, TMS as an internal standard): δ 1.86-1.91 (m, 1H), 2.19-2.24 (m, 1H), 2.31 (s, 3H), 2.72-2.74 (m, 1H), 2.83-2.85 (m, 1H), 3.19-3.20 (m, 1H), 3.31-3.32 (m, 1H), 3.77-3.84 (m, 2H), 6.89-7.07 (m, 3H).

<Synthesis of compound 30>

A compound 30 shown below was synthesized by a method mentioned below.

[Chem. 137]

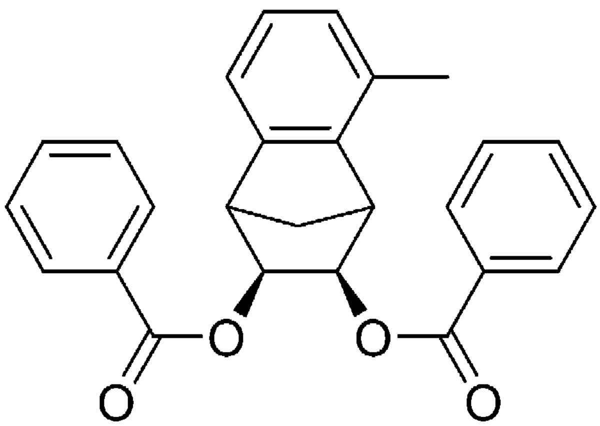

(Compound 30)

A 100 mL 3-neck flask including a magnetic stirring bar sufficiently dried by heating was equipped with a flat plug, a thermometer, and a three-way cock. Under a nitrogen atmosphere, 2.19 g (11.5 mmol, 1 equivalent) of the compound 29 and about 10 mL of pyridine were added thereto, and the flat plug was replaced with a dropping funnel. After the reaction solution was cooled to 0°C, 2.94 mL (25.3 mmol, 2.2 equivalents) of benzoyl chloride was slowly added dropwise. After the dropwise addition finished, the temperature was raised to room temperature, and stirring was conducted for 3 hours. After the reaction finished, the solution was cooled to 0°C, 20 mL of methanol was added, and stirring was conducted for an hour. After addition of about 20 mL of water and about 30 mL of dichloromethane, the solution was extracted with dichloromethane 3 times, and the gathered organic layer was washed with a saturated ammonium chloride aqueous solution twice. After drying over sodium sulfate, the solution was concentrated in a rotary evaporator. The resultant crude product was purified by silica gel column chromatography (eluent: hexane:ethyl acetate = 10:1) to result in 3.19 g (8.01 mmol, yield 70%, white solid) of the compound 30. The $^1$H-NMR data of the compound 30 obtained are shown below.

$^1$H NMR (270 MHz, $CDCl_3$, TMS as an internal standard): $\delta$ 2.09-2.14 (m, 1H), 2.41 (s, 3H), 2.56-2.60 (m, 1H), 3.55 (br s, 1H), 3.67 (br s, 1H), 5.10-5.18 (m, 2H), 6.99-7.17 (m, 3H), 7.24-7.33 (overlapping with signal of $CHCl_3$, m, 4H), 7.45-7.53 (m, 2H), 7.88-7.95 (m, 4H).

The melting completion temperature of the compound 30 obtained was 131°C.

[Example A11]

<Synthesis of compound 31>

A compound 31 shown below was synthesized by a method mentioned below.

[Chem. 138]

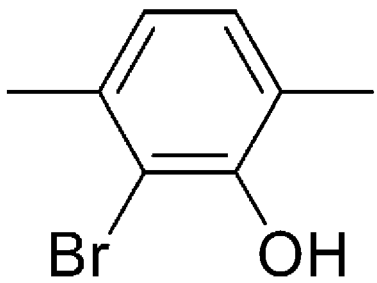

(Compound 31)

10.8 g (53.9 mmol, yield 90%) of the compound 31 was obtained in accordance with the operation and equivalence relationship described in <Synthesis of compound 22> except that 7.33 g (60.0 mmol) of 2,5-dimethylphenol was used instead of using 6.00 mL (44.1 mmol) of 2-isopropylphenol in <Synthesis of compound 22>. The obtained compound exhibited a good agreement with the spectrum of the identical compound synthesized in "Adv. Synth. Catal. 2008, 350, 1309-1315". The $^1$H-NMR data of the compound 31 obtained are shown below.

$^1$H NMR (270 MHz, $CDCl_3$, TMS as an internal standard): $\delta$ 2.27 (s, 3H), 2.36 (s, 3H), 5.65 (s, 1H), 6.71 (d, J = 7.6 Hz, 1H), 6.95 (d, J = 7.6 Hz, 1H).

<Synthesis of compound 32>

A compound 32 shown below was synthesized by a method mentioned below.

[Chem. 139]

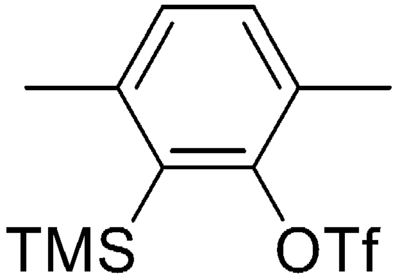

(Compound 32)

13.0 g of the compound 32 containing a small amount of impurities was obtained in accordance with the operation and equivalence relationship described in <Synthesis of compound 23> except that 10.8 g (53.9 mmol) of the

compound 31 was used instead of using 9.40 g (43.7 mmol) of the compound 22 in <Synthesis of compound 23>. The product in this reaction was used in the next reaction without further purification and identification.

<Synthesis of compound 33>

A compound 33 shown below was synthesized by a method mentioned below.

[Chem. 140]

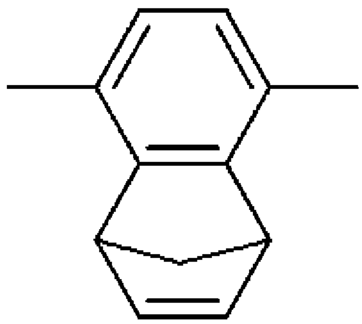

(Compound 33)

5.58 g (32.8 mmol) of the compound 33 was obtained in accordance with the operation and equivalence relationship described in <Synthesis of compound 24> except that 13.0 g (39.9 mmol as 1 equivalent) of the compound 32 containing a small amount of impurities was used instead of using 9.93 g (29.2 mmol) of the compound 23 in <Synthesis of compound 24>. The $^{1}$H-NMR data of the compound 33 obtained are shown below.

$^{1}$H NMR (270 MHz, $CDCl_3$, TMS as an internal standard): δ 2.13-2.17 (m, 1H), 2.23-2.27 (m, 7H), 3.97-4.00 (m, 2H), 6.66 (s, 2H), 6.78-6.79 (m, 2H).

<Synthesis of compound 34>

A compound 34 shown below was synthesized by a method mentioned below.

[Chem. 141]

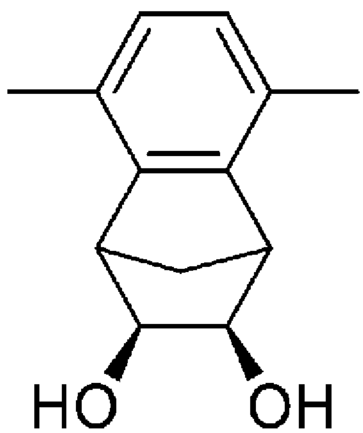

(Compound 34)

A 500 mL 3-neck flask including a magnetic stirring bar was equipped with a dropping funnel, a thermometer, and a three-way cock. Under a nitrogen atmosphere, 135 mL of $^{t}$BuOH, 40 mL of water, and 5.58 g (32.8 mmol, 1 equivalent) of the compound 33 were loaded thereto, and the reaction solution was cooled to 0°C. 1.64 g (41.0 mmol, 1.25 equivalents) of NaOH and 7.78 g (49.2 mmol, 1.5 equivalents) of $KMnO_4$ were dissolved in 150 mL of water, and the solution was slowly added dropwise to the reaction solution. After the dropwise addition finished, stirring was conducted under 0°C conditions for further 30 minutes, and then unreacted $KMnO_4$ was quenched using a saturated aqueous solution of sodium metabisulfite. After stirring was conducted at room temperature for a while, sodium hydrogen carbonate

was added until the pH of the reaction solution reached approximately 7 to 8, and the resulting white precipitate was removed by filtration. The filtered solution was extracted with ethyl acetate 3 times. The gathered organic layer was dried over sodium sulfate and then concentrated in a rotary evaporator. The resultant crude product was purified by silica gel column chromatography (eluent: hexane:ethyl acetate = 5:1) to result in 5.29 g (25.9 mmol, yield 79%) of the compound 34. The $^1$H-NMR data of the compound 34 obtained are shown below.

$^1$H NMR (270 MHz, $CDCl_3$, TMS as an internal standard): δ 1.82-1.87 (m, 1H), 2.18-2.22 (m, 1H), 2.27 (s, 6H), 2.73-2.78 (m, 2H), 3.29-3.03 (m, 2H), 3.76-3.80 (m, 2H), 6.81 (s, 2H).

<Synthesis of compound 35>

A compound 35 shown below was synthesized by a method mentioned below.

[Chem. 142]

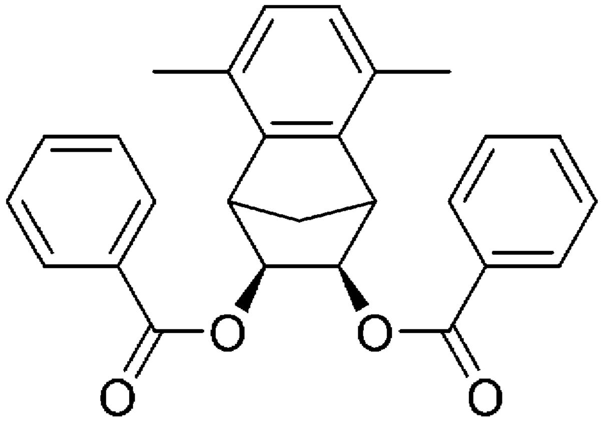

(Compound 35)

A 100 mL 3-neck flask including a magnetic stirring bar sufficiently dried by heating was equipped with a flat plug, a thermometer, and a three-way cock. Under a nitrogen atmosphere, 5.29 g (25.9 mmol, 1 equivalent) of the compound 34 and about 10 mL of pyridine were added thereto, and the flat plug was replaced with a dropping funnel. After the reaction solution was cooled to 0°C, 6.60 mL (57.0 mmol, 2.2 equivalents) of benzoyl chloride was slowly added dropwise. After the dropwise addition finished, the temperature was raised to room temperature, and stirring was conducted for 4 hours. After the reaction finished, the solution was cooled to 0°C, 20 mL of methanol was added, and stirring was conducted for an hour. After addition of about 20 mL of water and about 30 mL of dichloromethane, the solution was extracted with dichloromethane 3 times, and the gathered organic layer was washed with a saturated ammonium chloride aqueous solution twice. After drying over sodium sulfate, the solution was concentrated in a rotary evaporator. The resultant crude product was purified by silica gel column chromatography (eluent: hexane:ethyl acetate = 20:1) and recrystallization with hexane to result in 5.49 g (13.3 mmol, yield 510, white solid) of the compound 35. The $^1$H-NMR data of the compound 35 obtained are shown below.

$^1$H NMR (270 MHz, $CDCl_3$, TMS as an internal standard): δ 2.04-2.10 (m, 1H), 2.36 (s, 6H), 2.54-2.58 (m, 1H), 3.66 (br s, 2H), 5.10-5.11 (m, 2H), 6.91 (s, 2H), 7.26-7.32 (overlapping with signal of $CHCl_3$, m, 4H), 7.46-7.53 (m, 2H), 7.91-7.94 (m, 4H).

The melting completion temperature of the compound 35 obtained was 138°C.

[Example A12]

<Synthesis of compound 36>

A compound 36 shown below was synthesized by a method mentioned below.

[Chem. 143]

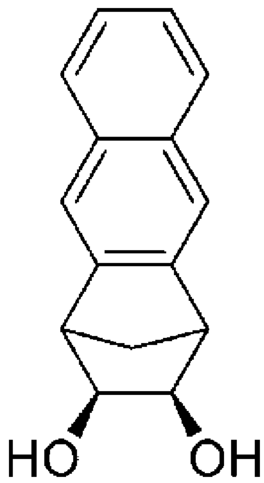

(Compound 36)

A 500 mL 3-neck flask including a magnetic stirring bar was equipped with a dropping funnel, a thermometer, and a three-way cock. Under a nitrogen atmosphere, 110 mL of $^{t}$BuOH, 35 mL of water, and 5.00 g (26.0 mmol, 1 equivalent) of 1,4-dihydro-1,4-methanoanthracene were loaded thereto, and the reaction solution was cooled to 0°C. 1.30 g (32.5 mmol, 1.25 equivalents) of NaOH and 6.16 g (39.0 mmol, 1.5 equivalents) of $KMnO_4$ were dissolved in 120 mL of water, and the solution was slowly added dropwise to the reaction solution. After the dropwise addition finished, stirring was conducted under 0°C conditions for further an hour, and then unreacted $KMnO_4$ was quenched using a saturated aqueous solution of sodium metabisulfite. After stirring was conducted at room temperature for a while, sodium hydrogen carbonate was added until the pH of the reaction solution reached approximately 7 to 8, and the resulting white precipitate was removed by filtration. The filtered solution was extracted with ethyl acetate 3 times. The gathered organic layer was dried over sodium sulfate and then concentrated in a rotary evaporator. The resultant crude product was purified by silica gel column chromatography (eluent: hexane:ethyl acetate = 10:1) to result in 2.57 g (11.4 mmol, yield 44%) of the compound 36. The $^{1}$H-NMR data of the compound 36 obtained are shown below.

$^{1}$H NMR (270 MHz, $CDCl_3$, TMS as an internal standard): $\delta$ 1.94-1.99 (m, 1H), 2.33-2.37 (m, 1H), 2.82 (br s, 2H), 3.37 (s, 2H), 3.92 (br s, 2H), 7.39-7.43 (m, 2H), 7.60 (br s, 2H), 7.74-7.77 (m, 2H).

<Synthesis of compound 37>

A compound 37 shown below was synthesized by a method mentioned below.

[Chem. 144]

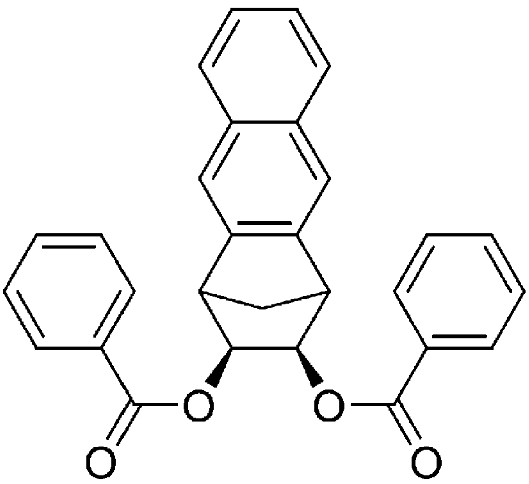

(Compound 37)

A 100 mL 3-neck flask including a magnetic stirring bar sufficiently dried by heating was equipped with a flat

plug, a thermometer, and a three-way cock. Under a nitrogen atmosphere, 2.57 g (11.4 mmol, 1 equivalent) of the compound 36 and about 10 mL of pyridine were added thereto, and the flat plug was replaced with a dropping funnel. After the reaction solution was cooled to 0°C, 2.90 mL (25.1 mmol, 2.2 equivalents) of benzoyl chloride was slowly added dropwise. After the dropwise addition finished, the temperature was raised to room temperature, and stirring was conducted overnight. After the reaction finished, the solution was cooled to 0°C, 10 mL of methanol was added, and stirring was conducted for an hour. After addition of about 20 mL of water and about 30 mL of dichloromethane, the solution was extracted with dichloromethane 3 times, and the gathered organic layer was washed with a saturated ammonium chloride aqueous solution twice. After drying over sodium sulfate, the solution was concentrated in a rotary evaporator. The resultant crude product was purified by silica gel column chromatography twice (eluent: first time: hexane:ethyl acetate = 20:1, then ethyl acetate only, second time: hexane:ethyl acetate = 5:1) and washing with hexane to result in 3.09 g (7.1 mmol, yield 62%, pale yellow solid) of the compound 37. The $^1$H-NMR data of the compound 37 obtained are shown below.

$^1$H NMR (270 MHz, $CDCl_3$, TMS as an internal standard): δ 2.19 (d, J =9.9 Hz, 1H), 2.71 (d, J = 9.2 Hz, 1H), 3.72 (br s, 2H), 5.26 (m, 2H), 7.27-7.32 (m, 4H), 7.45-7.53 (m, 4H), 7.76 (br s, 2H), 7.80-7.83 (m, 2H), 7.91-7.94 (m, 4H).

The melting completion temperature of the compound 37 obtained was 164°C.

[Example A13]

<Synthesis of compound 38>

A compound 38 shown below was synthesized by a method mentioned below.

[Chem. 145]

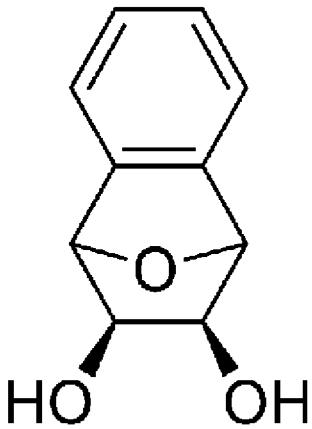

(Compound 38)

A 500 mL 3-neck flask including a magnetic stirring bar was equipped with a dropping funnel, a thermometer, and a three-way cock. Under a nitrogen atmosphere, 150 mL of $^t$BuOH, 50 mL of water, and 4.33 g (30.0 mmol, 1 equivalent) of 1,4-epoxy-1,4-dihydronaphthalene were loaded thereto, and the reaction solution was cooled to 0°C. 1.50 g (37.5 mmol, 1.25 equivalents) of NaOH and 7.11 g (45.0 mmol, 1.5 equivalents) of $KMnO_4$ were dissolved in 150 mL of water, and the solution was slowly added dropwise to the reaction solution. After the dropwise addition finished, stirring was conducted under 0°C conditions for further an hour, and then unreacted $KMnO_4$ was quenched using a saturated aqueous solution of sodium metabisulfite. After stirring was conducted at room temperature for a while, sodium hydrogen carbonate was added until the pH of the reaction solution reached approximately 7 to 8, and the resulting white precipitate was removed by filtration. The filtered solution was extracted with ethyl acetate 3 times. The gathered organic layer was dried over sodium sulfate and then concentrated in a rotary evaporator. The resultant crude product was purified by silica gel column chromatography (eluent: ethyl acetate) to result in 2.79 g (15.7 mmol, yield 53%) of the compound 38. The $^1$H-NMR data of the compound 38 obtained are shown below.

$^1$H NMR (270 MHz, $CDCl_3$, TMS as an internal standard): δ 2.71-2.77 (m, 2H), 3.93-3.98 (m, 2H), 5.17 (s, 2H), 7.19-7.24 (m, 2H), 7.27-7.31 (m, 2H).

<Synthesis of compound 39>

A compound 39 shown below was synthesized by a method mentioned below.

[Chem. 146]

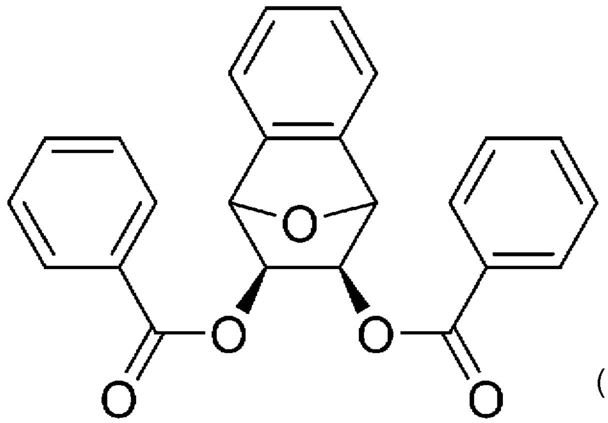

(Compound 39)

A 100 mL 3-neck flask including a magnetic stirring bar sufficiently dried by heating was equipped with a flat plug, a thermometer, and a three-way cock. Under a nitrogen atmosphere, 3.10 g (17.4 mmol, 1 equivalent) of the compound 38 and about 10 mL of pyridine were added thereto, and the flat plug was replaced with a dropping funnel. After the reaction solution was cooled to 0°C, 4.50 mL (38.3 mmol, 2.2 equivalents) of benzoyl chloride was slowly added dropwise. After the dropwise addition finished, the temperature was raised to room temperature, and stirring was conducted overnight. After the reaction finished, the solution was cooled to 0°C, 10 mL of methanol was added, and stirring was conducted for 30 minutes. After addition of about 20 mL of water and about 30 mL of dichloromethane, the solution was extracted with dichloromethane 3 times, and the gathered organic layer was washed with a saturated ammonium chloride aqueous solution twice. After drying over sodium sulfate, the solution was concentrated in a rotary evaporator. The resultant crude product was purified by recrystallization with acetone to result in 3.92 g (10.1 mmol, yield 58%, colorless crystal) of the compound 39. The $^1$H-NMR data of the compound 39 obtained are shown below.

$^1$H NMR (270 MHz, $CDCl_3$, TMS as an internal standard): $\delta$ 5.30 (s, 2H), 5.55 (s, 2H), 7.26-7.33 (overlapping with signal of $CHCl_3$, m, 6H), 7.43-7.54 (m, 4H), 7.94-7.97 (m, 4H).

The melting completion temperature of the compound 39 obtained was 188°C.

[Example A14]

<Synthesis of compound 40>

A compound 40 shown below was synthesized by a method mentioned below.

[Chem. 147]

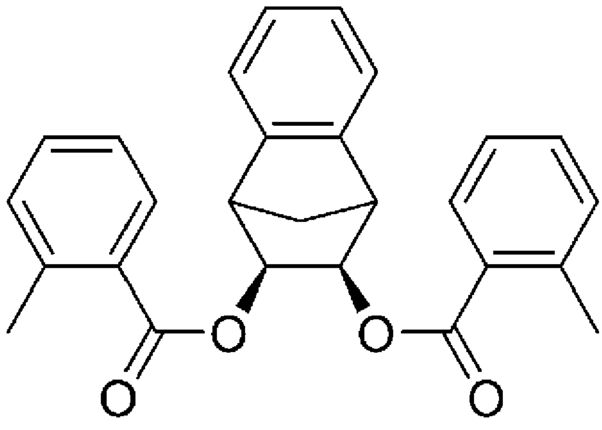

(Compound 40)

A 100 mL 3-neck flask including a magnetic stirring bar sufficiently dried by heating was equipped with a flat plug, a thermometer, and a three-way cock. Under a nitrogen atmosphere, 2.13 g (12.1 mmol, 1 equivalent) of the

compound 5 and about 10 mL of pyridine were added thereto, and the flat plug was replaced with a dropping funnel. After the reaction solution was cooled to 0°C, 3.42 mL (26.8 mmol, 2.2 equivalents) of o-toluoyl chloride was slowly added dropwise. After the dropwise addition finished, the temperature was raised to room temperature, and stirring was conducted overnight. After the reaction finished, the solution was cooled to 0°C, 10 mL of methanol was added, and stirring was conducted for 30 minutes. After addition of about 20 mL of water and about 30 mL of dichloromethane, the solution was extracted with dichloromethane 3 times, and the gathered organic layer was washed with a saturated ammonium chloride aqueous solution twice. After drying over sodium sulfate, the solution was concentrated in a rotary evaporator. The resultant crude product was purified by silica gel column chromatography (eluent: hexane:ethyl acetate = 5:1, then ethyl acetate only) to result in 3.75 g (9.08 mmol, yield 75%, white solid) of the compound 40. The $^1$H-NMR data of the compound 40 obtained are shown below.

$^1$H NMR (270 MHz, $CDCl_3$, TMS as an internal standard): δ 2.13-2.17 (m, 1H), 2.49 (s, 6H), 2.54-2.59 (m, 1H), 3.55 (s, 2H), 5.15-5.16 (m, 2H), 7.02 (t, J = 7.6 Hz, 2H), 7.15-7.20 (m, 4H), 7.30-7.36 (m, 4H), 7.77-7.80 (m, 2H).

The melting completion temperature of the compound 40 obtained was 119°C.

[Example A15]

<Synthesis of compound 41>

A compound 41 shown below was synthesized by a method mentioned below.

[Chem. 148]

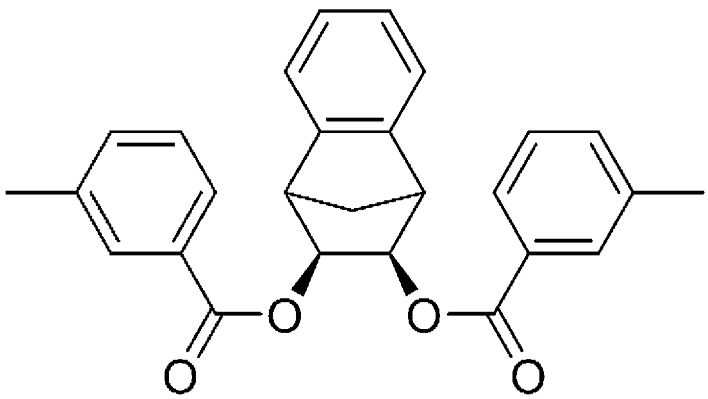

(Compound 41)

A 100 mL 3-neck flask including a magnetic stirring bar sufficiently dried by heating was equipped with a flat plug, a thermometer, and a three-way cock. Under a nitrogen atmosphere, 2.15 g (12.2 mmol, 1 equivalent) of the compound 5 and about 10 mL of pyridine were added thereto, and the flat plug was replaced with a dropping funnel. After the reaction solution was cooled to 0°C, 3.54 mL (26.8 mmol, 2.2 equivalents) of m-toluoyl chloride was slowly added dropwise. After the dropwise addition finished, the temperature was raised to room temperature, and stirring was conducted overnight. After the reaction finished, the solution was cooled to 0°C, 10 mL of methanol was added, and stirring was conducted for 30 minutes. After addition of about 20 mL of water and about 30 mL of dichloromethane, the solution was extracted with dichloromethane 3 times, and the gathered organic layer was washed with a saturated ammonium chloride aqueous solution twice. After drying over sodium sulfate, the solution was concentrated in a rotary evaporator. The resultant crude product was purified by silica gel column chromatography (eluent: hexane:ethyl acetate = 5:1) twice and recrystallization with ethanol to result in 2.60 g (6.30 mmol, yield 52%, white solid) of the compound 41. The $^1$H-NMR data of the compound 41 obtained are shown below.

$^1$H NMR (270 MHz, $CDCl_3$, TMS as an internal standard): δ 2.13-2.21 (m, 7H), 2.59-2.63 (m, 1H), 3.57 (s, 2H), 5.15-5.16 (m, 2H), 7.17-7.24 (m, 4H), 7.29-7.36 (m, 4H), 7.68 (br s, 2H), 7.76-7.79 (m, 2H).

The melting completion temperature of the compound 41 obtained was 84°C.

[Example A16]

<Synthesis of compound 42>

A compound 42 shown below was synthesized by a method mentioned below.

[Chem. 149]

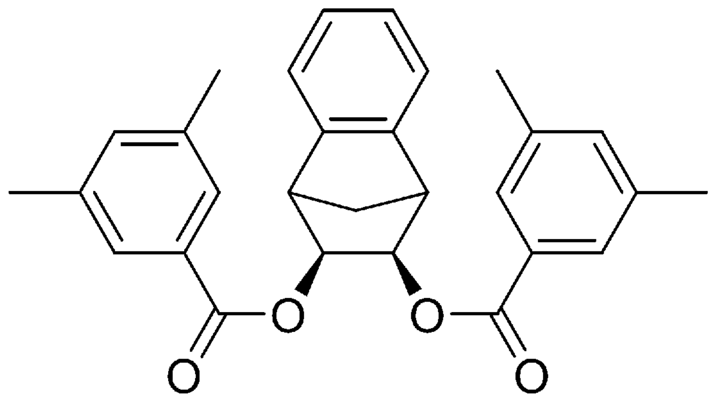

(Compound 42)

A 100 mL 3-neck flask including a magnetic stirring bar sufficiently dried by heating was equipped with a flat plug, a thermometer, and a three-way cock. Under a nitrogen atmosphere, 2.23 g (12.6 mmol, 1 equivalent) of the compound 5 and about 10 mL of pyridine were added thereto, and the flat plug was replaced with a dropping funnel. After the reaction solution was cooled to 0°C, 4.20 mL (28.4 mmol, 2.3 equivalents) of 3,5-dimethylbenzoyl chloride was slowly added dropwise. After the dropwise addition finished, the temperature was raised to room temperature, and stirring was conducted overnight. After the reaction finished, the solution was cooled to 0°C, 10 mL of methanol was added, and stirring was conducted for an hour. After addition of about 20 mL of water and about 30 mL of dichloromethane, the solution was extracted with dichloromethane 3 times, and the gathered organic layer was washed with a saturated ammonium chloride aqueous solution twice. After drying over sodium sulfate, the solution was concentrated in a rotary evaporator. The resultant crude product was purified by silica gel column chromatography twice (eluent: first time: hexane:ethyl acetate = 10:1, second time: hexane:ethyl acetate = 20:1) to result in 5.13 g (11.6 mmol, yield 92%, white solid) of the compound 42. The $^1$H-NMR data of the compound 42 obtained are shown below.

$^1$H NMR (270 MHz, $CDCl_3$, TMS as an internal standard): $\delta$ 2.12-2.19 (m, 13H), 2.59-2.63 (m, 1H), 3.56 (s, 2H), 5.11-5.12 (m, 2H), 7.12 (br s, 2H), 7.17-7.20 (m, 2H), 7.32-7.35 (m, 2H), 7.53 (br s, 4H).

The melting completion temperature of the compound 42 obtained was 118°C.

[Example A17]

<Synthesis of compound 43>

A compound 43 shown below was synthesized by a method mentioned below.

[Chem. 150]

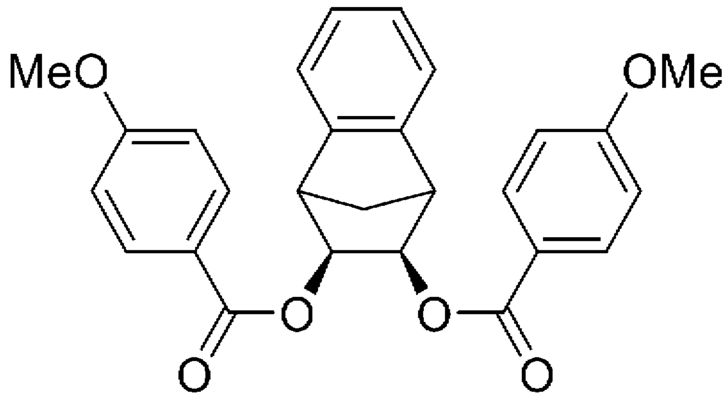

(Compound 43)

A 100 mL 3-neck flask including a magnetic stirring bar sufficiently dried by heating was equipped with a flat plug, a thermometer, and a three-way cock. Under a nitrogen atmosphere, 2.26 g (12.8 mmol, 1 equivalent) of the compound 5 and about 10 mL of pyridine were added thereto. After the reaction solution was cooled to 0°C, 5.00 g (29.3 mmol, 2.3 equivalents) of 4-methoxybenzoyl chloride was slowly loaded. After the loading finished, the temperature was

raised to room temperature, and stirring was conducted overnight. After the reaction finished, the solution was cooled to 0°C, 10 mL of methanol was added, and stirring was conducted for an hour. After addition of about 20 mL of water and about 30 mL of dichloromethane, the solution was extracted with dichloromethane 3 times, and the gathered organic layer was washed with a saturated ammonium chloride aqueous solution twice. After drying over sodium sulfate, the solution was concentrated in a rotary evaporator. The resultant crude product was purified by washing with ethanol to result in 4.67 g (10.5 mmol, yield 82%, pale yellow solid) of the compound 43. The $^1$H-NMR data of the compound 43 obtained are shown below.

$^1$H NMR (270 MHz, $CDCl_3$, TMS as an internal standard): δ 2.11-2.16 (m, 1H), 2.56-2.61 (m, 1H), 3.54 (s, 2H), 3.83 (s, 6H), 5.12-5.13 (m, 2H), 6.75-6.80 (m, 4H), 7.16-7.19 (m, 2H), 7.31-7.34 (m, 2H), 7.84-7.89 (m, 4H).

The melting completion temperature of the compound 43 obtained was 167°C.

[Example A18]

<Synthesis of compound 44>

A compound 44 shown below was synthesized by a method mentioned below.

[Chem. 151]

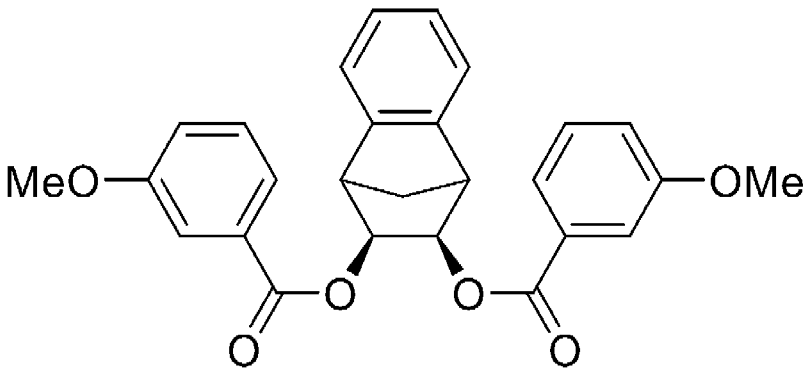

(Compound 44)

A 100 mL 3-neck flask including a magnetic stirring bar sufficiently dried by heating was equipped with a flat plug, a thermometer, and a three-way cock. Under a nitrogen atmosphere, 2.26 g (12.8 mmol, 1 equivalent) of the compound 5 and about 10 mL of pyridine were added thereto, and the flat plug was replaced with a dropping funnel. After the reaction solution was cooled to 0°C, 3.81 mL (27.9 mmol, 2.2 equivalents) of 3-methoxybenzoyl chloride was slowly added dropwise. After the dropwise addition finished, the temperature was raised to room temperature, and stirring was conducted overnight. After the reaction finished, the solution was cooled to 0°C, 10 mL of methanol was added, and stirring was conducted for an hour. After addition of about 20 mL of water and about 30 mL of dichloromethane, the solution was extracted with dichloromethane 3 times, and the gathered organic layer was washed with a saturated ammonium chloride aqueous solution twice. After drying over sodium sulfate, the solution was concentrated in a rotary evaporator. The resultant crude product was purified by silica gel column chromatography (eluent: hexane:ethyl acetate = 10:1) and recrystallization with a two-layer system of acetone-hexane to result in 3.07 g (6.91 mmol, yield 54%, white solid) of the compound 44. The $^1$H-NMR data of the compound 44 obtained are shown below.

$^1$H NMR (270 MHz, $CDCl_3$, TMS as an internal standard): δ 2.13-2.18 (m, 1H), 2.57-2.61 (m, 1H), 3.56 (s, 2H), 3.66 (s, 6H), 5.16-5.17 (m, 2H), 7.01-7.06 (m, 2H), 7.17-7.23 (m, 4H), 7.32-7.35 (m, 2H), 7.41-7.42 (m, 2H), 7.52-7.56 (m, 2H).

The melting completion temperature of the compound 44 obtained was 113°C.

[Example A19]

<Synthesis of compound 45>

A compound 45 shown below was synthesized by a method mentioned below.

[Chem. 152]

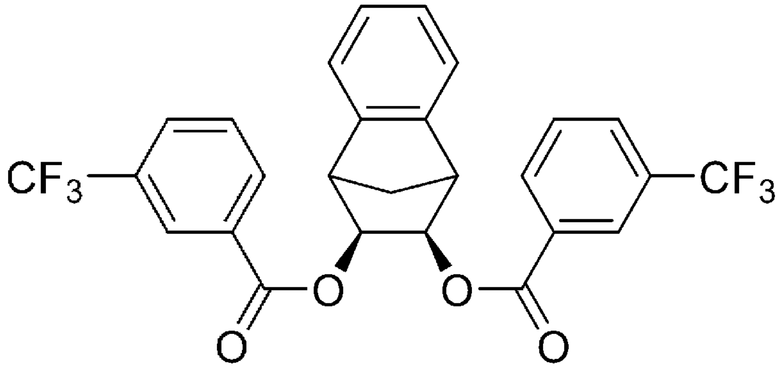

(Compound 45)

A 100 mL 3-neck flask including a magnetic stirring bar sufficiently dried by heating was equipped with a flat plug, a thermometer, and a three-way cock. Under a nitrogen atmosphere, 1.78 g (10.1 mmol, 1 equivalent) of the compound 5 and about 10 mL of pyridine were added thereto, and the flat plug was replaced with a dropping funnel. After the reaction solution was cooled to 0°C, 3.30 mL (22.3 mmol, 2.2 equivalents) of 3-(trifluoromethyl)benzoyl chloride was slowly added dropwise. After the dropwise addition finished, the temperature was raised to room temperature, and stirring was conducted overnight. After the reaction finished, the solution was cooled to 0°C, 10 mL of methanol was added, and stirring was conducted for an hour. After addition of about 20 mL of water and about 30 mL of dichloromethane, the solution was extracted with dichloromethane 3 times, and the gathered organic layer was washed with a saturated ammonium chloride aqueous solution twice. After drying over sodium sulfate, the solution was concentrated in a rotary evaporator. The resultant crude product was purified by silica gel column chromatography (eluent: hexane:ethyl acetate = 10:1) and recrystallization with hexane to result in 3.63 g (6.98 mmol, yield 70%, colorless crystal) of the compound 45. The $^1$H-NMR data of the compound 45 obtained are shown below.

$^1$H NMR (270 MHz, $CDCl_3$, TMS as an internal standard): $\delta$ 2.19-2.24 (m, 1H), 2.60-2.65 (m, 1H), 3.59 (br s, 2H), 5.22-5.23 (m, 2H), 7.19-7.24 (m, 2H), 7.34-7.37 (m, 2H), 7.46 (t, J = 7.6 Hz, 2H), 7.73-7.77 (m, 2H), 8.09-8.12 (m, 4H).

The melting completion temperature of the compound 45 obtained was 124°C.

[Example A20]

<Synthesis of compound 46>

A compound 46 shown below was synthesized by a method mentioned below.

[Chem. 153]

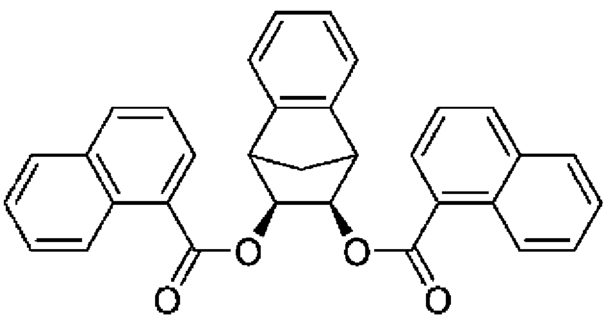

(Compound 46)

A 100 mL 3-neck flask including a magnetic stirring bar sufficiently dried by heating was equipped with a flat plug, a thermometer, and a three-way cock. Under a nitrogen atmosphere, 2.06 g (11.7 mmol, 1 equivalent) of the compound 5 and about 10 mL of pyridine were added thereto, and the flat plug was replaced with a dropping funnel. After the reaction solution was cooled to 0°C, 3.80 mL (25.3 mmol, 2.2 equivalents) of 1-naphthoyl chloride was slowly added dropwise. After the dropwise addition finished, the temperature was raised to room temperature, and stirring was conducted overnight. After the reaction finished, the solution was cooled to 0°C, 10 mL of methanol was added, and

stirring was conducted for an hour. After addition of about 20 mL of water and about 30 mL of dichloromethane, the solution was extracted with dichloromethane 3 times, and the gathered organic layer was washed with a saturated ammonium chloride aqueous solution twice. After drying over sodium sulfate, the solution was concentrated in a rotary evaporator. The resultant crude product was purified by silica gel column chromatography (eluent: hexane:ethyl acetate = 10:1) and washing with hexane to result in 4.73 g (9.76 mmol, yield 85%, white solid) of the compound 46. The $^1$H-NMR data of the compound 46 obtained are shown below.

$^1$H NMR (270 MHz, $CDCl_3$, TMS as an internal standard): δ 2.19-2.24 (m, 1H), 2.65-2.69 (m, 1H), 3.67 (s, 2H), 5.34-5.35 (m, 2H), 7.06-7.12 (m, 2H), 7.21-7.24 (m, 2H), 7.33-7.46 (m, 6H), 7.76-7.80 (m, 2H), 7.86 (d, J = 8.2, 2H), 8.01-8.05 (m, 2H), 8.79-8.82 (m, 2H).

The melting completion temperature of the compound 46 obtained was 158°C.

[Example A21]

<Synthesis of compound 47>

A compound 47 shown below was synthesized by a method mentioned below.

[Chem. 154]

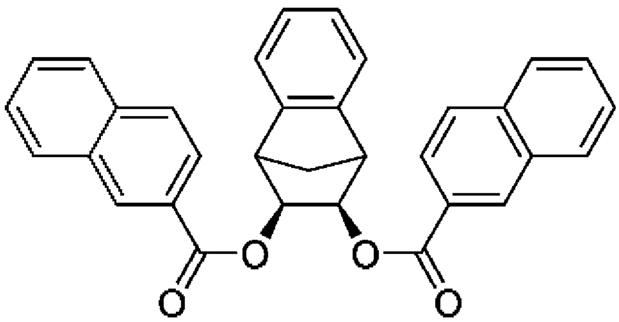

(Compound 47)

A 100 mL 3-neck flask including a magnetic stirring bar sufficiently dried by heating was equipped with a flat plug, a thermometer, and a three-way cock. Under a nitrogen atmosphere, 2.03 g (11.5 mmol, 1 equivalent) of the compound 5 and about 10 mL of pyridine were added thereto. After the reaction solution was cooled to 0°C, 4.80 g (25.2 mmol, 2.2 equivalents) of 2-naphthoyl chloride was slowly loaded. After the loading finished, the temperature was raised to room temperature, and stirring was conducted overnight. After the reaction finished, about 10 mL of dichloromethane was added, and stirring was conducted for further 3 hours. Then the solution was cooled to 0°C, 10 mL of methanol was added, and stirring was conducted for an hour. After addition of about 20 mL of water and about 30 mL of dichloromethane, the solution was extracted with dichloromethane 3 times, and the gathered organic layer was washed with a saturated ammonium chloride aqueous solution twice. After drying over sodium sulfate, the solution was concentrated in a rotary evaporator. The resultant crude product was purified by silica gel column chromatography twice (eluent: first time: hexane:ethyl acetate = 7:1, second time: hexane:ethyl acetate = 5:1) and washing with hexane to result in 2.05 g (4.23 mmol, yield 37%, white solid) of the compound 47. The $^1$H-NMR data of the compound 47 obtained are shown below.

$^1$H NMR (270 MHz, $CDCl_3$, TMS as an internal standard): δ 2.21-2.26 (m, 1H), 2.71-2.76 (m, 1H), 3.67 (br s, 2H), 5.26-5.27 (m, 2H), 7.20-7.23 (m, 2H), 7.30-7.40 (m, 6H), 7.48-7.54 (m, 2H), 7.71 (d, J = 8.6 Hz, 2H), 7.77-7.80 (m, 2H), 7.96-8.00 (m, 2H), 8.39 (br s, 2H).

The melting completion temperature of the compound 47 obtained was 201°C.

[Example A22]

<Synthesis of compound 48>

A compound 48 shown below was synthesized by a method mentioned below.

[Chem. 155]

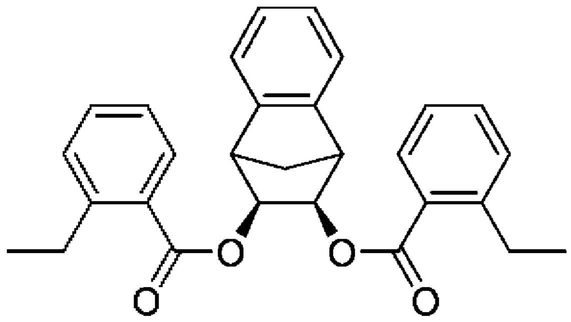

(Compound 48)

A 100 mL 3-neck flask including a magnetic stirring bar sufficiently dried by heating was equipped with a flat plug, a thermometer, and a three-way cock. Under a nitrogen atmosphere, 2.15 g (12.2 mmol, 1 equivalent) of the compound 5 and about 10 mL of pyridine were added thereto, and the flat plug was replaced with a dropping funnel. After the reaction solution was cooled to 0°C, 4.00 mL (26.8 mmol, 2.2 equivalents) of 2-ethylbenzoyl chloride was slowly added dropwise. After the dropwise addition finished, the temperature was raised to room temperature, and stirring was conducted overnight. After the reaction finished, the solution was cooled to 0°C, 10 mL of methanol was added, and stirring was conducted for 30 minutes. After addition of about 20 mL of water and about 30 mL of dichloromethane, the solution was extracted with dichloromethane 3 times, and the gathered organic layer was washed with a saturated ammonium chloride aqueous solution twice. After drying over sodium sulfate, the solution was concentrated in a rotary evaporator. The resultant crude product was purified by silica gel column chromatography (eluent: hexane:ethyl acetate = 20:1) to result in 4.34 g (9.85 mmol, yield 810, white solid) of the compound 48. The $^1$H-NMR data of the compound 48 obtained are shown below.

$^1$H NMR (270 MHz, $CDCl_3$, TMS as an internal standard): δ 1.15 (t, J = 7.3 Hz, 6H), 2.13-2.17 (m, 1H), 2.54-2.57 (m, 1H), 2.89 (q, J = 7.3 Hz, 4H), 3.54 (br s, 2H), 5.16 (br s, 2H), 7.01 (t, J = 7.6 Hz, 2H), 7.17-7.21 (m, 4H), 7.33-7.38 (m, 4H), 7.73 (d, J = 8.2Hz, 2H).

The melting completion temperature of the compound 48 obtained was 52°C.

[Example A23]

<Synthesis of compound 49>

A compound 49 shown below was synthesized by a method mentioned below.

[Chem. 156]

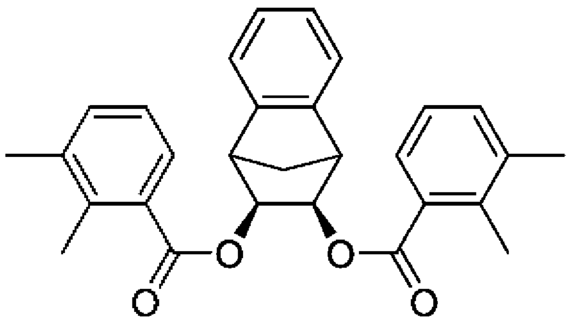

(Compound 49)

A 100 mL 3-neck flask including a magnetic stirring bar sufficiently dried by heating was equipped with a flat plug, a thermometer, and a three-way cock. Under a nitrogen atmosphere, 2.19 g (12.4 mmol, 1 equivalent) of the compound 5 and about 10 mL of pyridine were added thereto, and the flat plug was replaced with a dropping funnel. After the reaction solution was cooled to 0°C, 4.50 g (26.7 mmol, 2.2 equivalents) of 2,3-dimethylbenzoyl chloride was slowly added dropwise. After the dropwise addition finished, the temperature was raised to room temperature, and stirring was conducted overnight. After the reaction finished, the solution was cooled to 0°C, 10 mL of methanol was added, and stirring was conducted for an hour. After addition of about 20 mL of water and about 30 mL of dichloromethane, the solution was extracted with dichloromethane 3 times, and the gathered organic layer was washed with a saturated

ammonium chloride aqueous solution twice. After drying over sodium sulfate, the solution was concentrated in a rotary evaporator. The resultant crude product was purified by silica gel column chromatography (eluent: hexane:ethyl acetate = 20:1) and recrystallization with hexane to result in 4.62 g (10.5 mmol, yield 85%, white solid) of the compound 49. The $^1$H-NMR data of the compound 49 obtained are shown below.

$^1$H NMR (270 MHz, $CDCl_3$, TMS as an internal standard): δ 2.10-2.31 (m, 13H), 2.51-2.55 (m, 1H), 3.53-3.54 (m, 2H), 5.13-5.14 (m, 2H), 6.88-6.95 (m, 2H), 7.17-7.23 (m, 4H), 7.33-7.36 (m, 2H), 7.49-7.52 (m, 2H).

The melting completion temperature of the compound 49 obtained was 107°C.

[Example A24]

<Synthesis of compound 50>

A compound 50 shown below was synthesized by a method mentioned below.

[Chem. 157]

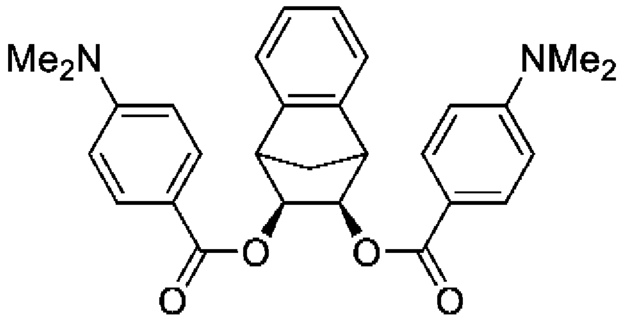

(Compound 50)

4.34 g (pale yellow solid) of the compound 50 was obtained in accordance with the operation and equivalence relationship described in <Synthesis of compound 6> except that 10 g of p-dimethylaminobenzoyl chloride was used instead of using 13.4 mL of benzoyl chloride and the reaction was conducted at the reaction temperature after addition of the reagents of 115°C for 7 days in <Synthesis of compound 6>. The $^1$H-NMR data of the compound 50 obtained are shown below.

$^1$H NMR (270 MHz, $CDCl_3$, TMS as an internal standard): δ 2.07-2.12 (m, 1H), 2.57-2.60 (m, 1H), 3.00 (s, 12H), 3.52 (br s, 2H), 5.10-5.11 (m, 2H), 6.52 (d, J = 8.9 Hz, 4H), 7.14-7.17 (m, 2H), 7.30-7.33 (m, 2H), 7.81 (d, J = 8.9 Hz, 4H).

The melting completion temperature of the compound 50 obtained was 252°C.

[Example A25]

<Synthesis of compound 51>

A compound 51 shown below was synthesized by a method mentioned below.

[Chem. 158]

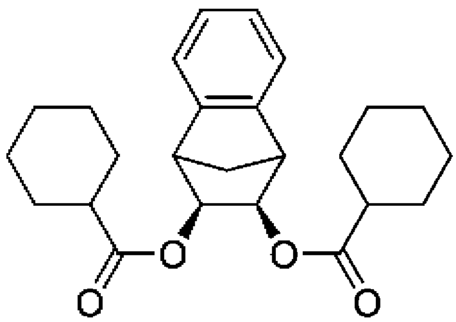

(Compound 51)

A 100 mL 3-neck flask including a magnetic stirring bar sufficiently dried by heating was equipped with a flat plug, a thermometer, and a three-way cock. Under a nitrogen atmosphere, 2.80 g (15.9 mmol, 1 equivalent) of the compound 5 and about 10 mL of pyridine were added thereto, and the flat plug was replaced with a dropping funnel. After the reaction solution was cooled to 0°C, 4.38 mL (32.3 mmol, 2 equivalents) of cyclohexanecarbonyl chloride was slowly added dropwise. After the dropwise addition finished, the temperature was raised to room temperature, and stirring was conducted overnight. After the reaction finished, the solution was cooled to 0°C, 20 mL of methanol was added, and stirring was conducted for an hour. After addition of about 20 mL of water and about 30 mL of dichloromethane, the solution was extracted with dichloromethane 3 times, and the gathered organic layer was washed with a saturated ammonium chloride aqueous solution twice. After drying over sodium sulfate, the solution was concentrated in a rotary evaporator. The resultant crude product was purified by silica gel column chromatography (eluent: hexane:ethyl acetate = 20:1) to result in 4.57 g (11.5 mmol, yield 72%, white solid) of the compound 51. The $^1$H-NMR data of the compound 51 obtained are shown below.

$^1$H NMR (270 MHz, $CDCl_3$, TMS as an internal standard): δ 1.19-1.53 (m, 10H), 1.62-1.83 (m, 6H), 1.89-2.01 (m, 5H), 2.26-2.35 (m, 3H), 3.31-3.32 (m, 2H), 4.75-4.76 (m, 2H), 7.10-7.13 (m, 2H), 7.23-7.27 (overlapping with signal of $CHCl_3$, m, 2H).

The melting completion temperature of the compound 51 obtained was 80°C.

[Example A26]

<Synthesis of compound 52>

A compound 52 shown below was synthesized by a method mentioned below.

[Chem. 159]

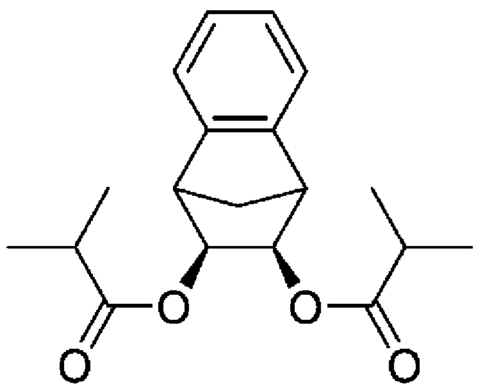

(Compound 52)

A 100 mL 3-neck flask including a magnetic stirring bar sufficiently dried by heating was equipped with a flat plug, a thermometer, and a three-way cock. Under a nitrogen atmosphere, 3.22 g (18.3 mmol, 1 equivalent) of the compound 5 and about 10 mL of pyridine were added thereto, and the flat plug was replaced with a dropping funnel. After the reaction solution was cooled to 0°C, 4.25 mL (40.3 mmol, 2.2 equivalents) of isobutyryl chloride was slowly added dropwise. After the dropwise addition finished, the temperature was raised to room temperature, and stirring was conducted overnight. After the reaction finished, the solution was cooled to 0°C, 20 mL of methanol was added, and stirring was conducted for 40 minutes. After addition of about 20 mL of water and about 30 mL of dichloromethane, the solution was extracted with dichloromethane 3 times, and the gathered organic layer was washed with a saturated ammonium chloride aqueous solution twice. After drying over sodium sulfate, the solution was concentrated in a rotary evaporator. The resultant crude product was purified by silica gel column chromatography (eluent: hexane:ethyl acetate = 15:1) to result in 5.70 g (18.0 mmol, yield 98%, pale yellow liquid) of the compound 52. The $^1$H-NMR data of the compound 52 obtained are shown below.

$^1$H NMR (270 MHz, $CDCl_3$, TMS as an internal standard): δ 1.17-1.22 (m, 12H), 1.98-2.03 (m, 1H), 2.30-2.34 (m, 1H), 2.57 (sep, J = 6.9 Hz, 2H), 3.32-3.33 (m, 2H), 4.77-4.78 (m, 2H), 7.11-7.16 (m, 2H), 7.23-7.27 (overlapping with signal of $CHCl_3$, m, 2H).

[Example A27]

<Synthesis of compound 53>

A compound 53 shown below was synthesized by a method mentioned below according to the following reaction formula.

[Chem. 160]

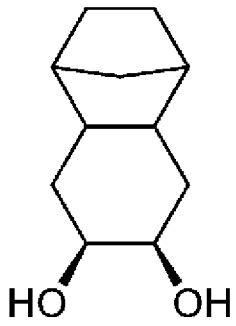

(Compound 53)

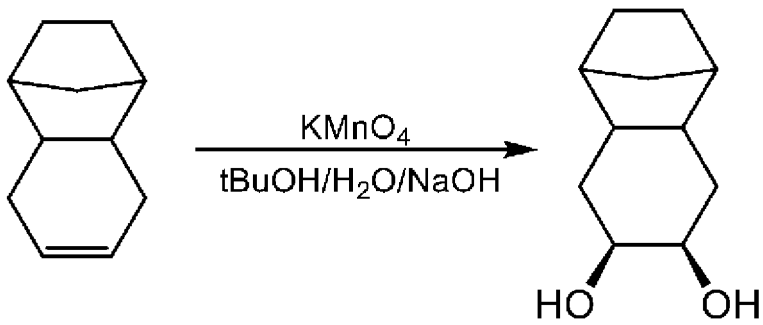


To a 1 L 3-neck flask, 14.8 g of tricyclo[6,2,1,0(2,7)]undec-4-ene, 500 mL of tert-butyl alcohol, and 100 mL of water were added, and the internal temperature was cooled to 0°C under stirring. To another flask, 23.7 g of potassium permanganate, 400 mL of water, and 4.8 g of sodium hydroxide were added and stirred to prepare a potassium permanganate aqueous solution. The potassium permanganate aqueous solution was added dropwise to the tricycloundecene solution prepared previously such that the internal temperature did not exceed 5°C. After the dropwise addition, stirring was continued at 0°C for an hour. Thereafter, a saturated sodium thiosulfate aqueous solution was added dropwise until the red purple color of the water layer disappeared. The precipitate formed was removed by filtration, and tert-butyl alcohol was distilled off under reduced pressure from the filtrate. Thereafter, the filtrate was extracted with ethyl acetate 3 times. The organic layer was washed with brine, dried over sodium sulfate, and then concentrated in a rotary evaporator. A compound 53 was continuously allowed to react without purification.

<Synthesis of compound 54>

A compound 54 shown below was synthesized by a method mentioned below according to the following reaction formula.

[Chem. 161]

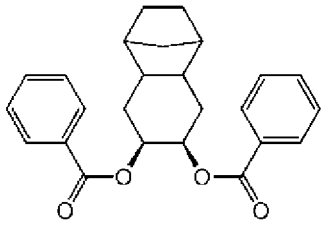

(Compound 54)

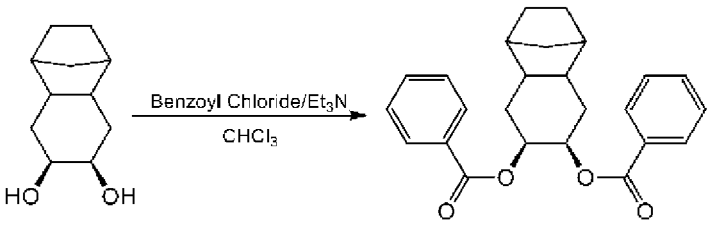


Under a nitrogen atmosphere, 5.5 g of the compound 53, 6.4 g of triethylamine, and 100 mL of chloroform were added to a 300 mL 3-neck flask and cooled to 0°C using an ice bath under stirring. 8.9 g of benzoyl chloride was added such that the internal temperature did not exceed 5°C. After the addition, the temperature was raised to room temperature, and stirring was conducted overnight. After disappearance of the raw material was confirmed by liquid chromatography, the solution was cooled again in an ice bath, and 5 mL of methanol was added thereto. Chloroform and water were added to the reaction solution to fractionate the organic layer, and the water layer was extracted with chloroform 3 times. The organic layer was washed with brine, dried over sodium sulfate, and then concentrated in a rotary evaporator. The reaction mixture was purified by silica gel column chromatography (eluent: hexane/ethyl acetate = 10/1) to result in 3.7 g (yield: 310, liquid) of the compound 54. The $^1$H-NMR data of the compound 54 obtained are shown below.

$^1$H NMR (270 MHz, $CDCl_3$, TMS as an internal standard): δ 7.97-7.91 (m, 4H), 7.55-7.47 (m, 2H), 7.39-7.33 (m, 4H), 5.49-5.47 (m, 2H), 2.19-1.14 (m, 14H).

[Example A28]

<Synthesis of compound 55>

A compound 55 shown below was synthesized by a method mentioned below according to the following reaction formula.

[Chem. 162]

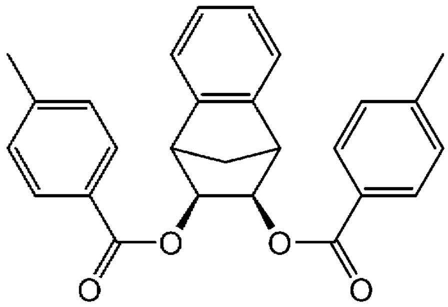

(Compound 55)

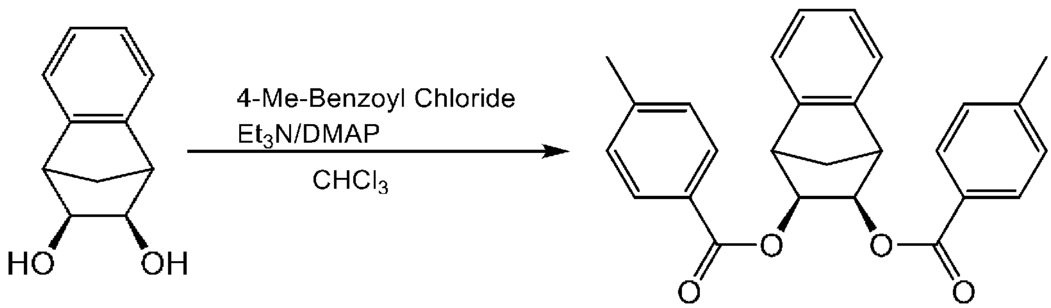


Under a nitrogen atmosphere, 3 g of the compound 5, 5.2 g of triethylamine, 0.21 g of 4-dimethylaminopyridine (DMAP), and 100 mL of chloroform were added to a 300 mL 3-neck flask and cooled to 0°C using an ice bath under stirring. 7.9 g of 4-methylbenzoyl chloride was added such that the internal temperature did not exceed 5°C. After the addition, the temperature was raised to room temperature, and stirring was conducted overnight. After disappearance of the raw material was confirmed by liquid chromatography, the solution was cooled again in an ice bath, and 5 mL of methanol was added thereto. Chloroform and water were added to the reaction solution to fractionate the organic layer, and the water layer was extracted with chloroform 3 times. The organic layer was washed with brine, dried over sodium sulfate, and then concentrated in a rotary evaporator. The reaction mixture was purified by silica gel column chromatography (eluent: hexane/ethyl acetate = 10/1) to give 4.3 g (10.4 mmol, yield: 61%, white powder) of the compound 55. The $^1$H-NMR data of the compound 55 obtained are shown below.

$^1$H NMR (270 MHz, $CDCl_3$, TMS as an internal standard): $\delta$ 7.80 (d, J=8.4Hz, 4H), 7.33 (dd, J=5.1Hz, 3.2Hz, 2H), 7.18 (dd, J=5.4Hz, 3.0HzHz, 2H), 7.09 (d, J=8.4Hz, 4H), 5.14 (d, J=1.6Hz, 2H), 3.55 (s, 2H), 2.59 (d, J=9.5Hz, 1H), 2.38 (s, 6H), 2.14 (d, J=9.7Hz, 1H).

The melting completion temperature of the compound 55 obtained was 186°C.

[Example A29]

<Synthesis of compound 56>

A compound 56 shown below was synthesized by a method mentioned below according to the following reaction formula.

[Chem. 163]

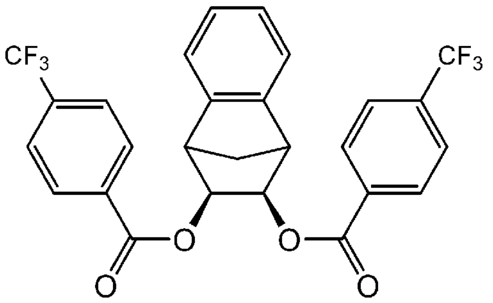

(Compound 56)

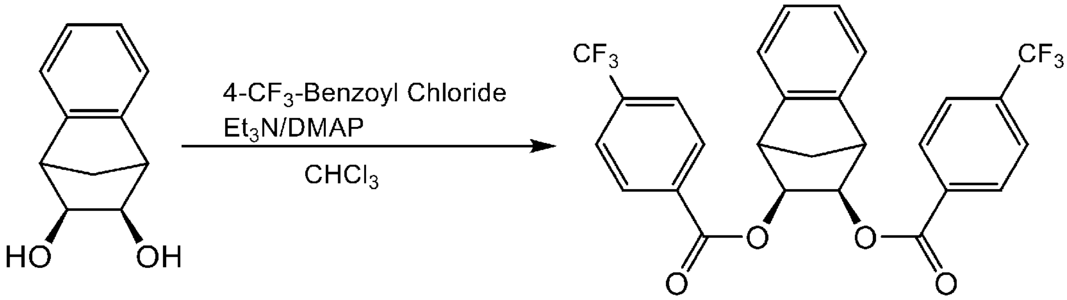


Under a nitrogen atmosphere, 3 g of the compound 5, 5.2 g of triethylamine, 0.21 g of 4-dimethylaminopyridine (DMAP), and 100 mL of chloroform were added to a 300 mL 3-neck flask and cooled to 0°C using an ice bath under stirring. 10.7 g of 4-trifluoromethylbenzoyl chloride was added such that the internal temperature did not exceed 5°C. After the addition, the temperature was raised to room temperature, and stirring was conducted overnight. After disappearance of the raw material was confirmed by liquid chromatography, the solution was cooled again in an ice bath, and 5 mL of methanol was added thereto. Chloroform and water were added to the reaction solution to fractionate the organic layer, and the water layer was extracted with chloroform 3 times. The organic layer was washed with brine, dried over sodium sulfate, and then concentrated in a rotary evaporator. The reaction mixture was purified by silica gel column chromatography (eluent: hexane/ethyl acetate = 10/1) to give 4.8 g (9.2 mmol, yield: 54%, white powder) of the compound 56. The $^{1}$H-NMR data of the compound 56 obtained are shown below.

$^{1}$H NMR (270 MHz, $CDCl_3$, TMS as an internal standard): δ 7.96 (d, J=8.4Hz, 4H), 7.54 (d, J=8.4Hz, 4H), 7.35 (dd, J=5.4Hz, 3.0Hz, 2H), 7.21 (dd, J=5.4Hz, 3.0Hz, 2H), 5.22 (d, J=1.4Hz, 2H), 3.58 (s, 2H), 2.60 (d, J=10.0Hz, 1H), 2.14 (d, J=10.3Hz, 1H).

The melting completion temperature of the compound 56 obtained was 146°C.

[Example A30]

<Synthesis of compound 57>

A compound 57 shown below was synthesized by a method mentioned below according to the following reaction formula.

[Chem. 164]

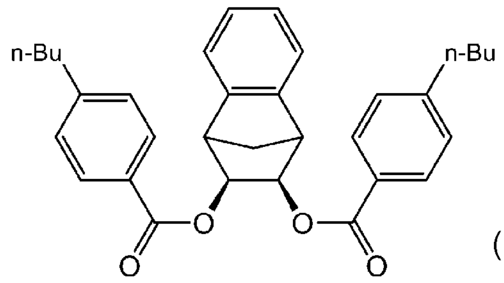

(Compound 57)

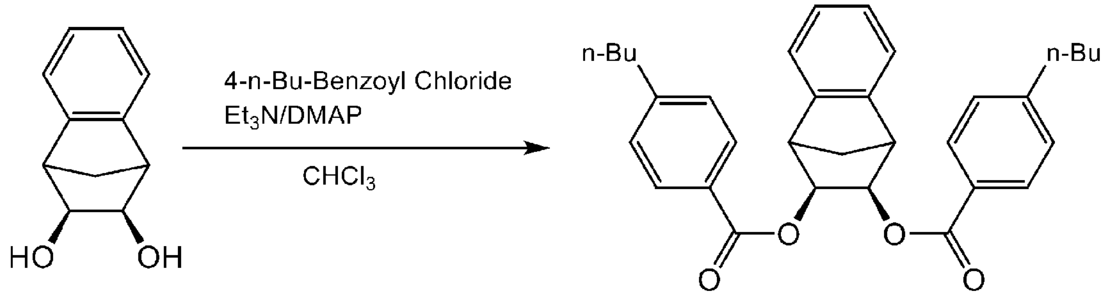


Under a nitrogen atmosphere, 3 g of the compound 5, 5.17 g of triethylamine, 0.21 g of 4-dimethylaminopyridine (DMAP), and 100 mL of chloroform were added to a 300 mL 3-neck flask and cooled to 0°C using an ice bath under stirring. 10.0 g of 4-butylbenzoyl chloride was added such that the internal temperature did not exceed 5°C. After the addition, the temperature was raised to room temperature, and stirring was conducted overnight. After disappearance of the raw material was confirmed by liquid chromatography, the solution was cooled again in an ice bath, and 5 mL of methanol was added thereto. Chloroform and water were added to the reaction solution to fractionate the organic layer, and the water layer was extracted with chloroform 3 times. The organic layer was washed with brine, dried over sodium sulfate, and then concentrated in a rotary evaporator. The reaction mixture was purified by silica gel column chromatography (eluent: hexane/ethyl acetate = 10/1) to give 3.8 g (7.7 mmol, yield: 45%, white powder) of the compound 57. The $^1$H-NMR data of the compound 57 obtained are shown below.

$^1$H NMR (270 MHz, $CDCl_3$, TMS as an internal standard): $\delta$ 7.81 (d, J=8.4Hz, 4H), 7.33 (dd, J=5.1Hz, 3.2Hz, 2H), 7.18 (dd, J=5.4Hz, 3.2Hz, 2H), 7.07 (d, J=8.4Hz, 4H), 5.14 (d, J=1.6Hz, 2H), 3.55 (s, 2H), 2.65-2.57 (m, 5H), 2.14 (d, J=9.7Hz, 1H), 1.64-1.53 (m, 4H), 1.41-1.27 (m, 4H), 0.93 (t, J=7.3Hz, 6H).

The melting completion temperature of the compound 57 obtained was 86°C.

[Example A31]

<Synthesis of compound 58>

A compound 58 shown below was synthesized by a method mentioned below according to the following reaction formula.

[Chem. 165]

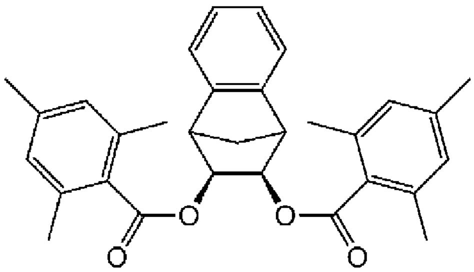

(Compound 58)

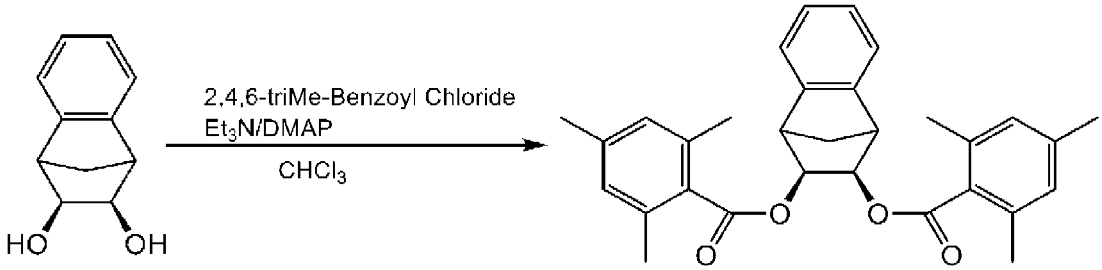


Under a nitrogen atmosphere, 3 g of the compound 5, 5.2 g of triethylamine, 0.21 g of 4-dimethylaminopyridine (DMAP), and 100 mL of chloroform were added to a 300 mL 3-neck flask and cooled to 0°C using an ice bath under stirring. 9.3 g of 2,4,6-trimethylbenzoyl chloride was added such that the internal temperature did not exceed 5°C. After the addition, the temperature was raised to room temperature, and stirring was conducted overnight. After disappearance of the raw material was confirmed by liquid chromatography, the solution was cooled again in an ice bath, and 5 mL of methanol was added thereto. Chloroform and water were added to the reaction solution to fractionate the organic layer, and the water layer was extracted with chloroform 3 times. The organic layer was washed with brine, dried over sodium sulfate, and then concentrated in a rotary evaporator. The reaction mixture was purified by silica gel column chromatography (eluent: hexane/ethyl acetate = 10/1) to give 4.6 g (9.8 mmol, yield: 58%, white powder) of the compound 58. The $^1H$-NMR data of the compound 58 obtained are shown below.

$^1H$ NMR (270 MHz, $CDCl_3$, TMS as an internal standard): δ 7.34 (dd, J=5.4Hz, 3.2Hz, 2H), 7.17 (dd, J=5.4Hz, 3.0Hz, 2H), 6.76 (s, 4H), 5.10 (d, J=1.6Hz, 2H), 3.55 (s, 2H), 2.42 (d, J=9.5Hz, 1H), 2.28-2.25 (m, 7H), 2.13 (s, 12H).

The melting completion temperature of the compound 58 obtained was 173°C.

[Example A32]

<Synthesis of compound 59>

A compound 59 shown below was synthesized by a method mentioned below according to the following reaction formula.

[Chem. 166]

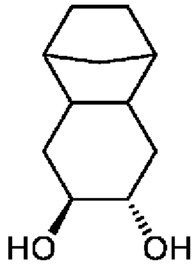

(Compound 59)

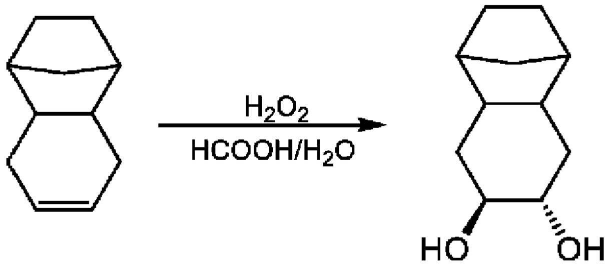


15 mL of a 30% hydrogen peroxide solution and 60 mL of 88% formic acid were added to a 300 mL 3-neck flask and heated to 40°C using an oil bath under stirring. 14.8 g of tricyclo[6,2,1,0(2,7)]undec-4-ene was added dropwise to the reaction solution such that the internal temperature did not exceed 50°C. After the dropwise addition, stirring was continued at 40°C for an hour. Thereafter, the reaction liquid was allowed to cool to room temperature and stirred for 17 hours. The reaction solution was concentrated under reduced pressure, 20 mL of a 2 mol/L sodium hydroxide aqueous solution, and 50 mL of ethyl acetate were added at 50°C or less thereto, and stirring was conducted at 50°C for an hour. The organic layer was fractionated, and the water layer was extracted with ethyl acetate 3 times. The organic layer was washed with brine, dried over sodium sulfate, and then concentrated in a rotary evaporator. The compound 59 was continuously allowed to react without purification.

<Synthesis of compound 60>

A compound 60 shown below was synthesized by a method mentioned below according to the following reaction formula.

[Chem. 167]

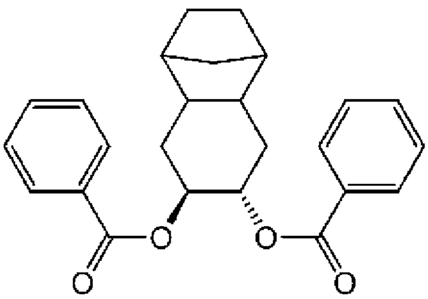

(Compound 60)

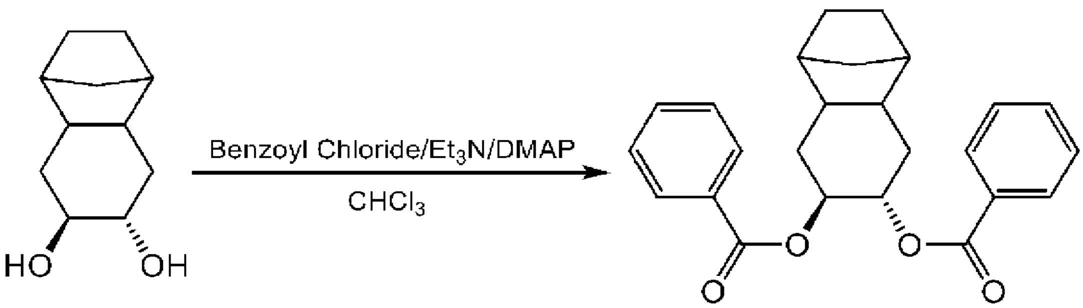

Under a nitrogen atmosphere, 3.3 g of the compound 59, 3.8 g of triethylamine, 0.22 g of 4-dimethylaminopyridine (DMAP), and 100 mL of chloroform were added to a 300 mL 3-neck flask and cooled to 0°C using an ice bath under stirring. 5.3 g of benzoyl chloride was added such that the internal temperature did not exceed 5°C. After the addition, the temperature was raised to room temperature, and stirring was conducted overnight. After disappearance of the raw material was confirmed by liquid chromatography, the solution was cooled again in an ice bath, and 5 mL of methanol was added thereto. Chloroform and water were added to the reaction solution to fractionate the organic layer, and the water layer was extracted with chloroform 3 times. The organic layer was washed with brine, dried over sodium sulfate, and then concentrated in a rotary evaporator. The reaction mixture was purified by silica gel column chromatography (eluent: hexane/ethyl acetate = 10/1) to give 5.3 g (13.6 mmol, yield: 74%, white powder) of the compound 60. The $^1$H-NMR data of the compound 60 obtained are shown below.

$^1$H NMR (270 MHz, $CDCl_3$, TMS as an internal standard): δ 8.06-8.01 (m, 4H), 7.55-7.39 (m, 6H), 5.50-5.45 (m, 1H), 5.21-5.13 (m, 1H), 2.06-1.09 (m, 14H).

The melting completion temperature of the compound 60 obtained was 125°C.

[Example A33]

<Synthesis of compound 61>

A compound 61 shown below was synthesized by a method mentioned below according to the following reaction formula.

[Chem. 168]

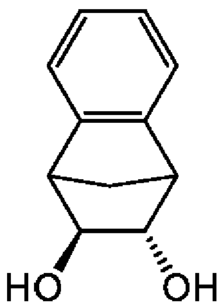

(Compound 61)

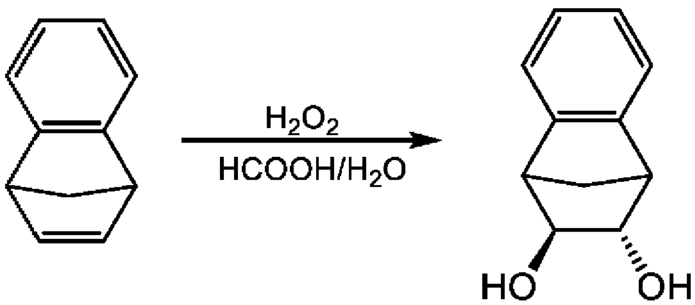


15 mL of a 30% hydrogen peroxide solution and 60 mL of 88% formic acid were added to a 300 mL 3-neck flask and heated to 40°C using an oil bath under stirring. 14.2 g of 1,4-dihydro-1,4-methanonaphthalene was added dropwise to the reaction solution such that the internal temperature did not exceed 50°C. After the dropwise addition, stirring was continued at 40°C for an hour. Thereafter, the reaction liquid was allowed to cool to room temperature and stirred for 17 hours. The reaction solution was concentrated under reduced pressure, 20 mL of a 2 mol/L sodium hydroxide aqueous solution, and 50 mL of ethyl acetate were added thereto at 50°C or less, and stirring was conducted at 50°C for an hour. The organic layer was fractionated, and the water layer was extracted with ethyl acetate 3 times. The organic layer was washed with brine, dried over sodium sulfate, and then concentrated in a rotary evaporator. The compound 61 was continuously allowed to react without purification.

<Synthesis of compound 62>

A compound 62 shown below was synthesized by a method mentioned below according to the following reaction

formula.

[Chem. 169]

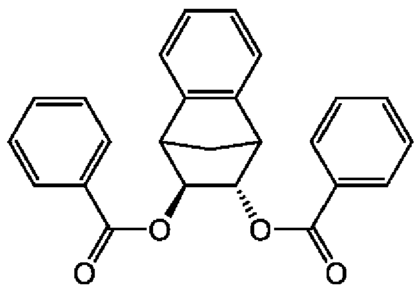

(Compound 62)

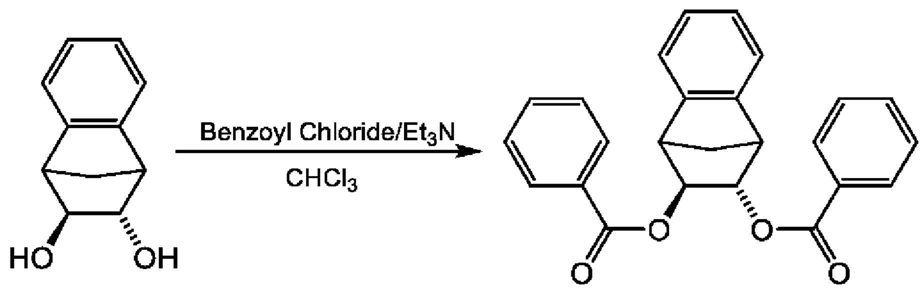


Under a nitrogen atmosphere, 8.8 g of the compound 61, 10.63 g of triethylamine, and 100 mL of chloroform were added to a 300 mL 3-neck flask and cooled to 0°C using an ice bath under stirring. 14.8 g of benzoyl chloride was added such that the internal temperature did not exceed 5°C. After the addition, the temperature was raised to room temperature, and stirring was conducted overnight. After disappearance of the raw material was confirmed by liquid chromatography, the solution was cooled again in an ice bath, and 5 mL of methanol was added thereto. Chloroform and water were added to the reaction solution to fractionate the organic layer, and the water layer was extracted with chloroform 3 times. The organic layer was washed with brine, dried over sodium sulfate, and then concentrated in a rotary evaporator. The reaction mixture was purified by silica gel column chromatography (eluent: hexane/ethyl acetate = 10/1) to give 4.1 g (10.7 mmol, yield: 210, white powder) of the compound 62. The $^1$H-NMR data of the compound 62 obtained are shown below.

$^1$H NMR (270 MHz, $CDCl_3$, TMS as an internal standard): $\delta$ 8.09-8.05 (m, 2H), 7.83-7.79 (m, 2H), 7.57-7.15 (m, 10H), 5.09-5.02 (m, 2H), 3.94 (s, 1H), 3.65-3.63 (m, 1H), 2.45-2.38 (m, 1H), 2.27-2.19 (m, 1H).

The melting completion temperature of the compound 62 obtained was 192°C.

[Example A34]

<Synthesis of compound 63>

A compound 63 shown below was synthesized by a method mentioned below.

[Chem. 170]

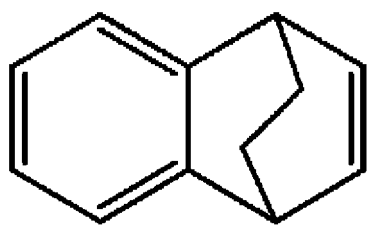

(Compound 63)

Under a nitrogen atmosphere, 35.2 g (149 mmol) of 1,2-dibromobenzene, 300 mL of dehydrated toluene, and

12.0 g of 1,3-cyclohexadiene were added to a 500 mL 3-neck flask and stirred. The internal temperature was cooled to 0°C, and 83 mL of a n-butyllithium hexane solution (1.6 M) was slowly added dropwise. After the dropwise addition, the temperature was gradually raised to room temperature, and stirring was conducted at room temperature for 12 hours. After the reaction, a saturated ammonium chloride aqueous solution was added, and then diethyl ether was added. The organic layer was separated and washed with water and brine in the order mentioned. After the organic layer was dried over magnesium sulfate, the magnesium sulfate was filtered off, and the obtained organic layer was concentrated in a rotary evaporator to thereby give 15.42 g of a crude product. The crude product was purified by silica gel column chromatography to give 4.9 g of the compound 63 as a mixture with impurities.

<Synthesis of compound 64>

A compound 64 shown below was synthesized by a method mentioned below.

[Chem. 171]

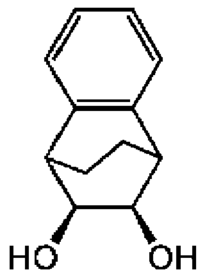

(Compound 64)

4.9 g of the compound 63 containing impurities obtained in <Synthesis of compound 63>, 125 mL of tert-butyl alcohol, and 31 mL of water were added to a 1000 mL 3-neck flask, and the internal temperature was cooled to 0°C under stirring. To another flask, 7.35 g of potassium permanganate, 150 mL of water, and 1.70 g of sodium hydroxide were added and stirred to prepare a potassium permanganate aqueous solution. The potassium permanganate aqueous solution containing the compound 63 prepared previously was slowly added dropwise such that the internal temperature did not exceed 5°C. After the dropwise addition, stirring was continued at 0°C for an hour. A saturated sodium metabisulfite aqueous solution was prepared and slowly added dropwise to the reaction solution until a white precipitate was formed. The precipitate formed was removed by filtration, and the filtrate was extracted with ethyl acetate 4 times. The organic layer was washed with brine and dried over magnesium sulfate. The magnesium sulfate was filtered off, and the obtained organic layer was concentrated in a rotary evaporator. The crude product was purified by silica gel column chromatography to give 0.72 g of the compound 64 as an isomer mixture.

<Synthesis of compounds 65-1 and 65-2>

Compounds 65-1 and 65-2 shown below were synthesized by a method mentioned below.

[Chem. 172]

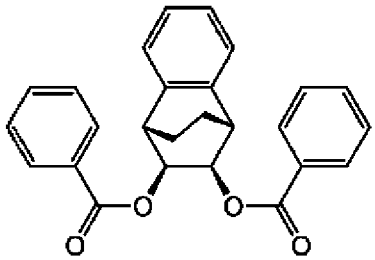

(Compound 65-1)

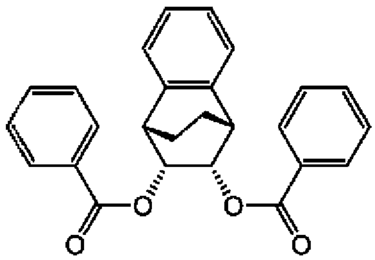

(Compound 65-2)

Under a nitrogen atmosphere, 0.65 g (3.3 mmol) of the compound 64 and 10.0 mL of dehydrated pyridine were added to a 50 mL 3-neck flask and stirred. Under cooling in an ice bath, 0.82 mL of benzoyl chloride was slowly added thereto. After the addition, the temperature was raised to room temperature, and stirring was conducted overnight. Under cooling in an ice bath again, 5 mL of methanol was added. To a beaker were added 100 mL of water and 100 mL of ethyl acetate to separate the organic layer. The organic layer was washed with water 3 times, washed with a saturated ammonium chloride aqueous solution and brine, and dried over magnesium sulfate. The magnesium sulfate was filtered, and the filtrate was concentrated in a rotary evaporator to thereby give a crude product. The crude product was purified by silica gel column chromatography to give 0.41 g (white solid) of the compound 65-1 and 0.53 g (white solid) of the compound 65-2. The stereostructures of the compounds 65-1 and 65-2 were determined by NOESY. The $^1$H-NMR data of the compound 65-1 and compound 65-2 are shown below.

(Compound 65-1)
$^1$H NMR (270 MHz, $CDCl_3$, TMS as an internal standard): $\delta$ 1.40-1.50 (m, 2H), 2.36-2.45 (m, 2H), 3.45 (s, 2H), 5.19 (s, 2H), 7.25-7.32 (m, 8H), 7.44-7.53 (m, 2H), 7.90-7.96 (m, 4H).
(Compound 65-2)
$^1$H NMR (270 MHz, $CDCl_3$, TMS as an internal standard): $\delta$ 1.52-1.62 (m, 2H), 1.92-2.00 (m, 2H), 3.41 (s, 2H), 5.55-5.57 (m, 2H), 7.08-7.16 (m, 4H), 7.22-7.38 (m, 6H), 7.52-7.57 (m, 4H).

The melting completion temperature of the compound 65-1 obtained was 143°C. The melting completion temperature of the compound 65-2 obtained was 193°C.

[Example A35]

<Synthesis of compound 66>

A compound 66 shown below was synthesized by a method mentioned below.

[Chem. 173]

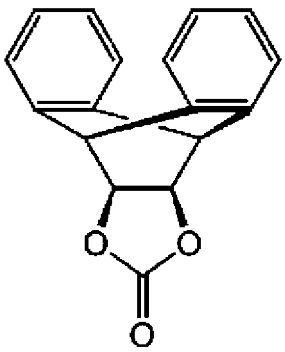

(Compound 66)

Under a nitrogen atmosphere, 3.8 g of anthracene, 2.9 g of vinylene carbonate, and 15 mL of toluene were added to a 30 mL pressure-resistant vessel and stirred under heating such that the internal temperature reached 180°C, and the stirring was continued for 45 hours. After cooling to room temperature, the solution was concentrated, and the solid was filtered off. The obtained solid was washed with hexane, and then dried to give 3.85 g of the compound 66. The $^{1}$H-NMR data of the compound 66 obtained are shown below.

$^{1}$H NMR (270 MHz, $CDCl_3$, TMS as an internal standard): 7.40-7.37 (m, 4H), 7.27-7.22 (m, 4H), 4.88 (m, 2H), 4.70 (m, 2H) .

<Synthesis of compound 67>

A compound 67 shown below was synthesized by a method mentioned below.

[Chem. 174]

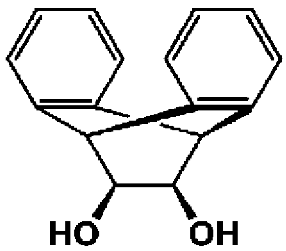

(Compound 67)

To a 100 mL 3-neck flask, 3.8 g of the compound 66, 7.2 mL of a 4 mol/L sodium hydroxide aqueous solution, and 28 mL of methanol were added. After the addition, stirring was conducted at room temperature for 30 minutes. After the methanol was distilled off, 20 mL of water was added, extraction was conducted using 30 mL of chloroform, and the organic layer was dried over sodium sulfate. The chloroform was distilled off, and drying was conducted to give 2.88 g of the compound 67. The $^{1}$H-NMR data of the compound 67 obtained are shown below.

$^{1}$H NMR (270 MHz, $CDCl_3$, TMS as an internal standard): 7.39-7.30 (m, 4H), 7.23-7.14 (m, 4H), 4.42 (s, 2H), 4.06 (s, 2H) , 2.10 (s, 2H).

<Synthesis of compound 68>

A compound 68 shown below was synthesized by a method mentioned below.

[Chem. 175]

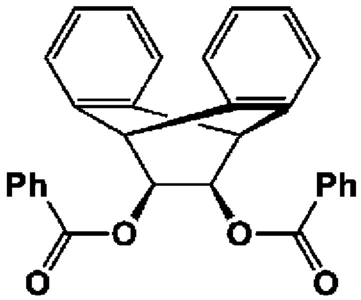

(Compound 68)

Under a nitrogen atmosphere, 2.9 g of the compound 67, 3.51 mL of triethylamine, and 12 mL of chloroform were added, and stirring was conducted at room temperature for 10 minutes. 2.92 mL of benzoyl chloride was added at room temperature, stirring was conducted under heating such that the internal temperature reached 85°C, and the stirring was continued for 12 hours. After cooling in an ice bath, 5 mL of saturated sodium hydrogen carbonate was added, and extraction was conducted using 10 mL of chloroform. The organic layer was dried over magnesium sulfate and then concentrated. The obtained solid was filtered off, washed with hexane, and then dried to give 5.0 g (11.2 mmol, yield: 92%, white powder) of the compound 68. The $^1$H-NMR data of the compound 68 obtained are shown below.

$^1$H NMR (270 MHz, $CDCl_3$, TMS as an internal standard): 7.59-7.56 (m, 4H), 7.44-7.36 (m, 6H), 7.26-7.21 (m, 4H), 7.18-7.12 (m, 4H), 5.52 (s, 2H), 4.68 (s, 2H).

The melting completion temperature of the compound 68 obtained was 174°C.

[Example A36]

<Synthesis of compound 69>

A compound 69 shown below was synthesized by a method mentioned below.

[Chem. 176]

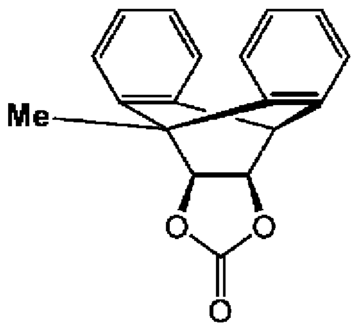

(Compound 69)

Under a nitrogen atmosphere, 3.8 g of 9-methylanthracene and 5.2 g of vinylene carbonate were added to a 30 mL pressure-resistant vessel and stirred under heating such that the internal temperature reached 220°C, and the stirring was continued for 9 hours. After cooling to room temperature, 5 mL of methanol was added and stirred, and then the solid was filtered off. The obtained solid was washed with methanol and then dried to give 5.6 g of the compound 69.

<Synthesis of compound 70>

A compound 70 shown below was synthesized by a method mentioned below.

[Chem. 177]

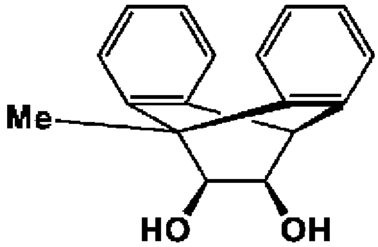

(Compound 70)

5.6 g of the compound 69, 8.0 g of sodium hydroxide, and 30 mL of pure water were added to a 50 mL 3-neck flask and stirred under heating such that the internal temperature reached 100°C, and the stirring was continued for 6 hours. After cooling to room temperature, 12 mol/L concentrated hydrochloric acid was added at 50°C or less for neutralization. The solid was filtered off, washed with pure water, and then dried to give 5.0 g of the compound 70.

<Synthesis of compound 71>

A compound 71 shown below was synthesized by a method mentioned below.

[Chem. 178]

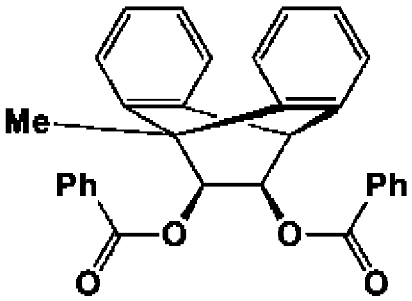

(Compound 71)

Under a nitrogen atmosphere, 5.0 g of the compound 70, 8.4 g of benzoyl chloride, and 50 mL of pyridine were added to a 200 mL 3-neck flask and stirred under heating such that the internal temperature reached 60°C, and the stirring was continued for 6 hours. After the pyridine was distilled off, chloroform was added thereto. After washing with 2 mol/L hydrochloric acid and a 2 mol/L sodium hydroxide aqueous solution, the organic layer was dried over magnesium sulfate. After concentration, the solid was filtered off, washed with hexane, and then dried to give 6.4 g (14.0 mmol, yield: 70%, white powder) of the compound 71. The $^1$H-NMR data of the compound 71 obtained are shown below.

$^1$H NMR (270 MHz, $CDCl_3$, TMS as an internal standard): 7.64-7.61 (m, 2H), 7.49-7.17 (m, 14H), 7.08-7.02 (m, 2H), 5.57 (dd, J = 7.8 Hz, 3.0 Hz, 1H), 5.29 (d, J = 7.6 Hz, 1H), 4.68 (d, J = 3.0 Hz, 1H), 2.02 (s, 3H).

The melting completion temperature of the compound 71 obtained was 173°C.

[Example A37]

<Synthesis of compound 72>

A compound 72 shown below was synthesized by a method mentioned below.

[Chem. 179]

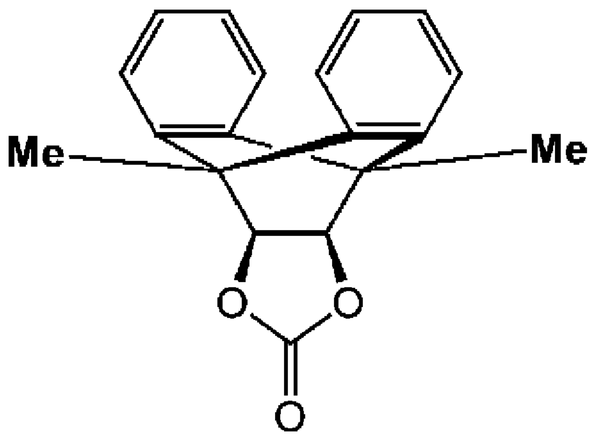

(Compound 72)

Under a nitrogen atmosphere, 4.0 g of 9,10-dimethylanthracene and 5.0 g of vinylene carbonate were added to a 30 mL pressure-resistant vessel and stirred under heating such that the internal temperature reached 220°C, and the stirring was continued for 9 hours. After cooling to room temperature, 5 mL of methanol was added and stirred, and then the solid was filtered off. The obtained solid was washed with methanol and then dried to give 5.6 g of the compound 72.

<Synthesis of compound 73>

A compound 73 shown below was synthesized by a method mentioned below.

[Chem. 180]

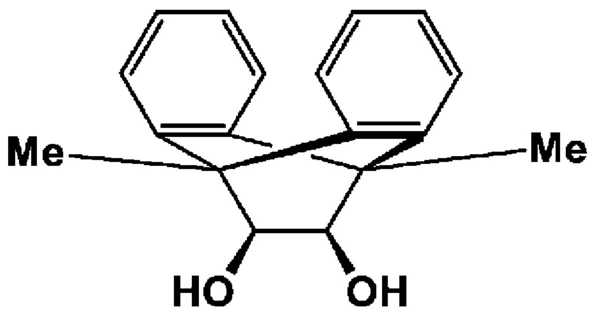

(Compound 73)

5.6 g of the compound 72, 7.6 g of sodium hydroxide, and 30 mL of pure water were added to a 50 mL 3-neck flask and stirred under heating such that the internal temperature reached 100°C, and the stirring was continued for 6 hours. After cooling to room temperature, 12 mol/L concentrated hydrochloric acid was added at 50°C or less for neutralization. The solid was filtered off, washed with pure water, and then dried to give 5.0 g of the compound 73.

<Synthesis of compound 74>

A compound 74 shown below was synthesized by a method mentioned below.

[Chem. 181]

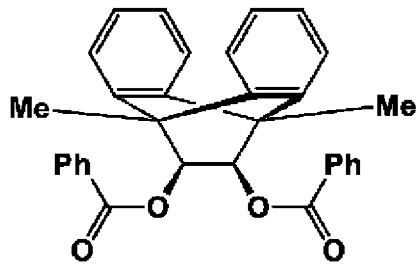

(Compound 74)

Under a nitrogen atmosphere, 5.0 g of the compound 73, 8.0 g of benzoyl chloride, and 50 mL of pyridine were added to a 200 mL 3-neck flask and stirred under heating such that the internal temperature reached 60°C, and the stirring was continued for 6 hours. After the pyridine was distilled off, chloroform was added thereto. After washing with 2 mol/L hydrochloric acid and a 2 mol/L sodium hydroxide aqueous solution, the organic layer was dried over magnesium sulfate. After concentration, the solid was filtered off, washed with hexane, and then dried to give 5.2 g (11.0 mmol, yield 58%, white powder) of the compound 74. The $^1$H-NMR data of the compound 74 obtained are shown below.

$^1$H NMR (270 MHz, $CDCl_3$, TMS as an internal standard): 7.52-7.49 (m, 4H), 7.46-7.42 (m, 4H), 7.35-7.27 (m, 6H), 7.12-7.06 (m, 4H), 5.40 (s, 2H), 2.00 (s, 6H).

The melting completion temperature of the compound 74 obtained was 218°C.

[Example A38]

<Synthesis of compound 75>

A compound 75 shown below was synthesized by a method mentioned below.

[Chem. 182]

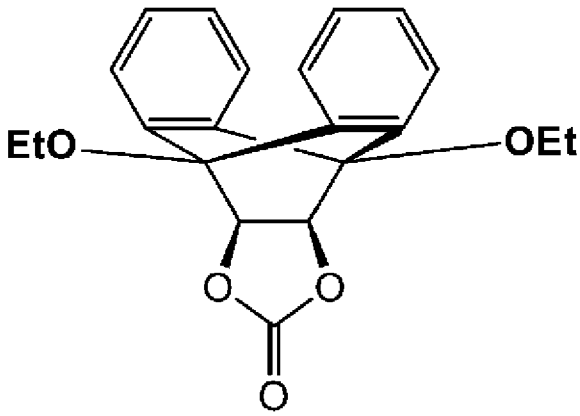

(Compound 75)

Under a nitrogen atmosphere, 8.0 g of 9, 10-diethoxyanthracene and 7.7 g of vinylene carbonate were added to a 30 mL pressure-resistant vessel and stirred under heating such that the internal temperature reached 220°C, and the stirring was continued for 9 hours. After cooling to room temperature, 5 mL of methanol was added and stirred, and then the solid was filtered off. The obtained solid was washed with methanol and then dried to give 10.5 g of the compound 75.

<Synthesis of compound 76>

A compound 76 shown below was synthesized by a method mentioned below.

[Chem. 183]

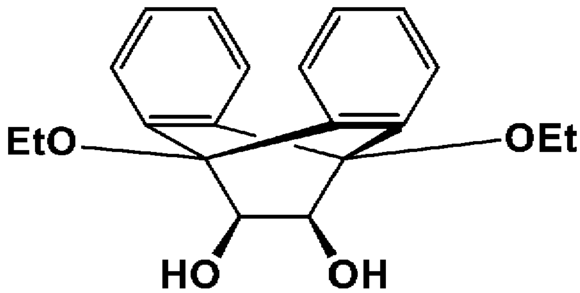

(Compound 76)

10.5 g of the compound 75, 12.0 g of sodium hydroxide and 30 mL of pure water were added to a 50 mL 3-neck flask and stirred under heating such that the internal temperature reached 100°C, and the stirring was continued for 6 hours. After cooling to room temperature, 12 mol/L concentrated hydrochloric acid was added at 50°C or less for neutralization. The solid was filtered off, washed with pure water, and then dried to give 9.8 g of the compound 76.

<Synthesis of compound 77>

A compound 77 shown below was synthesized by a method mentioned below.

[Chem. 184]

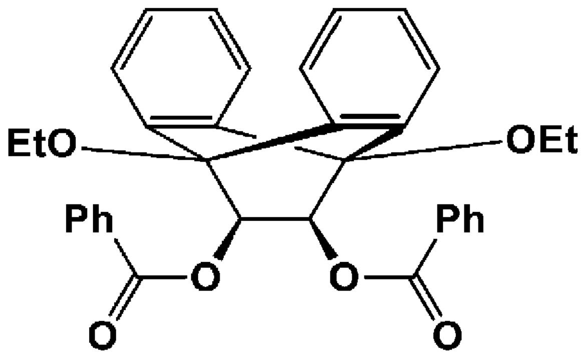

(Compound 77)

Under a nitrogen atmosphere, 9.8 g of the compound 76, 12.6 g of benzoyl chloride, and 50 mL of pyridine were added to a 200 mL 3-neck flask and stirred under heating such that the internal temperature reached 60°C, and the stirring was continued for 6 hours. After the pyridine was distilled off, chloroform was added thereto. After washing with 2 mol/L hydrochloric acid and a 2 mol/L sodium hydroxide aqueous solution, the organic layer was dried over magnesium sulfate. After concentration, the solid was filtered off, washed with hexane, and then dried to give 9.4 g (17.6 mmol, yield: 59%, white powder) of the compound 77. The $^{1}$H-NMR data of the compound 77 obtained are shown below.

$^{1}$H NMR (270 MHz, $CDCl_3$, TMS as an internal standard): 7.76-7.73 (m, 2H), 7.51-7.46 (m, 6H), 7.39-7.26 (m, 6H), 7.10-7.04 (m, 4H), 5.98 (s, 2H), 4.20 (dq, J = 8.4 Hz, 7.0 Hz, 2H), 3.93 (dq, J = 8.4 Hz, 7.0 Hz, 2H), 1.46 (t, J = 7.0 Hz, 6H) .

The melting completion temperature of the compound 77 obtained was 175°C.

[Example A39]

<Synthesis of compound 78>

A compound 78 shown below was synthesized by a method mentioned below.

[Chem. 185]

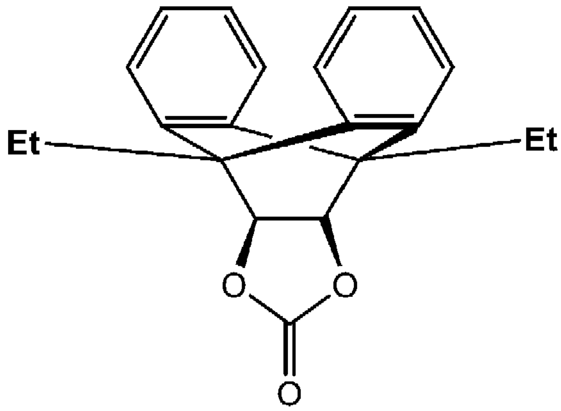

(Compound 78)

Under a nitrogen atmosphere, 7.0 g of 9, 10-diethylanthracene and 7.7 g of vinylene carbonate were added to a 30 mL pressure-resistant vessel and stirred under heating such that the internal temperature reached 220°C, and the stirring was continued for 9 hours. After cooling to room temperature, 5 mL of methanol was added and stirred, and then the solid was filtered off. The obtained solid was washed with methanol and then dried to give 9.6 g of the compound 78.

<Synthesis of compound 79>

A compound 79 shown below was synthesized by a method mentioned below.

[Chem. 186]

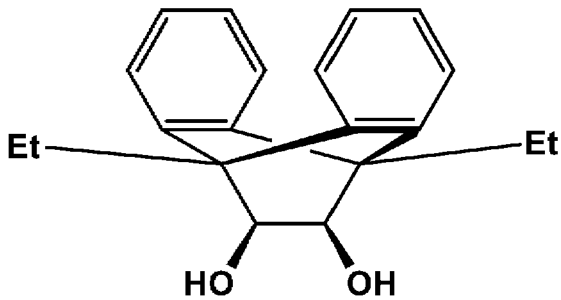

(Compound 79)

9.6 g of the compound 78, 12.0 g of sodium hydroxide, and 30 mL of pure water were added to a 50 mL 3-neck flask, and stirring was continued for 6 hours such that the internal temperature reached 100°C. After cooling to room temperature, 12 mol/L concentrated hydrochloric acid was added at 50°C or less for neutralization. The solid was filtered off, washed with pure water, and then dried to give 3.2 g of the compound 79.

<Synthesis of compound 80>

A compound 80 shown below was synthesized by a method mentioned below.

[Chem. 187]

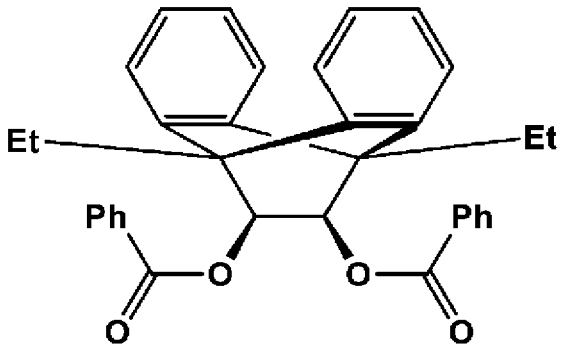

(Compound 80)

Under a nitrogen atmosphere, 3.2 g of the compound 79, 4.6 g of benzoyl chloride, and 50 mL of pyridine were added to a 200 mL 3-neck flask and stirred under heating such that the internal temperature reached 60°C, and the stirring was continued for 6 hours. After the pyridine was distilled off, chloroform was added thereto. After washing with 2 mol/L hydrochloric acid and a 2 mol/L sodium hydroxide aqueous solution, the organic layer was dried over magnesium sulfate. After concentration, the solid was filtered off, washed with hexane, and then dried to give 2.1 g (4.2 mmol, yield: 38%, white powder) of the compound 80. The $^{1}$H-NMR data of the compound 80 obtained are shown below.

$^{1}$H NMR (270 MHz, $CDCl_3$, TMS as an internal standard): 7.47-7.44 (m, 8H), 7.33-7.22 (m, 6H), 7.09-7.03 (m, 4H), 5.65 (s, 2H), 2.58 (q, J = 7.3 Hz, 4H), 1.37 (t, J = 7.3 Hz, 6H).

The melting completion temperature of the compound 80 obtained was 184°C.

[Example A40]

<Synthesis of compound 81>

A compound 81 shown below was synthesized by a method mentioned below.

[Chem. 188]

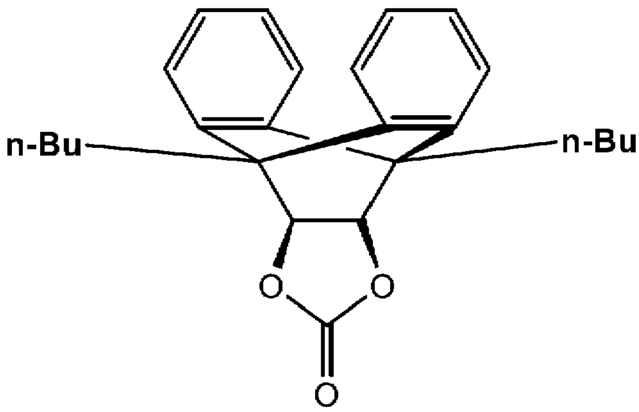

(Compound 81)

Under a nitrogen atmosphere, 8.7 g of 9, 10-di-n-butylanthracene and 7.7 g of vinylene carbonate were added to a 30 mL pressure-resistant vessel and stirred under heating such that the internal temperature reached 220°C, and the stirring was continued for 9 hours. After cooling to room temperature, 5 mL of methanol was added and stirred, and then the solid was filtered off. The obtained solid was washed with methanol and then dried to give 11.3 g of the compound 81.

<Synthesis of compound 82>

A compound 82 shown below was synthesized by a method mentioned below.

[Chem. 189]

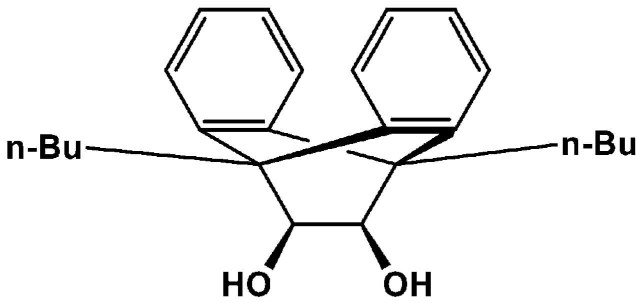

(Compound 82)

11.3 g of the compound 81, 12.0 g of sodium hydroxide, and 30 mL of pure water were added to a 50 mL 3-neck flask and stirred under heating such that the internal temperature reached 100°C, and the stirring was continued for 6 hours. After cooling to room temperature, 12 mol/L concentrated hydrochloric acid was added at 50°C or less for neutralization. The solid was filtered off, washed with pure water, and then dried to give 4.1 g of the compound 82.

<Synthesis of compound 83>

A compound 83 shown below was synthesized by a method mentioned below.

[Chem. 190]

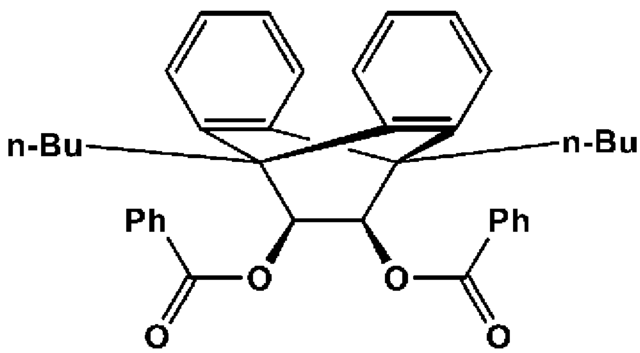

(Compound 83)

Under a nitrogen atmosphere, 4.1 g of the compound 82, 4.9 g of benzoyl chloride, and 50 mL of pyridine were added to a 200 mL 3-neck flask and stirred under heating such that the internal temperature reached 60°C, and the stirring was continued for 6 hours. After the pyridine was distilled off, chloroform was added thereto. After washing with 2 mol/L hydrochloric acid and a 2 mol/L sodium hydroxide aqueous solution, the organic layer was dried over magnesium sulfate. After concentration, the solid was filtered off, washed with hexane, and then dried to give 5.4 g (9.7 mmol, yield: 83%, white powder) of the compound 83. The $^1$H-NMR data of the compound 83 obtained are shown below.

$^1$H NMR (270 MHz, $CDCl_3$, TMS as an internal standard): 7.48-7.21 (m, 14H), 7.08-7.02 (m, 4H), 5.62 (s, 2H), 2.55-2.35 (m, 4H), 1.98-1.47 (m, 8H), 0.99 (t, J = 7.3 Hz, 6H).

The melting completion temperature of the compound 83 obtained was 176°C.

[Example A41]

<Synthesis of compound 84>

A compound 84 shown below was synthesized by a method mentioned below.

[Chem. 191]

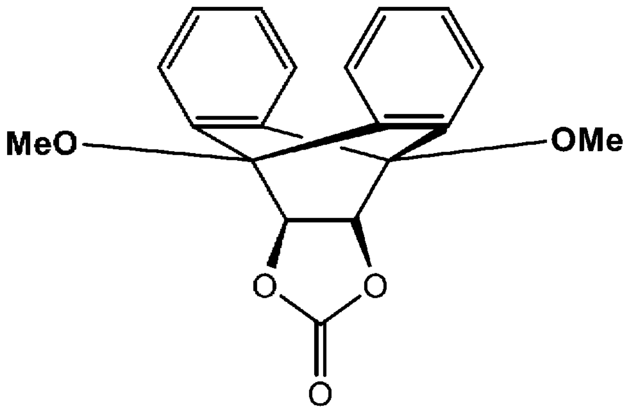

(Compound 84)

Under a nitrogen atmosphere, 7.2 g of 9,10-dimethoxyanthracene and 7.7 g of vinylene carbonate were added to a 30 mL pressure-resistant vessel and stirred under heating such that the internal temperature reached 220°C, and the stirring was continued for 9 hours. After cooling to room temperature, 5 mL of methanol was added and stirred, and then the solid was filtered off. The obtained solid was washed with methanol and then dried to give 9.7 g of the compound 84.

<Synthesis of compound 85>

A compound 85 shown below was synthesized by a method mentioned below.

[Chem. 192]

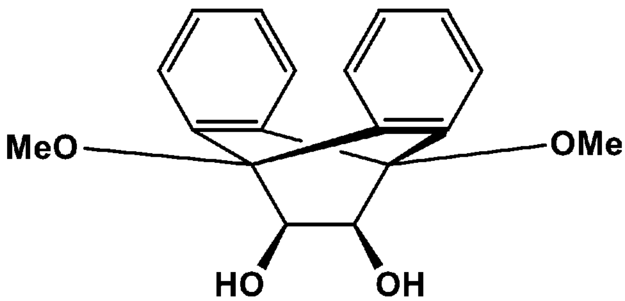

(Compound 85)

9.7 g of the compound 84, 12.0 g of sodium hydroxide, and 30 mL of pure water were added to a 50 mL 3-neck flask and stirred under heating such that the internal temperature reached 100°C, and the stirring was continued for 6 hours. After cooling to room temperature, 12 mol/L concentrated hydrochloric acid was added at 50°C or less for neutralization. The solid was filtered off, washed with pure water, and then dried to give 6.4 g of the compound 85.

<Synthesis of compound 86>

A compound 86 shown below was synthesized by a method mentioned below.

[Chem. 193]

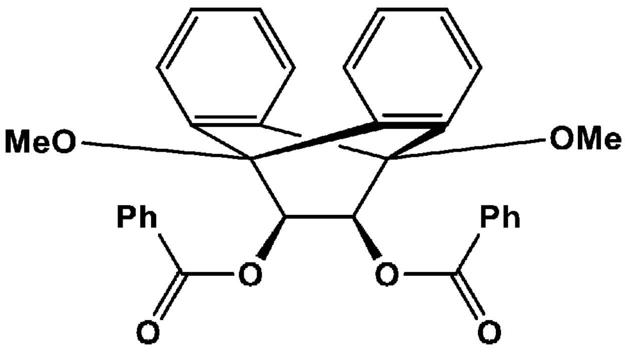

(Compound 86)

Under a nitrogen atmosphere, 6.4 g of the compound 85, 9.1 g of benzoyl chloride, and 50 mL of pyridine were added to a 200 mL 3-neck flask and stirred under heating such that the internal temperature reached 60°C, and the stirring was continued for 6 hours. After the pyridine was distilled off, chloroform was added thereto. After washing with 2 mol/L hydrochloric acid and a 2 mol/L sodium hydroxide aqueous solution, the organic layer was dried over magnesium sulfate. After concentration, the solid was filtered off, washed with hexane, and then dried to give 7.4 g (14.6 mmol, yield: 68%, white powder) of the compound 86. The $^1$H-NMR data of the compound 86 obtained are shown below.

$^1$H NMR (270 MHz, $CDCl_3$, TMS as an internal standard): 7.71-7.56 (m, 4H), 7.51-7.05 (m, 14H), 5.98 (s, 2H), 3.87 (s, 6H).

The melting completion temperature of the compound 86 obtained was 227°C.

[Example A42]

<Synthesis of compound 87>

A compound 87 shown below was synthesized by a method mentioned below.

[Chem. 194]

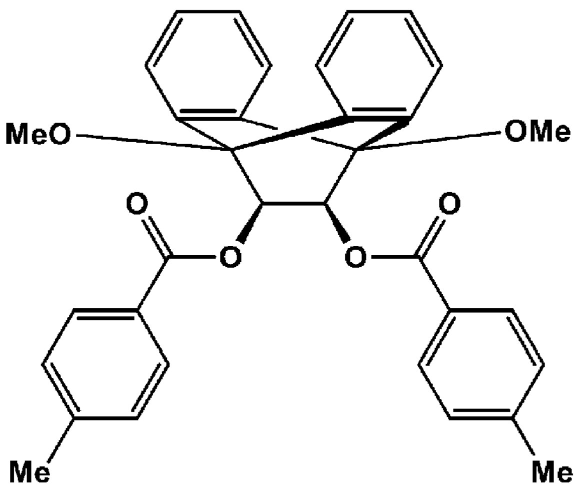

(Compound 87)

Under a nitrogen atmosphere, 4.1 g of the compound 85, 6.4 g of 4-methylbenzoyl chloride, and 50 mL of pyridine were added to a 200 mL 3-neck flask and stirred under heating such that the internal temperature reached 60°C, and the stirring was continued for 6 hours. After the pyridine was distilled off, chloroform was added thereto. After washing with 2 mol/L hydrochloric acid and a 2 mol/L sodium hydroxide aqueous solution, the organic layer was dried over magnesium sulfate. After concentration, the solid was filtered off, washed with hexane, and then dried to give 1.3 g (2.4 mmol, yield: 18%, white powder) of the compound 87. The $^1$H-NMR data of the compound 87 obtained are shown below.

$^1$H NMR (270 MHz, $CDCl_3$, TMS as an internal standard): 7.70-7.53 (m, 4H), 7.39-7.26 (m, 8H), 6.90-6.87 (m, 4H), 5.95 (s, 2H), 3.85 (s, 6H) 2.24 (s, 6H).

The melting completion temperature of the compound 87 obtained was 220°C.

[Example A43]

<Synthesis of compound 88>

A compound 88 shown below was synthesized by a method mentioned below.

[Chem. 195]

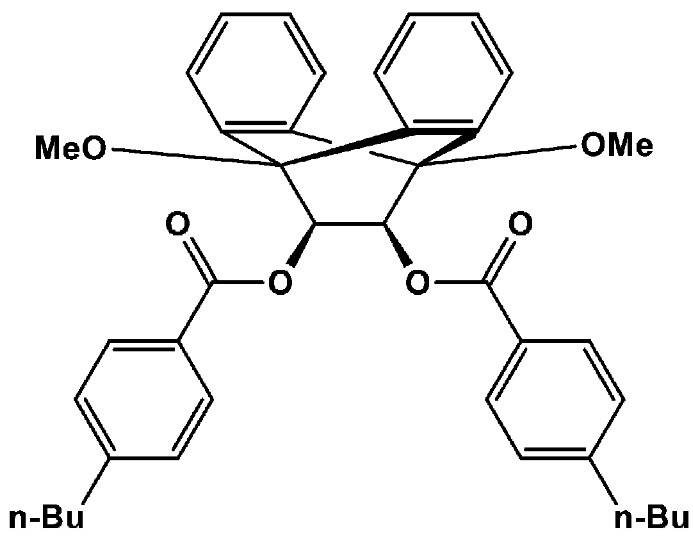

(Compound 88)

Under a nitrogen atmosphere, 4.1 g of the compound 85, 8.1 g of 4-n-butylbenzoyl, and 50 mL of pyridine were added to a 200 mL 3-neck flask and stirred under heating such that the internal temperature reached 60°C, and the stirring was continued for 6 hours. After the pyridine was distilled off, chloroform was added thereto. After washing with 2 mol/L hydrochloric acid and a 2 mol/L sodium hydroxide aqueous solution, the organic layer was dried over magnesium sulfate. After concentration, the solid was filtered off, washed with hexane, and then dried to give 1.9 g (3.1 mmol, yield: 22%, white powder) of the compound 88. The $^1$H-NMR data of the compound 88 obtained are shown below.

$^1$H NMR (270 MHz, $CDCl_3$, TMS as an internal standard): 7.70-7.55 (m, 4H), 7.39-7.25 (m, 8H), 6.89-6.86 (m, 4H), 5.95 (s, 2H), 3.86 (s, 6H) 2.51-2.45 (m, 4H), 1.56-1.19 (m, 8H), 0.88 (t, J = 7.0 Hz, 6H) .

The melting completion temperature of the compound 88 obtained was 158°C.

[Example A44]

<Synthesis of compound 89>

A compound 89 shown below was synthesized by a method mentioned below.

[Chem. 196]

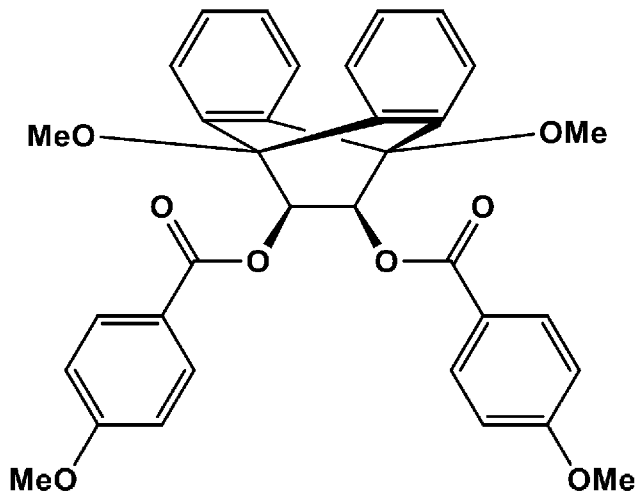

(Compound 89)

Under a nitrogen atmosphere, 15 g (50.3 mmol) of the compound 85 and 117 mL of dehydrated pyridine were added to a 200 mL 3-neck flask and cooled in an ice bath. 18.0 g (105.5 mmol) of 4-methoxybenzoyl chloride was added dropwise over about 5 minutes and stirred under heating in an oil bath warmed to 100°C for 20 hours. After the reaction finished followed by allowing to cool, 10 mL of dehydrated methanol was added thereto, and quenching was conducted at room temperature. After stirred at room temperature for approximately 30 minutes, the reaction solution was added dropwise to 600 mL of a mixed solution of hexane and water (hexane:water = 5:1) to give fine crystals. After the resulting crystals were gathered by filtration, the product gathered by filtration was washed with hexane and then dissolved in 100 mL of dichloromethane. The solution was washed twice with 100 mL of 1 N hydrochloric acid and once each with 100 mL of a saturated sodium hydrogen carbonate aqueous solution and 100 mL of brine in the order mentioned. Then, the obtained organic layer was dried over magnesium sulfate. After removal of the magnesium sulfate by a filtration operation followed by concentration in an evaporator, the resulting solid was dissolved again in 30 mL of chloroform, and the solution was added dropwise to 150 mL of methanol to give a solid. The obtained solid was purified by silica gel column chromatography (eluent: dichloromethane:methanol = gradient from 100:0 to 97:3) to give 20.17 g (yield 710, white solid) of the compound 89. The $^1$H-NMR data of the compound 89 obtained are shown below.

$^1$H NMR (270 MHz, $CDCl_3$, TMS as an internal standard): 7.70-7.67 (m, 2H), 7.58-7.55 (m, 2H), 7.46-7.43 (m, 4H), 7.37-7.33 (m, 2H), 7.31-7.28 (m, 2H), 6.59-6.56 (m, 4H), 5.94 (s, 2H), 3.86 (s, 6H) 3.73 (s, 6H).

The melting completion temperature of the compound 89 obtained was 221°C.

[Example A45]

<Synthesis of compound 90>

A compound 90 shown below was synthesized by a method mentioned below.

[Chem. 197]

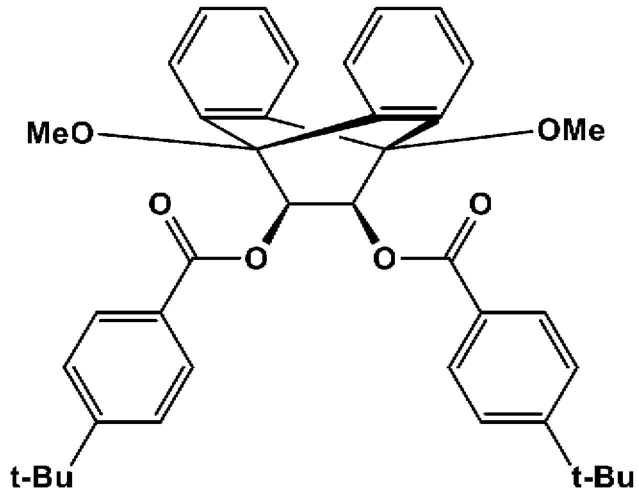

(Compound 90)

Under a nitrogen atmosphere, 5.0 g (16.8 mmol) of the compound 85 and 13 mL of dehydrated pyridine were added to a 100 mL 3-neck flask and stirred at room temperature. After dropwise addition of 7.0 g (35.6 mmol) of 4-tert-butylbenzoyl chloride, stirring was conducted at 100°C using an oil bath for 24 hours. After the reaction finished, 26 mL of methanol was added, and a precipitated solid was collected by filtration. The obtained solid was washed with 13 mL of 2 N hydrochloric acid, then suspended in 20 mL of methanol, and stirred at 60°C for about an hour. After allowing to cool, a white solid was collected by filtration to give 10.2 g (yield 98%, white solid) of the compound 90. The $^1$H-NMR data of the compound 90 obtained are shown below.

$^1$H NMR (270 MHz, $CDCl_3$, TMS as an internal standard): 7.71-7.67 (m, 2H), 7.58-7.55 (m, 2H), 7.46-7.28 (m, 8H), 7.12-7.09 (4H), 5.96 (s, 2H), 3.86 (s, 6H), 1.19 (s, 18H).

The melting completion temperature of the compound 90 obtained was 178°C.

[Example A46]

<Synthesis of compound 91>

A compound 91 shown below was synthesized by a method mentioned below.

[Chem. 198]

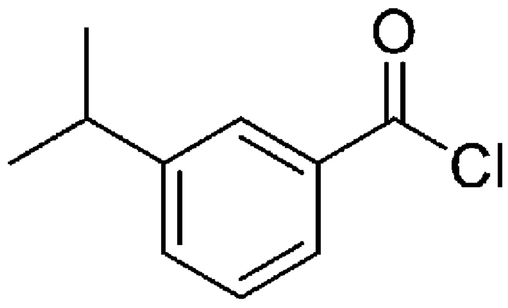

(Compound 91)

A 300 mL 3-neck flask including a magnetic stirring bar sufficiently dried by heating was equipped with a flat plug, a thermometer, and a three-way cock. Under a nitrogen atmosphere, 3.08 g (18.8 mmol) of 3-isopropylbenzoic acid, 60 mL of dichloromethane, and 2 drops of DMF were added thereto. After the reaction solution was cooled to 0°C, 2.57 mL (30 mmol) of oxalyl chloride was slowly added dropwise. After the dropwise addition finished, the temperature was raised to room temperature, and stirring was conducted at room temperature for 3 hours. The volatile compound in the reaction system was removed under reduced pressure to give the compound 91. The compound was used in <Synthesis of compound 92> without further purification.

<Synthesis of compound 92>

A compound 92 shown below was synthesized by a method mentioned below.

[Chem. 199]

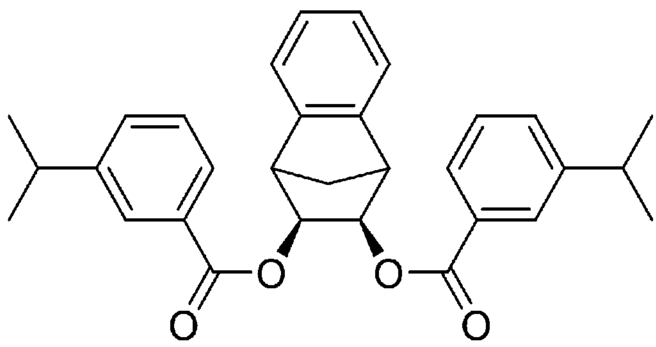

(Compound 92)

A 100 mL 3-neck flask including a magnetic stirring bar sufficiently dried by heating was equipped with a flat plug, a thermometer, and a three-way cock. Under a nitrogen atmosphere, 1.6 g of the compound 5 and 10 mL of dehydrated pyridine were added thereto. After the reaction solution was cooled to 0°C, 20 mL of a dichloromethane solution of the compound 91 synthesized in <Synthesis of compound 91> was slowly added to a pyridine solution of the compound 5, and stirring was conducted overnight. After the reaction finished, the solution was cooled to 0°C, 20 mL of methanol was added thereto, and stirring was conducted for an hour. After addition of 20 mL of water and 30 mL of dichloromethane, the solution was extracted with dichloromethane 3 times, and the gathered organic layer was washed with a saturated ammonium chloride aqueous solution twice. The organic layer was dried over sodium sulfate and then concentrated in a rotary evaporator. The resultant crude product was purified by silica gel column chromatography (eluent: hexane:ethyl acetate = 10:1) and recrystallization using hexane to result in 1.54 g (yield 34%, white solid) of the compound 92. The $^1$H-NMR data of the compound 92 obtained are shown below.

$^1$H NMR (270 MHz, $CDCl_3$, TMS as an internal standard): δ 1.12 (d, J = 6.9 Hz, 12H), 2.13-2.17 (m, 1H), 2.59-2.63 (m, 1H), 2.73 (sep, J = 6.9 Hz, 2H), 3.57 (s, 2H), 5.16 (s, 2H), 7.13-7.26 (overlapping with signal of $CHCl_3$, m, 4H), 7.33-7.36 (m, 4H), 7.76-7.79 (m, 4H).

The melting completion temperature of the compound 92 obtained was 93°C.

[Example A47]

<Synthesis of compound 93>

A compound 93 shown below was synthesized by a method mentioned below.

[Chem. 200]

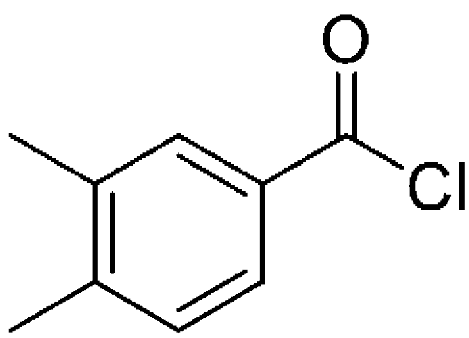

(Compound 93)

A compound 93 was synthesized in accordance with the operation and equivalence relationship described in <Synthesis of compound 91> except that 4.21 g (28.0 mmol) of 3,4-dimethylbenzoic acid was used instead of using 3-isopropylbenzoic acid in <Synthesis of compound 91>. The compound 93 obtained was used as was in <Synthesis of compound 94>.

<Synthesis of compound 94>

A compound 94 shown below was synthesized by a method mentioned below.

[Chem. 201]

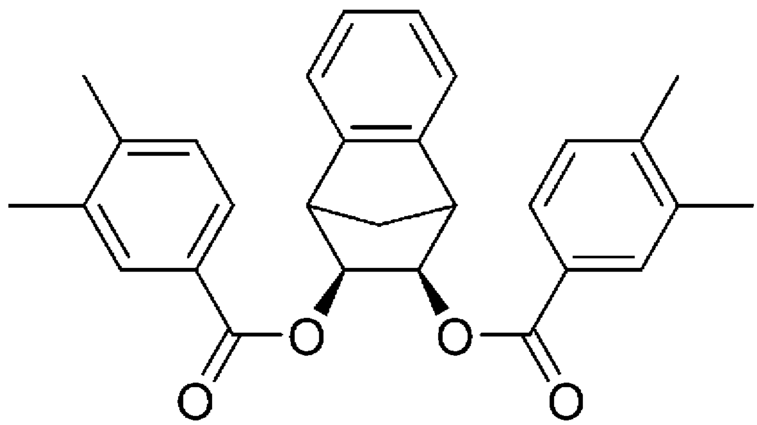

(Compound 94)

A 100 mL 3-neck flask including a magnetic stirring bar sufficiently dried by heating was equipped with a flat plug, a thermometer, and a three-way cock. Under a nitrogen atmosphere, 2.13 g (12.1 mmol) of the compound 5 and 10 mL of dehydrated pyridine were added thereto. After the reaction solution was cooled to 0°C, 20 mL of a dichloromethane solution of the compound 93 synthesized in <Synthesis of compound 93> was slowly added to a pyridine solution of the compound 5, and stirring was conducted overnight. After the reaction finished, the solution was cooled to 0°C, 20 mL of methanol was added thereto, and stirring was conducted for an hour. After addition of 20 mL of water and 30 mL of dichloromethane, the solution was extracted with dichloromethane 3 times, and the gathered organic layer was washed with a saturated ammonium chloride aqueous solution twice. The organic layer was dried over sodium sulfate and then concentrated in a rotary evaporator. The resultant crude product was purified by silica gel column chromatography (eluent: hexane:ethyl acetate = 10:1) to result in 2.64 g (6.0 mmol, yield 50%, pale yellow solid) of the compound 94. The $^1$H-NMR data of the compound 94 obtained are shown below.

$^1$H NMR (270 MHz, $CDCl_3$, TMS as an internal standard): δ 2.07 (s, 6H), 2.11-2.17 (m, 1H), 2.27 (s, 6H), 2.58-2.62 (m, 1H), 3.56 (s, 2H), 5.12-5.13 (m, 2H), 7.09 (d, J = 7.9 Hz, 2H), 7.16-7.19 (m, 2H), 7.32-7.35 (m, 2H), 7.62 (br s, 2H), 7.70-7.72 (m, 2H).

The melting completion temperature of the compound 94 obtained was 158°C.

[Example A48]

<Synthesis of compound 95>

A compound 95 shown below was synthesized by a method mentioned below according to the following reaction formula.

[Chem. 202]

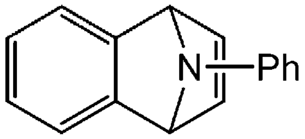

(Compound 95)

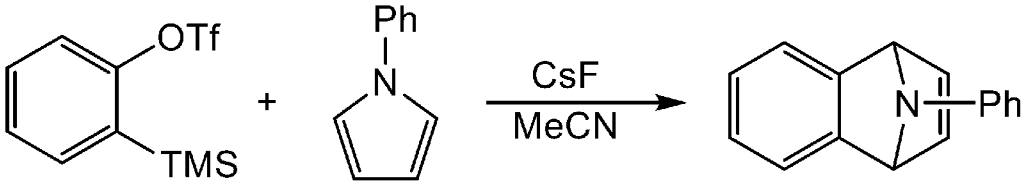


Under a nitrogen atmosphere, 200 mL of dehydrated acetonitrile, 6.36 g of 1-phenylpyrrole, and 6.74 g of cesium fluoride were added to a 1 L 3-neck flask and stirred at room temperature. Subsequently, 4.3 g of 2-(trimethylsilyl)phenyltriflate was slowly added, and then the reaction solution was stirred under heating at 40°C for 16 hours. The reaction solution was allowed to pass through a silica gel short column (eluent: ethyl acetate) and concentrated in a rotary evaporator. The resultant crude product was purified by silica gel column chromatography (eluent: hexane:ethyl acetate = 5:1) to result in 2.31 g (10.5 mmol, yield 710) of the compound 95.

<Synthesis of compound 96>

A compound 96 shown below was synthesized by a method mentioned below.

[Chem. 203]

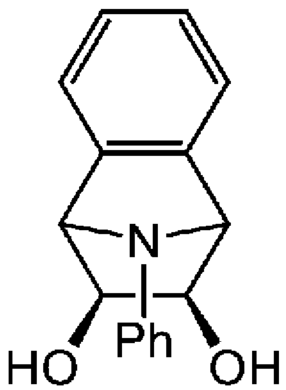

(Compound 96)

A 500 mL 3-neck flask including a magnetic stirring bar was equipped with a dropping funnel, a thermometer, and a three-way cock. Under a nitrogen atmosphere, 60 mL of a mixed solution of tert-butyl alcohol and acetone, 20 mL of water, and 2.51 g (11.4 mmol) of the compound 95 were added thereto, and the reaction solution was cooled to 0°C. 0.57 g (14.3 mmol) of sodium hydroxide and 2.70 g (17.1 mmol) of potassium permanganate were dissolved in 60 mL of water, and this solution was slowly added dropwise to the reaction solution prepared previously. After the dropwise addition finished, stirring was conducted under 0°C conditions for further an hour, and then unreacted potassium permanganate was quenched using a saturated aqueous solution of sodium metabisulfite. After stirring was conducted at room temperature for a while, sodium hydrogen carbonate was added until the pH of the reaction solution reached approximately 7 to 8, and the resulting white precipitate was removed by filtration. The filtered solution was extracted with ethyl acetate 3 times. The gathered organic layer was dried over sodium sulfate and then concentrated in a rotary evaporator. The resultant crude product was purified by silica gel column chromatography (eluent: hexane:ethyl acetate = 1:1) to result in 2.04 g (8.1 mmol, yield 710) of the compound 96.

<Synthesis of compound 97>

A compound 97 shown below was synthesized by a method mentioned below.

[Chem. 204]

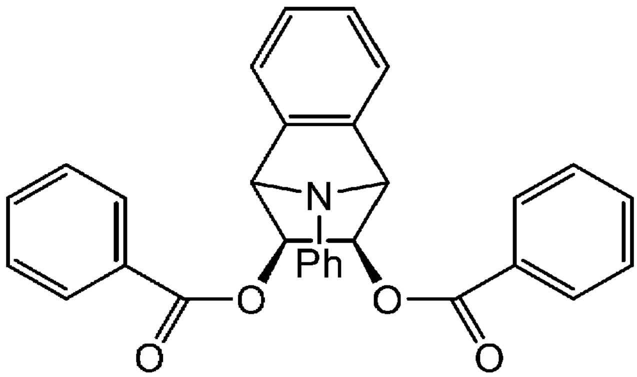

(Compound 97)

A 100 mL 3-neck flask including a magnetic stirring bar sufficiently dried by heating was equipped with a flat plug, a thermometer, and a three-way cock. Under a nitrogen atmosphere, 2.18 g (8.6 mmol) of the compound 96 and 10 mL of dehydrated pyridine were added thereto, and the flat plug was replaced with a dropping funnel. After the reaction solution was cooled to 0°C, 2.20 mL (18.9 mmol) of benzoyl chloride was slowly added dropwise. After the dropwise addition finished, the temperature was raised to room temperature, and stirring was conducted overnight. After the reaction finished, the solution was cooled to 0°C, 10 mL of methanol was added, and stirring was conducted for 30 minutes. After addition of 20 mL of water and 30 mL of dichloromethane, the solution was extracted with dichloromethane 3 times, and the gathered organic layer was washed with a saturated ammonium chloride aqueous solution twice. The organic layer was dried over sodium sulfate and then concentrated in a rotary evaporator. The resultant crude product was purified by silica gel column chromatography (eluent: hexane:ethyl acetate = 10:1) to result in 1.80 g (3.9 mmol, yield 45%, white solid) of the compound 97. The $^1H$-NMR data of the compound 97 obtained are shown below.

$^1H$ NMR (270 MHz, $CDCl_3$, TMS as an internal standard): $\delta$ 5.27-5.29 (m, 4H), 6.77-6.82 (m, 1H), 6.89-6.92 (m, 2H), 7.11-7.17 (m, 2H), 7.20-7.30 (overlapping with signal of $CHCl_3$, m, 6H), 7.41-7.51 (m, 4H), 7.90-7.94 (m, 4H).

The melting completion temperature of the compound 97 obtained was 158°C.

[Example A49]

<Synthesis of compound 98>

A compound 98 shown below was synthesized by a method mentioned below according to the following reaction formula.

[Chem. 205]

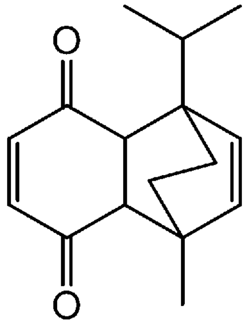

(Compound 98)

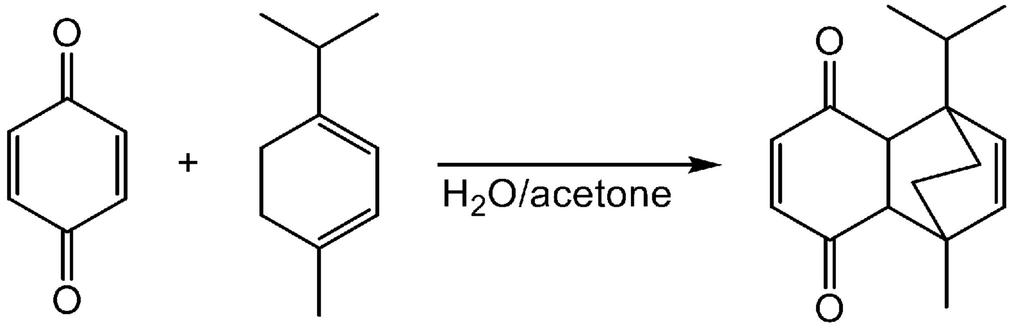


Under a nitrogen atmosphere, 14.0 g of p-benzoquinone (0.130 mol) and 17.6 g of α-terpinene (0.129 mol) were added to a 2 L 3-neck flask, then 700 mL of water was added thereto, and stirring was conducted at room temperature. 420 mL of acetone was slowly added dropwise, and stirring was continued at room temperature overnight. After 700 mL of ethyl acetate was added and stirring was conducted, the organic layer and the water layer were separated. The organic layer was washed with brine and then dried over magnesium sulfate. Concentration was conducted in a rotary evaporator to thereby give a crude product. The resultant crude product was combined with a resultant crude product using 5.0 g of p-benzoquinone in accordance with the similar operation and equivalence relationship (41.87 g in total), and the combined crude products were purified by silica gel column chromatography to give 30.0 g of the compound 98.

<Synthesis of compound 99>

A compound 99 shown below was synthesized by a method mentioned below according to the following reaction formula.

[Chem. 206]

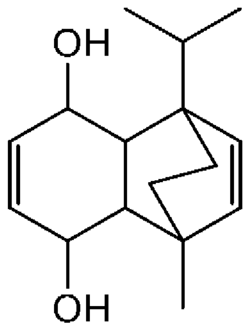

(Compound 99)

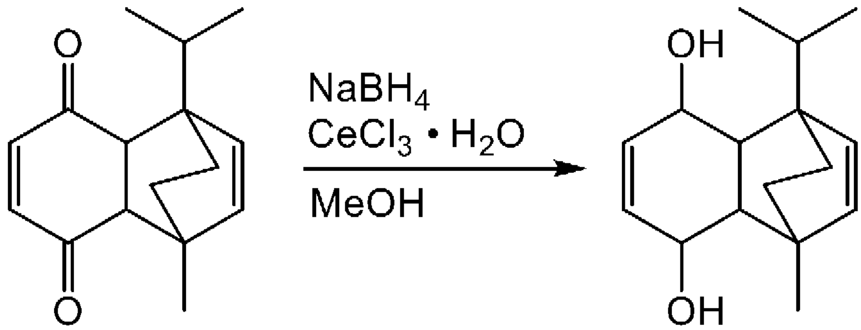


Under a nitrogen atmosphere, 28.0 g (0.115 mol) of the compound 98 was added to a 500 mL 3-neck flask followed by addition of 1078 mL of dehydrated methanol, and stirring was conducted at room temperature for a while. After addition of 92.9 g (0.249 mol) of cerium chloride heptahydrate, the solution was cooled with ice, and 9.92 g (0.262 mol) of sodium borohydride was slowly added. After the addition finished, the solution was stirred under ice cooling for an hour and quenched with 1 N hydrochloric acid. A saturated sodium hydrogen carbonate aqueous solution was added for neutralization, and the volatile substance was removed in a rotary evaporator. The remaining aqueous solution was extracted with ethyl acetate. The obtained organic layer was washed with a saturated sodium hydrogen carbonate aqueous solution and brine and dried over magnesium sulfate. After the magnesium sulfate was filtered off, concentration was conducted in a rotary evaporator. Purification was conducted by recrystallization using ethanol and silica gel column chromatography (hexane:ethyl acetate = 90:10 → 60:40) to give 24.9 g (yield 88%) of the compound 99 as an isomer mixture.

<Synthesis of compound 100>

A compound 100 shown below was synthesized by a method mentioned below according to the following reaction formula.

[Chem. 207]

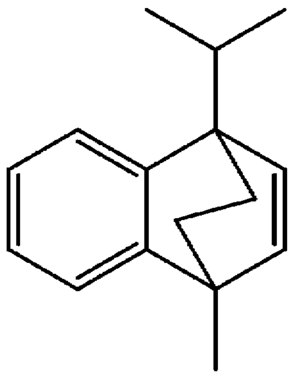

(Compound 100)

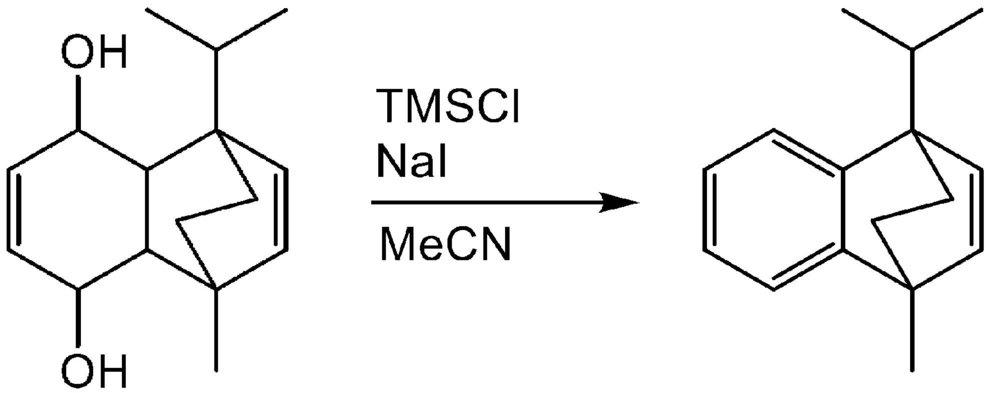


Under a nitrogen atmosphere, 21.3 g (85.8 mmol) of the compound 99 was added to a 100 mL 3-neck flask, then 25.7 g (171.5 mmol) of sodium iodide and 326 mL of acetonitrile were added, and stirring was conducted at room temperature for a while. 21.7 mL (171.0 mmol) of chlorotrimethylsilane was added dropwise thereto, and stirring was conducted at room temperature after the dropwise addition finished. The solution was quenched with a sodium thiosulfate solution, and the resulting solution was extracted with chloroform. The organic layer was washed with water and brine and then dried over magnesium sulfate. After the magnesium sulfate was filtered off, concentration was conducted in a rotary evaporator. The concentrate was combined with the crude product from a different lot and purified by silica gel column chromatography (eluent: hexane) to give 9.13 g of the compound 100 containing a small amount of impurities. Without further purification, the compound 100 was used in the next <Synthesis of compounds 101-1 and 101-2>.

<Synthesis of compounds 101-1 and 101-2>

Compounds 101-1 and 101-2 shown below were synthesized by a method mentioned below.

[Chem. 208]

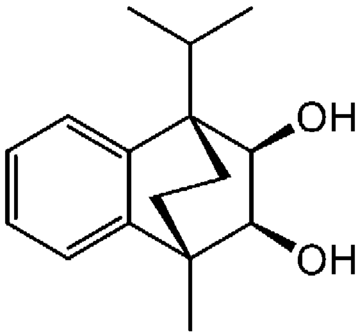

(Compound 101-1)

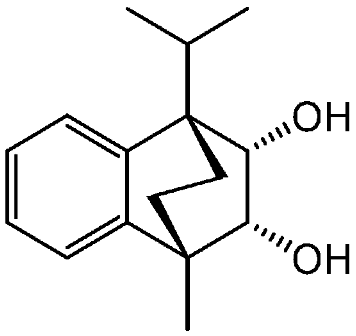

(Compound 101-2)

Under a nitrogen atmosphere, 9.13 g obtained in <Synthesis of compound 100> was added to a 1 L 3-neck flask, then 167 mL of tert-butyl alcohol and 42 mL of water were added, and the solution was cooled in an ice bath. To another flask, an aqueous solution including 10.0 g (63.2 mmol) of potassium permanganate, 2.18 g (54.5 mol) of sodium hydroxide, and 209 mL of water was prepared and slowly added dropwise to the reaction solution previously prepared. After the dropwise addition finished, stirring was continued still under cooling in an ice bath for 20 minutes. Under cooling in an ice bath, sodium metabisulfite was slowly added until the color of the potassium permanganate of the reaction solution disappeared. An operation of adding ethyl acetate to the reaction solution and separating only the supernatant organic layer was repeated 4 times. The gathered organic layer was dried over magnesium sulfate and then concentrated in a rotary evaporator. The resultant crude product was purified by silica gel column chromatography (hexane:ethyl acetate = 100:0 $\rightarrow$ 60:40) to give 1.74 g (yield 16%) of the compound 101-1 and 1.77 g (yield 17%) of the compound 101-2.

<Synthesis of compound 102>

A compound 102 shown below was synthesized by a method mentioned below.

[Chem. 209]

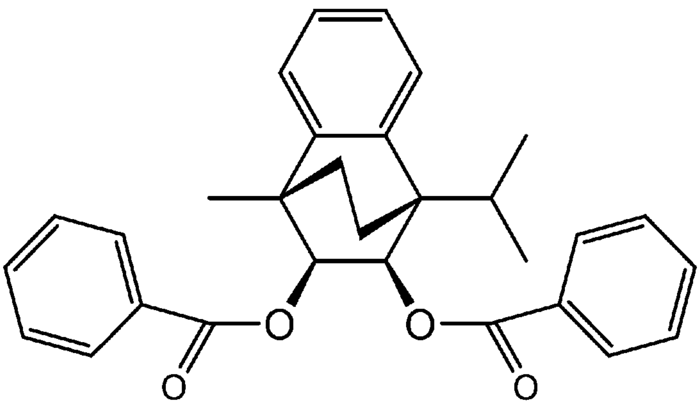

(Compound 102)

Under a nitrogen atmosphere, 1.74 g (7.06 mmol) of the compound 101-1 was added to a 200 mL 3-neck flask

and dissolved in 7 mL of dehydrated pyridine. Under cooling in an ice bath, 1.72 mL (14.8 mmol) of benzoyl chloride was slowly added dropwise such that the internal temperature did not exceed 5°C. After the dropwise addition, the temperature was raised to room temperature, and stirring was conducted for a while. After the reaction solution was cooled with ice, methanol was slowly added for quenching. Then, water and dichloromethane were added, the organic layer side was washed 3 times with 1 N hydrochloric acid, once with a saturated sodium hydrogen carbonate aqueous solution, and once with brine in the order mentioned. The organic layer after the washing was dried over magnesium sulfate, filtered, and then concentrated in a rotary evaporator. The resultant crude product was purified by silica gel column chromatography to give 1.10 g (yield 35%, white solid) of the compound 102. The $^1$H-NMR data of the compound 102 obtained are shown below.

$^1$H NMR (270 MHz, $CDCl_3$, TMS as an internal standard): δ 1.19-1.47 (m, 11 H), 2.27-2.58 (m, 3H), 5.15 (dd, J = 1.6, 8.6 Hz, 1H), 5.29 (dd, J = 1.6, 8.6 Hz, 1H), 7.11-7.17 (m, 2H), 7.29-7.40 (m, 6H), 7.43-7.50 (m, 2H), 7.77-7.80 (m, 2H), 7.88-7.92 (m, 2H).

The peaks considered to be the melting points of the compound 102 obtained were observed at 143°C and 150°C.

<Synthesis of compound 103>

A compound 103 shown below was synthesized by a method mentioned below.

[Chem. 210]

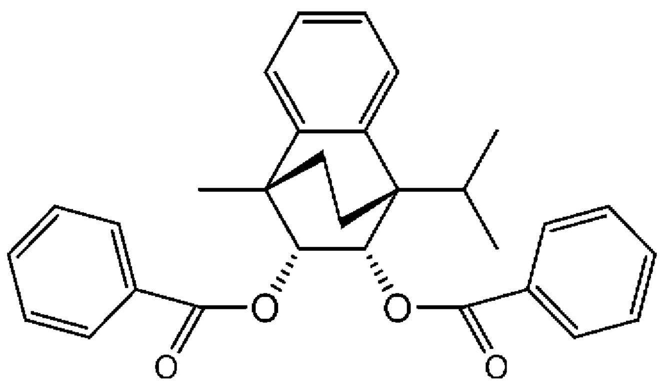

(Compound 103)

2.36 g (yield 73%, white solid) of the compound 103 was obtained in accordance with the operation and equivalence relationship described in <Synthesis of compound 102> except that 1.77 g of the compound 102-2 (7.18 mmol) was used instead of using 1.74 g the compound 101-1 in <Synthesis of compound 102>. The $^1$H-NMR data of the compound 103 obtained are shown below.

$^1$H NMR (270 MHz, $CDCl_3$, TMS as an internal standard): δ 1.04 (d, J = 6.9 Hz, 3H), 1.16 (d, J = 6.9 Hz, 3H), 1.41 (s, 3H), 1.48-1.58 (m, 2H), 1.77-1.94 (m, 2H), 2.73-2.83 (m, 1H), 5.55 (d, J = 7.9 Hz, 1H), 5.62 (d, J = 7.9 Hz, 1H), 6.94-7.00 (m, 2H), 7.09-7.15 (m, 2H), 7.21-7.52 (overlapping with signal of $CHCl_3$, m, 10H) .

The melting completion temperature of the compound 103 obtained was 149°C.

[Example A50]

<Synthesis of compound 104>

A compound 104 shown below was synthesized by a method mentioned below according to the following reaction formula.

[Chem. 211]

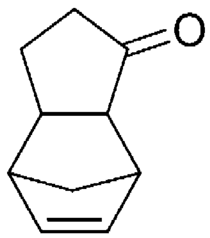

(Compound 104)

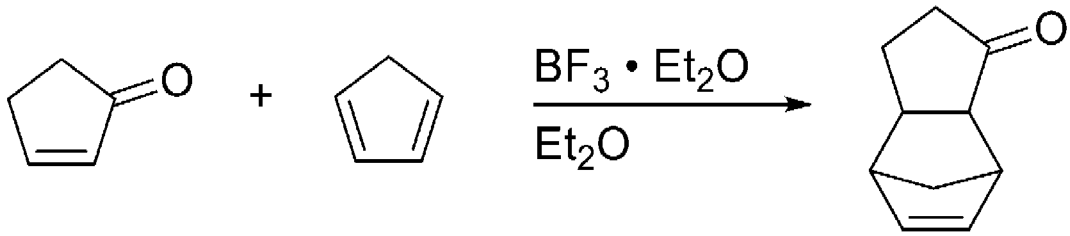


Under a nitrogen atmosphere, 70 g (0.85 mol) of 2-cyclopenten-1-one and 900 mL of dehydrated diethyl ether were added to a 2 L 4-neck flask and stirred while the internal temperature was cooled to 0°C. Then, 94 g (1.42 mol) of cyclopentadiene was added thereto. As the cyclopentadiene used in the reaction, a product obtained by pyrolyzing dicyclopentadiene in tetradecane at 160°C or more was rapidly used. 48.4 g (0.34 mol) of a boron trifluoride - ethyl ether complex was added dropwise over 10 minutes while the internal temperature remained at 0 to 5°C. After the dropwise addition finished, the temperature was raised to room temperature, and stirring was continued in this state for 17 hours. After the reaction finished, 900 mL of pure water was added, and stirring was conducted for 30 minutes. The solution was separated into an organic layer and a water layer, and the collected water layer was extracted 3 times using 500 mL of diethyl ether. The entire organic layer was gathered and then subjected to liquid-separation and washing with 1 L of brine. The obtained organic layer was dried over magnesium sulfate and filtered, and the filtrate was concentrated using a rotary evaporator. The resultant crude product was purified by silica gel column chromatography (hexane:ethyl acetate = 50:1) to give 82.9 g (yield 66%) of the compound 104.

<Synthesis of compound 105>

A compound 105 shown below was synthesized by a method mentioned below according to the following reaction formula.

[Chem. 212]

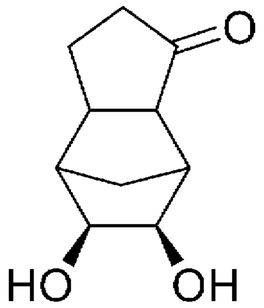

(Compound 105)

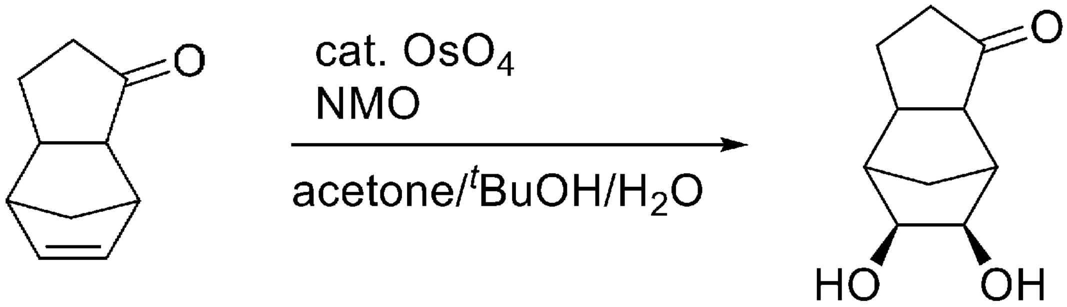


Under a nitrogen atmosphere, 24 g (162 mmol) of the compound 104, 41.7 g (178 mmol) of a 50% 4-methylmorpholine N-oxide (hereinafter, referred to as "NMO") aqueous solution, 60 mL of pure water, 60 mL of acetone, and 120 mL of tert-butyl alcohol were added to a 500 mL 4-neck flask and stirred. After addition of 124 mg (0.5 mmol) of osmium oxide, stirring was conducted at room temperature for 2 days. After the reaction finished, 2 g of sodium hydrosulfite, 24 g of Florisil, and 160 mL of pure water were added, and stirring was conducted for 30 minutes. The filtrate was collected by filtration under reduced pressure, and the pH of the filtrate was adjusted to 7 using 1 N sulfuric acid. The organic solvent was removed from the filtrate under reduced pressure at an external temperature of 40°C, and the pH of the remaining aqueous solution was adjusted to 3 again with 1 N sulfuric acid. Excess sodium chloride and 500 mL of ethyl acetate were added and stirred, the solution was filtered under reduced pressure, and undissolved sodium chloride was filtered off. The solution was liquid-separated into an organic layer and a water layer, and the collected water layer was extracted 3 times with 400 mL of ethyl acetate. The organic layer was gathered, dried over sodium sulfate, and filtered. Then, the filtrate was concentrated in a rotary evaporator. The resultant crude product was purified by silica gel column chromatography (ethyl acetate:dichloromethane = 1:1) to give 13.2 g (yield 45%) of the compound 105.

<Synthesis of compound 106>

A compound 106 shown below was synthesized by a method mentioned below.

[Chem. 213]

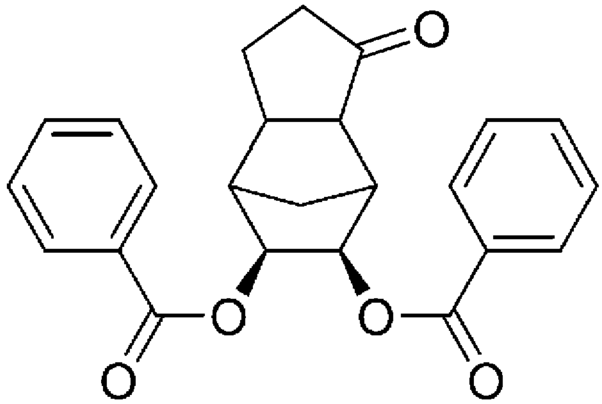

(Compound 106)

Under a nitrogen atmosphere, 3.33 g (18.3 mmol) of the compound 105 was added to a 3-neck flask followed by addition of 10 mL of dehydrated pyridine, and stirring was conducted. Under cooling in an ice bath, 4.68 mL (40.3 mmol) of benzoyl chloride was slowly added. After the addition, the temperature was raised to room temperature, and stirring was conducted for 5 hours. Under cooling in an ice bath again, 10 mL of methanol was added for quenching. Thereafter, water and dichloromethane were added, and the solution was transferred into a separation funnel. The solution was washed with water and a saturated ammonium chloride aqueous solution, and the organic layer was dried over magnesium sulfate and then concentrated in a rotary evaporator. 8.29 g of the resultant crude product was purified by silica gel column chromatography (eluent: hexane:ethyl acetate = 85:15 → 65:35) and recrystallization with an acetone solvent to give 3.16 g (yield 44%, pale yellow solid) of the compound 106. The $^1$H-NMR data of the compound 106 obtained are shown below.

$^1$H NMR (270 MHz, $CDCl_3$, TMS as an internal standard): δ 1.63-1.67 (m, 1H), 2.08-2.62 (m, 6H), 2.76-2.82 (m, 1H), 2.89-2.99 (m, 2H), 5.11-5.13 (m, 1H), 5.45-5.48 (m, 1H), 7.17 (t, J = 7.6 Hz, 2H), 7.33 (t, J = 7.6 Hz, 2H), 7.42 (t, J = 7.6 Hz, 1H), 7.52 (t, J = 7.6 Hz, 1H). 7.74-7.77 (m, 2H), 7.90-7.92 (m, 2H).

The melting completion temperature of the compound 106 obtained was 127°C.

[Example A51]

<Synthesis of compound 107>

A compound 107 shown below was synthesized by a method mentioned below according to the following reaction formula.

[Chem. 214]

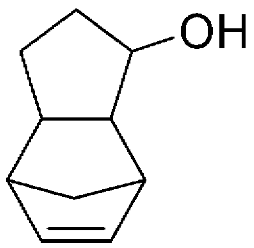

(Compound 107)

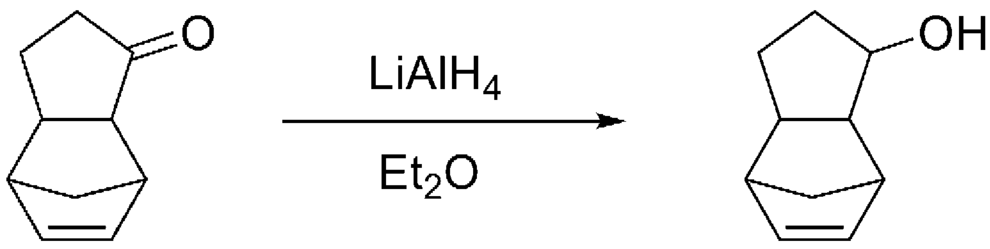


Under a nitrogen atmosphere, 1.5 L of dehydrated diethyl ether was added to a 5 L 4-neck flask and cooled

until the internal temperature reached 0°C. Subsequently, 22.2 g (585 mmol) of lithium aluminum hydride was added. 51 g (344 mmol) of the compound 104 was dissolved in 500 mL of dehydrated diethyl ether, and the solution was added dropwise to the solution prepared previously over 30 minutes. At this time, the liquid temperature was allowed to be within the range of 0 to 5°C. After the dropwise addition finished, the temperature was raised to room temperature, and stirring was continued for 1 hours. After the reaction finished, the liquid temperature was cooled again to 0°C, and 200 mL of methanol was added dropwise over an hour with attention to generation of hydrogen. Subsequently, 2 L of a saturated potassium sodium tartrate aqueous solution was added slowly, and stirring was conducted at room temperature for 2 hours. The solution was separated into an organic layer and a water layer, and the collected water layer was extracted 3 times with 500 mL of diethyl ether. The organic layer was gathered and subjected to liquid-separation and washing with 1 L of brine. The organic layer was dried over sodium sulfate and filtered. Then, the filtrate was concentrated in a rotary evaporator, and the concentrate was vacuum-dried at an external temperature of 40°C for 2 hours to result in 48.0 g (yield 93%) of the compound 107.

<Synthesis of compound 108>

A compound 108 shown below was synthesized by a method mentioned below according to the following reaction formula.

[Chem. 215]

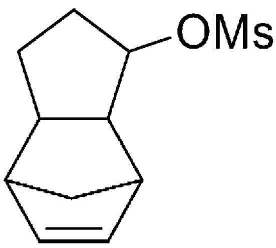

(Compound 108)

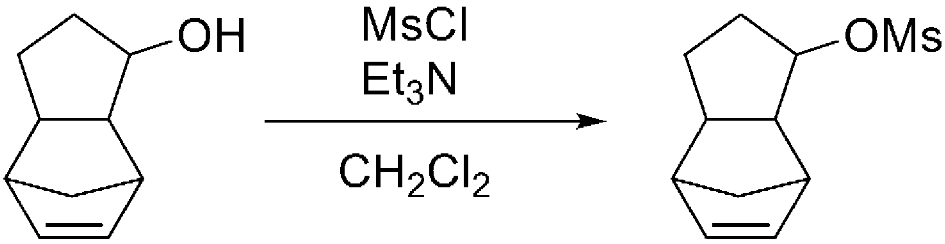


Under a nitrogen atmosphere, 47.9 g (319 mmol) of the compound 107, 48.5 g (479 mmol) of triethylamine, and 1 L of dichloromethane were added to a 2 L 4-neck flask and cooled until the internal temperature reached 0°C. Subsequently, 40.2 g (351 mmol) of methanesulfonyl chloride was added dropwise over 30 minutes with attention to allowing the internal temperature to be from 0 to 5°C. After the dropwise addition finished, stirring was conducted for 30 minutes with the internal temperature remaining at 5°C. After the reaction finished, 500 mL of pure water was added, and stirring was conducted for 30 minutes. The organic layer was separated, added to 500 mL of 10% hydrochloric acid cooled with an ice bath, and stirred. Subsequently, the organic layer was separated, added to 500 mL of a saturated sodium hydrogen carbonate aqueous solution, and stirred. The organic layer was separated again and subjected to liquid-separation and washing with 500 mL of brine. The organic layer was dried over sodium sulfate and filtered. Then, the filtrate was concentrated in a rotary evaporator, and the concentrate was vacuum-dried at an external temperature of 40°C for 2 hours to result in 70.4 g (yield 97%) of the compound 108.

<Synthesis of compound 109>

A compound 109 shown below was synthesized by a method mentioned below according to the following reaction formula.

[Chem. 216]

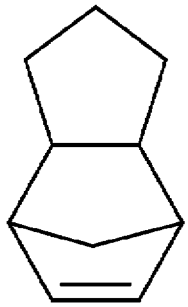

(Compound 109)

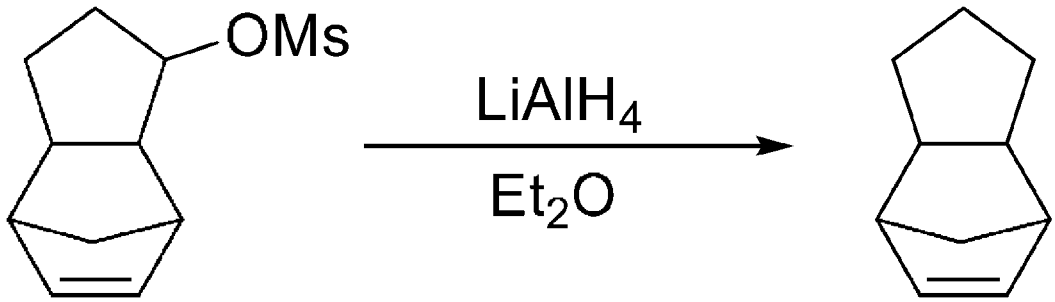


Under a nitrogen atmosphere, 1 L of dehydrated diethyl ether was added to a 5 L 4-neck flask, and the internal temperature was brought to 0°C. Subsequently, 17.5 g (460 mmol) of lithium aluminum hydride was added. 70.0 g (307 mmol) of the compound 108 was dissolved in 500 mL of dehydrated diethyl ether, and this solution was added dropwise to the solution prepared previously over 30 minutes. At this time, the internal temperature was allowed to be within the range of 0 to 5°C. After the dropwise addition finished, stirring was conducted at room temperature for 3 hours. After the reaction finished, the internal temperature was cooled again to 0°C, and 2 L of a saturated potassium sodium tartrate aqueous solution was added dropwise over 2 hours with attention to generation of hydrogen. Thereafter, stirring was conducted at room temperature for further 2 hours. The solution was separated into an organic layer and a water layer, and the collected water layer was extracted 4 times with 400 mL of diethyl ether. The gathered organic layer was subjected to liquid-separation and washing with 1 L of brine. The organic layer was dried over sodium sulfate and filtered, and then the filtrate was concentrated under atmospheric pressure at an external temperature of 50°C. After the concentration, distillation under reduced pressure was conducted at an external temperature of 60°C and 10 mmHg. Collection of the components distilled out at 40°C resulted in 37 g (yield 90%, colorless liquid) of the compound 109.

<Synthesis of compound 110>

A compound 110 shown below was synthesized by a method mentioned below.

[Chem. 217]

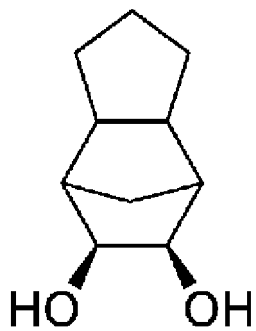

(Compound 110)

15 g (112 mmol) of the compound 109, 108 mL of pure water, and 600 mL of tert-butyl alcohol were added to a 2 L 4-neck flask and cooled with ice. 5.87 g (145 mmol) of sodium hydroxide and 19.43 g (123 mmol) of potassium permanganate were dissolved in 660 mL of water. The solution was placed in a dropping funnel and slowly added dropwise to the mixed solution prepared previously such that the internal temperature reached 3°C or less. After the dropwise addition finished, completion of the reaction was confirmed by GC analysis. Then, a saturated sodium metabisulfite aqueous solution was added until the color of the potassium permanganate disappeared, and the solution was then filtered with Celite. The filtrate was concentrated, and the obtained water layer was extracted with ethyl acetate 3 times. The gathered organic layer was dried over sodium sulfate and filtered, and then the filtrate was concentrated in a rotary evaporator. The resultant crude product was purified by silica gel column chromatography (dichloromethane:ethyl acetate = 2:1) to give 11.95 g (yield 64%, white solid) of the compound 110.

<Synthesis of compound 111>

A compound 111 shown below was synthesized by a method mentioned below.

[Chem. 218]

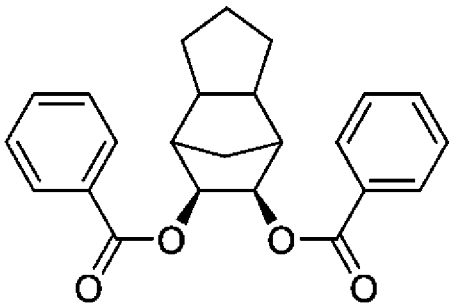

(Compound 111)

Under a nitrogen atmosphere, 3.0 g (17.8 mmol) of the compound 110 was added to a 3-neck flask followed by addition of 10 mL of dehydrated pyridine, and stirring was conducted. Under cooling in an ice bath, 4.25 mL (36.6 mmol) of benzoyl chloride was slowly added over 10 minutes. After the addition, the temperature was raised to room temperature, and stirring was conducted overnight. Under cooling in an ice bath again, 3 mL of methanol was added for quenching. Thereafter, water and ethyl acetate were added, and the solution was transferred into a separation funnel. The solution was washed with water, a saturated ammonium chloride aqueous solution, and brine in the order mentioned, and the organic layer was dried over magnesium sulfate and then concentrated in a rotary evaporator. The resultant crude product was purified by silica gel column chromatography (hexane:ethyl acetate = 10:1) to give 6.47 g (yield 96%, white solid) of the compound 111. The $^1$H-NMR data of the compound 111 obtained are shown below.

$^1$H NMR (270 MHz, $CDCl_3$, TMS as an internal standard): $\delta$ 1.58-1.87 (m, 7H), 2.22-2.26 (m, 1H), 2.44 (br s, 2H), 2.58 (br s, 2H), 5.38 (d, J = 1.6 H, 2H), 7.22-7.28 (overlapping with signal of $CHCl_3$, m, 4H), 7, 46 (t, J = 7.6 Hz, 2H), 7.84-7.87 (m, 4H).

The melting completion temperature of the compound 111 obtained was 97°C.

[Example A52]

<Synthesis of compound 112>

A compound 112 shown below was synthesized by a method mentioned below according to the following reaction formula.

[Chem. 219]

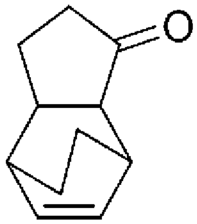

(Compound 112)

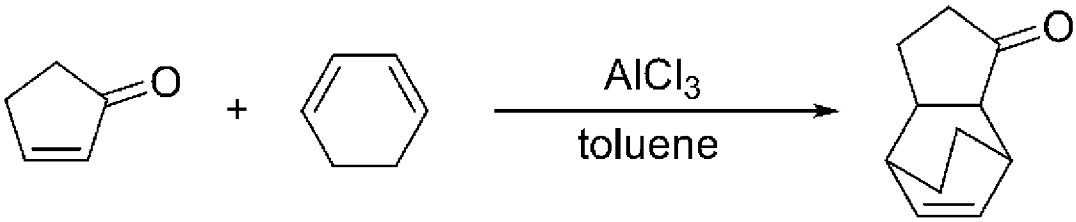


Under a nitrogen atmosphere, 33.3 g (0.25 mol) of aluminum chloride and 1.5 L of dehydrated toluene were added to a 5 L 4-neck flask and stirred at room temperature. After 41 g (0.50 mol) of 2-cyclopenten-1-one was dissolved in 1 L of dehydrated toluene, the solution was added to the reaction solution previously prepared and stirred at room temperature for 40 minutes. Subsequently, 240.4 g (3.0 mol) of 1,3-cyclohexadiene was added, the internal temperature was raised to 60°C, and stirring was conducted under heating for 12 hours. After the reaction finished, the solution was cooled using an ice bath, and 2 L of 1 N hydrochloric acid was added. Then, stirring was conducted at room temperature for 30 minutes. The solution was separated into an organic layer and a water layer, and the collected water layer was extracted twice with 500 mL of toluene. The gathered organic layer was subjected to liquid-separation and washing once with 1 L of brine and once with 1 L of a saturated sodium hydrogen carbonate aqueous solution. The organic layer was dried over sodium sulfate and filtered, and then the filtrate was concentrated in a rotary evaporator. The resultant crude product was purified by silica gel column chromatography (hexane:ethyl acetate = 20:1) to give 36 g (yield 44%) of the compound 112 as an isomer mixture.

<Synthesis of compound 113>

A compound 113 shown below was synthesized by a method mentioned below.

[Chem. 220]

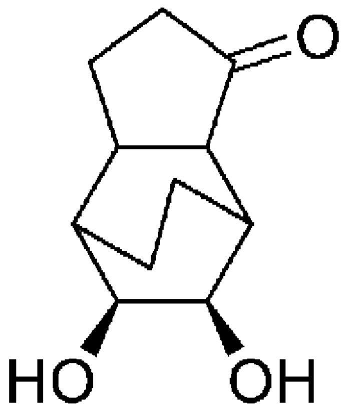

(Compound 113)

Under a nitrogen atmosphere, 35.5 g (219 mmol) of the compound 112, 50 mL (241 mmol) of a 50% NMO aqueous solution, 81 mL of pure water, 81 mL of acetone, and 162 mL of tert-butyl alcohol were added to a 500 mL 4-neck flask and stirred. Subsequently, 170 mg (0.66 mmol) of osmium oxide was added, and stirring was conducted under heating at an internal temperature of 40°C for 40 hours. After the reaction finished, 2.7 g of sodium hydrosulfite, 33 g of Florisil, and 216 mL of pure water were added, and stirring was conducted for 30 minutes. The filtrate was collected by filtration under reduced pressure, and the pH of the filtrate was adjusted to 7 using 1 N sulfuric acid. The organic solvent was removed from the filtrate under reduced pressure at an external temperature of 40°C, and the pH

of the remaining aqueous solution was adjusted to 3 again with 1 N sulfuric acid. Excess sodium chloride and 600 mL of ethyl acetate were added and stirred, the solution was filtered under reduced pressure, and undissolved sodium chloride was filtered off. The solution was liquid-separated into an organic layer and a water layer, and the collected water layer was extracted 3 times with 600 mL of ethyl acetate. The organic layer was gathered, dried over sodium sulfate, and filtered. Then, the filtrate was concentrated in a rotary evaporator. The resultant crude product was purified by silica gel column chromatography (ethyl acetate:dichloromethane = 1:1) and washing using a hexane solvent to give 27.0 g (yield 64%) of the compound 113.

<Synthesis of compound 114>

A compound 114 shown below was synthesized by a method mentioned below.

[Chem. 221]

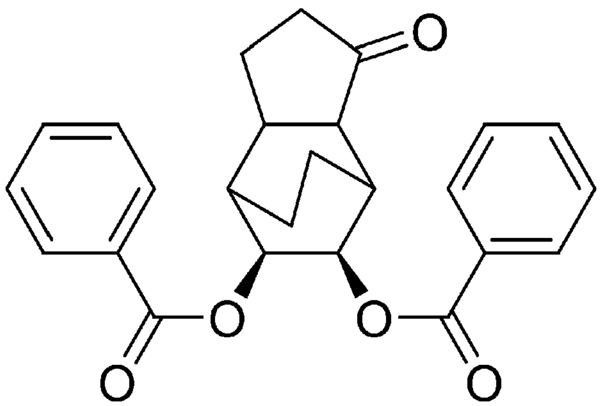

(Compound 114)

Under a nitrogen atmosphere, 4.0 g (20.4 mmol) of the compound 113 was added to a 100 mL 3-neck flask followed by addition of 10 mL of dehydrated pyridine, and stirring was conducted. Under cooling in an ice bath, 4.89 mL of benzoyl chloride (42.1 mmol) was slowly added. After the addition, the temperature was raised to room temperature, and stirring was conducted overnight. Under cooling in an ice bath again, 5 mL of methanol was added for quenching. Thereafter, water and ethyl acetate were added, and the solution was separated into a water layer and an organic layer. The organic layer was washed once each with a saturated ammonium chloride aqueous solution, a saturated sodium hydrogen carbonate aqueous solution, and brine in the order mentioned. After the organic layer was dried over magnesium sulfate and filtered off, the organic layer was concentrated in a rotary evaporator. The crude product was purified by silica gel column chromatography (hexane:ethyl acetate = 10:1 → 5:1) to give 7.49 g (yield 910, pale yellow solid) of the compound 114. The $^1$H-NMR data of the compound 114 obtained are shown below.

$^1$H NMR (270 MHz, $CDCl_3$, TMS as an internal standard): δ 1.48-1.55 (overlapping with signal of $H_2O$, m, 2H), 1.98-2.54 (m, 9H), 2.68-2.81 (m, 1H), 5.01-5.05 (m, 1H), 5.52-5.56 (m, 1H), 7.18-7.34 (overlapping with signal of $CHCl_3$, m, 4H), 7.41-7.52 (m, 2H), 7.79-7.92 (m, 4H).

The melting completion temperature of the compound 114 obtained was 122°C.

[Example A53]

<Synthesis of compound 115>

A compound 115 shown below was synthesized by a method mentioned below.

[Chem. 222]

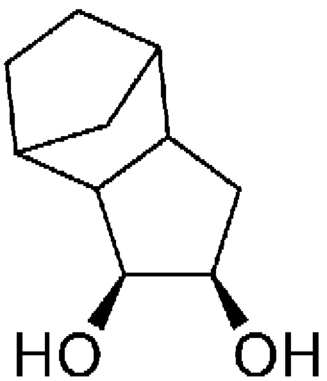

(Compound 115)

Under a nitrogen atmosphere, 2.68 g (20 mmol) of 5,6-dihydrodicyclopentadiene, 100 mL of tert-butyl alcohol, and 40 mL of water were added to a 1 L 3-neck flask, and the reaction solution was cooled to 0°C. 1.0 g (25 mmol) of sodium hydroxide and 4.74 g (30 mmol) of potassium permanganate were dissolved in 100 mL of water, and the solution was slowly added dropwise to the reaction solution previously prepared. After the dropwise addition finished, stirring was conducted at the same temperature for an hour, and a saturated sodium metabisulfite aqueous solution was added dropwise until the color of unreacted potassium permanganate disappeared. After stirring was conducted at room temperature for a while, sodium hydrogen carbonate was added until the pH of the reaction solution reached approximately 7 to 8, and the resulting white precipitate was removed by filtration. The filtered solution was extracted with ethyl acetate 3 times. The gathered organic layer was dried over sodium sulfate and then concentrated in a rotary evaporator. The crude product (2.51 g) containing the compound 115 was used in the next <Synthesis of compound 116> without further purification.

<Synthesis of compound 116>

A compound 116 shown below was synthesized by a method mentioned below.

[Chem. 223]

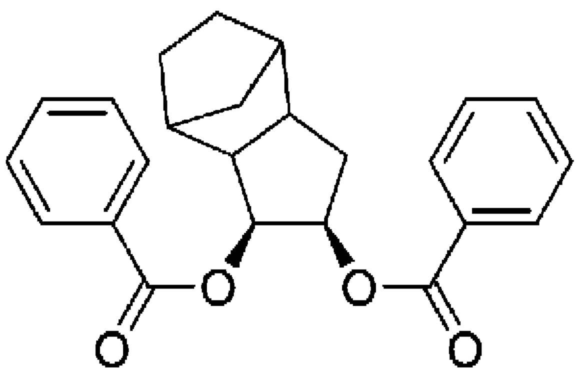

(Compound 116)

Under a nitrogen atmosphere, 2.51 g of the resultant crude product in <Synthesis of compound 115> was added to a 100 mL 3-neck flask followed by addition of 10 mL of dehydrated pyridine, and stirring was conducted. Under cooling in an ice bath, 4.04 mL (34.8 mmol) of benzoyl chloride was slowly added. After the addition, the temperature was raised to room temperature, and stirring was conducted overnight. Under cooling in an ice bath again, 10 mL of methanol was added for quenching. Thereafter, water and dichloromethane were added, and the solution was separated into a water layer and an organic layer. The organic layer was washed with a saturated ammonium chloride aqueous solution twice. The organic layer was dried over sodium sulfate and filtered off. Then, the organic layer was concentrated in a rotary evaporator. The crude product was purified by silica gel column chromatography twice (first time: hexane:ethyl acetate = 10:1, second time: hexane:ethyl acetate = 20:1) to give 3.03 g of the compound 116 (colorless liquid). The $^1$H-NMR data of the compound 116 obtained are shown below.

$^1$H NMR (270 MHz, $CDCl_3$, TMS as an internal standard): $\delta$ 1.50-1.61 (overlapping with signal of $H_2O$, m, 6H), 1.97-2.14 (m, 2H), 2.25 (br s, 1H), 2.44 (br s, 1H), 2.61-2.69 (m, 1H), 2.72-2.83 (m, 1H), 5.47 (t, J = 4.0 Hz, 1H), 5.61-5.67

(m, 1H), 7.32-7.40 (m, 4H), 7.48-7.55 (m, 2H), 7.92-7.98 (m, 4H).

[Example A54]

<Preparation of solid titanium catalyst component [$\alpha$1]>

After a 1 L glass vessel was sufficiently purged with nitrogen, 85.8 g of anhydrous magnesium chloride, 321 g of decane, and 352 g of 2-ethylhexyl alcohol were placed thereinto and subjected to a heating reaction at 130°C for 3 hours to give a homogeneous solution. 241 g of this solution and 6.43 g of ethyl benzoate were added to the glass vessel and mixed under stirring at 50°C for an hour. The homogeneous solution thus obtained was cooled to room temperature. Then, 38.3 mL of this entire homogeneous solution was added dropwise into 100 mL of titanium tetrachloride retained at -20°C under stirring at a speed of 350 rpm for 45 minutes. After the addition was finished, the temperature of this mixture was raised to 80°C over 3.8 hours. When the temperature reached 80°C, 0.97 g of the compound 6 was added into the mixture. The temperature was raised again to 120°C over 40 minutes, and the same temperature was retained under stirring for 35 minutes. After the reaction finished, the solid portion was recovered by hot filtration. This solid portion was resuspended in 100 mL of titanium tetrachloride, and then the suspension was subjected to a heating reaction again at 120°C for 35 minutes. After the reaction finished, the solid portion was recovered again by hot filtration and sufficiently washed with decane at 100°C and decane at room temperature until no free titanium compound was detected in the decane solution after washing the solid. A solid titanium catalyst component [$\alpha$1] prepared by the procedure above was preserved as a decane slurry, and a portion thereof was dried in order to examine the catalyst composition. The composition of the solid titanium catalyst component [$\alpha$1] thus obtained included 0.28% by mass titanium, 1.7% by mass magnesium, and 0.12% by mass 2-ethylhexyl alcohol residues.

<Main polymerization>

After the addition of 500 g of propylene and 1 NL of hydrogen at room temperature to a two-liter autoclave, the mixture which was obtained by mixing 7 mL of heptane, 0.5 mmol of triethyl aluminum, 0.08 mmol of cyclohexylmethyldimethoxysilane, and 0.004 mmol of solid titanium catalyst component [$\alpha$1] (in terms of titanium atom) at 25°C for 10 minutes was added thereto, and the temperature inside the autoclave was raised quickly to 70°C. After polymerization at 70°C for 1.5 hours, the reaction was stopped by the addition of a small amount of methanol, and then propylene was purged. Finally, the obtained polymer particles were dried under reduced pressure at 80°C overnight. The polymerization results are as follows.

Activity: 48.6 kg-PP/g-catalyst
Bulk specific gravity: 490 kg/$m^3$
MFR (ASTM1238E standard, 230°C, 2.16 kg load): 0.57 g/10 minutes
Amount of decane-insoluble component: 1.87 wt%
Tm: 163.92°C
Tmf: 172.08°C
Mw/Mn: 10.64
Mz/Mw: 4.85
Methods for measuring the physical properties described above are as follows.

(1) Bulk specific gravity:
The bulk specific gravity was measured in accordance with JIS K-6721.
(2) Melt flow rate (MFR):
In accordance with ASTM D1238E, the measurement temperature for a propylene polymer was set at 230°C.
(3) Amount of decane-soluble (insoluble) component:
To a glass measurement vessel, about 3 g of propylene polymer (It was measured to the unit of $10^{-4}$ g. This weight was denoted as b (g) in the following equation.), 500 mL of decane, and a small amount of a heat-resistant stabilizer soluble in decane were loaded. Under a nitrogen atmosphere, the temperature was raised to 150°C over 2 hours under stirring with a stirrer to dissolve the propylene polymer, the temperature was retained at 150°C for 2 hours, and then the solution was gradually cooled to 23°C over 8 hours. The liquid containing the precipitate of the propylene polymer obtained was filtered under reduced pressure with a 25G-4 standard glass filter manufactured by Iwata Garasu Corporation. 100 mL of the filtrate was harvested and dried under reduced pressure to give a portion of a decane soluble component. The weight thereof was weighed to the $10^{-4}$ g unit (this weight was denoted as a(g) in the following equation). After this operation, the amount of the decane-soluble component was determined by the following equation.

$$\text{Decane-soluble component content} = 100 \times (500 \times a) / (100 \times b)$$

$$\text{Decane-insoluble component content} = 100 - 100 \times (500 \times a) / (100 \times b)$$

(4) Molecular weight distribution:

Gel permeation chromatograph: HLC-8321 GPC/HT model manufactured by TOSOH CORPORATION
Detector: differential refractometer
Column: TSKgel GMH6-HT × 2 and TSKgel GMH6-HTL × 2 manufactured by TOSOH CORPORATION were serially connected.
Mobile phase medium: o-dichlorobenzene
Flow rate: 1.0 mL/minute
Measurement temperature: 140°C
Method of creating calibration curve: a standard polystyrene sample was used.
Sample concentration: 0.1% (w/w)
Amount of sample solution: 0.4 mL
Measurement was conducted under the conditions described above. The chromatogram obtained was analyzed by a known method to calculate the weight average molecular weight (Mw), the number average molecular weight (Mn), the Z average molecular weight (Mz), and the Mw/Mn value and Mz/Mw value as indices for the molecular weight distribution (MWD). The measurement time for one sample was 60 minutes.

(5) Melting point of polymer (Tm):
The melting point (Tm), crystallization temperature (Tc), and amount of heat of fusion (ΔH) of the polymer in the present invention were measured by a differential scanning calorimeter (DSC) in a DSC220C apparatus manufactured by Seiko Instruments Inc. 3 to 10 mg of a specimen was sealed in an aluminum pan and heated from room temperature to 200°C at 100°C/minute. The specimen was retained at 200°C for 5 minutes and then cooled to 30°C at 10°C/minute. The peak temperature in this cooling test was defined as the crystallization temperature (Tc). Subsequently, after retained at 30°C for 5 minutes, the specimen was subjected to a second heating to 200°C at 10°C/minute. In this second heating test, the peak temperature was employed as the melting point (Tm), and the heat generation quantity was employed as the amount of heat of fusion (ΔH).

The final melting point (Tmf) of the polymer in the present invention was measured by a differential scanning calorimeter (DSC) in a DSC220C apparatus manufactured by Seiko Instruments Inc. 3 to 10 mg of a specimen was sealed in an aluminum pan and heated from room temperature to 240°C at 80°C/minute. The specimen was retained at 240°C for 1 minute and then cooled to 0°C at 80°C/minute. After retained at 0°C for 1 minute, the specimen was heated to 150°C at 80°C/minute and retained for 5 minutes. Finally, the specimen was heated to 180°C at 1.35°C/minute, and the intersection of the tangent of the inflection point on the high-temperature side of the peak provided in this final heating test and the base line was employed as the final melting point (Tmf).

Tmf can be considered one parameter for evaluating the ease of crystallization and the crystal structure, for example, of a polymer in the ultra-high molecular weight region, which is said to tend to be difficult to crystallize. More specifically, it can be considered that, as this Tmf value is higher, the ultra-high molecular weight polymer component is more likely to form crystals that is strong and has high heat resistance.

[Example B1]

<Synthesis of compounds 201 and 202>

Synthesis of compounds 201 and 202 shown below was conducted by a method mentioned below.

[Chem. 224]

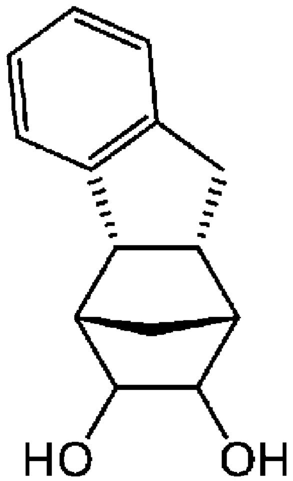

(Compound 201)

In (Compound 201), the thick line represents the near side of the paper plane, the dotted line represents the far side of the paper plane, and the compound 201 corresponds to diol compound derived from the endo form shown in the formula (34) above.

[Chem. 225]

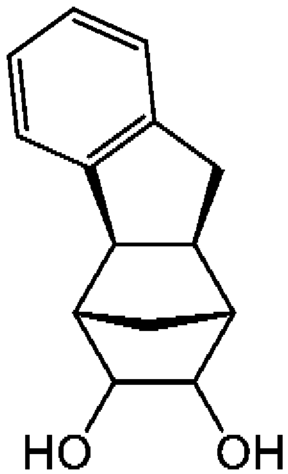

(Compound 202)

In (Compound 202), the thick line represents the near side of the paper plane, and the compound 202 corresponds to diol compound derived from the exo form shown in the formula (34) above.

A 2-liter flask was equipped with a mechanical stirrer and purged with nitrogen by allowing nitrogen to flow inside. 25.4 g of an olefin ((a compound in which, in the formula (33), $R^4$, $R^9$, and $R^{31}$ to $R^{34}$ are hydrogen atoms, and X is $CH_2$), 440 ml of tert-butyl alcohol, and 110 ml of water were added into the flask, and the internal temperature was cooled to 0°C. To another 1 L beaker, 30 g of potassium permanganate, 600 ml of water, and 6.60 g of sodium hydroxide were added to prepare a potassium permanganate alkali aqueous solution. The 2-liter flask was equipped with a dropping funnel, and the potassium permanganate alkali aqueous solution prepared was loaded to the dropping funnel. The potassium permanganate alkali aqueous solution was slowly added dropwise such that the internal temperature did not exceed 5°C. After the dropwise addition finished, stirring was conducted at an internal temperature of 0°C for an hour. To another flask, a saturated sodium metabisulfite aqueous solution was prepared and slowly added dropwise to the previous reaction solution until a white precipitate was formed. After the dropwise addition, the temperature was raised to room temperature to cause a white solid to precipitate. The supernatant organic layer was collected, and then an operation of extraction from the water layer with ethyl acetate was conducted twice. The organic layer was combined and washed with water and brine. The organic layer was dried over magnesium sulfate. Subsequently, the organic layer was concentrated to give 27.41 g of a crude product. The resultant crude product was purified by silica gel column

chromatography to give 22.71 g of an intended product (isomer mixture). The product obtained was purified again by a silica gel column to separate isomers, and 10.9 g of the compound 201 and 2.9 g of the compound 202 were isolated. The $^1$H-NMR data of the compounds 201 and 202 obtained are shown below.

(Compound 201)

$^1$H NMR (270 MHz, $CDCl_3$, TMS as an internal standard): δ 1.39-1.51 (m, 1 H), 1.89-2.01 (m, 1 H), 2.19-2.27 (m, 1 H), 2.30-2.38 (m, 1 H), 2.47-2.58 (m, 2 H), 2.70-3.08 (m, 3H), 3.21-3.32 (m, 1H), 3.58-3.76 (m, 2 H), 7.06-7.36 (m, 4 H).

(Compound 202)

$^1$H NMR (270 MHz, $CDCl_3$, TMS as an internal standard): δ 1.00-1.10 (m, 1 H), 1.55-1.64 (m, 1 H), 2.07 (br s, 1 H), 2.23-2.35 (m, 2 H), 2.50 (dd, J = 14.5, 5.3 Hz, 2 H) 2.63 (dd, J = 17.1, 3.6 Hz 1H), 3.06 (d, J = 7.9, 1 H), 3.28 (dd, J = 17.4, 10.5Hz 1 H), 3.79-3.87 (m, 1 H), 3.88-3.96 (m, 1 H), 7.09-7.20 (m, 4 H).

<Synthesis of compound 203>

Synthesis of a compound 203 shown below was conducted by a method mentioned below.

[Chem. 226]

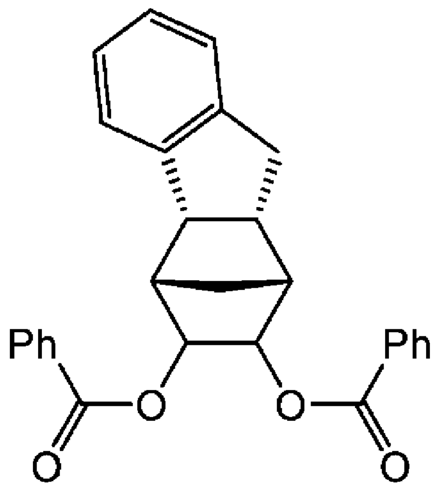

(Compound 203)

The inside of a 200 ml flask was purged with nitrogen, 5 g of the compound 201 was added followed by addition of 30 ml of dehydrated pyridine. Under cooling in an ice bath, 5.69 ml of benzoyl chloride was slowly added dropwise. After the dropwise addition, the temperature was raised to room temperature, and stirring was conducted overnight. Under cooling again in an ice bath, methanol was added for quenching. After chloroform and water were added and stirred, the organic layer was separated. The organic layer was washed with a saturated ammonium chloride aqueous solution and brine and then dried over magnesium sulfate. Subsequently, the organic layer was concentrated to give 10.61 g of a crude product. The crude product was purified by silica gel column chromatography to give 6.83 g of the compound 203. The $^1$H-NMR data of the compound 203 obtained are shown below.

(Compound 203)

$^1$H NMR (270 MHz, $CDCl_3$, TMS as an internal standard): δ 1.68-1.74 (m, 1 H), 2.29-2.34 (m, 1 H), 2.57-2.59 (m, 1 H), 2.90-3.20 (m, 4 H), 3.83 (dd, J = 10.2, 5.6 Hz, 1 H) 4.66 (dd, J = 5.9, 1.3 Hz 1H), 5.03 (dd, J = 5.9, 1.7 Hz 1 H), 7.20-7.49 (m, 10 H), 7.79-7.86 (m, 4 H).

The melting completion temperature of the compound 203 was 108.7°C.

<Synthesis of compound 204>

Synthesis of a compound 204 shown below was conducted by a method mentioned below.

[Chem. 227]

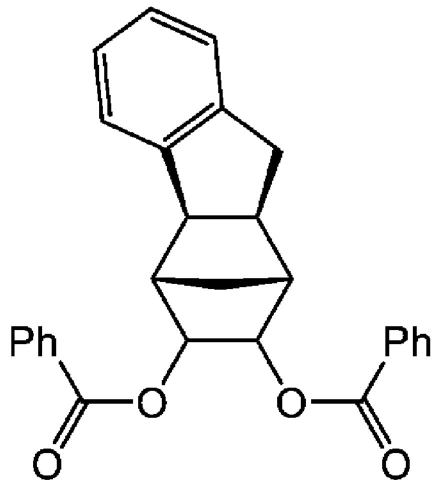

(Compound 204)

3.85 g (yield 89%) of the compound 204 was obtained in accordance with the operation and equivalence relationship described in <Synthesis of compound 203> except that 2.2 g of the compound 202 was used instead of using the compound 201 in <Synthesis of compound 203>. The $^1$H-NMR data of the compound 204 obtained are shown below.

(Compound 204)

$^1$H NMR (270 MHz, $CDCl_3$, TMS as an internal standard): δ 1.27-1.33 (m, 1 H), 1.92-1.98 (m, 1 H), 2.44 (br s, 1 H), 2.57-2.76 (m, 3 H), 3.34-3.45 (m, 2 H) 5.19-5.23 (m, 1H), 5.30-5.35 (m, 1 H), 7.17-7.32 (m, 8 H), 7.43-7.52 (m, 2 H), 7.84-7.92 (m, 4 H) .

The melting completion temperature of the compound 204 was 168.2°C.

[Example B2]

<Preparation of solid titanium catalyst component [α1]>

After a 1 L glass vessel was sufficiently purged with nitrogen, 85.8 g of anhydrous magnesium chloride, 321 g of decane, and 352 g of 2-ethylhexyl alcohol were placed thereinto and subjected to a heating reaction at 130°C for 3 hours to give a homogeneous solution. 241 g of this solution and 6.43 g of ethyl benzoate were added to the glass vessel and mixed under stirring at 50°C for an hour. The homogeneous solution thus obtained was cooled to room temperature. Then, 38.3 ml of this entire homogeneous solution was added dropwise into 100 ml of titanium tetrachloride retained at -20°C under stirring at a speed of 350 rpm for 45 minutes. After the addition was finished, the temperature of this mixture was raised to 80°C over 3.8 hours. When the temperature reached 80°C, 0.91 g of the compound 203 was added into the mixture. The temperature was raised again to 120°C over 40 minutes, and the same temperature was retained under stirring for 35 minutes. After the reaction finished, the solid portion was recovered by hot filtration. This solid portion was resuspended in 100 ml of titanium tetrachloride, and then the suspension was subjected to a heating reaction again at 120°C for 35 minutes. After the reaction finished, the solid portion was recovered again by hot filtration and sufficiently washed with decane at 100°C and decane at room temperature until no free titanium compound was detected in the decane solution after washing the solid. A solid titanium catalyst component [α1] prepared by the procedure above was preserved as a decane slurry.

<Main polymerization>

After the addition of 500 g of propylene and 1 NL of hydrogen at room temperature to a two-liter autoclave, the mixture which was obtained by mixing 7 ml of heptane, 0.5 mmol of triethyl aluminum, 0.08 mmol of cyclohexylmethyldimethoxysilane, and 0.004 mmol of solid titanium catalyst component [α1] (in terms of titanium atom) at 25°C for 10 minutes was added thereto, and the temperature inside the autoclave was raised quickly to 70°C. After polymerization at 70°C for 1.5 hours, the reaction was stopped by the addition of a small amount of methanol, and then propylene was purged. Finally, the obtained polymer particles were dried under reduced pressure at 80°C overnight. The polymerization

results are as follows.

Activity: 72.7 kg-PP/g-catalyst
Bulk specific gravity: 490 kg/$m^3$
MFR (ASTM1238e standard, 230°C, 2.16 kg load): 0.45 g/10 minutes
Amount of decane-insoluble component: 0.49 wt%
Tm: 165.39°C
Tmf: 172.33°C
Mw/Mn: 11.25
Mz/Mw: 4.41
Methods for measuring the physical properties are as described in Example A54.

[Example B3]

<Synthesis of compound 205>

Synthesis of a compound 205 shown below was conducted by a method mentioned below.

[Chem. 228]

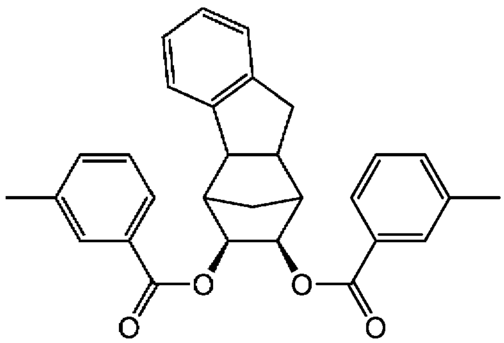

(Compound 205)

The inside of a 100 ml flask was purged with nitrogen, 3.0 g (1 equivalent) of a mixture of the compound 201 and compound 202 was added, and then 15 ml of dehydrated pyridine and 15 ml of chloroform were added. Under cooling in an ice bath, 4.5 g (2.1 equivalents) of 3-methylbenzoyl chloride was slowly added dropwise. After the dropwise addition, the temperature was raised to room temperature, and stirring was conducted overnight. Under cooling again in an ice bath, methanol was added for quenching. After chloroform and water were added and stirred, the organic layer was separated. The organic layer was washed with a saturated ammonium chloride aqueous solution and brine and then dried over magnesium sulfate. Subsequently, the organic layer was concentrated to give 6.78 g of a crude product. The crude product was purified by silica gel column chromatography to give 5.94 g (yield 95%) of the compound 205 (endo form:exo form = 86:14) as an isomer mixture of an endo form and an exo form. The $^1$H-NMR data of the compound 205 obtained are shown below.

(Compound 205)

$^1$H NMR (270 MHz, $CDCl_3$, TMS as an internal standard): endo-form: $\delta$ 1.68-1.73 (m, 1 H), 2.13-2.19 (m, 6 H), 2.30-2.35 (m, 1 H), 2.57-2.59 (m, 1 H), 2.89-3.20 (m, 4 H), 3.80-3.86 (dd, J = 10.6, 5.9 Hz, 1 H), 4.63-4.65 (m, 1 H), 5.00-5.03 (m, 1 H), 7.14-7.34 (m, 8 H), 7.56-7.76 (m, 4 H); exo-form: 1.26-1.32 (m, 1 H), 1.92-1.96 (m, 1 H), 2.13-2.19 (m, 6 H), 2.42 (br s, 1 H), 2.61-2.74 (m, 3 H), 3.33-3.43 (m, 2 H), 5.16-5.18 (m, 1 H), 5.27-5.30 (m, 1 H), 7.14-7.34 (m, 8 H), 7.56-7.76 (m, 4 H).

The melting completion temperature of the compound 205 was 116.0°C.

[Example B4]

<Synthesis of compound 206>

Synthesis of a compound 206 shown below was conducted by a method mentioned below.

[Chem. 229]

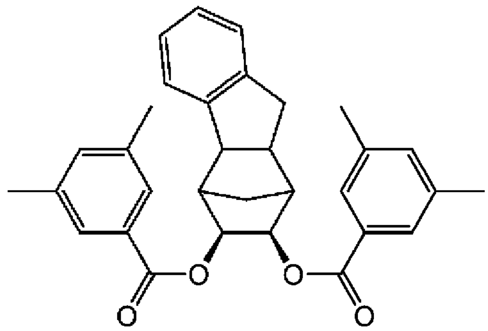

(Compound 206)

6.37 g (yield 96%) of the compound 206 (endo form:exo form = 85:15) was obtained as an isomer mixture of an endo form and an exo form in accordance with the operation and equivalence relationship described in <Synthesis of compound 205> except that 4.91 g (2.1 equivalents) of 3,5-dimethylbenzoyl chloride was used instead of using 3-methylbenzoyl chloride in <Synthesis of compound 205>. The $^1$H-NMR data of the compound 206 obtained are shown below.

(Compound 206)

$^1$H NMR (270 MHz, $CDCl_3$, TMS as an internal standard): endo-form: δ 1.68-1.72 (m, 1 H), 2.13-2.18 (m, 12 H), 2.31-2.34 (m, 1 H), 2.56-2.58 (m, 1 H), 2.88-3.19 (m, 4 H), 3.79-3.85 (dd, J = 10.6, 5.6 Hz, 1 H), 4.60-4.63 (m, 1 H), 4.98-5.00 (m, 1 H), 7.07-7.09 (m, 2 H), 7.17-7.32 (m, 4 H), 7.42-7.50 (m, 4 H); exo-form: 1.24-1.29 (m, 1 H), 1.90-1.96 (m, 1 H), 2.13-2.18 (m, 12 H), 2.42 (br s, 1 H), 2.60-2.74 (m, 3 H), 3.33-3.43 (m, 2 H), 5.14-5.16 (m, 1 H), 5.24-5.27 (m, 1 H), 7.07-7.09 (m, 2 H), 7.17-7.32 (m, 4 H), 7.42-7.50 (m, 4 H).

The peaks considered to be the melting points of the compound 206 were observed at 153.0°C and 191.4°C.

[Example B5]

<Synthesis of compound 207>

Synthesis of a compound 207 shown below was conducted by a method mentioned below.

[Chem. 230]

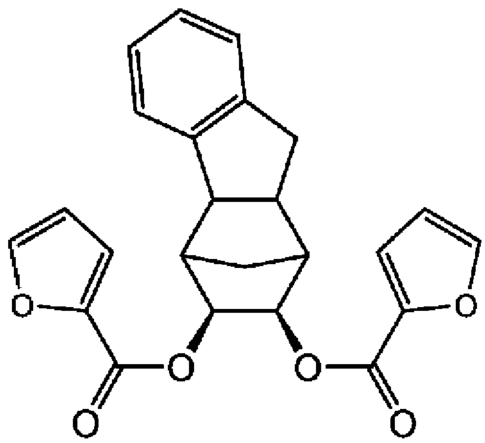

(Compound 207)

5.40 g (yield 94%) of the compound 207 (endo form:exo form = 86:14) was obtained as an isomer mixture of an endo form and an exo form in accordance with the operation and equivalence relationship described in <Synthesis of compound 205> except that 3.80 g (2.1 equivalents) of 2-furoyl chloride was used instead of using 3-methylbenzoyl chloride in <Synthesis of compound 205>. The $^1$H-NMR data of the compound 207 obtained are shown below.

(Compound 207)

$^1$H NMR (270 MHz, $CDCl_3$, TMS as an internal standard): endo-form: δ 1.66-1.70 (m, 1 H), 2.24-2.28 (m, 1 H), 2.54-2.55 (m, 1 H), 2.85-3.08 (m, 4 H), 3.78-3.83 (dd, J = 10.6, 5.6 Hz, 1 H), 4.55-4.57 (m, 1 H), 4.95-4.97 (m, 1 H), 6.37-6.42 (m, 2 H), 6.86-6.97 (m, 2 H), 7.16-7.30 (m, 4 H), 7.44-7.49 (m, 2 H); exo-form: 1.23-1.28 (m, 1 H), 1.86-1.91 (m, 1 H), 2.39 (br s, 1 H), 2.57-2.72 (m, 3 H), 3.31-3.42 (m, 2 H), 5.11-5.13 (m, 1 H), 5.22-5.24 (m, 1 H), 6.37-6.42 (m, 2 H), 6.86-6.97 (m, 2 H), 7.16-7.30 (m, 4 H), 7.44-7.49 (m, 2 H).

The peaks considered to be the melting points of the compound 207 were observed at 107.6°C and 120.5°C.

[Example B6]

<Synthesis of compound 208>

Synthesis of a compound 208 shown below was conducted by a method mentioned below.

[Chem. 231]

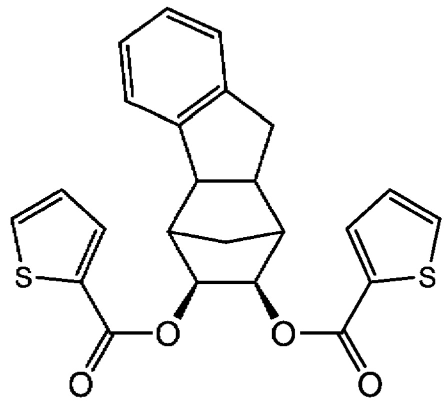

(Compound 208)

5.72 g (yield 94%) of the compound 208 (endo form:exo form = 86:14) was obtained as an isomer mixture of an endo form and an exo form in accordance with the operation and equivalence relationship described in <Synthesis of compound 205> except that 4.27 g (2.1 equivalents) of 2-thenoyl chloride was used instead of using 3-methylbenzoyl chloride in <Synthesis of compound 205>. The $^1$H-NMR data of the compound 208 obtained are shown below.

(Compound 208)

$^1$H NMR (270 MHz, $CDCl_3$, TMS as an internal standard): endo-form: δ 1.66-1.70 (m, 1 H), 2.24-2.29 (m, 1 H), 2.55-2.56 (m, 1 H), 2.87-3.14 (m, 4 H), 3.78-3.84 (dd, J = 10.2, 5.6 Hz, 1 H), 4.57-4.59 (m, 1 H), 4.94-4.97 (m, 1 H), 6.95-7.01 (m, 2 H), 7.16-7.32 (m, 4 H), 7.43-7.48 (m, 2 H), 7.58-7.67 (m, 2 H); exo-form: 1.24-1.28 (m, 1 H), 1.87-1.92 (m, 1 H), 2.40 (br s, 1 H), 2.56-2.73 (m, 3 H), 3.32-3.42 (m, 2 H), 5.11-5.14 (m, 1 H), 5.23-5.25 (m, 1 H), 6.95-7.01 (m, 2 H), 7.16-7.32 (m, 4 H), 7.43-7.48 (m, 2 H), 7.58-7.67 (m, 2 H).

The peaks considered to be the melting points of the compound 208 were observed at 110.9°C and 141.5°C.

[Example B7]

<Synthesis of compound 209>

Synthesis of a compound 209 shown below was conducted by a method mentioned below.

[Chem. 232]

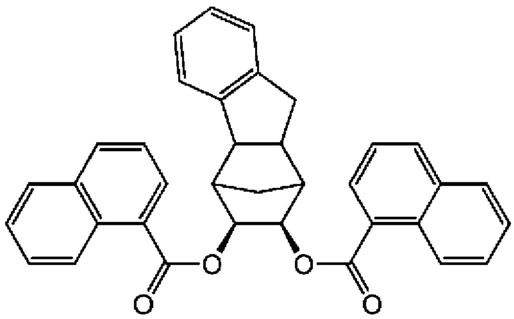

(Compound 209)

5.77 g (yield 88%) of the compound 209 (endo form:exo form = 88:12) was obtained as an isomer mixture of an endo form and an exo form in accordance with the operation and equivalence relationship described in <Synthesis of compound 205> except that 5.0 g (2.1 equivalents) of 1-naphthoyl chloride was used instead of using 3-methylbenzoyl chloride in <Synthesis of compound 205>. The $^1$H-NMR data of the compound 209 obtained are shown below.

(Compound 209)

$^1$H NMR (270 MHz, $CDCl_3$, TMS as an internal standard): endo-form: δ 1.75-1.79 (m, 1 H), 2.36-2.40 (m, 1 H), 2.69-2.70 (m, 1 H), 2.96-3.29 (m, 4 H), 3.86-3.92 (dd, J = 10.2, 5.6 Hz, 1 H), 4.82-4.85 (m, 1 H), 5.19-5.22 (m, 1 H), 6.98-7.43 (m, 10 H), 7.73-7.98 (m, 6 H), 8.70-8.80 (m, 2 H); exo-form: 1.33-1.37 (m, 1 H), 1.99-2.03 (m, 1 H), 2.53 (br s, 1 H), 2.70-2.79 (m, 3 H), 3.38-3.47 (m, 2 H), 5.36-5.38 (m, 1 H), 5.48-5.50 (m, 1 H), 6.98-7.43 (m, 10 H), 7.73-7.98 (m, 6 H), 8.70-8.80 (m, 2 H).

The melting completion temperature of the compound 209 was 149.8°C.

[Example B8]

<Synthesis of compound 210>

Synthesis of a compound 210 shown below was conducted by a method mentioned below.

[Chem. 233]

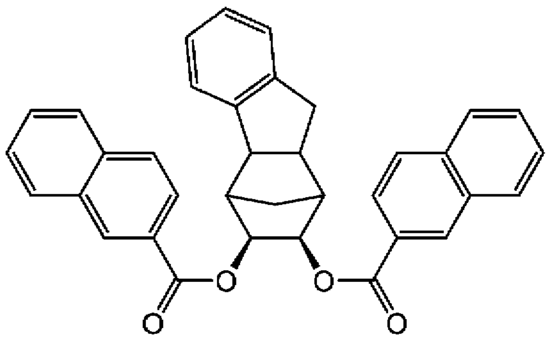

(Compound 210)

4.89 g (yield 75%) of the compound 210 including an endo form only was obtained in accordance with the operation and equivalence relationship described in <Synthesis of compound 205> except that 5.0 g (2.1 equivalents) of 2-naphthoyl chloride was used instead of using 3-methylbenzoyl chloride in <Synthesis of compound 205>. The $^1$H-NMR data of the compound 210 obtained are shown below.

(Compound 210)

$^1$H NMR (270 MHz, $CDCl_3$, TMS as an internal standard): δ 1.76-1.80 (m, 1 H), 2.43-2.47 (m, 1 H), 2.67-2.68 (m, 1 H), 2.95-3.25 (m, 4 H), 3.85-3.91 (dd, J = 10.6, 5.9 Hz, 1 H), 4.74-4.76 (m, 1 H), 5.11-5.14 (m, 1 H), 7.21-7.54 (m,

10 H), 7.64-7.80 (m, 4 H), 7.88-7.95 (m, 2 H), 8.27-8.35 (m, 2 H).

The melting completion temperature of the compound 210 was 173.8°C.

[Example B9]

<Synthesis of compound 211>

Synthesis of a compound 211 shown below was conducted by a method mentioned below.

[Chem. 234]

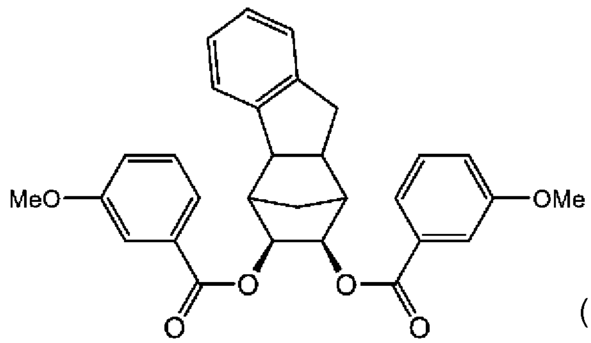

(Compound 211)

4.70 g (yield 70%) of the compound 211 (endo form:exo form = 80:20) was obtained as an isomer mixture of an endo form and an exo form in accordance with the operation and equivalence relationship described in <Synthesis of compound 205> except that 5.0 g (2.1 equivalents) of 3-methoxybenzoyl chloride was used instead of using 3-methylbenzoyl chloride in <Synthesis of compound 205>. The $^1$H-NMR data of the compound 211 obtained are shown below.

(Compound 211)

$^1$H NMR (270 MHz, $CDCl_3$, TMS as an internal standard): endo-form: $\delta$ 1.69-1.72 (m, 1 H), 2.28-2.32 (m, 1 H), 2.57-2.58 (m, 1 H), 2.88-3.18 (m, 4 H), 3.60-3.66 (m, 6 H), 3.80-3.86 (dd, J = 10.2, 5.6 Hz, 1 H), 4.64-4.66 (m, 1 H), 5.01-5.03 (m, 1 H), 6.98-7.03 (m, 2 H), 7.13-7.37 (m, 8 H), 7.44-7.53 (m, 2 H); exo-form: 1.26-1.30 (m, 1 H), 1.91-1.95 (m, 1 H), 2.42 (br s, 1 H), 2.60-2.73 (m, 3 H), 3.33-3.43 (m, 2 H), 3.60-3.66 (m, 6 H), 5.18-5.20 (m, 1 H), 5.29-5.31 (m, 1 H), 6.98-7.03 (m, 2 H), 7.13-7.37 (m, 8 H), 7.44-7.53 (m, 2 H).

The melting completion temperature of the compound 211 was 124.3°C.

[Example B10]

<Synthesis of compound 212>

Synthesis of a compound 212 shown below was conducted by a method mentioned below.

[Chem. 235]

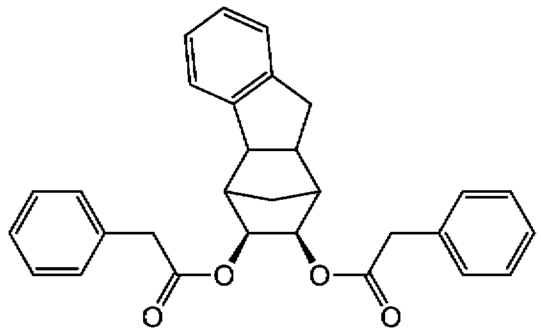

(Compound 212)

1.32 g (yield 210) of the compound 212 (endo form:exo form = 78:22) as an isomer mixture of an endo form and an exo form was obtained as a colorless liquid in accordance with the operation and equivalence relationship described in <Synthesis of compound 205> except that 5.75 g (2.7 equivalents) of phenylacetyl chloride was used instead of using 3-methylbenzoyl chloride in <Synthesis of compound 205>. The $^1$H-NMR data of the compound 212 obtained are shown below.

(Compound 212)

$^1$H NMR (270 MHz, $CDCl_3$, TMS as an internal standard): endo-form: δ 1.52-1.55 (m, 1 H), 1.98-2.02 (m, 1 H), 2.29-2.33 (m, 1 H), 2.63-2.65 (m, 1 H), 2.75-2.97 (m, 3 H), 3.17-3.26 (m, 4 H), 3.66-3.72 (dd, J = 10.6, 5.6 Hz, 1 H), 4.27-4.30 (m, 1 H), 4.64-4.67 (m, 1 H), 7.13-7.33 (m, 14 H); exo-form: 1.09-1.13 (m, 1 H), 1.60-1.64 (m, 1 H), 2.15 (br s, 1 H), 2.33-2.57 (m, 3 H), 3.17-3.26 (m, 6 H), 5.20-5.22 (m, 1 H), 5.31-5.33 (m, 1 H), 7.13-7.33 (m, 14 H).

[Example B11]

<Synthesis of compound 213>

Synthesis of a compound 213 shown below was conducted by a method mentioned below.

[Chem. 236]

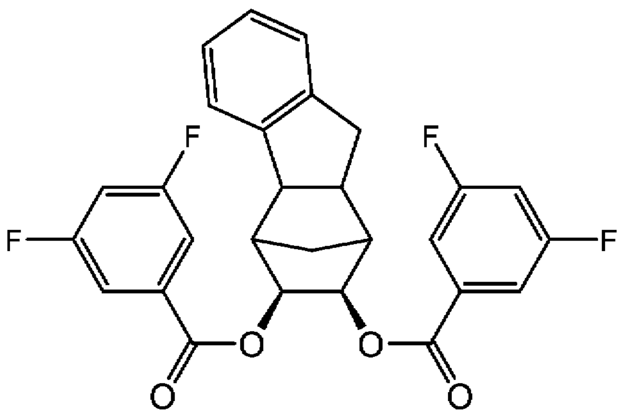

(Compound 213)

6.02 g (yield 87%) of the compound 213 (endo form:exo form = 96:4) as an isomer mixture of an endo form and an exo form was obtained in accordance with the operation and equivalence relationship described in <Synthesis of compound 205> except that 5.14 g (2.1 equivalents) of 3,5-difluorobenzoyl chloride was used instead of using 3-methylbenzoyl chloride in <Synthesis of compound 205>. The $^1$H-NMR data of the compound 213 obtained are shown below.

(Compound 213)

$^1$H NMR (270 MHz, $CDCl_3$, TMS as an internal standard): endo-form: δ 1.74-1.77 (m, 1 H), 2.24-2.29 (m, 1 H), 2.57-2.59 (m, 1 H), 2.89-3.11 (m, 4 H), 3.82-3.88 (dd, J = 10.6, 5.6 Hz, 1 H), 4.62-4.64 (m, 1 H), 5.00-5.02 (m, 1 H), 6.87-6.99 (m, 2 H), 7.18-7.38 (m, 8 H); exo-form: 1.30-1.35 (m, 1 H), 1.87-1.91 (m, 1 H), 2.43 (br s, 1 H), 2.60-2.75 (m, 3 H), 3.35-3.45 (m, 2 H), 5.17-5.20 (m, 1 H), 5.29-5.31 (m, 1 H), 6.87-6.99 (m, 2 H), 7.18-7.38 (m, 8 H).

The melting completion temperature of the compound 213 was 127.7°C.

[Example B12]

<Synthesis of compound 214>

Synthesis of a compound 214 shown below was conducted by a method mentioned below.

[Chem. 237]

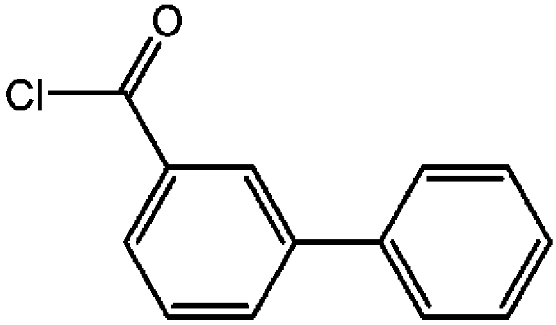

(Compound 214)

The inside of a 100 ml flask was purged with nitrogen, 5.95 g (1 equivalent) of 3-phenylbenzoic acid was added, and then 60 ml of dichloromethane and 2 drops of dimethylformamide were added. Under cooling in an ice bath, 3.09 ml (1.2 equivalents) of oxalyl chloride was slowly added dropwise. After the dropwise addition, the temperature was raised to room temperature, and stirring was conducted for 2 hours. The volatile compound in the reaction system was removed under reduced pressure to give the compound 214. The compound was used in Synthesis of compound 215 without further purification.

<Synthesis of compound 215>

Synthesis of a compound 215 shown below was conducted by a method mentioned below.

[Chem. 238]

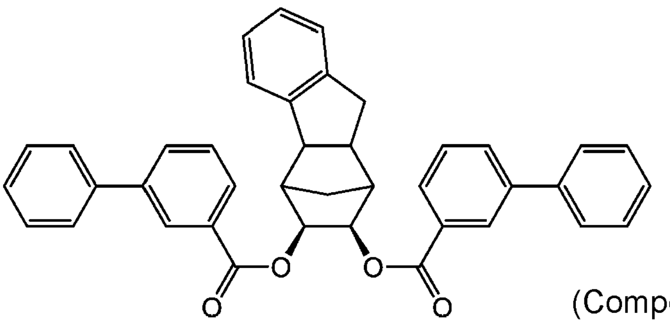

(Compound 215)

3 ml of dehydrated pyridine was added to a 100 ml flask under a nitrogen atmosphere, the flask containing the entire amount (2.2 equivalents) of the compound 214 synthesized in <Synthesis of compound 214>. Under cooling in an ice bath, 10 ml of a solution of 2.92 g (1 equivalent) of the mixture of the compound 201 and compound 202 in dichloromethane was slowly added. After the dropwise addition, the temperature was raised to room temperature, and stirring was conducted overnight. Under cooling again in an ice bath, methanol was added for quenching. After chloroform and water were added and stirred, the organic layer was separated. The organic layer was washed with a saturated ammonium chloride aqueous solution and brine and then dried over magnesium sulfate. Subsequently, the organic layer was concentrated to give 9.87 g of a crude product. The crude product was purified by silica gel column chromatography to give 6.82 g (yield 88%) of the compound 215 (endo form:exo form = 88:12) as an isomer mixture of an endo form and an exo form. The $^1$H-NMR data of the compound 215 obtained are shown below.

(Compound 215)

$^1$H NMR (270 MHz, $CDCl_3$, TMS as an internal standard): endo-form: δ 1.72-1.75 (m, 1 H), 2.33-2.37 (m, 1 H), 2.60-2.61 (m, 1 H), 2.92-3.21 (m, 4 H), 3.82-3.88 (dd, J = 9.9, 5.3 Hz, 1 H), 4.69-4.72 (m, 1 H), 5.08-5.11 (m, 1 H), 7.17-7.39 (m, 16 H), 7.59-7.64 (m, 2 H), 7.81-7.85 (m, 2 H), 8.02-8.07 (m, 2 H); exo-form: 1.26-1.33 (m, 1 H), 1.96-1.99 (m, 1 H), 2.45 (br s, 1 H), 2.63-2.75 (m, 3 H), 3.35-3.45 (m, 2 H), 5.25-5.38 (m, 1 H), 5.36-5.38 (m, 1 H), 7.17-7.39 (m, 16 H), 7.59-7.64 (m, 2 H), 7.81-7.85 (m, 2 H), 8.02-8.07 (m, 2 H).

The compound 215 exhibits behavior of gradually melting from 62.5°C.

[Example B13]

<Synthesis of compound 216>

Synthesis of a compound 216 shown below was conducted by a method mentioned below.

[Chem. 239]

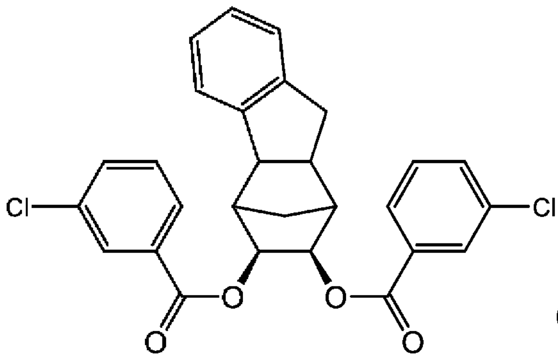

(Compound 216)

2.22 g (yield 32%) of the compound 216 (endo form:exo form = 83:17) was obtained as an isomer mixture of an endo form and an exo form in accordance with the operation and equivalence relationship described in <Synthesis of compound 205> except that 5.36 g (2.2 equivalents) of 3-chlorobenzoyl chloride was used and only 10 ml of pyridine was used as the solvent instead of using 3-methylbenzoyl chloride in <Synthesis of compound 205>. The $^1$H-NMR data of the compound 216 obtained are shown below.

(Compound 216)

$^1$H NMR (270 MHz, $CDCl_3$, TMS as an internal standard): endo-form: δ 1.71-1.75 (m, 1 H), 2.28-2.32 (m, 1 H), 2.57-2.58 (m, 1 H), 2.89-3.10 (m, 4 H), 3.80-3.86 (dd, J = 10.2, 5.6 Hz, 1 H), 4.63-4.65 (m, 1 H), 5.01-5.04 (m, 1 H), 7.16-7.33 (m, 6 H), 7.40-7.45 (m, 2 H), 7.71-7.80 (m, 4 H); exo-form: 1.28-1.32 (m, 1 H), 1.90-1.95 (m, 1 H), 2.42 (br s, 1 H), 2.60-2.74 (m, 3 H), 3.32-3.42 (m, 2 H), 5.17-5.20 (m, 1 H), 5.29-5.31 (m, 1 H), 7.16-7.33 (m, 6 H), 7.40-7.45 (m, 2 H), 7.71-7.80 (m, 4 H).

The melting completion temperature of the compound 216 was 139.4°C.

[Example B14]

<Synthesis of compound 217>

Synthesis of a compound 217 shown below was conducted by a method mentioned below.

[Chem. 240]

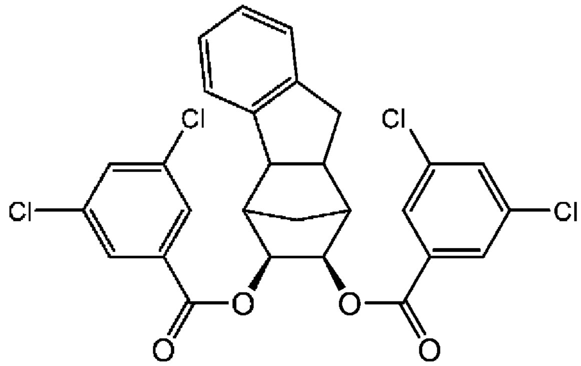

(Compound 217)

4.50 g (yield 58%) of the compound 217 including an endo form only was obtained in accordance with the operation and equivalence relationship described in <Synthesis of compound 205> except that 6.41 g (2.2 equivalents) of 3,5-dichlorobenzoyl chloride was used and only 10 ml of pyridine was used as the solvent instead of using 3-methylbenzoyl chloride in <Synthesis of compound 205>. The $^{1}$H-NMR data of the compound 217 obtained are shown below.

(Compound 217)

$^{1}$H NMR (270 MHz, $CDCl_3$, TMS as an internal standard): $\delta$ 1.74-1.78 (m, 1 H), 2.26-2.30 (m, 1 H), 2.57-2.58 (m, 1 H), 2.89-3.10 (m, 4 H), 3.82-3.88 (dd, J = 9.9, 5.6 Hz, 1 H), 4.60-4.62 (m, 1 H), 4.98-5.01 (m, 1 H), 7.23-7.32 (m, 4 H), 7.45-7.48 (m, 2 H), 7.62-7.67 (m, 4 H).

The melting completion temperature of the compound 217 was 208.2°C.

[Example B15]

<Synthesis of compound 218>

Synthesis of a compound 218 shown below was conducted by a method mentioned below.

[Chem. 241]

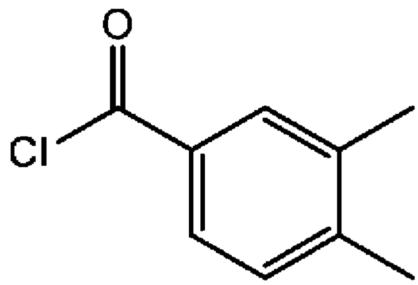

(Compound 218)

Synthesis of the compound 218 was conducted in accordance with the operation and equivalence relationship described in <Synthesis of compound 214> except that 4.63 g (1 equivalent) of 3,4-dimethylbenzoic acid was used instead of using 3-phenylbenzoic acid in <Synthesis of compound 214>. The compound 218 obtained was used as was in Synthesis of compound 219.

<Synthesis of compound 219>

Synthesis of a compound 219 shown below was conducted by a method mentioned below.

[Chem. 242]

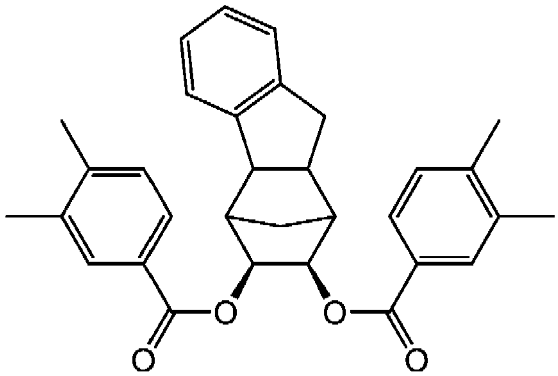

(Compound 219)

The inside of a 100 ml flask was purged with nitrogen, 2.57 g (1 equivalent) of the mixture of the compound 201 and compound 202 was added, and then 20 ml of a solution of the entire amount (2.2 equivalents) of the compound 218 synthesized in <Synthesis of compound 218> in dichloromethane was added. Under cooling in an ice bath, 10 ml of dehydrated pyridine was slowly added dropwise. After the dropwise addition, the temperature was raised to room temperature, and stirring was conducted overnight. Under cooling again in an ice bath, methanol was added for quenching. After dichloromethane and water were added and stirred, the organic layer was separated. The organic layer was washed with a saturated ammonium chloride aqueous solution and brine and then dried over magnesium sulfate. Subsequently, the organic layer was concentrated to give 10.3 g of a crude product. The crude product was purified by silica gel column chromatography to give 4.80 g (yield 83%) of the compound 219 (endo form:exo form = 87:13) as an isomer mixture of an endo form and an exo form. The $^1$H-NMR data of the compound 219 obtained are shown below.

(Compound 219)

$^1$H NMR (270 MHz, $CDCl_3$, TMS as an internal standard): endo-form: δ 1.66-1.70 (m, 1 H), 2.07-2.08 (m, 6 H), 2.23-2.25 (m, 6 H), 2.29-2.33 (m, 1 H), 2.56-2.57 (m, 1 H), 2.88-3.18 (m, 4 H), 3.79-3.85 (dd, J = 10.9, 5.6 Hz, 1 H), 4.61-4.63 (m, 1 H), 4.97-5.00 (m, 1 H), 7.02-7.08 (m, 2 H), 7.16-7.34 (m, 4 H), 7.51-7.66 (m, 4 H); exo-form: 1.24-1.27 (m, 1 H), 1.91-1.95 (m, 1 H), 2.07-2.08 (m, 6 H), 2.23-2.25 (m, 6 H), 2.41 (br s, 1 H), 2.59-2.74 (m, 3 H), 3.30-3.42 (m, 2 H), 5.14-5.16 (m, 1 H), 5.25-5.28 (m, 1 H), 7.02-7.08 (m, 2 H), 7.16-7.34 (m, 4 H), 7.51-7.66 (m, 4 H).

The melting completion temperature of the compound 219 was 131.1°C.

[Example B16]

<Synthesis of compound 220>

Synthesis of a compound 220 shown below was conducted by a method mentioned below.

[Chem. 243]

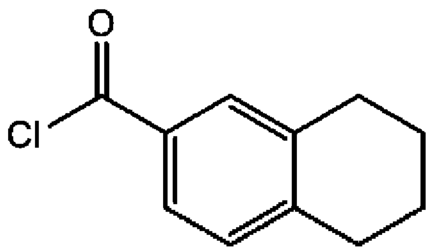

(Compound 220)

The compound 220 was synthesized in accordance with the operation and equivalence relationship described in <Synthesis of compound 214> except that 4.85 g (1 equivalent) of 5,6,7,8-tetrahydro-2-naphthoic acid was used instead of using 3-phenylbenzoic acid in <Synthesis of compound 214>. The compound 220 obtained was used as was in Synthesis of compound 221.

<Synthesis of compound 221>

Synthesis of a compound 221 shown below was conducted by a method mentioned below.

[Chem. 244]

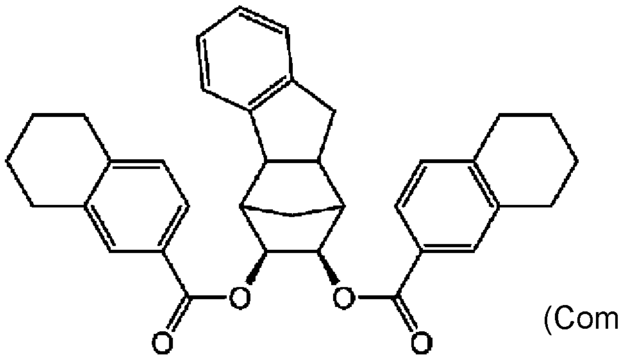

(Compound 221)

4.30 g (yield 74%) of the compound 221 (endo form:exo form = 86:14) was obtained as an isomer mixture of an endo form and an exo form in accordance with the operation and equivalence relationship described in <Synthesis of compound 219> except that the compound 220 (2.2 equivalents) was used instead of using the compound 218 in <Synthesis of compound 219>. The $^1$H-NMR data of the compound 221 obtained are shown below.

(Compound 221)

$^1$H NMR (270 MHz, $CDCl_3$, TMS as an internal standard): endo-form: δ 1.66-1.73 (m, 9 H), 2.28-2.32 (m, 1 H), 2.37-2.77 (m, 9 H), 2.88-3.19 (m, 4 H), 3.78-3.84 (dd, J = 10.2, 5.3 Hz, 1 H), 4.60-4.62 (m, 1 H), 4.97-4.99 (m, 1 H), 6.96-7.01 (m, 2 H), 7.14-7.32 (m, 4 H), 7.41-7.50 (m, 2 H), 7.58-7.63 (m, 2 H); exo-form: 1.23-1.27 (m, 1 H), 1.66-1.73 (m, 8 H), 1.90-1.94 (m, 1 H), 2.37-2.77 (m, 12 H), 3.30-3.42 (m, 2 H), 5.13-5.15 (m, 1 H), 5.24-5.27 (m, 1 H), 6.96-7.01 (m, 2 H), 7.14-7.32 (m, 4 H), 7.41-7.50 (m, 2 H), 7.58-7.63 (m, 2 H).

The melting completion temperature of the compound 221 was 151.2°C.

[Example B17]

<Synthesis of compound 222>

Synthesis of a compound 222 shown below was conducted by a method mentioned below.

[Chem. 245]

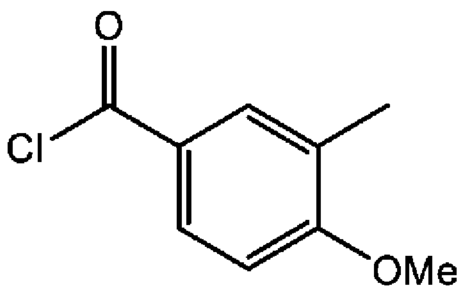

(Compound 222)

The compound 222 was synthesized in accordance with the operation and equivalence relationship described in <Synthesis of compound 214> except that 5.0 g (1 equivalent) of 4-methoxy-3-methylbenzoic acid was used instead of using 3-phenylbenzoic acid in <Synthesis of compound 214>. The compound 222 obtained was used as was in Synthesis of compound 223.

<Synthesis of compound 223>

Synthesis of a compound 223 shown below was conducted by a method mentioned below.

[Chem. 246]

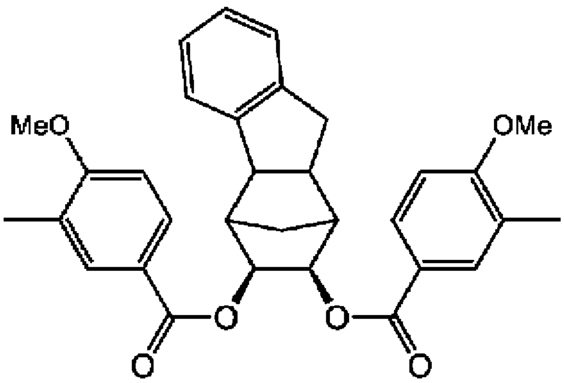

(Compound 223)

2.10 g (yield 33%) of the compound 223 including an endo form only was obtained in accordance with the operation and equivalence relationship described in <Synthesis of compound 219> except that the compound 222 (2.2 equivalents) was used instead of using the compound 218 in <Synthesis of compound 219>. The $^1$H-NMR data of the compound 223 obtained are shown below.

(Compound 223)

$^1$H NMR (270 MHz, $CDCl_3$, TMS as an internal standard): $\delta$ 1.66-1.70 (m, 1 H), 1.95 (s, 3 H), 2.02 (s, 3 H), 2.29-2.33 (m, 1 H), 2.55-2.56 (m, 1 H), 2.87-3.19 (m, 4 H), 3.78-3.88 (m, 7 H), 4.59-4.61 (m, 1 H), 4.96-4.98 (m, 1 H), 6.67-6.73 (m, 2 H), 7.21-7.33 (m, 4 H), 7.51-7.58 (m, 2 H), 7.72-7.79 (m, 2 H) .
The melting completion temperature of the compound 223 was 187.1°C.

**Industrial Applicability**

The novel ester compound according to the present invention is a compound useful for production of resin additives, cosmetics and external preparations for skin, microbicidal compositions, antioxidants, chelators, and Ziegler-Natta catalysts. The compound can be utilized particularly as a catalyst component for Ziegler-Natta catalysts. The compound enables production of a catalyst that offers excellent stereoregularity and productivity on polymerization of polypropylene, and thus has a significantly high industrial value.

**Claims**

1. An ester compound represented by the following general formula (1):

[Chem. 1]

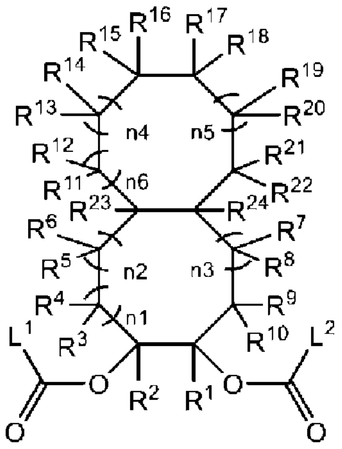

(1)

wherein, in the formula (1), $R^1$ to $R^{24}$ are each independently a hydrogen atom, a halogen atom, a hydrocarbon group, or a hetero atom-containing hydrocarbon group; $R^1$ to $R^{10}$, $R^{23}$, and $R^{24}$ are optionally bonded to one another to form a ring or optionally form a multiple bond at which adjacent substituents are directly bonded; $R^{11}$ to $R^{24}$ are optionally bonded to one another to form a ring, or adjacent substituents are optionally bonded to one another to form a multiple bond; at least one set in $R^1$ to $R^{24}$ is bonded to one another to form a ring structure; n2 to n5 each independently represent an integer of 0 to 2; n1 and n6 each independently represent an integer of 0 or 1; and $L^1$ and $L^2$ are each independently a hydrocarbon group or a hetero atom-containing hydrocarbon group.

2. The ester compound according to claim 1, wherein $L^1$ and $L^2$ are each independently a hydrocarbon group or a hetero atom-containing hydrocarbon group having 1 to 20 carbon atoms.

3. The ester compound according to claim 1, wherein $L^1$ and $L^2$ are each independently a hydrocarbon group or a hetero atom-containing hydrocarbon group having 4 or more carbon atoms.

4. The ester compound according to claim 1, represented by any of the following general formulas (2) to (4):

[Chem. 2]

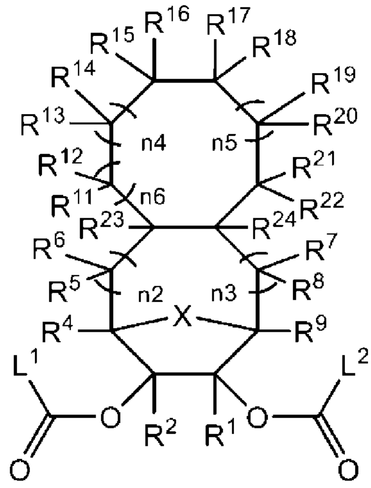

(2)

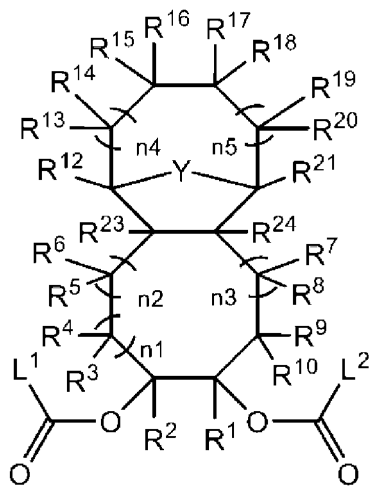

(3)

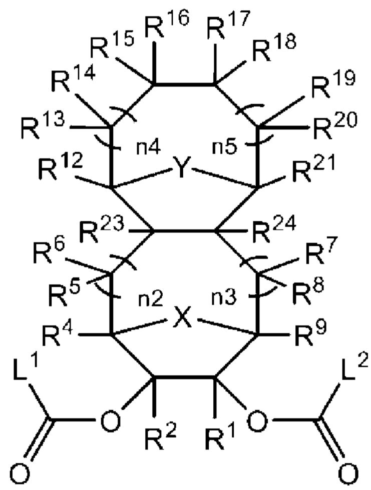

(4)

wherein, in the formulas (2) to (4), $R^1$ to $R^{24}$ are each independently a hydrogen atom, a halogen atom, a hydrocarbon group, or a hetero atom-containing hydrocarbon group; $R^1$ to $R^{10}$, $R^{23}$, and $R^{24}$ are optionally bonded to one another to form a ring or optionally form a multiple bond at which adjacent substituents are directly bonded; $R^{11}$ to $R^{24}$ are optionally bonded to one another to form a ring, or adjacent substituents are optionally bonded to one another to

form a multiple bond; X and Y are each independently a hydrocarbon group, a hetero atom, or a hetero atom-containing hydrocarbon group; n2 to n5 each independently represent an integer of 0 to 2; n1 and n6 each independently represent an integer of 0 or 1; and $L^1$ and $L^2$ are each independently a hydrocarbon group or a hetero atom-containing hydrocarbon group having 4 or more carbon atoms.

**5.** The ester compound according to claim 3 or 4, wherein n1 and n6 are 1, and n2 to n5 are all 0.

**6.** The ester compound according to claim 1, represented by the following general formula (5) or (6):

[Chem. 3]

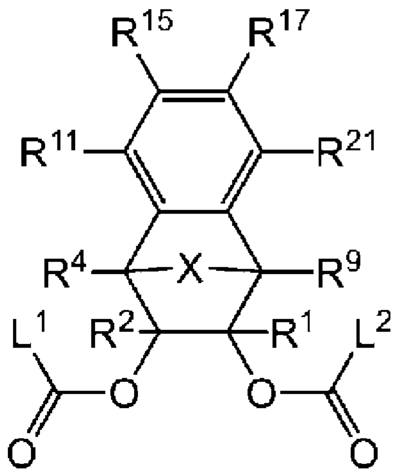

(5)

wherein, in the formula (5), $R^1$ and $R^2$ are each independently a hydrogen atom or a hydrocarbon group, $R^4$ and $R^9$ are each independently a hydrogen atom, a hydrocarbon group, or a hetero atom-containing hydrocarbon group, $R^{11}$, $R^{15}$, $R^{17}$, and $R^{21}$ are each independently a hydrogen atom, a halogen atom, hydrocarbon group, or a hetero atom-containing hydrocarbon group; $R^{11}$, $R^{15}$, $R^{17}$, and $R^{21}$ are optionally bonded to one another to form a ring; X is a hydrocarbon group, a hetero atom, or a hetero atom-containing hydrocarbon group; and $L^1$ and $L^2$ are each independently a hydrocarbon group or a hetero atom-containing hydrocarbon group having 4 or more carbon atoms;

[Chem. 4]

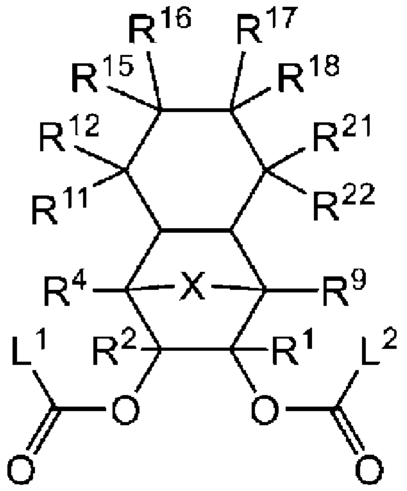

(6)

wherein, in the formula (6), $R^1$ and $R^2$ are each independently a hydrogen atom or a hydrocarbon group, $R^4$, $R^9$, $R^{11}$, $R^{12}$, $R^{15}$ to $R^{18}$, $R^{21}$, and $R^{22}$ are each independently a hydrogen atom, a hydrocarbon group, or a hetero atom-

containing hydrocarbon group; $R^{11}$, $R^{12}$, $R^{15}$ to $R^{18}$, $R^{21}$, and $R^{22}$ are optionally bonded to one another to form a ring or optionally form a multiple bond at which adjacent substituents are bonded to one another; X is a hydrocarbon group, a hetero atom, or a hetero atom-containing hydrocarbon group; and $L^1$ and $L^2$ are each independently a hydrocarbon group or a hetero atom-containing hydrocarbon group having 4 or more carbon atoms.

**7.** The ester compound according to claim 1, represented by the following general formula (7) or (8):

[Chem. 5]

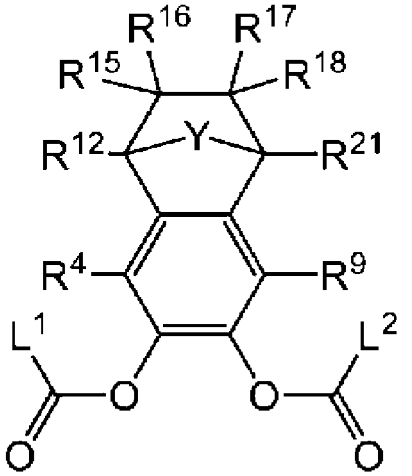

(7)

wherein, in the formula (7), $R^4$, $R^9$, $R^{12}$, $R^{15}$ to $R^{18}$, and $R^{21}$ are each independently a hydrogen atom, a hydrocarbon group, or a hetero atom-containing hydrocarbon group; $R^{15}$ to $R^{18}$ are optionally bonded to one another to form a ring or optionally form a multiple bond at which adjacent substituents are bonded to one another; Y is a hydrocarbon group, a hetero atom, or a hetero atom-containing hydrocarbon group; and $L^1$ and $L^2$ are each independently a hydrocarbon group or a hetero atom-containing hydrocarbon group having 4 or more carbon atoms;

[Chem. 6]

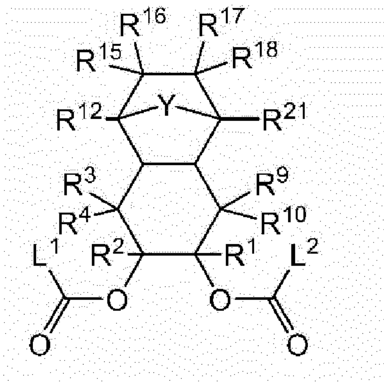

(8)

wherein, in the formula (8), $R^1$ and $R^2$ are each independently a hydrogen atom or a hydrocarbon group, $R^3$, $R^4$, $R^9$, $R^{10}$, $R^{12}$, $R^{15}$ to $R^{18}$, and $R^{21}$ are each independently a hydrogen atom, a hydrocarbon group, or a hetero atom-containing hydrocarbon group; $R^{15}$ to $R^{18}$ are optionally bonded to one another to form a ring or optionally form a multiple bond at which adjacent substituents are bonded to one another; Y is a hydrocarbon group, a hetero atom, or a hetero atom-containing hydrocarbon group; and $L^1$ and $L^2$ are each independently a hydrocarbon group or a hetero atom-containing hydrocarbon group having 4 or more carbon atoms.

**8.** The ester compound according to claim 1, represented by the following general formula (9):

[Chem. 7]

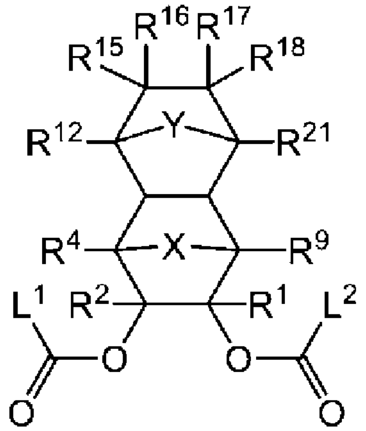

(9)

wherein, in the formula (9), $R^1$ and $R^2$ are each independently a hydrogen atom or a hydrocarbon group, $R^4$, $R^9$, $R^{12}$, $R^{15}$ to $R^{18}$, and $R^{21}$ are each independently a hydrogen atom, a hydrocarbon group, or a hetero atom-containing hydrocarbon group; $R^{15}$ to $R^{18}$ are optionally bonded to one another to form a ring or optionally form a multiple bond at which adjacent substituents are bonded to one another; X and Y are each independently a hydrocarbon group, a hetero atom, or a hetero atom-containing hydrocarbon group; and $L^1$ and $L^2$ are each independently a hydrocarbon group or a hetero atom-containing hydrocarbon group having 4 or more carbon atoms.

**9.** The ester compound according to claim 1, represented by the following general formula (31):

[Chem. 8]

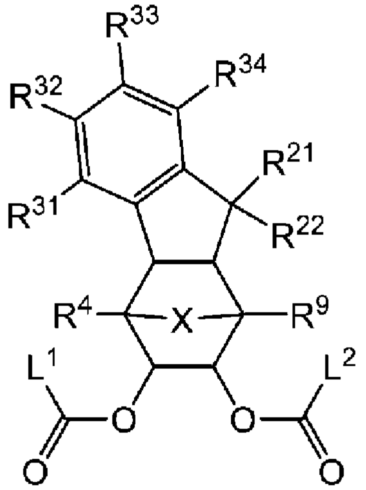

(3 1)

wherein, in the formula (31), $R^{31}$ to $R^{34}$ are each independently a hydrogen atom, a halogen atom, a hydrocarbon group, or a hetero atom-containing hydrocarbon group, $R^4$, $R^9$, $R^{21}$, and $R^{22}$ are each independently a hydrogen atom, a hydrocarbon group, or a hetero atom-containing hydrocarbon group, $R^4$, $R^9$, $R^{21}$, $R^{22}$, and $R^{31}$ to $R^{34}$ are optionally bonded to one another to form a ring; $L^1$ and $L^2$ are each independently a hydrocarbon group or a hetero atom-containing hydrocarbon group; and X is a hydrocarbon group, hetero atom or a hetero atom-containing hydrocarbon group.

**10.** The ester compound according to any one of claims 4 and 6 to 9, wherein X and Y are each independently a divalent group selected from the following general formula group (10):

[Chem. 9]

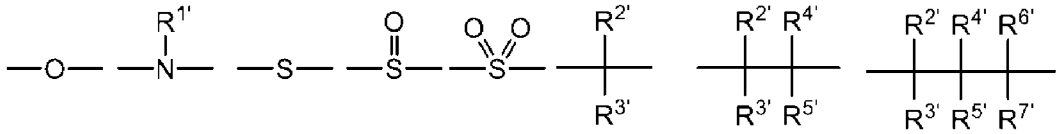

(10)

wherein, in the group (10), $R^{1'}$ to $R^{7'}$ are each independently a hydrogen atom, a hydrocarbon group, or a hetero atom-containing hydrocarbon group, and $R^{2'}$ to $R^{7'}$ are optionally bonded to one another to form a ring, or adjacent substituents are optionally directly bonded to form a multiple bond.

**11.** The ester compound according to any one of claims 4 and 6 to 9, wherein X and Y are a divalent group selected from the following general formula group (11):

[Chem. 10]

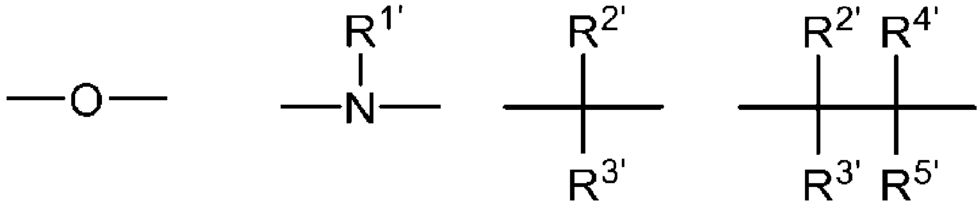

(11)

wherein, in the group (11), $R^{1'}$ to $R^{5'}$ are each independently a hydrogen atom, a hydrocarbon group having 1 to 20 carbon atoms, or a hetero atom-containing hydrocarbon group having 1 to 20 carbon atoms, and $R^{2'}$ to $R^{5'}$ are optionally bonded to one another to form a ring, or adjacent substituents are optionally directly bonded to form a multiple bond.

**12.** The ester compound according to claim 9, wherein X is a divalent group represented by the following general formula (13) :

[Chem. 11]

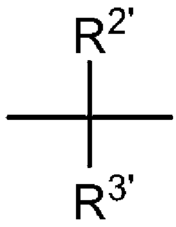

(13)

wherein, in the formula (13), $R^{2'}$ and $R^{3'}$ are each independently a hydrogen atom, a hydrocarbon group having 1 to 20 carbon atoms, or a hetero atom-containing hydrocarbon group having 1 to 20 carbon atoms, and $R^{2'}$ and $R^{3'}$ are optionally bonded to one another to form a ring.

**13.** The ester compound according to claim 10, wherein $R^{1'}$ to $R^{7'}$ are each independently a hydrogen atom or a hydrocarbon group having 1 to 10 carbon atoms.

**14.** The ester compound according to claim 11, wherein $R^{1'}$ to $R^{5'}$ are each independently a hydrogen atom or a hydrocarbon group having 1 to 10 carbon atoms.

**15.** The ester compound according to claim 12, wherein $R^{2'}$ and $R^{3'}$ are each independently a hydrogen atom or a

hydrocarbon group having 1 to 10 carbon atoms.

**16.** The ester compound according to claim 12, wherein $R^{2'}$ and $R^{3'}$ are all hydrogen atoms.

**17.** The ester compound according to any one of claims 1 to 16, wherein $R^1$ to $R^{24}$ are each independently a hydrogen atom, a hydrocarbon group having 1 to 20 carbon atoms, or a hetero atom-containing hydrocarbon group having 1 to 20 carbon atoms.

**18.** The ester compound according to any one of claims 1 to 16, wherein $R^1$ to $R^{24}$ are each independently a hydrogen atom, a hydrocarbon group having 1 to 10 carbon atoms, or a hetero atom-containing hydrocarbon group having 1 to 10 carbon atoms.

**19.** The ester compound according to claim 9, wherein $R^{31}$ to $R^{34}$ are each independently a hydrogen atom, a halogen atom, a hydrocarbon group having 1 to 20 carbon atoms, or a hetero atom-containing hydrocarbon group having 1 to 20 carbon atoms.

**20.** The ester compound according to claim 9, wherein $R^{31}$ to $R^{34}$ are each independently a hydrogen atom, a hydrocarbon group having 1 to 10 carbon atoms, or a hetero atom-containing hydrocarbon group having 1 to 10 carbon atoms.

**21.** The ester compound according to claim 9, wherein $R^{31}$ to $R^{34}$ are all hydrogen atoms, $R^4$, $R^9$, $R^{21}$, and $R^{22}$ are each independently a hydrogen atom, a hydrocarbon group having 1 to 6 carbon atoms, or a hetero atom-containing hydrocarbon group having 1 to 6 carbon atoms, and $L^1$ and $L^2$ are each independently a hydrocarbon group having 1 to 10 carbon atoms or a hetero atom-containing hydrocarbon group having 1 to 10 carbon atoms.

**22.** The ester compound according to claim 9, wherein $R^{31}$ to $R^{34}$, $R^{21}$ and $R^{22}$ are all hydrogen atoms, $R^4$ and $R^9$ are each independently a hydrogen atom or a hydrocarbon group having 1 to 6 carbon atoms, and $L^1$ and $L^2$ are each independently selected from hydrocarbon groups having 1 to 10 carbon atoms.

**23.** The ester compound according to any one of claims 1 to 8, wherein $R^1$ and $R^2$ are hydrogen atoms.

**24.** The ester compound according to any one of claims 1 to 8, wherein $R^1$, $R^2$, $R^{23}$, and $R^{24}$ are all hydrogen atoms, and $R^3$ to $R^{22}$ are each independently a hydrogen atom or a substituted or unsubstituted alkyl group having 1 to 4 carbon atoms.

**25.** The ester compound according to any one of claims 1 to 8, wherein $L^1$ and $L^2$ are each independently a hydrocarbon group or a hetero atom-containing hydrocarbon group having 4 to 20 carbon atoms.

**26.** The ester compound according to any one of claims 1 to 8, wherein $L^1$ and $L^2$ are each independently a hydrocarbon group or a hetero atom-containing hydrocarbon group having 4 to 10 carbon atoms.

**27.** The ester compound according to claim 4, 6, or 6, wherein the $R^4$ and/or $R^9$ are/is a hydrocarbon group or a hetero atom-containing hydrocarbon group.

**28.** The ester compound according to claim 4, 6, or 8, wherein the $R^4$ and/or $R^9$ are/is a hydrocarbon group or an oxygen atom-containing hydrocarbon group.

**INTERNATIONAL SEARCH REPORT**

International application No.
**PCT/JP2021/031281**

**A. CLASSIFICATION OF SUBJECT MATTER**

***C07C 229/60***(2006.01)i; ***C07D 317/36***(2006.01)i; ***C07D 333/38***(2006.01)i; ***C07D 493/08***(2006.01)i; ***C07D 307/68***(2006.01)i; ***C07C 69/24***(2006.01)i; ***C07C 69/75***(2006.01)i; ***C07C 69/76***(2006.01)i; ***C07C 69/78***(2006.01)i; ***C07C 69/92***(2006.01)i
FI: C07C69/78 CSP; C07C69/76 Z; C07C69/92; C07C229/60; C07C69/75 Z; C07C69/24; C07D493/08 A; C07D317/36; C07C69/76 A; C07D307/68; C07D333/38

According to International Patent Classification (IPC) or to both national classification and IPC

**B. FIELDS SEARCHED**

Minimum documentation searched (classification system followed by classification symbols)

C07C229/60; C07D317/36; C07D333/38; C07D493/08; C07D307/68; C07C69/24; C07C69/75; C07C69/76; C07C69/78; C07C69/92

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | EP 2194070 A1 (SUED-CHEMIE AG) 09 June 2010 (2010-06-09) claims | 1-6, 10, 11, 17, 18, 23-26 |
| A | | 7, 9, 19-22, 27, 28 |
| X | CN 101195668 A (CHINA NATIONAL PETROLEUM CORPORATION) 11 June 2008 (2008-06-11) claims, examples | 1-6, 10, 11, 17, 18, 23-26 |
| A | | 7, 9, 19-22, 27, 28 |
| X | JP 2013-20238 A (SHIN-ETSU CHEMICAL CO., LTD.) 31 January 2013 (2013-01-31) synthesis example, resist polymers 3, 6 | 1-6, 8, 10-18, 23, 25, 26 |
| X | WO 2011/005822 A1 (EXXONMOBIL RESEARCH AND ENGINEERING COMPANY) 13 January 2011 (2011-01-13) example 10 | 1-4, 10-18, 23-26 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 October 2021** | **19 October 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/031281**

**C. DOCUMENTS CONSIDERED TO BE RELEVANT**

| Category* | Citation of document, with indication, where appropriate, of the relevant passages | Relevant to claim No. |
|---|---|---|
| A | CN 105585644 A (PETROCHINA COMPANY LIMITED) 18 May 2016 (2016-05-18) entire text | 1-28 |
| A | CN 103665209 A (PETROCHINA COMPANY LIMITED) 26 March 2014 (2014-03-26) entire text | 1-28 |

Form PCT/ISA/210 (second sheet) (January 2015)

**INTERNATIONAL SEARCH REPORT**
**Information on patent family members**

International application No.
**PCT/JP2021/031281**

| Patent document cited in search report | Publication date (day/month/year) | Patent family member(s) | Publication date (day/month/year) |
|---|---|---|---|
| EP 2194070 A1 | 09 June 2010 | JP 2010-132904 A<br>US 2010/0144991 A1<br>CN 101759817 A | |
| CN 101195668 A | 11 June 2008 | (Family: none) | |
| JP 2013-20238 A | 31 January 2013 | US 2012/0315581 A1<br>synthesis example, resist polymers 3, 6<br>KR 10-2012-0138664 A | |
| WO 2011/005822 A1 | 13 January 2011 | US 2011/0009548 A1 | |
| CN 105585644 A | 18 May 2016 | (Family: none) | |
| CN 103665209 A | 26 March 2014 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005226076 A
- JP 2000516987 A
- JP 2002542347 A
- JP 2005517746 A
- JP 2011529888 A
- JP 2014500390 A
- JP 2008247796 A
- WO 2008062553 A
- US 20180149973 A1
- US 20020162991 A1
- JP 2008037756 A


### Non-patent literature cited in the description


- *Journal of the American Chemical Society,* 1952, vol. 74, 1027-1029
- *Journal of Organic Chemistry,* 1971, vol. 36, 3979-3987
- *Organic Letters,* 2004, vol. 6, 1589-1592
- *Journal of the American Chemical Society,* 1957, vol. 79, 2822-2824
- *Organic Synthesis,* 1991, vol. 70, 47-53
- *Organic Synthesis,* 1997, vol. 75, 153-160
- *Catalysis Letters,* 2012, vol. 142, 124-130
- *Organic Synthesis,* 1997, vol. 74, 91-100
- Synthesis of Organic Compound II, Alcohols and amines. *The Fourth Series of Experimental Chemistry,* vol. 20, 39
- *Journal of Organic Chemistry,* 1959, vol. 24, 54-55
- *Angewandte Chemie International Edition,* 1978, vol. 17, 522-524
- *Bulletin of the Chemical Society of Japan,* 1967, vol. 40, 2380-2382
- *Organic Synthesis,* 1952, vol. 32, 41
- *Macromolecules,* 2017, vol. 50, 580-586
- *Journal of Organic Chemistry,* 1980, vol. 45, 2301-2304
- *Journal of Organic Chemistry,* 2009, vol. 74, 405-407
- *Journal of Organic Chemistry,* 1988, vol. 53, 2120-2122
- *Journal of Organic Chemistry,* 1963, vol. 28, 2572-2577
- *European Journal of Organic Chemistry,* 2017, vol. 24, 3501-3504
- *J. Med. Chem.,* 2017, vol. 60, 3618-3625
- *Bull. Chem. Soc. Jpn.,* 1993, vol. 66, 1576-1579
- *Angew. Chem. Int. Ed.,* 2011, vol. 50, 5674-5677
- *Org. Lett.,* 2013, vol. 15, 5722-5725
- *J. Org. Chem.,* 2017, vol. 82, 9715-9730
- *J. Org. Chem.,* 2015, vol. 80, 11618-11623
- *Adv. Synth. Catal.,* 2008, vol. 350, 1309-1315